(19) Europäisches Patentamt
European Patent Office
Office européen des brevets

(11) EP 1 115 704 B1

(12) EUROPÄISCHE PATENTSCHRIFT

(45) Veröffentlichungstag und Bekanntmachung des Hinweises auf die Patenterteilung:
**18.06.2003 Patentblatt 2003/25**

(21) Anmeldenummer: **99947404.2**

(22) Anmeldetag: **22.09.1999**

(51) Int Cl.[7]: **C07D 209/34**, A61K 31/40, C07D 209/48, C07D 401/12, C07D 403/12

(86) Internationale Anmeldenummer:
**PCT/EP99/07040**

(87) Internationale Veröffentlichungsnummer:
**WO 00/018734 (06.04.2000 Gazette 2000/14)**

(54) **NEUE SUBSTITUIERTE INDOLINONE MIT EINER INHIBIERENDEN WIRKUNG AUF VERSCHIEDENE KINASEN UND CYCLIN/CDK-KOMPLEXE**

NOVEL SUBSTITUTED INDOLINONES WITH AN INHIBITORY EFFECT ON VARIOUS KINASES AND CYCLIN/CDK COMPLEXES

NOUVELLES INDOLINONES SUBSTITUEES A EFFET INHIBITEUR SUR DIFFERENTES KINASES ET DIFFERENTS COMPLEXES CYCLINE/CDK

(84) Benannte Vertragsstaaten:
**AT BE CH CY DE DK ES FI FR GB GR IE IT LI LU MC NL PT SE**
Benannte Erstreckungsstaaten:
**LT LV RO SI**

(30) Priorität: **25.09.1998 DE 19844003**
**07.08.1999 DE 19937496**

(43) Veröffentlichungstag der Anmeldung:
**18.07.2001 Patentblatt 2001/29**

(73) Patentinhaber: **Boehringer Ingelheim Pharma GmbH & Co.KG**
**55218 Ingelheim am Rhein (DE)**

(72) Erfinder:
- **WALTER, Rainer**
  **D-88400 Biberach (DE)**
- **GRELL, Wolfgang**
  **D-88400 Biberach (DE)**
- **HECKEL, Armin**
  **D-88400 Bibrach (DE)**
- **HIMMELSBACH, Frank**
  **D-7951 Mittelbiberach (DE)**
- **EBERLEIN, Wolfgang**
  **D-88400 Biberach (DE)**
- **ROTH, Gerald**
  **D-88400 Biberach (DE)**
- **VAN MEEL, Jacobus, C., A.**
  **A-2340 Mödling (AT)**
- **REDEMANN, Norbert**
  **D-88400 Biberach (DE)**
- **SPEVAK, Walter**
  **A-2105 Oberrohrbach (AT)**
- **TONTSCH-GRUNT, Ulrike**
  **A-2500 Baden (AT)**
- **VON RÜDEN, Thomas**
  **D-81152 Planegg (DE)**

(74) Vertreter: **Laudien, Dieter, Dr.**
**Boehringer Ingelheim GmbH,**
**B Patent**
**55216 Ingelheim am Rhein (DE)**

(56) Entgegenhaltungen:
**WO-A-96/22976** **WO-A-96/40116**

- **HANS GÜNTER AURICH: "Photochemische Umlagerung eines 5-Imino- -isoxazolins" JUSTUS LIEBIGS ANNALEN DER CHEMIE., Bd. 732, - 1970 Seiten 195-198, XP002127303 VERLAG CHEMIE GMBH. WEINHEIM., DE ISSN: 0075-4617**

Bemerkungen:
Die Akte enthält technische Angaben, die nach dem Eingang der Anmeldung eingereicht wurden und die nicht in dieser Patentschrift enthalten sind.

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 1 115 704 B1

## Beschreibung

Die vorliegende Erfindung betrifft neue substituierte Indolinone der allgemeinen Formel

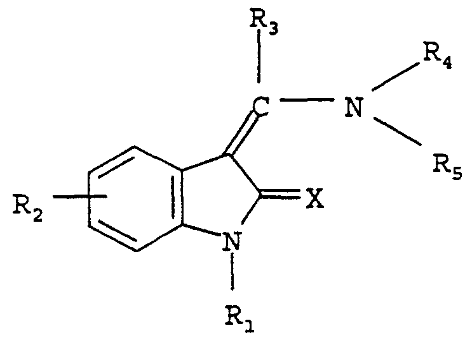

(I),

deren Isomere, deren Salze, insbesondere deren physiologisch verträgliche Salze, welche wertvolle Eigenschaften aufweisen.

Die obigen Verbindungen der allgemeinen Formel I, in denen $R_1$ ein Wasserstoffatom, eine $C_{1-3}$-Alkylgruppe oder einen Prodrugrest darstellt, weisen wertvolle pharmakologische Eigenschaften auf, insbesondere eine inhibierende Wirkung auf verschiedene Kinasen, vor allem auf Komplexe von CDK's (CDK1, CDK2, CDK3, CDK4, CDK6, CDK7, CDK8 und CDK9) mit ihren spezifischen Cyclinen (A, B1, B2, C, D1, D2, D3, E, F, G1, G2, H, I und K), auf virales Cyclin (siehe L. Mengtao in J. Virology 71(3), 1984-1991 (1997))und auf Rezeptor-Tyrosinkinasen wie HER2, EGFR, FGFR, IGF-1R und KDR, und die übrigen Verbindungen der obigen allgemeinen Formel I, in denen $R_1$ kein Wasserstoffatom, keine $C_{1-3}$-Alkylgruppe und keinen Prodrugrest darstellt, stellen wertvolle Zwischenprodukte zur Herstellung der vorstehend erwähnten Verbindungen dar, welche wertvolle pharmakologische Eigenschaften aufweisen.

H.G. Aurich (Liebigs Ann. Chem 732, 195-198 (1970)) beschreibt in seiner Mitteilung über die photochemische Umlagerung eines 5-Imino-$\Delta^3$-isoxazolins als Zwischenstufe die Verbindung 3-[(α-Phenylamino)-benzyliden]-indolin-2-on.

Weitere Indolinone zur Modulierung der Tyrosinkinase Signal-Transduktion zur Regulierung, Modulierung und/oder Hemmung abnormer Zellproliferation werden in der WO 96/40116 beschrieben.

Die WO 96/22976 beschreibt wasserlösliche 3-Aryliden-2-oxindole als Tyrosinkinase-Hemmer.

Gegenstand der vorliegenden Erfindung sind somit die obigen Verbindungen der allgemeinen Formel I, wobei die Verbindungen, in denen $R_1$ ein Wasserstoffatom, eine $C_{1-3}$-Alkylgruppe oder einen Prodrugrest wie eine $C_{1-4}$-Alkoxycarbonyl- oder $C_{2-4}$-Alkanoylgruppe darstellt, wertvolle pharmakologische Eigenschaften aufweisen, die die pharmakologisch wirksamen Verbindungen enthaltende Arzneimittel, deren Verwendung und Verfahren zu ihrer Herstellung.

In der obigen allgemeinen Formel I bedeuten
X ein Sauerstoff- oder Schwefelatom,
$R_1$ ein Wasserstoffatom, $C_{1-3}$-Alkyl- oder Hydroxygruppe,
$R_2$ ein Wasserstoff-, Fluor-, Chlor-, Brom- oder Jodatom, eine $C_{1-3}$-Alkyl- oder Nitrogruppe,
$R_3$ eine Phenyl- oder Naphthylgruppe, die jeweils durch Fluor-, Chlor-, Brom- oder Jodatome, durch $C_{1-3}$-Alkyl-, $C_{1-3}$-Alkoxy-, Carboxy-, Cyano-, Trifluormethyl-, Nitro-, Amino-, $C_{1-3}$-Alkylamino-, Di-($C_{1-3}$-alkyl)-amino-, $C_{1-3}$-Alkylsulfonylamino-, Amino-$C_{1-3}$-alkyl-, 2-Carboxy-phenylcarbonylaminomethyl-, $C_{1-3}$-Alkylamino-$C_{1-3}$-alkyl-, $C_{2-4}$-Alkanoylamino-$C_{1-3}$-alkyl-, N-($C_{2-4}$-Alkanoyl)-$C_{1-3}$-alkylamino-$C_{1-3}$-alkyl-, Di-($C_{1-3}$-Alkyl)-amino-$C_{1-3}$-alkyl-, Carboxy-$C_{2-3}$-alkenyl-, N-(Carboxy-$C_{1-3}$-alkyl)-aminocarbonyl-, N-(Carboxy-$C_{1-3}$-alkyl)-N-($C_{1-3}$-alkyl)-aminocarbonyl- oder Imidazolyl-$C_{1-3}$-alkylgruppen mono- oder disubstituiert sein können, wobei die Substituenten gleich oder verschieden sein können,
$R_4$ ein Wasserstofatom oder eine $C_{1-3}$-Alkylgruppe und
$R_5$ ein gegebenenfalls durch eine $C_{1-3}$-Alkylgruppe substituierte Phenyl- oder Naphthylgruppe, die jeweils zusätzlich im aromatischen Teil
durch ein Fluor-, Chlor-, Brom- oder Jodatom, durch eine $C_{1-3}$-Alkyl-, $C_{1-3}$-Alkoxy-, Cyano-, Nitro- oder Trifluormethylgruppe,
durch eine $C_{1-3}$-Alkoxygruppe, die durch eine Carboxy-, Aminocarbonyl-, $C_{1-3}$-Alkylaminocarbonyl- oder Di-($C_{1-3}$-Alkyl)-aminocarbonylgruppe oder in 2- oder 3-Stellung auch durch eine Amino-, $C_{1-3}$-Alkylamino-, Di-($C_{1-3}$-Alkyl)-amino-, Phenyl-$C_{1-3}$-alkylamino-, N-(Phenyl-$C_{1-3}$-alkyl)-N-($C_{1-3}$-alkyl)-amino-, Pyrrolidino-, Piperidino- oder Hexamethyleniminogruppe substituiert ist,

durch eine gegebenenfalls durch eine Di-($C_{1-3}$-Alkyl)-aminogruppe substituierte $C_{2-3}$-Alkenylgruppe, die im Alkenylteil zusätzlich durch ein Chlor- oder Bromatom substituiert sein kann, durch eine gegebenenfalls durch eine Di-($C_{1-3}$-Alkyl)-aminogruppe substituierte $C_{2-3}$-Alkinylgruppe,

durch eine $C_{1-3}$-Alkylgruppe, die durch eine 3- bis 7-gliedrige Cycloalkyleniminogruppe, durch eine Dehydropiperidino-, Morpholino-, Thiomorpholino-, 1-Oxido-thiomorpholino-, 1,1-Dioxido-thiomorpholino-, Piperazino-, N-($C_{1-3}$-Alkyl)-piperazino-, N-($C_{1-3}$-Alkanoyl)-piperazino- oder N-($C_{1-5}$-Alkoxycarbonyl)-piperazinogruppe substituiert ist, wobei die vorstehend erwähnten Substituenten durch eine $C_{1-3}$-Alkyl-, Phenyl- oder Phenyl-$C_{1-3}$-alkylgruppe substituiert und die vorstehend erwähnten Piperidino- oder Hexamethyleniminogruppen zusätzlich durch eine $C_{1-3}$-Alkylgruppe oder in 3- oder 4-Stellung durch eine Hydroxy-, $C_{1-3}$-Alkoxy-, Hydroxy-$C_{1-3}$-alkyl-, Carboxy-, Aminocarbonyl-, N-($C_{1-3}$-Alkyl)-aminocarbonyl- oder N,N-Di-($C_{1-3}$-Alkyl)-aminocarbonylgruppe substituiert sein können,

durch eine durch eine Hydroxy-, $C_{1-3}$-Alkoxy-, Carboxy- oder Cyanogruppe substituierte $C_{1-3}$-Alkylgruppe, wobei eine durch eine Carboxygruppe substituierte $C_{1-3}$-Alkylgruppe zusätzlich im Alkylteil durch eine Amino- oder $C_{1-5}$-Alkoxycarbonylaminogruppe substituiert sein kann,

durch eine gegebenenfalls durch eine oder zwei $C_{1-3}$-Alkylgruppen substituierte Aminocarbonylamino-, Amidino- oder Guanidinogruppe,

durch eine Piperidino-, Hexamethylenimino-, Morpholino-, Piperazino- oder N-($C_{1-3}$-Alkyl)-piperazinogruppe,

durch eine Formyl-, Carboxy- oder Trifluoracetylgruppe,

durch eine Carbonylgruppe, die

durch eine $C_{1-3}$-Alkyl-, $C_{1-3}$-Alkoxy-$C_{1-3}$-alkyl-, Amino-, $C_{1-5}$-Alkylamino- oder Di-($C_{1-3}$-Alkyl)-aminogruppe substituiert ist, wobei die vorstehend erwähnten Amino- und $C_{1-3}$-Alkylaminogruppen zusätzlich am Stickstoffatom durch eine Carboxy-$C_{1-3}$-alkylgruppe oder durch eine $C_{2-3}$-Alkylgruppe, die in 2- oder 3-Stellung durch eine Hydroxy-, $C_{1-3}$-Alkoxy-, Amino-, $C_{1-3}$-Alkylamino- oder Di-($C_{1-3}$-Alkyl)-aminogruppe substituiert sein können,

durch eine Pyrrolidinocarbonyl-, Piperidinocarbonyl-, Hexamethyleniminocarbonyl-, Morpholinocarbonyl-, Piperazinocarbonyl-, N-($C_{1-3}$-Alkyl)-piperazinocarbonyl- oder N-(Phenyl-$C_{1-3}$-alkyl)-piperazinocarbonylgruppe,

durch eine Amidosulfonyl-, Pyrrolidinosulfonyl-, Piperidinosulfonyl- oder Hexamethyleniminosulfonylgruppe, durch eine $C_{1-3}$-Alkylamidosulfonyl- oder Di-($C_{1-3}$-alkyl)-amidosulfonylgruppe, in denen ein Alkylteil jeweils durch eine Carboxy-, Aminocarbonyl-, N-($C_{1-3}$-Alkyl)-aminocarbonyl- oder N,N-Di-($C_{1-3}$-Alkyl)-aminocarbonylgruppe oder auch in 2- oder 3-Stellung durch eine $C_{1-3}$-Alkylamino- oder Di-($C_{1-3}$-Alkyl)-aminogruppe substituiert sein kann,

durch eine Amino-, $C_{1-5}$-Alkylamino-, $C_{3-7}$-Cycloalkylamino-, Phenyl-$C_{1-3}$-alkylamino-, Phenylamino-, 6-gliedrige Heteroarylamino-, Amino-$C_{1-3}$-alkyl-, N-($C_{1-5}$-Alkyl)-amino-$C_{1-3}$-alkyl-, Di-($C_{1-5}$-alkyl)-amino-$C_{1-3}$-alkyl-, $C_{3-7}$-Cycloalkylamino-$C_{1-3}$-alkyl-, N-($C_{1-5}$-Alkyl)-$C_{3-7}$-cycloalkylamino-$C_{1-3}$-alkyl-, Phenylamino-$C_{1-3}$-alkyl-, N-($C_{1-3}$-Alkyl)-phenylamino-$C_{1-3}$-alkyl-, Phenyl-$C_{1-3}$-alkylamino-$C_{1-3}$-alkyloder N-($C_{1-5}$-Alkyl)-phenyl-$C_{1-3}$-alkylamino-$C_{1-3}$-alkylgruppe oder durch eine gegebenenfalls am Stickstoffatom durch $C_{1-5}$-Alkylgruppe substituierte 6-gliedrige Heteroarylamino-$C_{1-3}$-alkylgruppe, wobei der N-Alkylteil der vorstehend erwähnten Gruppen jeweils durch eine Cyano-, Carboxy-, Aminocarbonyl-, $C_{1-3}$-Alkylaminocarbonyl-, Di-($C_{1-3}$-alkyl)-aminocarbonyl-, 2-[Di-($C_{1-3}$-alkyl)-amino]-ethylaminocarbonyl-, 3-[Di-($C_{1-3}$-alkyl)-amino]-propylaminocarbonyl-, N-{2-[Di-($C_{1-3}$-alkyl)-amino]-ethyl}-N-($C_{1-3}$-alkyl)-aminocarbonyl- oder N-{3-[Di-($C_{1-3}$-alkyl)-amino]-propyl}-N-($C_{1-3}$-alkyl)-aminocarbonylgruppe oder in 2- oder 3-Stellung durch eine Hydroxy-, $C_{1-3}$-Alkoxy-, Amino-, $C_{1-3}$-Alkylamino-, Di-($C_{1-3}$-alkyl)-amino-, Pyrrolidino-, Piperidino-, Hexamethylenimino-, Morpholino-, Piperazino- oder N-($C_{1-3}$-Alkyl)-piperazinogruppe substituiert sein können und das Stickstoffatom der vorstehend erwähnten Amino-, N-($C_{1-5}$-Alkyl)-amino-, $C_{3-7}$-Cycloalkylamino-, Phenyl-$C_{1-3}$-alkylamino-, Phenylamino-, 6-gliedrige Heteroarylamino-, Amino-$C_{1-3}$-alkyl- und N-($C_{1-5}$-Alkylamino)-$C_{1-3}$-alkylgruppen zusätzlich

durch eine $C_{1-5}$-Alkoxycarbonylgruppe,

durch eine Formyl-, Trifluoracetyl- oder Benzoylgruppe,

durch eine Carboxy-$C_{1-3}$-alkyl-, Aminocarbonyl-$C_{1-3}$-alkyl-, N-($C_{1-3}$-Alkyl)-aminocarbonyl-$C_{1-3}$-alkyl- oder N, N-Di-($C_{1-3}$-Alkyl)-aminocarbonyl-$C_{1-3}$-alkylgruppe,

durch eine $C_{1-5}$-Alkylgruppe, die mit Ausnahme der 1-Stellung durch eine Hydroxy-, $C_{1-3}$-Alkoxy-, Amino-, $C_{1-3}$-Alkylamino- oder Di-($C_{1-3}$-alkyl)-aminogruppe substituiert sein kann,

durch eine $C_{2-4}$-Alkanoylgruppe, die im Alkanoylteil durch eine Carboxy-, Hydroxy-, $C_{1-3}$-Alkoxy-, Phenyl-, Amino-, Phthalimido-, $C_{1-3}$-Alkylamino-, Di-($C_{1-3}$-alkyl)-amino-, Pyrrolidino-, Piperidino-, Hexamethylenimino- oder Morpholinogruppe oder durch eine gegebenenfalls am Stickstoffatom durch eine $C_{1-3}$-Alkyl- oder Phenyl-$C_{1-3}$-alkylgruppe substituierte Piperazinogruppe substituiert sein kann, wobei der Alkylteil der vorstehend erwähnten $C_{1-3}$-Alkylaminound Di-($C_{1-3}$-alkyl)-aminosubstituenten in 2- oder 3-Stellung durch eine Hydroxy-, $C_{1-3}$-Alkoxy-, Amino-, $C_{1-5}$-Alkoxycarbonylamino-, $C_{1-3}$-Alkylamino-, Di-($C_{1-3}$-alkyl)-amino-, Phenyl-, Pyrrolidino-, Piperidino-, Hexamethyleniminooder Morpholinogruppe substituiert sein kann,

durch eine $C_{1-5}$-Alkylsulfonylgruppe, in der der Alkylteil mit Ausnahme der 1-Stellung durch eine Di-($C_{1-3}$-alkyl)-amino-, Pyrrolidino-, Piperidino-, Hexamethylenimino- oder Morpholinogruppe substituiert sein kann,

durch eine gegebenenfalls im Phenylteil durch ein Fluor-, Chlor- oder Bromatom oder durch eine $C_{1-3}$-Alkyl- oder $C_{1-3}$-Alkoxygruppe substituierte Phenyl-($C_{1-3}$)-alkylsulfonyl- oder Phenylsulfonylgruppe substituiert sein kann,

substituiert sein können,

wobei zusätzlich eine vorhandene Carboxy-, Amino- oder Iminogruppe durch einen in-vivo abspaltbaren Rest substituiert sein kann.

Unter einem von einer Imino- oder Aminogruppe in-vivo abspaltbaren Rest ist beispielsweise eine Hydroxygruppe, eine Acylgruppe wie die Benzoyl- oder Pyridinoylgruppe oder eine $C_{1-16}$-Alkanoylgruppe wie die Formyl-, Acetyl-, Propionyl-, Butanoyl-, Pentanoyl- oder Hexanoylgruppe, eine Allyloxycarbonylgruppe, eine $C_{1-16}$-Alkoxycarbonylgruppe wie die Methoxycarbonyl-, Ethoxycarbonyl-, Propoxycarbonyl-, Isopropoxycarbonyl-, Butoxycarbonyl-, tert.Butoxycarbonyl-, Pentoxycarbonyl-, Hexyloxycarbonyl-, Octyloxycarbonyl-, Nonyloxycarbonyl-, Decyloxycarbonyl-, Undecyloxycarbonyl-, Dodecyloxycarbonyloder Hexadecyloxycarbonylgruppe, eine Phenyl-$C_{1-6}$-alkoxycarbonylgruppe wie die Benzyloxycarbonyl-, Phenylethoxycarbonyloder Phenylpropoxycarbonylgruppe, eine $C_{1-3}$-Alkylsulfonyl-$C_{2-4}$-alkoxycarbonyl-, $C_{1-3}$-Alkoxy-$C_{2-4}$-alkoxy-$C_{2-4}$-alkoxycarbonyl- oder $R_aCO$-O-($R_bCR_c$)-O-CO-Gruppe, in der

$R_a$ eine $C_{1-8}$-Alkyl-, $C_{5-7}$-Cycloalkyl-, Phenyl- oder Phenyl-$C_{1-3}$-alkylgruppe,

$R_b$ ein Wasserstoffatom, eine $C_{1-3}$-Alkyl-, $C_{5-7}$-Cycloalkyl- oder Phenylgruppe und

$R_c$ ein Wasserstoffatom, eine $C_{1-3}$-Alkyl- oder $R_aCO$-O-($R_bCR_c$)-C-Gruppe, in der $R_a$ bis $R_c$ wie vorstehend erwähnt definiert sind, darstellen,

und zusätzlich für eine Aminogruppe die Phthalimidogruppe zu verstehen, wobei die vorstehend erwähnten Esterreste ebenfalls als in-vivo in eine Carboxygruppe überführbare Gruppe verwendet werden können.

Hierbei werden die Verbindungen der allgemeinen Formel I von der prioritätsbegründenden deutschen Anmeldung Nr. 198 44 000.3 umfaßt, in der

X ein Sauerstoff- oder Schwefelatom,

$R_1$ ein Wasserstoffatom oder eine $C_{1-3}$-Alkylgruppe,

$R_2$ ein Wasserstoff-, Fluor-, Chlor-, Brom- oder Jodatom, eine $C_{1-3}$-Alkyl- oder Nitrogruppe,

$R_3$ eine Phenyl- oder Naphthylgruppe, die jeweils durch Fluor-, Chlor- oder Bromatome, durch $C_{1-3}$-Alkyl-, $C_{1-3}$-Alkoxy-, Cyano-, Trifluormethyl-, Nitro-, Amino-, $C_{1-3}$-Alkylamino-, Di-($C_{1-3}$-alkyl)-amino-, $C_{1-3}$-Alkylsulfonylamino-, Amino-$C_{1-3}$-alkyl-, $C_{1-3}$-Alkylamino-$C_{1-3}$-alkyl-, $C_{2-4}$-Alkanoyl-amino-$C_{1-3}$-alkyl-, N-($C_{2-4}$-Alkanoyl)-$C_{1-3}$-alkylamino-$C_{1-3}$-alkyl- oder Di-($C_{1-3}$-Alkyl)-amino-$C_{1-3}$-alkylgruppen mono- oder disubstituiert sein können, wobei die Substituenten gleich oder verschieden sein können,

$R_4$ ein Wasserstofatom oder eine $C_{1-3}$-Alkylgruppe und

$R_5$ ein gegebenenfalls durch eine $C_{1-3}$-Alkylgruppe substituierte Phenyl- oder Naphthylgruppe, die jeweils zusätzlich im aromatischen Teil

durch ein Fluor-, Chlor-, Brom- oder Jodatom, durch eine $C_{1-3}$-Alkyl-, $C_{1-3}$-Alkoxy-, Cyano-, Nitro- oder Trifluormethylgruppe,

durch eine gegebenenfalls durch eine $C_{1-3}$-Alkyl-, Phenyloder Phenyl-$C_{1-3}$-alkylgruppe substituierte Piperidino-, Hexamethylenimino-, Morpholino-, Piperazinogruppe substituierte $C_{1-3}$-Alkylgruppe, wobei die vorstehend erwähnten Piperidino- oder Hexamethyleniminogruppen zusätzlich in 3- oder 4-Stellung durch eine Hydroxy-, $C_{1-3}$-Alkoxy- oder Carboxygruppe substituiert sein können,

durch eine gegebenenfalls durch eine Hydroxy-, $C_{1-3}$-Alkoxy-, Carboxy- oder Cyanogruppe substituierte $C_{1-3}$-Alkylgruppe,

durch eine gegebenenfalls durch eine oder zwei $C_{1-3}$-Alkylgruppen substituierte Aminocarbonylamino-, Amidino- oder Guanidinogruppe,

durch eine Piperidino-, Hexamethylenimino-, Morpholino-, Piperazino- oder N-($C_{1-3}$-Alkyl)-piperazinogruppe,

durch eine Formyl-, Carboxy- oder Trifluoracetylgruppe,

durch eine Carbonylgruppe, die durch eine $C_{1-3}$-Alkyl-, $C_{1-3}$-Alkoxy-$C_{1-3}$-alkyl-, Amino-, $C_{1-5}$-Alkylamino- oder Di-($C_{1-3}$-Alkyl)-aminogruppe substiuiert ist, wobei die vorstehend erwähnten Amino- und $C_{1-3}$-Alkylaminogruppen zusätzlich am Stickstoffatom durch eine Carboxy-$C_{1-3}$-alkyl-, Amino-$C_{1-3}$-alkyl-, $C_{1-3}$-Alkylamino-$C_{1-3}$-alkyl- oder Di-($C_{1-3}$-Alkyl)-amino-$C_{1-3}$-alkylgruppe substituiert sein können,

durch eine Piperidinocarbonyl-, Hexamethyleniminocarbonyl-, Morpholinocarbonyl-, Piperazinocarbonyl-, N-($C_{1-3}$-Alkyl)-piperazinocarbonyl- oder N-(Phenyl-$C_{1-3}$-alkyl)-piperazinocarbonylgruppe,

durch eine Amino-, $C_{1-5}$-Alkylamino-, Amino-$C_{1-3}$-alkyl-, N-($C_{1-3}$-Alkylamino)-$C_{1-3}$-alkyl- oder Di-($C_{1-5}$-alkylamino)-$C_{1-3}$-alkylgruppe, wobei der Alkylteil der vorstehend erwähnten $C_{1-3}$-Alkylaminoteile durch eine Cyano-, Carboxy-, Aminocarbonyl-, $C_{1-3}$-Alkylaminocarbonyl-, Di-($C_{1-3}$-alkyl)-aminocarbonyl-, 2-[Di-($C_{1-3}$-alkyl)-amino]-ethylaminocarbonyl- oder 3-[Di-($C_{1-3}$-alkyl)-amino]-propylaminocarbonylgruppe oder in 2- oder 3-Stellung durch eine Hydroxy-, $C_{1-3}$-Alkoxy-, Di-($C_{1-3}$-alkyl)-amino-, Piperidino-, Hexamethylenimino-, Morpholino-, Piperazino- oder N-($C_{1-3}$-Alkyl)-piperazinogruppe substituiert sein können und das Stickstoffatom der vorstehend erwähnten Amino-, $C_{1-3}$-Alkylamino-, Amino-$C_{1-3}$-alkyl- oder N-($C_{1-5}$-Alkylamino)-$C_{1-3}$-alkylteile zusätzlich

durch eine $C_{1-5}$-Alkoxycarbonylgruppe,

durch eine Formyl- oder Trifluoracetylgruppe, durch eine $C_{1-5}$-Alkylgruppe, die mit Ausnahme der 1-Stellung durch eine Hydroxy-, $C_{1-3}$-Alkoxy-, Amino-, $C_{1-3}$-Alkylamino- oder Di-($C_{1-3}$)-alkylaminogruppe substituiert sein kann,

durch eine $C_{2-4}$-Alkanoylgruppe, die im Alkanoylteil durch eine Carboxy-, Hydroxy-, $C_{1-3}$-Alkoxy-, Amino-, $C_{2-4}$-Alkanoylamino-, $C_{1-5}$-Alkoxycarbonylamino-, $C_{1-3}$-Alkylamino-, Di-($C_{1-3}$-alkyl)-amino-, Piperidino-, Hexamethyleniminooder Morpholinogruppe oder durch eine gegebenenfalls am Stickstoffatom durch eine $C_{1-3}$-Alkyl- oder Phenyl-$C_{1-3}$-alkylgruppe substituierte Piperazinogruppe substituiert sein kann,

durch eine $C_{1-3}$-Alkylsulfonyl-, Amidosulfonyl-, $C_{1-3}$-Alkylamidosulfonyl- oder Di-($C_{1-3}$-alkyl)-amidosulfonylgruppe,

durch eine gegebenenfalls im Phenylteil durch ein Fluor-, Chlor- oder Bromatom oder durch eine $C_{1-3}$-Alkyl- oder $C_{1-3}$-Alkoxygruppe substituierte Phenyl-($C_{1-3}$)-alkylsulfonyl- oder Phenylsulfonylgruppe substituiert sein kann,
substituiert sein können,
bedeuten, wobei zusätzlich eine vorhandene Carboxy-, Amino- oder Iminogruppe durch einen in-vivo abspaltbaren Rest substituiert sein kann,
deren Isomere und deren Salze.

Bevorzugte Verbindungen der allgemeinen Formel I sind diejenigen, in denen
X ein Sauerstoff- oder Schwefelatom,
$R_1$ ein Wasserstoffatom, eine $C_{1-3}$-Alkyl-, Hydroxy-, $C_{1-4}$-Alkoxycarbonyl- oder $C_{2-4}$-Alkanoylgruppe,
$R_2$ ein Wasserstoff-, Fluor-, Chlor-, Brom- oder Jodatom, eine $C_{1-3}$-Alkyl- oder Nitrogruppe,
$R_3$ eine Phenyl- oder Naphthylgruppe, die jeweils durch Fluor-, Chlor-, Brom- oder Jodatome, durch $C_{1-3}$-Alkyl-, Imidazolylmethyl-, 2-Carboxy-ethenyl-, 2-($C_{1-3}$-Alkoxycarbonyl)-ethenyl-, $C_{1-3}$-Alkoxy-, Cyano-, Carboxy-, $C_{1-3}$-Alkoxycarbonyl-, Trifluormethyl-, Nitro-, Amino-, Phthalimidomethyl-, 2-Carboxy-phenylcarbonylaminomethyl-, $C_{1-3}$-Alkylamino-, Di-($C_{1-3}$-alkyl)-amino-, $C_{1-3}$-Alkylsulfonylamino-, Amino-$C_{1-3}$-alkyl-, $C_{1-3}$-Alkylamino-$C_{1-3}$-alkyl-, $C_{2-4}$-Alkanoylamino-$C_{1-3}$-alkyl-, N-($C_{2-4}$-Alkanoyl)-$C_{1-3}$-alkylamino-$C_{1-3}$-alkyl-, Di-($C_{1-3}$-Alkyl)-amino-$C_{1-3}$-alkyl-, Carboxy-$C_{1-3}$-alkylaminocarbonyl- oder $C_{1-3}$-Alkoxycarbonyl-$C_{1-3}$-alkylaminocarbonylgruppen mono- oder disubstituiert sein können, wobei die Substituenten gleich oder verschieden sein können,
$R_4$ ein Wasserstoffatom oder eine $C_{1-3}$-Alkylgruppe und
$R_5$ ein gegebenenfalls durch eine $C_{1-3}$-Alkylgruppe substituierte Phenyl- oder Naphthylgruppe, die jeweils zusätzlich im aromatischen Teil

durch ein Fluor-, Chlor-, Brom- oder Jodatom, durch eine $C_{1-3}$-Alkyl-, $C_{1-3}$-Alkoxy-, Cyano-, Nitro- oder Trifluormethylgruppe, wobei die vorstehend erwähnte Alkylgruppe gleichzeitig durch eine Carboxy- oder $C_{1-3}$-Alkoxycarbonylgruppe und eine Amino- oder $C_{1-4}$-Alkoxycarbonylaminogruppe substituiert sein kann,

eine $C_{1-3}$-Alkylgruppe, die durch eine 4- bis 7-gliedrige Cycloalkyleniminogruppe, durch eine Dehydropiperidino-, Morpholino-, Thiomorpholino-, 1-Oxido-thiomorpholino-, 1,1-Dioxido-thiomorpholino-, Piperazino- oder N-($C_{1-4}$-Alkoxycarbonyl)-piperazinogruppe substituiert ist, wobei die vorstehend erwähnten Piperidino-, Hexamethylenimino-, Morpholino-, Thiomorpholino-, 1-Oxido-thiomorpholino-, 1,1-Dioxido-thiomorpholino- und Piperazinogruppen durch eine $C_{1-3}$-Alkyl-, Phenyl- oder Phenyl-$C_{1-3}$-alkylgruppe und die vorstehend erwähnten Piperidinogruppen zusätzlich durch eine $C_{1-3}$-Alkylgruppe oder in 3- oder 4-Stellung durch eine Hydroxy-, $C_{1-3}$-Alkoxy-, Hydroxy-$C_{1-3}$-alkyl-, Carboxy-, Aminocarbonyl-, N-($C_{1-3}$-Alkyl)-aminocarbonyl- oder N,N-Di-($C_{1-3}$-Alkyl)-aminocarbonylgruppe substituiert sein können,
durch eine gegebenenfalls durch eine Hydroxy-, $C_{1-3}$-Alkoxy-, Carboxy-, $C_{1-3}$-Alkoxycarbonyl- oder Cyanogruppe substituierte $C_{1-3}$-Alkylgruppe,
durch eine gegebenenfalls durch eine oder zwei $C_{1-3}$-Alkylgruppen substituierte Aminocarbonylamino-, Amidino- oder Guanidinogruppe,
durch eine Piperidino-, Hexamethylenimino-, Morpholino-, Piperazino- oder N-($C_{1-3}$-Alkyl)-piperazinogruppe,
durch eine Formyl-, Carboxy-, $C_{1-3}$-Alkoxycarbonyl- oder Trifluoracetylgruppe,
durch eine Carbonylgruppe, die

durch eine $C_{1-3}$-Alkyl-, $C_{1-3}$-Alkoxy-$C_{1-3}$-alkyl-, Amino-, $C_{1-5}$-Alkylamino- oder Di-($C_{1-3}$-Alkyl)-aminogruppe substituiert ist, wobei die vorstehend erwähnten Amino- und $C_{1-3}$-Alkylaminogruppen zusätzlich am Stickstoffatom durch eine Carboxy-$C_{1-3}$-alkyl- oder $C_{1-3}$-Alkoxycarbonyl-$C_{1-3}$-alkylgruppe oder durch eine $C_{2-3}$-Alkylgruppe, die in 2- oder 3-Stellung durch eine Hydroxy-, $C_{1-3}$-Alkoxy-, Amino-, $C_{1-3}$-Alkylamino- oder Di-($C_{1-3}$-Alkyl)-aminogruppe substituiert sein können,
durch eine Pyrrolidinocarbonyl-, Pyrrolidinosulfonyl-, Piperidinocarbonyl-, Hexamethyleniminocarbonyl-, Morpholinocarbonyl-, Piperazinocarbonyl-, N-($C_{1-3}$-Alkyl)-piperazinocarbonyloder N-(Phenyl-$C_{1-3}$-alkyl)-piperazinocarbonylgruppe,
durch eine Amidosulfonyl-, $C_{1-3}$-Alkylamidosulfonyl- oder Di-($C_{1-3}$-alkyl)-amidosulfonylgruppe, in denen ein Alkylteil durch eine Carboxy-, $C_{1-3}$-Alkoxycarbonyl-, Aminocarbonyl-, $C_{1-3}$-Alkylaminocarbonyl- oder Di-($C_{1-3}$-alkyl)-aminocarbonylgruppe oder in 2- oder 3-Stellung durch eine Amino-, $C_{1-3}$-Alkylamino- oder Di-($C_{1-3}$-alkyl)-aminogruppe substituiert sein kann,
durch eine Amino-, $C_{1-5}$-Alkylamino-, Amino-$C_{1-3}$-alkyl-, N-($C_{1-3}$-Alkyl)-amino-$C_{1-3}$-alkyl-, N-(2-Hydroxyethyl)-amino-$C_{1-3}$-alkyl-, N-(3-Hydroxypropyl)-amino-$C_{1-3}$-alkyl-, Di-($C_{1-5}$-alkyl)-amino-$C_{1-3}$-alkyl-, N-($C_{3-7}$-Cycloalkyl)-ami-

no-$C_{1-3}$-alkyl-, N-($C_{3-7}$-Cycloalkyl)-N-($C_{1-3}$-alkyl)-amino-$C_{1-3}$-alkyl- oder N-(Phenyl-$C_{1-3}$-alkyl)-amino-$C_{1-3}$-alkylgruppe, wobei der N-Alkylteil der vorstehend erwähnten Gruppen durch eine Cyano-, Carboxy-, $C_{1-3}$-Alkylcarbonyl-, Aminocarbonyl-, $C_{1-3}$-Alkylaminocarbonyl-, Di-($C_{1-3}$-alkyl)-aminocarbonyl-, 2-[Di-($C_{1-3}$-alkyl)-amino]-ethylaminocarbonyl-, 3-[Di-($C_{1-3}$-alkyl)-amino]-propylaminocarbonyl-, N-{2-[Di-($C_{1-3}$-alkyl)-amino]-ethyl}-N-($C_{1-3}$-alkyl)-aminocarbonyl- oder N-{3-[Di-($C_{1-3}$-alkyl)-amino]-propyl}-N-($C_{1-3}$-alkyl)-aminocarbonylgruppe oder in 2- oder 3-Stellung durch eine Hydroxy-, $C_{1-3}$-Alkoxy-, Amino-, $C_{1-3}$-Alkylamino-, Di-($C_{1-3}$-alkyl)-amino- oder Morpholinogruppe substituiert sein können, wobei das Stickstoffatom der vorstehend erwähnten Amino-, $C_{1-3}$-Alkylamino-, Amino-$C_{1-3}$-alkyl- oder N-($C_{1-5}$-Alkylamino)-$C_{1-3}$-alkylteile zusätzlich

durch eine $C_{1-5}$-Alkoxycarbonylgruppe,

durch eine Formyl-, Trifluoracetyl- oder Benzoylgruppe,

durch eine $C_{1-5}$-Alkylgruppe, die mit Ausnahme der 1-Stellung durch eine Hydroxy-, $C_{1-3}$-Alkoxy-, Amino-, $C_{1-3}$-Alkylamino- oder Di-($C_{1-3}$)-alkylaminogruppe substituiert sein kann,

durch eine $C_{2-4}$-Alkanoylgruppe, die im Alkanoylteil durch eine Hydroxy-, $C_{1-3}$-Alkoxy-, Amino-, $C_{2-4}$-Alkanoylamino-, $C_{1-5}$-Alkoxycarbonylamino-, Phthalimido-, $C_{1-3}$-Alkylamino-, Di-($C_{1-3}$-alkyl)-amino-, N-($C_{1-3}$-alkyl)-phenylamino-, Pyrrolidino-, Piperidino- oder Morpholinogruppe oder durch eine gegebenenfalls am Stickstoffatom durch eine $C_{1-3}$-Alkyl- oder Phenyl-$C_{1-3}$-alkylgruppe substituierte Piperazinogruppe substituiert sein kann, wobei der N-Alkylteil der vorstehend erwähnten Gruppen in 2- oder 3-Stellung durch eine Methoxy-, Di-($C_{1-3}$-alkyl)-amino- oder Morpholinogruppe substituiert sein kann,

durch eine $C_{1-5}$-Alkylsulfonylgruppe, in der der Alkylteil mit Ausnahme der 1-Stellung durch eine Di-($C_{1-3}$-alkyl)-amino-, Pyrrolidino-, Piperidino-, Hexamethylenimino- oder Morpholinogruppe substituiert sein kann,

durch eine Pyridinyl- oder Pyrimidinylgruppe,

durch eine gegebenenfalls im Phenylteil durch eine $C_{1-3}$-Alkylgruppe substituierte Phenyl-, Phenyl-($C_{1-3}$)-alkylsulfonyl- oder Phenylsulfonylgruppe substituiert sein kann,

durch eine $C_{1-3}$-Alkoxygruppe, die durch eine Carboxy-, $C_{1-3}$-Alkoxycarbonyl-, Aminocarbonyl-, $C_{1-3}$-Alkylaminocarbonyl- oder Di-($C_{1-3}$-Alkyl)-aminocarbonylgruppe oder in 2- oder 3-Stellung durch eine Amino-, $C_{1-3}$-Alkylamino-, Di-($C_{1-3}$-Alkyl)-amino-, N-($C_{1-3}$-Alkyl)-N-(phenyl-$C_{1-3}$-alkyl)-amino-, Piperidino- oder Hexamethyleniminogruppe substituiert ist,

durch eine Prop-1-enyl-, 2-Chlor-prop-1-enyl- oder Prop-1-inyl-Gruppe, die in 3-Stellung durch eine Di-($C_{1-3}$-Alkyl)-aminogruppe substituiert ist,

substituiert sein können,

bedeuten, deren Isomere und deren Salze.

Besonders bevorzugte Verbindungen der allgemeinen Formel I sind diejenigen, in denen

X ein Sauerstoffatom,

$R_1$ ein Wasserstoffatom, eine $C_{1-3}$-Alkyl-, $C_{1-4}$-Alkoxycarbonyl- oder $C_{2-4}$-Alkanoylgruppe,

$R_2$ ein Wasserstoff-, Fluor-, Chlor-, Brom- oder Jodatom, eine $C_{1-3}$-Alkyl- oder Nitrogruppe,

$R_3$ eine Phenylgruppe, die durch Fluor-, Chlor-, Brom- oder Jodatome, durch $C_{1-3}$-Alkyl-, Trifluormethyl-, Imidazolylmethyl-, 2-Carboxy-ethenyl-, 2-$C_{1-3}$-Alkoxycarbonyl-ethenyl-, $C_{1-3}$-Alkoxy-, Cyano-, Carboxy-, $C_{1-3}$-Alkoxycarbonyl-, Nitro-, Amino-, Phthalimidomethyl-, 2-Carboxy-benzoylaminomethyl-, $C_{1-3}$-Alkylamino-, Di-($C_{1-3}$-alkyl)-amino-, $C_{1-3}$-Alkylsulfonylamino-, Amino-$C_{1-3}$-alkyl-, $C_{1-3}$-Alkylamino-$C_{1-3}$-alkyl-, $C_{2-4}$-Alkanoylamino-$C_{1-3}$-alkyl-, N-($C_{2-4}$-Alkanoyl)-$C_{1-3}$-alkylamino-$C_{1-3}$-alkyl-, Di-($C_{1-3}$-Alkyl)-amino-$C_{1-3}$-alkyl-, Carboxy-$C_{1-3}$-alkylaminocarbonyl- oder $C_{1-3}$-Alkoxycarbonyl-$C_{1-3}$-alkylaminocarbonylgruppen mono- oder disubstituiert sein können, wobei die Substituenten gleich oder verschieden sein können,

$R_4$ ein Wasserstoffatom oder eine $C_{1-3}$-Alkylgruppe und

$R_5$ ein gegebenenfalls durch eine $C_{1-3}$-Alkylgruppe substituierte Phenyl- oder Naphthylgruppe, die jeweils zusätzlich im aromatischen Teil

durch ein Fluor-, Chlor-, Brom- oder Jodatom, durch eine $C_{1-3}$-Alkoxy-, Cyano-, Nitro- oder Trifluormethylgruppe,

eine $C_{1-3}$-Alkylgruppe, die durch eine 4- bis 7-gliedrige Cycloalkyleniminogruppe, durch eine Dehydropiperidino-, Morpholino-, Thiomorpholino-, 1-Oxido-thiomorpholino-, 1,1-Dioxido-thiomorpholino-, Piperazino- oder N-($C_{1-4}$-Alkoxycarbonyl)-piperazinogruppe substituiert ist, wobei die vorstehend erwähnten Piperidino-, Hexamethylenimino-, Morpholino- und Piperazinogruppen durch eine $C_{1-3}$-Alkyl-, Phenyl- oder Phenyl-$C_{1-3}$-alkylgruppe und die vorstehend erwähnten Piperidinogruppen zusätzlich durch eine $C_{1-3}$-Alkylgruppe oder in 3-oder 4-Stellung durch eine Hydroxy-, $C_{1-3}$-Alkoxy-, Hydroxy-$C_{1-3}$-alkyl-, Carboxy-, Aminocarbonyl-, N-($C_{1-3}$-Alkyl)-aminocarbonyl- oder N,N-Di-($C_{1-3}$-Alkyl)-aminocarbonylgruppe substituiert sein können,

durch eine gegebenenfalls durch eine Hydroxy-, $C_{1-3}$-Alkoxy-, Carboxy-, $C_{1-3}$-Alkoxycarbonyl- oder Cyanogruppe substituierte $C_{1-3}$-Alkylgruppe,

durch eine gegebenenfalls durch eine oder zwei $C_{1-3}$-Alkylgruppen substituierte Aminocarbonylamino-, Amidino- oder Guanidinogruppe,

durch eine Piperidino-, Hexamethylenimino-, Morpholino-, Piperazino- oder N-($C_{1-3}$-Alkyl)-piperazinogruppe,

durch eine Formyl-, Carboxy-, $C_{1-3}$-Alkoxycarbonyl- oder Trifluoracetylgruppe,
durch eine Carbonylgruppe, die
durch eine $C_{1-3}$-Alkyl-, $C_{1-3}$-Alkoxy-$C_{1-3}$-alkyl-, Amino-, $C_{1-5}$-Alkylamino- oder Di-($C_{1-3}$-Alkyl)-aminogruppe substituiert ist, wobei die vorstehend erwähnten Amino- und $C_{1-3}$-Alkylaminogruppen zusätzlich am Stickstoffatom durch eine Carboxy-$C_{1-3}$-alkyl-, $C_{1-3}$-Alkoxycarbonyl-$C_{1-3}$-alkyl- oder $C_{1-3}$-Alkoxycarbonyl-$C_{1-3}$-alkylgruppe oder durch eine $C_{2-3}$-Alkylgruppe, die in 2- oder 3-Stellung durch eine Hydroxy-, $C_{1-3}$-Alkoxy-, Amino-, $C_{1-3}$-Alkylamino- oder Di-($C_{1-3}$-Alkyl)-aminogruppe substituiert sein können,
durch eine Pyrrolidinocarbonyl-, Pyrrolidinosulfonyl-, Piperidinocarbonyl- oder Hexamethyleniminocarbonylgruppe,
durch eine Amidosulfonyl-, $C_{1-3}$-Alkylamidosulfonyl- oder Di-($C_{1-3}$-alkyl)-amidosulfonylgruppe, in denen ein Alkylteil durch eine Carboxy-, $C_{1-3}$-Alkoxycarbonyl- oder Dimethylaminocarbonylgruppe oder in 2- oder 3-Stellung durch eine Dimethylaminogruppe substituiert sein kann,
durch eine geradkettige $C_{1-2}$-Alkylgruppe, die endständig durch eine Amino-, Benzylamino-, Pyridylamino- oder Pyrimidylaminogruppe, durch eine $C_{1-4}$-Alkylaminogruppe, in der der Alkylteil in Position 2, 3 oder 4 durch eine Hydroxy- oder Methoxygruppe substituiert sein kann, oder durch eine im $C_{1-2}$-Alkylteil durch eine Carboxy-, $C_{1-3}$-Alkoxycarbonyl- oder Di-($C_{1-3}$-Alkyl)-aminocarbonylgruppe substituierte $C_{1-2}$-Alkylaminogruppe substituiert ist, wobei in den vorstehend erwähnten Gruppen ein am Aminstickstoffatom vorhandenes Wasserstoffatom zusätzlich
durch eine $C_{3-6}$-Cycloalkylgruppe, durch eine $C_{1-4}$-Alkylgruppe, in der der Alkylteil in Position 2, 3 oder 4 durch eine Hydroxygruppe substituiert sein kann, durch eine gegebenenfalls durch eine Methoxy-, Carboxy-, $C_{1-3}$-Alkoxycarbonyl-, Amino-, Methylamino-, Dimethylamino-, Acetylamino-, $C_{1-5}$-Alkoxycarbonylamino-, N-Methyl-$C_{1-5}$-alkoxycarbonylamino- oder Morpholinocarbonylaminogruppe substituierte $C_{1-2}$-Alkylcarbonylgruppe, durch eine $C_{1-5}$-Alkoxycarbonyl-, $C_{1-4}$-Alkylsulfonyl-, Phenylsulfonyl- oder Tolylsulfonylgruppe ersetzt sein kann,
durch eine 3-Dimethylaminopropyl- oder 3-Dimethylamino-prop-1-enylgruppe,
durch eine Ethylgruppe, die in 1-Stellung durch eine Aminooder $C_{1-5}$-Alkoxycarbonylaminogruppe substituiert ist,
durch eine Ethylgruppe, die in 2-Stellung durch eine Aminooder $C_{1-5}$-Alkoxycarbonylaminogruppe und durch eine Carboxyoder $C_{1-3}$-Alkoxycarbonylgruppe substituiert ist,
durch eine Amino- oder $C_{1-3}$-Alkylaminogruppe, in der der Alkylteil durch eine Cyano-, Carboxy-, $C_{1-3}$-Alkoxycarbonyl-, Aminocarbonyl-, Methylaminocarbonyl- oder Dimethylaminocarbonylgruppe oder in 2- oder 3-Stellung durch eine Amino-, Methylamino-, Dimethylamino-, Acetylamino-, N-Methyl-acetylamino- oder Morpholinogruppe, durch eine gegebenenfalls in 2-oder 3-Position des $C_{1-3}$-Alkylteils durch eine Dimethylaminogruppe substituierte N-($C_{1-3}$-Alkyl)-aminocarbonyl- oder N-($C_{1-3}$-Alkyl)-methylaminocarbonylgruppe substituiert sein kann, wobei ein am Aminstickstoffatom vorhandenes Wasserstoffatom in den vorstehend erwähnten Gruppen zusätzlich
durch eine Formyl-, Trifluoracetyl-, Benzoyl-, $C_{1-4}$-Alkoxycarbonyl- oder $C_{1-4}$-Alkylaminocarbonylgruppe,
durch eine $C_{2-4}$-Alkanoylgruppe, die endständig durch eine Amino-, Acetylamino-, $C_{1-4}$-Alkoxycarbonylamino-, Pyrrolidino-, Piperidino-, Morpholino-, Piperazino-, 4-Methylpiperazino-, 4-Benzylpiperazino- oder Phthalimidogruppe oder durch eine $C_{1-3}$-Alkylamino-, N-Acetyl-$C_{1-3}$-alkyl-amino- oder Di-($C_{1-3}$-alkyl)-aminogruppe substituiert sein kann, wobei in den vorstehend erwähnten $C_{1-3}$-Alkylamino-, N-Acetyl-$C_{1-3}$-alkyl-amino- und Di-($C_{1-3}$-alkyl)-aminogruppen jeweils ein $C_{1-3}$-Alkylteil zusätzlich durch eine Phenylgruppe oder in 2- oder 3-Position durch eine Methoxy-, Dimethylamino- oder Morpholinogruppe substituiert sein kann,
durch eine $C_{1-4}$-Alkylsulfonylgruppe, in der der Alkylteil in 2- oder 3-Position zusätzlich durch eine Dimethylamino-, Piperidino- oder Morpholinogruppe substituiert sein kann,
durch eine Phenylsulfonyl- oder Toluolsulfonylgruppe ersetzt sein kann,
durch eine $C_{1-3}$-Alkoxygruppe, die durch eine Carboxy-, $C_{1-3}$-Alkoxycarbonyl-, Aminocarbonyl-, Methylaminocarbonyloder Dimethylaminocarbonylgruppe oder in 2- oder 3-Stellung durch eine Amino-, Methylamino-, Dimethylamino-, N-Methylbenzylamino-, Piperidino- oder Hexamethyleniminogruppe substituiert ist,
durch eine $C_{1-3}$-Alkylaminocarbonyl- oder Di-($C_{1-3}$-Alkyl)-aminocarbonylgruppe, in der ein $C_{1-3}$-Alkylteil in 2- oder 3-Stellung durch eine Methoxy- oder Dimethylaminogruppe substituiert sein kann,
substituiert sein können,
bedeuten, deren Isomere und deren Salze.

Ganz besonders bevorzugte Verbindungen der allgemeinen Formel I sind diejenigen, in denen
X ein Sauerstoffatom
$R_1$ ein Wasserstoffatom,
$R_2$ ein Wasserstoff-, Chlor- oder Bromatom, eine Methyl- oder Nitrogruppe,
$R_3$ eine Phenylgruppe, die durch ein Fluor-, Chlor- oder Bromatom, durch ein Methyl-, Methoxy-, Aminomethyl-, Acetylaminomethyl-, Carboxy-, Methoxycarbonyl- oder Imidazolylmethylgruppe substituiert sein kann,
$R_4$ ein Wasserstoffatom,
$R_5$ eine Phenylgruppe, die
durch ein Fluor-, Chlor- oder Bromatom, durch eine Methyl-, Methoxy-, Nitro-, Cyano- oder Trifluormethylgruppe,
durch eine Methyl oder Ethylgruppe, die jeweils durch eine Carboxy-, $C_{1-3}$-Alkoxycarbonyl-, Cyano-, Azetidin-

1-yl-, Pyrrolidino-, Piperidino-, 4-Phenylpiperidino-, 3,6-Dihydro-2H-pyridin-1-yl-, Hexamethylenimino, Morpholino-, Thiomorpholino-, 1-Oxido-thiomorpholino-, Piperazino-, 4-Methylpiperazino- oder 4-Acetylpiperazinogruppe substituiert ist, wobei die vorstehend erwähnten Piperidinogruppen zusätzlich durch eine oder zwei Methylgruppen oder in 3- oder 4-Position durch eine Hydroxy-, Methoxy-, Carboxy-, Hydroxymethyl-, $C_{1-3}$-Alkoxycarbonyl-, Aminocarbonyl-, Methylaminocarbonyl- oder Dimethylaminocarbonylgruppe substituiert sein können,

durch eine geradkettige $C_{1-2}$-Alkygruppe, die endständig durch eine Amino- oder Benzylaminogruppe, durch eine $C_{1-4}$-Alkylaminogruppe, in der der Alkylteil in den Positionen 2, 3 oder 4 durch eine Hydroxy- oder Methoxygruppe substituiert sein kann, durch eine im $C_{1-2}$-Alkylteil durch eine Carboxy-, $C_{1-3}$-Alkoxycarbonyl- oder Dimethylaminocarbonylgruppe substituierte $C_{1-2}$-Alkylaminogruppe substituiert ist, wobei in den vorstehend erwähnten Gruppen ein am Aminstickstoff vorhandenes Wasserstoffatom zusätzlich

durch eine $C_{3-6}$-Cycloalkylgruppe, durch eine $C_{1-4}$-Alkylgruppe, in der der Alkylteil in den Positionen 2, 3 oder 4 durch eine Hydroxygruppe substituiert sein kann, oder durch eine gegebenenfalls durch eine Amino-, Methylaminooder Dimethylaminogruppe substituierte $C_{1-2}$-Alkylcarbonylgruppe ersetzt sein kann,

durch eine 3-Dimethylamino-prop-1-enylgruppe,

durch eine Ethylgruppe, die in 1-Position durch eine Aminooder $C_{1-4}$-Alkoxycarbonylaminogruppe substituiert ist,

durch eine Amino- oder $C_{1-3}$-Alkylaminogruppe, in der der Alkylteil endständig durch eine Carboxy-, Aminocarbonyl-, Methylaminocarbonyl- oder Dimethylaminocarbonylgruppe oder in 2- oder 3-Stellung durch eine Amino-, Methylamino-, Dimethylamino-, Acetylamino-, N-Acetyl-methylamino- oder Morpholinogruppe oder durch eine gegebenenfalls in 2- oder 3-Position durch eine Dimethylaminogruppe substituierte N-($C_{1-3}$-Alkyl)-aminocarbonyl- oder N-($C_{1-3}$-Alkyl)-methylaminocarbonylgruppe substituiert sein kann, wobei ein am Aminstickstoff vorhandenes Wasserstoffatom in den vorstehend erwähnten Gruppen zusätzlich

durch eine Formyl- oder Benzoylgruppe,

durch eine $C_{2-4}$-Alkanoylgruppe, die endständig durch eine Amino-, Acetylamino-, Pyrrolidino-, Piperidino-, Morpholino-, Piperazino- oder 4-Methylpiperazinogruppe oder durch eine $C_{1-3}$-Alkylamino-, N-Acetyl-$C_{1-3}$-alkylaminooder Di-($C_{1-3}$-alkyl)-aminogruppe substituiert sein kann, wobei in den vorstehend erwähnten $C_{1-3}$-Alkylamino-, N-Acetyl-$C_{1-3}$-alkylamino- oder Di-($C_{1-3}$-alkyl)-aminogruppen ein $C_{1-3}$-Alkylteil zusätzlich in 2- oder 3-Position durch eine Methoxy-, Dimethylamino- oder Morpholinogruppe substituiert sein kann,

durch eine $C_{1-4}$-Alkylsulfonylgruppe, die in 2- oder 3-Stellung durch eine Dimethylaminogruppe substituiert sein kann, ersetzt sein kann,

durch eine Pyrrolindinosulfonylgruppe, eine Aminosulfonyl-, $C_{1-3}$-Alkylaminosulfonyl- oder Di-($C_{1-3}$-alkyl)-aminosulfonylgruppe, in denen jeweils ein $C_{1-3}$-Alkylteil durch eine Carboxy-, $C_{1-3}$-Alkoxycarbonyl-, Aminocarbonyl-, Methylaminocarbonyl- oder Dimethylaminocarbonylgruppe oder mit Ausnahme der 1-Stellung durch eine Dimethylaminogruppe substituiert sein kann,

durch eine $C_{2-3}$-Alkoxygruppe, die in 2- oder 3-Stellung durch eine Dimethylamino- oder Piperidinogruppe substituiert ist,

durch eine Aminocarbonyl-, $C_{1-3}$-Alkylaminocarbonyl- oder Di-($C_{1-3}$-alkyl)-aminocarbonylgruppe, in denen jeweils die $C_{1-3}$-Alkylteile mit Ausnahme der 1-Position durch eine Methoxy- oder Dimethylaminogruppe substituiert sein können, substituiert sein kann, bedeuten,

insbesondere diejenigen Verbindungen der obigen allgemeinen Formel I sind diejenigen, in denen

X und $R_2$ bis $R_4$ wie vorstehend erwähnt definiert sind,

$R_1$ ein Wasserstoffatom und

$R_5$ eine Phenylgruppe bedeutet, die

durch eine Methyl oder Ethylgruppe, die jeweils durch eine Azetidin-1-yl-, Pyrrolidino-, Piperidino-, Hexamethylenimino-, Morpholino-, 1-Oxido-thiomorpholino-, Piperazino-, 4-Methylpiperazino- oder 4-Acetylpiperazinogruppe substituiert ist, wobei die vorstehend erwähnten Piperidinogruppen zusätzlich durch eine oder zwei Methylgruppen oder in 4-Position durch eine Hydroxy-, Methoxy-, Hydroxymethyl-, Aminocarbonyl-, Methylaminocarbonyl- oder Dimethylaminocarbonylgruppe substituiert sein können,

durch eine geradkettige $C_{1-2}$-Alkygruppe, die endständig durch eine Aminogruppe oder durch eine $C_{1-3}$-Alkylaminogruppe substituiert ist, wobei der Alkylteil der $C_{1-3}$-Alkylaminogruppe in den Positionen 2 oder 3 durch eine Hydroxy- oder Methoxygruppe substituiert sein kann und in den vorstehend erwähnten Gruppen das am Aminstickstoff vorhandene Wasserstoffatom zusätzlich

durch eine $C_{3-6}$-Cycloalkylgruppe, durch eine $C_{1-3}$-Alkylgruppe, in der der Alkylteil in den Positionen 2 oder 3 durch eine Hydroxygruppe substituiert sein kann, oder durch eine durch eine Amino-, Methylamino- oder Dimethylaminogruppe substituierte $C_{1-2}$-Alkylcarbonylgruppe ersetzt sein kann,

durch eine Ethylgruppe, die in 1-Position durch eine Aminogruppe substituiert ist,

durch eine Amino- oder $C_{1-3}$-Alkylaminogruppe, in der der Alkylteil endständig durch eine Carboxy-, Aminocarbonyl-, Methylaminocarbonyl-, Dimethylaminocarbonyl-, N-(2-Dimethylamino-ethyl)-aminocarbonyl- oder N-(2-Dimethylamino-ethyl)-N-methyl-aminocarbonylgruppe oder in 2- oder 3-Stellung durch eine Amino-, Methylamino-, Dimethylamino-,

Acetylamino-, N-Acetyl-methylamino- oder Morpholinogruppe substituiert sein kann, wobei das vorhandene Wasserstoffatom am Aminstickstoff der vorstehend erwähnten Gruppen zusätzlich

durch eine $C_{2-4}$-Alkanoylgruppe, die endständig durch eine Amino-, Acetylamino-, Pyrrolidino-, Piperidino-, Morpholino-, Piperazino- oder 4-Methylpiperazinogruppe oder durch eine $C_{1-3}$-Alkylamino-, N-Acetyl-$C_{1-3}$-alkylaminooder Di-($C_{1-3}$--alkyl)-aminogruppe substituiert sein kann, wobei in den vorstehend erwähnten $C_{1-3}$-Alkylamino-, N-Acetyl-$C_{1-3}$-alkylamino- oder Di-($C_{1-3}$--alkyl)-aminogruppen ein $C_{1-3}$-Alkylteil zusätzlich in 2- oder 3-Position durch eine Methoxy-, Dimethylamino- oder Morpholinogruppe substituiert sein kann,

durch eine $C_{1-4}$-Alkylsulfonylgruppe, die in 2- oder 3-Stellung durch eine Dimethylaminogruppe substituiert sein kann, ersetzt sein kann,

durch eine Pyrrolindinosulfonylgruppe, eine Aminosulfonyl-, $C_{1-3}$-Alkylaminosulfonyl- oder Di-($C_{1-3}$-alkyl)-aminosulfonylgruppe, in denen jeweils ein $C_{1-3}$-Alkylteil durch eine Carboxy-, Methoxycarbonyl-, Aminocarbonyl-, Methylaminocarbonyl- oder Dimethylaminocarbonylgruppe oder mit Ausnahme der 1-Stellung durch eine Dimethylaminogruppe substituiert sein kann,

durch eine $C_{1-3}$-Alkoxygruppe, die in 2- oder 3-Stellung durch eine Dimethylamino- oder Piperidinogruppe substituiert ist,

durch eine Aminocarbonyl-, $C_{1-3}$-Alkylaminocarbonyl- oder Di-($C_{1-3}$-alkyl)-aminocarbonylgruppe, in denen jeweils ein $C_{1-3}$-Alkylteil mit Ausnahme der 1-Position durch eine Methoxy- oder Dimethylaminogruppe substituiert sein können, substituiert sein kann, bedeuten,

deren Isomere und deren Salze.

Als besonders bevorzugte Verbindungen der allgemeinen Formel I seien beispielsweise folgende erwähnt:

(a) (Z)-3-[1-(4-Dimethylaminomethyl-phenylamino)-1-phenyl-methyliden] -5-nitro-2-indolinon,

(b) (Z)-3-[1-(4-Piperidinomethyl-phenylamino)-1-phenyl-methyliden]-5-nitro-2-indolinon,

(c) (Z)-3-{1-[4-(2-Morpholinoethyl)-phenylamino]-1-phenyl-methyliden}-5-nitro-2-indolinon,

(d) (Z)-3-{1-[4-(2-Dimethylamino-ethyl)-phenylamino]-1-phenylmethyliden}-5-nitro-2-indolinon und

(e) (Z)-3-{1-[4-(N-(2-Dimethylamino-ethyl)-N-methylsulfonylamino)-phenylamino]-1-phenyl-methyliden}-2-indolinon

sowie deren Salze.

Erfindungsgemäß erhält man die neuen Verbindungen beispielsweise nach folgenden im Prinzip literaturbekannten Verfahren:

a. Umsetzung einer Verbindung der allgemeinen Formel

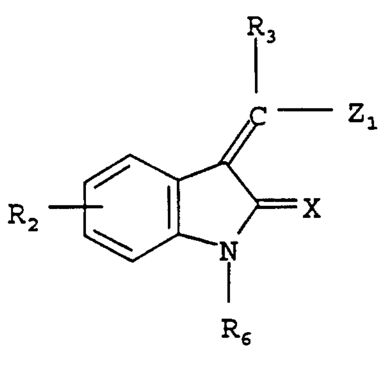 (II),

in der

X, $R_2$ und $R_3$ wie eingangs erwähnt definiert sind,

$R_6$ ein Wasserstoffatom, eine Schutzgruppe für das Stickstoffatom der Lactamgruppe oder eine Bindung an eine Festphase und

$Z_1$ ein Halogenatom, eine Hydroxy-, Alkoxy- oder Aralkoxygruppe, z.B. ein Chlor- oder Bromatom, eine Methoxy-, Ethoxy- oder Benzyloxygruppe, bedeuten,

mit einem Amin der allgemeinen Formel

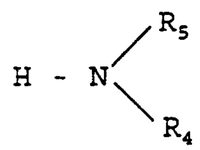

(III),

in der

$R_4$ und $R_5$ wie eingangs erwähnt definiert sind,

und erforderlichenfalls anschließende Abspaltung einer verwendeten Schutzgruppe für das Stickstoffatom der Lactamgruppe oder von einer Festphase.

Als Schutzgruppe für das Stickstoffatom der Lactamgruppe kommt beispielsweise eine Acetyl-, Benzoyl-, Ethoxycarbonyl-, tert.Butyloxycarbonyl- oder Benzyloxycarbonylgruppe und

als Festphase ein Rink-Harz wie ein p-Benzyloxybenzylalkoholharz, wobei die Bindung zweckmäßigerweise über ein Zwischenglied wie ein 2,5-Dimethoxy-4-hydroxy-benzylderivat erfolgt, in Betracht.

Die Umsetzung wird zweckmäßigerweise in einem Lösungsmittel wie Dimethylformamid, Toluol, Acetonitril, Tetrahydrofuran, Dimethylsulfoxid, Methylenchlorid oder deren Gemischen gegebenenalls in Gegenwart einer inerten Base wie Triethylamin, N-Ethyl-diisopropylamin oder Natriumhydrogencarbonat bei Temperaturen zwischen 20 und 175°C durchgeführt, wobei eine verwendete Schutzgruppe infolge Umamidierung gleichzeitig abgespalten werden kann.

Bedeutet $Z_1$ in einer Verbindung der allgemeinen Formel II ein Halogenatom, dann wird die Umsetzung vorzugsweise in Gegenwart einer inerten Base bei Temperaturen zwischen 20 und 120°C, durchgeführt.

Bedeutet $Z_1$ in einer Verbindung der allgemeinen Formel II eine Hydroxy-, Alkoxy- oder Aralkoxygruppe, dann wird die Umsetzung vorzugsweise bei Temperaturen zwischen 20 und 200°C, durchgeführt.

Die gegebenenfalls erforderliche anschließende Abspaltung einer verwendeten Schutzgruppe wird zweckmäßigerweise entweder hydrolytisch in einem wäßrigen oder alkoholischen Lösungsmittel, z.B. in Methanol/Wasser, Ethanol/Wasser, Isopropanol/Wasser, Tetrahydrofuran/Wasser, Dioxan/Wasser, Dimethylformamid/Wasser, Methanol oder Ethanol in Gegenwart einer Alkalibase wie Lithiumhydroxid, Natriumhydroxid oder Kaliumhydroxid bei Temperaturen zwischen 0 und 100°C, vorzugsweise bei Temperaturen zwischen 10 und 50°C,

oder vorteilhafterweise durch Umamidierung mit einer primären oder sekundären organischen Base wie Methylamin, Butylamin, Dimethylamin oder Piperidin in einem Lösungsmittel wie Methanol, Ethanol, Dimethylformamid und deren Gemischen oder in einem Überschuß des eingesetzten Amins bei Temperaturen zwischen 0 und 100°C, vorzugsweise bei Temperaturen zwischen 10 und 50°C, durchgeführt.

Die Abspaltung von einer verwendeten Festphase erfolgt vorzugsweise mittels Trifluoressigsäure und Wasser in Gegenwart von einem Dialkylsulfid wie Dimethylsulfid bei Temperaturen zwischen 0 und 35°C, vorzugsweise bei Raumtemperatur.

b. Zur Herstellung einer Verbindung der allgemeinen Formel I, die eine Aminomethylgruppe enthält und X ein Sauerstoffatom darstellt:

Reduktion einer Verbindung der allgemeinen Formel

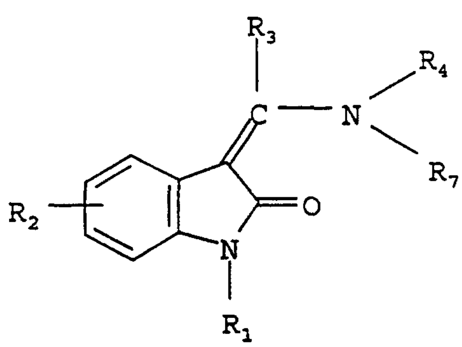

(IV),

in der

$R_1$ bis $R_4$ wie eingangs erwähnt definiert sind und
$R_7$ mit der Maßgabe die für $R_5$ eingangs erwähnten Bedeutungen aufweist, daß $R_5$ eine Cyanogruppe enthält.

Die Reduktion wird vorzugsweise mittels katalytischer Hydrierung mit Wasserstoff in Gegenwart eines Katalysators wie Palladium/Kohle oder Platin in einem Lösungsmittel wie Methanol, Ethanol, Essigsäureethylester, Dimethylformamid, Dimethylformamid/Aceton oder Eisessig gegebenenfalls unter Zusatz einer Säure wie Salzsäure bei Temperaturen zwischen 0 und 50°C, vorzugsweise jedoch bei Raumtemperatur, und bei einem Wasserstoffdruck von 1 bis 7 bar, vorzugsweise jedoch von 3 bis 5 bar.

c. Zur Herstellung einer Verbindung der allgemeinen Formel I, in der $R_1$ ein Wasserstoffatom und X ein Sauerstoffatom darstellen:

Reduktion einer Verbindung der allgemeinen Formel

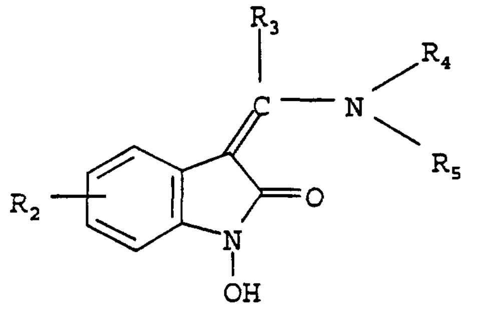

(V),

in der
$R_2$ bis $R_5$ wie eingangs erwähnt definiert sind.

Die Reduktion wird vorzugsweise mittels katalytischer Hydrierung mit Wasserstoff in Gegenwart eines Katalysators wie Palladium/Kohle oder Platin in einem Lösungsmittel wie Methanol, Ethanol, Essigsäureethylester, Dimethylformamid, Dimethylformamid/Aceton oder Eisessig bei Temperaturen zwischen 0 und 50°C, vorzugsweise jedoch bei Raumtemperatur, und bei einem Wasserstoffdruck von 1 bis 7 bar, vorzugsweise jedoch von 3 bis 5 bar, durchgeführt.

Erhält man erfindungsgemäß eine Verbindung der allgemeinen Formel I, die eine Alkoxycarbonylgruppe enthält, so kann diese mittels Hydrolyse in eine entsprechende Carboxyverbindung übergeführt werden, oder
eine Verbindung der allgemeinen Formel I, die eine Amino- oder Alkylaminogruppe enthält, so kann diese mittels Alkylierung oder reduktiver Alkylierung in eine entsprechende Alkylaminooder Dialkylaminoverbindung übergeführt werden, oder
eine Verbindung der allgemeinen Formel I, die eine Amino- oder Alkylaminogruppe enthält, so kann diese mittels Acylierung in eine entsprechende Acylverbindung übergeführt werden, oder
eine Verbindung der allgemeinen Formel I, die eine Carboxygruppe enthält, so kann diese mittels Veresterung oder Amidierung in eine entsprechende Ester- oder Aminocarbonylverbindung übergeführt werden, oder
eine Verbindung der allgemeinen Formel I, in der $R_3$ einen Phenylrest darstellt, der ein Chlor-, Brom- oder Jodatom enthält, so kann diese durch Umsetzung mit einer Alkenylverbindung in eine entsprechende alkenylierte Verbindung übergeführt werden, oder
eine Verbindung der allgemeinen Formel I, in der $R_3$ einen Phenylrest darstellt, der ein Chlor-, Brom- oder Jodatom enthält, so kann diese durch Umsetzung mit einer Alkinylverbindung in eine entsprechende alkenylierte Verbindung übergeführt werden.

Die anschließende Hydrolyse erfolgt vorzugsweise in einem wäßrigen Lösungsmittel, z.B. in Wasser, Isopropanol/Wasser, Tetrahydrofuran/Wasser oder Dioxan/Wasser, in Gegenwart einer Säure wie Trifluoressigsäure, Salzsäure oder Schwefelsäure oder in Gegenwart einer Alkalibase wie Lithiumhydroxid, Natriumhydroxid oder Kaliumhydroxid bei Temperaturen zwischen 0 und 100°C, vorzugsweise bei Temperaturen zwischen 10 und 50°C.

Die anschließende reduktive Alkylierung wird vorzugsweise in einem geeigneten Lösungsmittel wie Methanol, Methanol/Wasser, Methanol/Wasser/Ammoniak, Ethanol, Ether, Tetrahydrofuran, Dioxan oder Dimethylformamid gegebenenfalls unter Zusatz einer Säure wie Salzsäure in Gegenwart von katalytisch angeregtem Wasserstoff, z.B. von Wasserstoff in Gegenwart von Raney-Nickel, Platin oder Palladium/Kohle, oder in Gegenwart eines Metallhydrids wie Natriumborhydrid, Lithiumborhydrid oder Lithiumaluminiumhydrid bei Temperaturen zwischen 0 und 100°C, vorzugsweise bei Temperaturen zwischen 20 und 80°C, durchgeführt.

Die anschließende Alkylierung wird mit eimem Alkylierungsmittel wie eimem Alkylhalogenid oder Dialkylsulfat wie Methyljodid, Dimethylsulfat oder Propylbromid vorzugsweise in einem Lösungsmittel wie Methanol, Ethanol, Methylenchlorid, Tetrahydrofuran, Toluol, Dioxan, Dimethylsulfoxid oder Dimethylformamid gegebenenfalls in Gegenwart

einer anorganischen oder einer tertiären organischen Base wie Triethylamin, N-Ethyl-diisopropylamin oder Dimethylaminopyridin, vorzugsweise bei Temperaturen zwischen 20°C und der Siedetemperatur des verwendeten Lösungsmittel, durchgeführt.

Die anschließende Acylierung wird vorzugsweise in einem Lösungsmittel wie Methylenchlorid, Diethylether, Tetrahydrofuran, Toluol, Dioxan, Acetonitril, Dimethylsulfoxid oder Dimethylformamid gegebenenfalls in Gegenwart einer anorganischen oder einer tertiären organischen Base, vorzugsweise bei Temperaturen zwischen 20°C und der Siedetemperatur des verwendeten Lösungsmittel, durchgeführt. Hierbei wird die Acylierung mit einer entsprechenden Säure vorzugsweise in Gegenwart eines wasserentziehenden Mittels, z.B. in Gegenwart von Chlorameisensäureisobutylester, Orthokohlensäuretetraethylester, Orthoessigsäuretrimethylester, 2,2-Dimethoxypropan, Tetramethoxysilan, Thionylchlorid, Trimethylchlorsilan, Phosphortrichlorid, Phosphorpentoxid, N,N'-Dicyclohexylcarbodiimid, N,N'-Dicyclohexylcarbodiimid/N-Hydroxysuccinimid, N,N'-Dicyclohexylcarbodiimid/1-Hydroxy-benztriazol, 2-(1H-Benzotriazol-1-yl)-1,1,3,3-tetramethyluronium-tetrafluorborat, 2-(1H-Benzotriazol-1-yl)-1,1,3,3-tetramethyluronium-tetrafluorborat/1-Hydroxy-benztriazol, N,N'-Carbonyldiimidazol oder Triphenylphosphin/Tetrachlorkohlenstoff, und gegebenenfalls unter Zusatz einer Base wie Pyridin, 4-Dimethylamino-pyridin, N-Methyl-morpholin oder Triethylamin zweckmäßigerweise bei Temperaturen zwischen 0 und 150°C, vorzugsweise bei Temperaturen zwischen 0 und 100°C, und die Acylierung mit einer entsprechenden reaktionsfähigen Verbindung wie deren Anhydrid, Ester, Imidazolide oder Halogenide gegebenenfalls in Gegenwart einer tertiären organischen Base wie Triethylamin, N-Ethyl-diisopropylamin oder N-Methyl-morpholin bei Temperaturen zwischen 0 und 150°C, vorzugsweise bei Temperaturen zwischen 50 und 100°C, durchgeführt.

Die anschließende Veresterung oder Amidierung wird zweckmäßigerweise durch Umsetzung eines reaktionsfähigen entsprechenden Carbonsäurederivates mit einem entsprechenden Alkohol oder Amin wie vorstehend beschrieben durchgeführt.

Die anschließende Alkenylierung wird vorzugsweise in einem Lösungsmittel wie Dimethylformamid, Dimethylacetamid oder Acetonitril in Gegenwart eines Palladiumkatalysators wie Bis-(triphenylphosphin)-palladium-dichlorid und vorzugsweise in Gegenwart einer geeigneten Base wie beispielsweise Triethylamin, Tributylamin, N-Ethyldiisopropylamin oder Natriumacetat bei Temperaturen zwischen 20 und 120°C durchgeführt (siehe R.F. Heck, Org. Reactions 27, 345-390 (1982).

Die anschließende Alkinylierung wird vorzugsweise in einem Lösungsmittel wie Benzol, Toluol, Dimethylformamid oder Chloroform in Gegenwart eines Palladiumkatalysators wie Tetrakistriphenylphosphin-palladium und von Kupfer-(I)-jodid vorzugsweise in Gegenwart einer geeigneten Base wie Triethylamin bei Temperaturen zwischen 20 und 100°C durchgeführt (siehe auch N.A. Bumagin et al. Synthesis 1984, 728-729; K. Sonogashira et al. Tetrahedron Lett. 1975, 4467).

Die Herstellung alkenylsubstituierter Arylamine erfolgt unter den Bedingungen einer Palladium-katalysierten Kupplung. Dazu werden Arylhalogenid und Alkenylverbindung mit einer katalytischen Menge eines Palladium-Katalysator wie Bis-(triphenylphosphin)-palladium-dichlorid in einem Lösungsmittel wie DMF, Dimethylacetamid oder Acetonitril in Gegenwart einer inerten Base wie beispielsweise Triethylamin, Tributylamin, N-Ethyl-diisopropylamin oder Natriumacetat bei Temperaturen zwischen 20 und 120°C umgesetzt.

Bei den vorstehend beschriebenen Umsetzungen können gegebenenfalls vorhandene reaktive Gruppen wie Carboxy-, Amino-, Alkylamino- oder Iminogruppen während der Umsetzung durch übliche Schutzgruppen geschützt werden, welche nach der Umsetzung wieder abgespalten werden.

Beispielsweise kommt als Schutzrest für eine Carboxylgruppe die Trimethylsilyl-, Methyl-, Ethyl-, tert.Butyl-, Benzyl- oder Tetrahydropyranylgruppe und
als Schutzrest für eine Amino-, Alkylamino- oder Iminogruppe die Acetyl-, Trifluoracetyl-, Benzoyl-, Ethoxycarbonyl-, tert.Butoxycarbonyl-, Benzyloxycarbonyl-, Benzyl-, Methoxybenzyl- oder 2,4-Dimethoxybenzyl-gruppe und für die Aminogruppe zusätzlich die Phthalylgruppe in Betracht.

Die gegebenenfalls anschließende Abspaltung eines verwendeten Schutzrestes erfolgt beispielsweise hydrolytisch in einem wäßrigen Lösungsmittel, z.B. in Wasser, Isopropanol/Wasser, Tetrahydrofuran/Wasser oder Dioxan/Wasser, in Gegenwart einer Säure wie Trifluoressigsäure, Salzsäure oder Schwefelsäure oder in Gegenwart einer Alkalibase wie Lithiumhydroxid, Natriumhydroxid oder Kaliumhydroxid bei Temperaturen zwischen 0 und 100°C, vorzugsweise bei Temperaturen zwischen 10 und 50°C.

Die Abspaltung eines Benzyl-, Methoxybenzyl- oder Benzyloxycarbonylrestes erfolgt jedoch beispielsweise hydrogenolytisch, z.B. mit Wasserstoff in Gegenwart eines Katalysators wie Palladium/Kohle in einem Lösungsmittel wie Methanol, Ethanol, Essigsäureethylester, Dimethylformamid, Dimethylformamid/Aceton oder Eisessig gegebenenfalls unter Zusatz einer Säure wie Salzsäure oder Eisessig bei Temperaturen zwischen 0 und 50°C, vorzugsweise jedoch bei Raumtemperatur, und bei einem Wasserstoffdruck von 1 bis 7 bar, vorzugsweise jedoch von 3 bis 5 bar.

Die Abspaltung einer Methoxybenzylgruppe kann auch in Gegenwart eines Oxidationsmittels wie Cer(IV)ammoniumnitrat in einem Lösungsmittel wie Methylenchlorid, Acetonitril oder Acetonitril/Wasser bei Temperaturen zwischen 0 und 50°C, vorzugsweise jedoch bei Raumtemperatur, erfolgen.

Die Abspaltung eines 2,4-Dimethoxybenzylrestes erfolgt jedoch vorzugsweise in Trifluoressigsäure in Gegenwart von Anisol.

Die Abspaltung eines tert.Butyl- oder tert.Butyloxycarbonylrestes erfolgt vorzugsweise durch Behandlung mit einer Säure wie Trifluoressigsäure oder Salzsäure gegebenenfalls unter Verwendung eines Lösungsmittels wie Methylenchlorid, Dioxan, Essigester oder Ether.

Die Abspaltung eines Phthalylrestes erfolgt vorzugsweise in Gegenwart von Hydrazin oder eines primären Amins wie Methylamin, Ethylamin oder n-Butylamin in einem Lösungsmittel wie Methanol, Ethanol, Isopropanol, Toluol/Wasser oder Dioxan bei Temperaturen zwischen 20 und 50°C.

Ferner können erhaltene chirale Verbindungen der allgemeinen Formel I in ihre Enantiomeren und/oder Diastereomeren aufgetrennt werden.

So lassen sich beispielsweise die erhaltenen Verbindungen der allgemeinen Formel I, welche in Racematen auftreten, nach an sich bekannten Methoden (siehe Allinger N. L. und Eliel E. L. in "Topics in Stereochemistry", Vol. 6, Wiley Interscience, 1971) in ihre optischen Antipoden und Verbindungen der allgemeinen Formel I mit mindestes 2 asymmetrischen Kohlenstoffatomen auf Grund ihrer physikalisch-chemischen Unterschiede nach an sich bekannten Methoden, z.B. durch Chromatographie und/oder fraktionierte Kristallisation, in ihre Diastereomeren auftrennen, die, falls sie in racemischer Form anfallen, anschließend wie oben erwähnt in die Enantiomeren getrennt werden können.

Die Enantiomerentrennung erfolgt vorzugsweise durch Säulentrennung an chiralen Phasen oder durch Umkristallisieren aus einem optisch aktiven Lösungsmittel oder durch Umsetzen mit einer, mit der racemischen Verbindung Salze oder Derivate wie z.B. Ester oder Amide bildenden optisch aktiven Substanz, insbesondere Säuren und ihre aktivierten Derivate oder Alkohole, und Trennen des auf diese Weise erhaltenen Gemisches diastereomerer Salze oder Derivate, z.B. auf Grund von verschiedenen Löslichkeiten, wobei aus den reinen diastereomeren Salzen oder Derivaten die freien Antipoden durch Einwirkung geeigneter Mittel freigesetzt werden können. Besonders gebräuchliche, optisch aktive Säuren sind z.B. die D- und L-Formen von Weinsäure, Dibenzoylweinsäure, Di-o-Tolylweinsäure, Apfelsäure, Mandelsäure, Camphersulfonsäure, Glutaminsäure, N-Acetylglutaminsäure, Asparaginsäure, N-Acetylasparaginsäure oder Chinasäure. Als optisch aktiver Alkohol kommt beispielsweise (+)- oder (-)-Menthol und als optisch aktiver Acylrest in Amiden beispielsweise der (+)- oder (-)-Menthyloxycarbonylrest in Betracht.

Desweiteren können die erhaltenen Verbindungen der Formel I in ihre Salze, insbesondere für die pharmazeutische Anwendung in ihre physiologisch verträglichen Salze mit anorganischen oder organischen Säuren, übergeführt werden. Als Säuren kommen hierfür beispielsweise Salzsäure, Bromwasserstoffsäure, Schwefelsäure, Phosphorsäure, Fumarsäure, Bernsteinsäure, Milchsäure, Zitronensäure, Weinsäure, Maleinsäure oder Methansulfonsäure in Betracht.

Außerdem lassen sich die so erhaltenen neuen Verbindungen der Formel I, falls diese eine Carboxygruppe enthalten, gewünschtenfalls anschließend in ihre Salze mit anorganischen oder organischen Basen, insbesondere für die pharmazeutische Anwendung in ihre physiologisch verträglichen Salze, überführen. Als Basen kommen hierbei beispielsweise Natriumhydroxid, Kaliumhydroxid, Cyclohexylamin, Ethanolamin, Diethanolamin und Triethanolamin in Betracht.

Die als Ausgangsprodukte verwendeten Verbindungen der allgemeinen Formeln I bis VIII sind teilweise literaturbekannt oder man erhält diese nach literaturbekannten Verfahren oder werden in den Beispielen beschrieben.

Wie bereits eingangs erwähnt, weisen die neuen Verbindungen der allgemeinen Formel I, in der $R_1$ ein Wasserstoffatom oder einen Prodrugrest darstellt, wertvolle pharmakologische Eigenschaften auf, insbesondere eine inhibierende Wirkung auf verschiedene Kinasen, vor allem auf Komplexe von CDK's (CDK1, CDK2, CDK3, CDK4, CDK6, CDK7, CDK8 und CDK9) mit ihren spezifischen Cyclinen (A, B1, B2, C, D1, D2, D3, E, F, G1, G2, H, I und K), auf virales Cyclin (siehe L. Mengtao in J. Virology 71(3), 1984-1991 (1997))und auf Rezeptor-Tyrosinkinasen wie HER2, EGFR, FGFR, IGF-1R und KDR, auf die Proliferation kultivierter humaner Tumor-Zellen sowie nach oraler Gabe auf das Wachstum von Tumoren in Nacktmäusen, die mit humanen Tumorzellen infiziert worden waren.

Beispielsweise wurden die in Tabelle 1 aufgeführten Verbindungen auf ihre biologischen Eigenschaften wie folgt geprüft:

<u>Test 1</u>

<u>Inhibierung von Cyclin/CDK-Enzym, -Aktivität in vitro</u>

High Five™ Insekten-Zellen (BTI-TN-5B1-4), die mit einem hohen Titer an rekombinantem Baculovirus infiziert waren, wurden für die Produktion von aktiven humanen Cyclin/CDK Holoenzymen benutzt. Durch die Verwendung eines Baculovirus-Vektors, der zwei Promoter enthielt (polyhedrin enhancer promoter, P10-enhancer promoter), wurden GST-tagged Cycline (z.B. Cyclin D1 oder Cyclin D3) mit der entsprechenden $His_6$-tagged CDK-Untereinheit (z.B. für CDK4 oder CDK6) in derselben Zelle exprimiert. Das aktive Holoenzym wurde durch Affinitäts-Chromatographie an Glutathion-Sepharose isoliert. Rekombinantes GST-tagged pRB (aa 379-928) wurde in E. coli produziert und durch

Affinitäts-Chromatographie an Glutathion-Sepharose gereinigt.

Die Substrate, die für die Kinase-Assays verwendet wurden, hingen von den spezifischen Kinasen ab. Histone H1 (Sigma) wurde verwendet als Substrat für Cyclin E/CDK2, Cyclin A/CDK2, Cyclin B/CDK1 und für v-Cyclin/CDK6. GST-tagged pRB (aa 379-928) wurde verwendet als Substrat für Cyclin D1/CDK4, Cyclin D3/CDK4, Cyclin D1/CDK6 und für Cyclin D3/CDK6.

Lysate der mit rekombinanten Baculovirus-infizierten Insekten-Zellen oder auch rekombinante Kinasen (erhalten aus den Lysaten durch Reinigung) wurden zusammen mit radioaktiv markiertem ATP in Gegenwart eines geeigneten Substrates mit verschiedenen Konzentrationen des Inhibitors in einer 1%igen DMSO-Lösung (Dimethylsulfoxid) 45 Minuten lang bei 30°C inkubiert. Die Substrat Proteine mit assoziierter Radioaktivität wurden mit 5%iger TCA (Trichloressigsäure) in hydrophoben PVDF multi-well Mikrotiter Platten (Millipore) oder mit 0.5%iger Phosphorsäure-Lösung auf Whatman P81 Filtern ausgefällt. Nach Zugabe von Scintillations-Flüssigkeit wurde die Radioaktivität in einem Wallace 1450 Microbeta Flüssig-Scintillations-Zähler gemessen. Pro Konzentration der Substanz wurden Doppel-Messungen durchgeführt; $IC_{50}$-Werte für die Enzym-Inhibition wurden berechnet.

Test 2

Inhibierung der Profileration von kultivierten humanen Tumorzellen

Zellen der Leiomyosarcoma Tumorzell-Linie SK-UT-1B (erhalten von der American Type Culture Collection (ATCC)) wurden in Minimum Essential Medium mit nicht-essentiellen Aminosäuren (Gibco), ergänzt mit Natrium-Pyruvat (1 mMol), Glutamin (2 mMol) und 10% fötalem Rinderserum (Gibco) kultiviert und in der log-Wachstumsphase geerntet. Anschließend wurden die SK-UT-1B-Zellen in Cytostar® multi-well Platten (Amersham) mit einer Dichte von 4000 cells per well eingebracht und über Nacht in einem Inkubator inkubiert. Verschiedene Konzentrationen der Verbindungen (gelöst in DMSO; Endkonzentration: <1%) wurden zu den Zellen zugegeben. Nach 48 Stunden Inkubation wurde $^{14}C$-Thymidin (Amersham) zu jedem well zugesetzt, und es wurde weitere 24 Stunden inkubiert. Die Menge an $^{14}C$-Thymidin, die in Gegenwart des Inhibitors in die Tumorzellen eingebaut wurde und die die Zahl der Zellen in der S-Phase repräsentiert, wurde in einem Wallace 1450 Microbeta Flüssig Scintillations Zähler gemessen. $IC_{50}$-Werte für die Inhibierung der Proliferation (= Inhibierung von eingebautem $^{14}C$-Thymidin) wurden - unter Korrektur für die Hintergrundstrahlung - berechnet. Alle Messungen wurden zweifach ausgeführt.

Test 3

In vivo Effekte an Tumor-tragenden Nacktmäusen

$10^6$ Zellen [SK-UT-1B, oder non-small cell Lungen-Tumor NCI-H460 (erhalten von ATCC)] in einem Volumen von 0.1 ml wurden in männliche und/oder weibliche Nacktmäuse (NMRI nu/nu; 25 bis 35 g; N = 10-20) subkutan injiziert; alternativ wurden kleine Stückchen von SK-UT-1B- oder NCI-H460-Zellklumpen subkutan implantiert. Eine bis drei Wochen nach Injektion bzw. Implantation wurde ein Kinase-Inhibitor täglich für die Dauer von 2 bis 4 Wochen oral (per Schlundsonde) appliziert. Die Tumor-Größe wurde dreimal pro Woche mit einer digitalen Schieblehre gemessen. Der Effekt eines Kinase-Hemmers auf das Tumor-Wachstum wurde als Prozentinhibierung im Vergleich zu einer mit Placebo behandelten Kontroll-Gruppe bestimmt.

Die nachfolgende Tabelle enthält die gefundenen Ergebnisse des in vitro-Tests 2:

| Verbindung (Beispiel Nr.) | Hemmung der SKUT-1B-Proliferation $IC_{50}$ [µM] |
|---|---|
| 117 | 0.34 |
| 170 | 0.22 |
| 133 | 0.48 |
| 134 | 0.56 |
| 188 | 0.15 |

Auf Grund ihrer biologischen Eigenschaften eignen sich die neuen Verbindungen der allgemeinen Formel I, deren Isomere und deren physiologisch verträgliche Salze zur Behandlung von Erkrankungen, die durch exzessive oder anomale Zellproliferation charakterisiert sind.

Zu solchen Erkrankungen gehören (ohne Anspruch auf Vollständigkeit): Virale Infektionen (z.B. HIV und Kaposi Sarkoma); Entzündung und Autoimmun-Erkrankungen (z.B. Colitis, Arthritis, Alzheimer Erkrankung, Glomerulonephritis und Wund-Heilung); bakterielle, fungale und/oder parasitäre Infektionen; Leukämien, Lymphoma, und solide Tumore; Haut-Erkrankungen (z.B. Psoriasis); Knochen-Erkrankungen; kardiovaskuläre Erkrankungen (z.B. Restenose und Hypertrophie). Ferner sind sie nützlich als Schutz von proliferierenden Zellen (z.B. Haar-, Intestinal-, Blut- und Progenitor-Zellen) gegen DNA-Schädigung durch Strahlung, UV-Behandlung und/oder zytostatischer Behandlung.

Die neuen Verbindungen können zur Kurz- oder Langzeitbehandlung der vorstehend erwähnten Krankheiten auch gegebenenfalls in Kombination mit anderen "State-of-art" Verbindungen wie anderen Cytostatika verwendet werden.

Die zur Erzielung einer entsprechenden Wirkung erforderliche Dosierung beträgt zweckmäßigerweise bei intravenöser Gabe 0,1 bis 30 mg/kg, vorzugsweise 0,3 bis 10 mg/kg, und bei oraler Gabe 0,1 bis 100 mg/kg, vorzugsweise 0,3 bis 30 mg/kg, jeweils 1 bis 4 x täglich. Hierzu lassen sich die erfindungsgemäß hergestellten Verbindungen der Formel I, gegebenenfalls in Kombination mit anderen Wirksubstanzen, zusammen mit einem oder mehreren inerten üblichen Trägerstoffen und/oder Verdünnungsmitteln, z.B. mit Maisstärke, Milchzucker, Rohrzucker, mikrokristalliner Zellulose, Magnesiumstearat, Polyvinylpyrrolidon, Zitronensäure, Weinsäure, Wasser, Wasser/Ethanol, Wasser/Glycerin, Wasser/Sorbit, Wasser/Polyethylenglykol, Propylenglykol, Cetylstearylalkohol, Carboxymethylcellulose oder fetthaltigen Substanzen wie Hartfett oder deren geeigneten Gemischen, in übliche galenische Zubereitungen wie Tabletten, Dragées, Kapseln, Pulver, Suspensionen, Zäpfchen oder als Lösungen für Injektionen oder Infusionen einarbeiten.

Die nachfolgenden Beispiele sollen die Erfindung näher erläutern.

Verwendete Abkürzungen:

CDI = N,N'-Carbonyldiimidazol
DMF = Dimethylformamid
HOBt = 1-Hydroxy-1H-benzotriazol
TBTU = O-(Benzotriazol-1-yl)-N,N,N',N'-bis(tetramethylen)-uroniumhexafluorophosphat
THF = Tetrahydrofuran

Beispiel 1

(Z)-3-(1-Anilino-1-phenyl-methyliden)-2-indolinon

a) 1-Acetyl-2-indolinon

13.3 g (0.1 Mol) 2-Indolinon und 30 ml Acetanhydrid werden 3 Stunden bei 170°C gerührt. Nach dem Abkühlen wird mit 150 ml Eiswasser versetzt, das kristalline Produkt abgesaugt, mit Wasser gewaschen und getrocknet.
Ausbeute: 16.6 g (95 % der Theorie),
Schmelzpunkt: 129-130°C

b) 1-Acetyl-3-(1-ethoxy-1-phenyl-methyliden)-2-indolinon

35.0 g (0.2 Mol) 1-Acetyl-2-indolinon werden in 300 ml Acetanhydrid gelöst und nach Zugabe von 135 g (0.6 Mol) Orthobenzoesäuretriethylester 22 Stunden zum Rückfluß erhitzt. Das Lösungsmittel wird abdestilliert und der Rückstand mit Petrolether verdünnt. Nach 18 Stunden stehen bei Raumtemperatur wird der kristalline Niederschlag abgesaugt, gewaschen und getrocknet.
Ausbeute: 41.2 g (67 % der Theorie).

c) (Z)-3-(1-Anilino-1-phenyl-methyliden)-2-indolinon

450 mg (1.5 mMol) 1-Acetyl-3-(1-ethoxy-1-phenyl-methyliden)-2-indolinon und 0.41 ml (4.5 mMol) Anilin werden in 7 ml DMF 90 Minuten bei 120°C gerührt. Nach Abkühlen auf Raumtemperatur werden 7 ml Methanol und 3 ml 1N Natronlauge zugesetzt. Man rührt 20 Minuten, verdünnt anschließend mit Wasser, saugt das kristalline Reaktionsprodukt ab und trocknet.
Ausbeute: 49 % der Theorie,
Schmelzpunkt: 325°C
$C_{21}H_{16}N_2O$ (312.37)
Massenspektrum: $M^+$ = 312

Beispiel 2

(Z)-3-[1-(4-Methoxy-phenylamino)-1-phenyl-methyliden]-2-indolinon

a) 1-Acetyl-3-(1-hydroxy-1-phenyl-methyliden)-2-indolinon

880 mg (5 mMol) 1-Acetyl-2-indolinon und 610 mg (5 mMol) Benzoesäure werden in 15 ml DMF gelöst und nach Zugabe von 1.8 g (5.5 mMol) TBTU, 840 mg (5.5 mMol) HOBt und 3.2 g (25 mMol) N-Ethyl-N,N-diisopropyl-amin 16 Stunden bei Raumtemperatur gerührt. Die Lösung wird in verdünnte Salzsäure eingerührt, der Niederschlag abgesaugt und bei 60°C getrocknet.
Ausbeute: 1.1 g (80 % der Theorie),
Schmelzpunkt: 126-129°C

b) 1-Acetyl-3-(1-chlor-1-phenyl-methyliden)-2-indolinon

5.6 g (20 mMol) 1-Acetyl-3-(1-hydroxy-1-phenyl-methyliden)-2-indolinon werden in 45 ml Toluol suspendiert und unter Eiskühlung mit 4.2 g (20 mMol) Phosphorpentachlorid versetzt und anschließend 18 Stunden bei Raumtemperatur gerührt. Der nach Eiskühlung ausgefallene Niederschlag wird abgesaugt und getrocknet.
Ausbeute. 5.3 g (89 % der Theorie).

c) (Z)-3-[1-(4-Methoxy-phenylamino)-1-phenyl-methyliden]-2-indolinon

0.18 g (1.5 mMol) 4-Methoxyanilin und 0.2 g (0.28 mMol) Triethylamin werden in 5 ml Dichlormethan gelöst und bei 5°C mit einer Lösung von 0.45 g (1.5 mMol) 1-Acetyl-3-(1-chlor-1-phenyl-methyliden)-2-indolinon in 10 ml Dichlormethan versetzt und anschließend 3 Stunden bei Raumtemperatur gerührt. Nach Entfernen des Lösungsmittels im Vakuum wird der Rückstand in Essigester/Wasser aufgenommen. Die organische Phase wird mit Wasser gewaschen, getrocknet und vom Lösungsmittel im Vakuum befreit. Danach wird in 15 ml Methanol gelöst, mit 3 ml 1N Natronlauge versetzt,3 Stunden bei Raumtemperatur gerührt und mit Wasser und Essigester verdünnt. Die organische Phase wird getrocknet und eingedampft. Der Rückstand wird in Essigester erhitzt, nach dem Abkühlen abgesaugt und getrocknet.
Ausbeute: 100 mg (20 % der Theorie),
Schmelzpunkt: 267-270°C
$C_{22}H_{18}N_2O_2$ (342.40)
Massenspektrum: $M^+ = 342$

| | | | |
|---|---|---|---|
| Ber. | C 77.17 | H 5.30 | N 8.18 |
| Gef. | 76.43 | 5.39 | 8.06 |

Beispiel 3

(Z)-3-[1-(3-Methoxy-phenylamino)-1-phenyl-methyliden]-2-indolinon

Hergestellt analog Beispiel 2 aus 1-Acetyl-3-(1-chlor-1-phenyl-methyliden)-2-indolinon und 3-Methoxyanilin in THF und anschließender Behandlung mit Natronlauge.
Ausbeute: 69 % der Theorie,
Schmelzpunkt: 218-221°C
$C_{22}H_{18}N_2O_2$ (342.40)
Massenspektrum: $M^+ = 342$

| | | | |
|---|---|---|---|
| Ber. | C 77.17 | H 5.30 | N 8.18 |
| Gef. | 76.74 | 5.30 | 7.74 |

Beispiel 4

(Z)-3-[1-(2-Methoxy-phenylamino)-1-phenyl-methyliden]-2-indolinon

Hergestellt analog Beispiel 1 aus 1-Acetyl-3-(1-ethoxy-1-phenyl-methyliden)-2-indolinon und 2-Methoxyanilin

in DMF und anschließender Behandlung mit Natronlauge in Methanol.
Ausbeute: 44 % der Theorie,
Schmelzpunkt: 237°C
$C_{22}H_{18}N_2O_2$ (342.40)
Massenspektrum: $M^+$ = 342
$R_f$-Wert: 0.47 (Kieselgel; Petrolether/Essigester = 4:6) $C_{22}H_{18}N_2O_2$ x $H_2O$ (360.42)

| | | | |
|---|---|---|---|
| Ber. | C 73.32 | H 5.59 | N 7.77 |
| Gef. | 73.51 | 5.61 | 7.66 |

Beispiel 5

(Z)-3-[1-(3-Methoxymethyl-phenylamino)-1-phenyl-methyliden]-2-indolinon

Hergestellt analog Beispiel 2 aus 1-Acetyl-3-(1-chlor-1-phenyl-methyliden)-2-indolinon und 3-Methoxymethyl-anilin-hydrochlorid in THF und anschließender Behandlung mit Natronlauge in Methanol.
Ausbeute: 28 % der Theorie,
Schmelzpunkt: 182-184°C
$C_{23}H_{20}N_2O_2$ (356.43)
Massenspektrum: $M^+$ = 356

| | | | |
|---|---|---|---|
| Ber. | C 77.51 | H 5.66 | N 7.86 |
| Gef. | 77.12 | 5.91 | 7.74 |

Beispiel 6

(Z)-3-[1-(3-Methyl-phenylamino)-1-phenyl-methyliden]-2-indolinon

Hergestellt analog Beispiel 2 aus 1-Acetyl-3-(1-chlor-1-phenyl-methyliden)-2-indolinon und m-Toluidin in Dichlormethan und anschließender Behandlung mit Natronlauge in Methanol.
Ausbeute: 3 % der Theorie,
Schmelzpunkt: 218-220°C
$C_{22}H_{18}N_2O$ (326.40)
Massenspektrum: $M^+$ = 326

Beispiel 7

(Z)-3-[1-(2-Methoxycarbonyl-phenylamino)-1-phenyl-methyliden]-2-indolinon

Hergestellt analog Beispiel 1 aus 1-Acetyl-3-(1-ethoxy-1-phenyl-methyliden)-2-indolinon und Anthranilsäuremethylester in DMF und anschließender kurzer Behandlung mit Natronlauge in Methanol.
Ausbeute: 12 % der Theorie,
Schmelzpunkt: 241-244°C
$C_{23}H_{18}N_2O_3$ (370.41)
Massenspektrum: $M^+$ = 370

| | | | |
|---|---|---|---|
| Ber. | C 74.58 | H 4.90 | N 7.56 |
| Gef. | 73.87 | 4.85 | 7.44 |

Beispiel 8

(Z)-3-[1-(2-Carboxy-phenylamino)-1-phenyl-methyliden]-2-indolinon

176 mg (0.48 mMol) (Z)-3-[1-(2-Methoxycarbonyl-phenylamino)-1-phenyl-methyliden]-2-indolinon werden in 15 ml Methanol und 2 ml Dioxan gelöst und nach Zugabe von 1.4 ml 1N Natronlauge zwei Stunden bei 80°C gerührt. Anschließend wird unter Kühlung mit 1.4 ml 1N Salzsäure neutralisiert, das ausgefallene Produkt abgesaugt, mit Wasser gewaschen und getrocknet.
Ausbeute: 100 mg (59 % der Theorie),
Schmelzpunkt: 227-230°C
$C_{22}H_{16}N_2O_3$ (356.38)
Massenspektrum: $M^+$ = 356
$R_f$-Wert: 0.30 (Kieselgel; Dichlormethan/Methanol/Eisessig = 19:1:0.1)

Beispiel 9

(Z)-3-[1-(3-Carboxy-phenylamino)-1-phenyl-methyliden]-2-indolinon

a) 1-Benzoyl-3-(1-hydroxy-1-phenyl-methyliden)-2-indolinon

26.6 g (0.2 Mol) 2-Indolinon und 53.8 g (0.44 Mol) 4-Dimethylamino-pyridin werden in 400 ml DMF gelöst und nach Zugabe von 30.9 g (0.22 Mol) Benzoylchlorid in 100 ml DMF 45 Minuten bei 45°C gerührt. Die Lösung wird auf 3 l Wasser und 100 ml konz. Salzsäure gegossen, der ausgefallene Niederschlag abgesaugt, aus Eisessig umkristallisiert und getrocknet.
Ausbeute: 11.8 g (17 % der Theorie),
Schmelzpunkt: 185-187°C

b) 1-Benzoyl-3-(1-chlor-1-phenyl-methyliden)-2-indolinon

Hergestellt analog Beispiel 2b aus 1-Benzoyl-3-(1-hydroxy-1-phenyl-methyliden)-2-indolinon und Phosphorpentachlorid in Toluol.
Ausbeute: 99 % der Theorie,
Schmelzpunkt: 170-176°C

c) (Z)-3-[1-(3-Carboxy-phenylamino)-1-phenyl-methyliden]-2-indolinon

Hergestellt analog Beispiel 2c aus 1-Benzoyl-3-(1-chlor-1-phenyl-methyliden)-2-indolinon und 3-Aminobenzoesäureethylester und anschließende vollständige Verseifung mit Natronlauge in Methanol.
Ausbeute: 60 % der Theorie,
$C_{22}H_{16}N_2O_3$ (356.38)
Massenspektrum: $M^+$ = 356
$R_f$-Wert: 0.33 (Kieselgel; Petrolether/Essigester = 3:2)

Beispiel 10

(Z)-3-{1-[3-(Aminocarbonyl)phenylamino]-1-phenyl-methyliden}-2-indolinon

Hergestellt analog Beispiel 9 aus 1-Benzoyl-3-(1-chlor-1-phenyl-methyliden)-2-indolinon und 3-Aminobenzoesäureamid in THF und anschließender Behandlung mit Natronlauge in Methanol.
Ausbeute: 76 % der Theorie,
Schmelzpunkt: 258-263°C
$C_{22}H_{17}N_3O_2$ (355.40)
Massenspektrum: $M^+$ = 355

Beispiel 11

(Z)-3-[1-(3-Ethoxycarbonylmethyl-phenylamino)-1-phenyl-methyliden]-2-indolinon

a) 3-(1-Ethoxy-1-phenyl-methyliden)-2-indolinon

6.15 g (20 mMol) 1-Acetyl-3-(1-ethoxy-1-phenyl-methyliden)-2-indolinon werden in wenig Ethanol suspendiert. Man gibt 10 ml 4N Natronlauge zu und rührt 1.5 Stunden bei Raumtemperatur. Nach Zugabe von 100 ml Wasser wird der Niederschlag abgesaugt, mit Wasser und wenig Ether gewaschen und bei 80°C getrocknet.
Ausbeute 2.8 g (56% der Theorie),
Schmelzpunkt: 168-169°C

b) (Z)-3-[1-(3-Ethoxycarbonylmethyl-phenylamino)-1-phenyl-methyliden]-2-indolinon

Hergestellt analog Beispiel 1c aus 3-(1-Ethoxy-1-phenyl-methyliden)-2-indolinon und 3-Aminophenylessigsäureethylester in DMF.
Ausbeute: 71 % der Theorie,
Schmelzpunkt: 178-181°C
$C_{25}H_{22}N_2O_3$ (398.47)
Massenspektrum: $M^+$ = 398
$R_f$-Wert: 0.52 (Kieselgel; Dichlormethan/Methanol = 24:1)

| | | | |
|---|---|---|---|
| Ber. | C 75.36 | H 5.56 | N 7.03 |
| Gef. | 75.23 | 5.69 | 6.95 |

Beispiel 12

(Z)-3-[1-(3-Carboxymethyl-phenylamino)-1-phenyl-methyliden]-2-indolinon

Hergestellt analog Beispiel 8 durch Verseifung von (Z)-3-[1-(3-Ethoxycarbonylmethyl-phenylamino)-1-phenylmethyliden]-2-indolinon in Natronlauge.
Ausbeute: 90 % der Theorie,
Schmelzpunkt: 268-270°C
$C_{23}H_{18}N_2O_3$ (370.41)
Massenspektrum: $M^+$ = 370
$R_f$-Wert: 0.21 (Kieselgel; Dichlormethan/Methanol = 19:1)

| | | | |
|---|---|---|---|
| Ber. | C 74.58 | H 4.90 | N 7.56 |
| Gef. | 74.54 | 4.94 | 7.59 |

Beispiel 13

(Z)-3-[1-(4-Ethoxycarbonyl-phenylamino)-1-phenyl-methyliden]-2-indolinon

Hergestellt analog Beispiel 2 aus 1-Acetyl-3-(1-chlor-1-phenyl-methyliden)-2-indolinon und 4-Aminobenzoesäureethylester in Dichlormethan und anschließender Behandlung mit Natronlauge in Methanol.
Ausbeute: 19 % der Theorie,
Schmelzpunkt: 227-228°C
$C_{24}H_{20}N_2O_3$ (384.44)
Massenspektrum: $M^+$ = 384

| | | | |
|---|---|---|---|
| Ber. | C 74.98 | H 5.24 | N 7.29 |
| Gef. | 74.37 | 5.08 | 7.02 |

Beispiel 14

(Z)-3-[1-(3-Ethoxycarbonyl-phenylamino)-1-phenyl-methyliden]-2-indolinon

Hergestellt analog Beispiel 9c und 8 aus 1-Benzoyl-3-(1-chlor-1-phenyl-methyliden)-2-indolinon und 3-Aminobenzoesäureethylester in THF und anschließender Behandlung mit Natronlauge in Methanol.
Ausbeute: 45 % der Theorie,
Schmelzpunkt: 194-195°C
$C_{24}H_{20}N_2O_3$ (384.44)
Massenspektrum: $M^+$ = 384

| | | | |
|---|---|---|---|
| Ber. | C 74.98 | H 5.24 | N 7.29 |
| Gef. | 74.01 | 5.28 | 6.96 |

Beispiel 15

(Z)-3-[1-(4-Ethoxycarbonylmethyl-phenylamino)-1-phenyl-methyliden]-2-indolinon

Hergestellt analog Beispiel 1 aus 1-Acetyl-3-(1-ethoxy-1-phenyl-methyliden)-2-indolinon und 4-Aminophenylessigsäureethylester in DMF und anschließender Behandlung mit Piperidin.
Ausbeute: 64 % der Theorie,
Schmelzpunkt: 167-168°C
$C_{25}H_{22}N_2O_3$ (398.47)
Massenspektrum: $M^+ = 398$

| | | | |
|---|---|---|---|
| Ber. | C 75.36 | H 5.56 | N 7.03 |
| Gef. | 75.41 | 5.63 | 7.10 |

Beispiel 16

(Z)-3-[1-(4-Carboxymethyl-phenylamino)-1-phenyl-methyliden]-2-indolinon

Hergestellt analog Beispiel 8 aus (Z)-3-[1-(4-Ethoxycarbonylmethyl-phenylamino)-1-phenyl-methyliden]-2-indolinon und Natronlauge in Ethanol.
Ausbeute: 81 % der Theorie,
Schmelzpunkt: 214-216°C
$C_{23}H_{18}N_2O_3$ (370.41)
Massenspektrum: $M^+ = 370$

| | | | |
|---|---|---|---|
| Ber. | C 74.58 | H 4.90 | N 7.56 |
| Gef. | 74.82 | 4.78 | 7.74 |

Beispiel 17

(Z)-3-[1-(4-Carboxy-phenylamino)-1-phenyl-methyliden]-2-indolinon

Hergestellt analog Beispiel 8 aus (Z)-3-[1-(4-Ethoxycarbonylphenylamino]-1-phenyl-methyliden]-2-indolinon und Natronlauge in Ethanol.
Ausbeute: 96 % der Theorie,
Schmelzpunkt: 312-316°C
$C_{22}H_{16}N_2O_3$ (356.38)
Massenspektrum: $M^+ = 356$

| | | | |
|---|---|---|---|
| Ber. | C 74.15 | H 4.53 | N 7.86 |
| Gef. | 73.23 | 4.48 | 7.61 |

Beispiel 18

(Z)-3-[1-(4-Dimethylaminocarbonyl-phenylamino)-1-phenyl-methyliden]-2-indolinon

285 mg (0.8 mMol) (Z)-3-[1-(4-Carboxyphenylamino)-1-phenylmethyliden]-2-indolinon und 330 mg (4 mMol) Dimethylamin-hydrochlorid werden in 8 ml DMF gelöst und nach Zugabe von 385 mg (1.2 mMol) TBTU, 184 mg (1.2 mMol) HOBt und 1.03 g (8 mMol) N-Ethyl-N,N-diisopropylamin 14 Stunden bei Raumtemperatur gerührt. Die Lösung wird mit Wasser verdünnt, das ausgefallene Produkt abgesaugt, mit Wasser und Ethanol gewaschen und getrocknet.
Ausbeute: 270 mg (88 % der Theorie),
Schmelzpunkt: 240-243°C
$C_{24}H_{21}N_3O_2$ (383.45)
Massenspektrum: $M^+ = 383$

| | | | |
|---|---|---|---|
| Ber. | C 75.18 | H 5.52 | N 10.96 |

(fortgesetzt)

| | | | |
|---|---|---|---|
| Gef. | 75.19 | 5.60 | 10.94 |

Beispiel 19

(Z)-3-[1-(4-Methylaminocarbonyl-phenylamino)-1-phenyl-methyliden]-2-indolinon

Hergestellt analog Beispiel 18 aus (Z)-3-[1-(4-Carboxyphenylamino)-1-phenyl-methyliden]-2-indolinon, Methylamin-hydrochlorid, TBTU, HOBt und N-Ethyl-N,N-diisopropylamin in DMF.
Ausbeute: 68 % der Theorie,
Schmelzpunkt: 290-293°C
$C_{23}H_{19}N_3O_2$ (369.43)
Massenspektrum: $M^+ = 369$

| | | | |
|---|---|---|---|
| Ber. | C 74.78 | H 5.19 | N 11.37 |
| Gef. | 75.58 | 5.19 | 11.22 |

Beispiel 20

(Z)-3-[1-(4-Aminocarbonyl-phenylamino)-1-phenyl-methyliden]-2-indolinon

356 mg (1 mMol) (Z)-3-[1-(4-Carboxyphenylamino)-1-phenyl-methyliden]-2-indolinon werden in 10 ml DMF gelöst und mit 194 mg (1 mMol) CDI versetzt. Man rührt 2 Stunden bei Raumtemperatur, gibt 2 ml methanolische Ammoniaklösung zu rührt 16 Stunden bei Raumtemperatur. Danach versetzt man mit Wasser, saugt den Niederschlag ab, wäscht mit Wasser und wenig Ether und trocknet bei 80°C.
Ausbeute: 270 mg (76 % der Theorie),
Schmelzpunkt: 321-323°C
$C_{22}H_{17}N_3O_2$ (355.40)
Massenspektrum: $M^+ = 355$

| | | | |
|---|---|---|---|
| Ber. | C 74.35 | H 4.82 | N 11.82 |
| Gef. | 74.04 | 4.93 | 11.27 |

Beispiel 21

(Z)-3-[1-(3-Methylaminocarbonyl-phenylamino)-1-phenyl-methyliden]-2-indolinon

Hergestellt analog Beispiel 18 aus (Z)-3-[1-(3-Carboxyphenylamino)-1-phenyl-methyliden]-2-indolinon, Methylamin-hydrochlorid, TBTU, HOBt und Triethylamin in DMF.
Ausbeute: 41 % der Theorie,
Schmelzpunkt: 250-252°C
$C_{23}H_{19}N_3O_2$ (369.43)
Massenspektrum: $M^+ = 369$

Beispiel 22

(Z)-3-[1-(3-Dimethylaminocarbonyl-phenylamino)-1-phenyl-methyliden]-2-indolinon

Hergestellt analog Beispiel 21 aus (Z)-3-[1-(3-Carboxyphenylamino)-1-phenyl-methyliden]-2-indolinon, Dimethylamin-hydrochlorid, TBTU, HOBt und Triethylamin in DMF.
Ausbeute: 87 % der Theorie,
Schmelzpunkt: 261-263°C
$C_{24}H_{21}N_3O_2$ (383.45)
Massenspektrum: $M^+ = 383$
$R_f$-Wert: 0.51 (Kieselgel; Essigester)

| | | | |
|---|---|---|---|
| Ber. | C 75.18 | H 5.52 | N 10.96 |
| Gef. | 75.05 | 5.58 | 10.93 |

Beispiel 23

(Z)-3-[1-(3-Ethoxycarbonylmethylaminocarbonyl-phenylamino)-1-phenyl-methyliden]-2-indolinon

Hergestellt analog Beispiel 21 aus (Z)-3-[1-(3-Carboxyphenylamino)-1-phenyl-methyliden]-2-indolinon, Glycinethylester, TBTU, HOBt und Triethylamin in DMF.
Ausbeute: 91 % der Theorie,
Schmelzpunkt: 233-235°C
$C_{26}H_{23}N_3O_4$ (441.49)
Massenspektrum: $M^+$ = 441
$R_f$-Wert: 0.55 (Kieselgel; Essigester)

| | | | |
|---|---|---|---|
| Ber. | C 70.73 | H 5.25 | N 9.52 |
| Gef. | 70.69 | 5.33 | 9.52 |

Beispiel 24

(Z)-3-[1-(3-Carboxymetylaminocarbonyl-phenylamino)-1-phenylmethyliden]-2-indolinon

Hergestellt analog Beispiel 8 aus (Z)-3-[1-(3-Ethoxycarbonylmethylaminocarbonyl-phenylamino)-1-phenylmethyliden]-2-indolinon und Natronlauge in Ethanol.
Ausbeute: 81 % der Theorie,
Schmelzpunkt: 248-250°C
$C_{24}H_{19}N_3O_4$ (413.44)
Massenspektrum: $(M-H)^-$ = 412

Beispiel 25

(Z)-3-{1-[3-(N-Ethoxycarbonylmethyl-N-methyl-aminocarbonyl)-phenylamino]-1-phenyl-methyliden}-2-indolinon

Hergestellt analog Beispiel 21 aus (Z)-3-[1-(3-Carboxyphenylamino)-1-phenyl-methyliden]-2-indolinon, Sarkosinethylester, TBTU, HOBt und Triethylamin in DMF.
Ausbeute: 91 % der Theorie,
Schmelzpunkt: 148-150°C
$C_{27}H_{25}N_3O_4$ (455.52)
Massenspektrum: $M^+$ = 455

| | | | |
|---|---|---|---|
| Ber. | C 71.19 | H 5.53 | N 9.22 |
| Gef. | 70.75 | 5.63 | 9.38 |

Beispiel 26

(Z)-3-{1-[3-(N-Carboxymethyl-N-methyl-aminocarbonyl)-phenylamino]-1-phenyl-methyliden}-2-indolinon

Hergestellt analog Beispiel 8 aus (Z)-3-{1-[3-(N-Ethoxycarbonylmethyl-N-methyl-aminocarbonyl)-phenylamino]-1-phenylmethyliden}-2-indolinon und Natronlauge in Ethanol.
Ausbeute: 89 % der Theorie,
Schmelzpunkt: 218-220°C
$C_{25}H_{21}N_3O_4$ (427.46)
Massenspektrum: $(M-H)^-$ = 426

Beispiel 27

(Z)-3-{1-[(3-(2-Dimethylaminoethyl-aminocarbonyl)-phenylamino]-1-phenyl-methyliden}-2-indolinon

Hergestellt analog Beispiel 21 aus (Z)-3-[1-(3-Carboxyphenylamino)-1-phenyl-methyliden]-2-indolinon, N, N-Dimethylethylendiamin, TBTU, HOBt und Triethylamin in DMF.
Ausbeute: 66 % der Theorie,
Schmelzpunkt: 203-205°C
$C_{26}H_{26}N_4O_2$ (426.52)
Massenspektrum: $M^+$ = 426
$R_f$-Wert: 0.17 (Kieselgel; Essigester/Methanol = 6:4)

| | | | |
|---|---|---|---|
| Ber. | C 73.22 | H 6.14 | N 13.14 |
| Gef. | 72.42 | 6.29 | 12.85 |

Beispiel 28

(Z)-3-[1-(4-tert.Butoxycarbonylamino-phenylamino)-1-phenyl-methyliden]-2-indolinon

Hergestellt analog Beispiel 1 aus 1-Acetyl-3-(1-ethoxy-1-phenyl-methyliden)-2-indolinon und 4-tert.Butoxycarbonylaminoanilin in DMF und anschließender Behandlung mit Natronlauge in Methanol.
Ausbeute: 64 % der Theorie,
Schmelzpunkt: 244-246°C
$C_{26}H_{25}N_3O_3$ (427.51)
Massenspektrum: $M^+$ = 427

| | | | |
|---|---|---|---|
| Ber. | C 73.05 | H 5.86 | N 9.83 |
| Gef. | 72.80 | 5.84 | 9.92 |

Beispiel 29

(Z)-3-[1-(4-Formylamino-phenylamino)-1-phenyl-methyliden]-2-indolinon

a) (Z)-3-[1-(4-Aminophenylamino)-1-phenyl-methyliden] -2-indolinon

1.7 g (4 mMol) (Z)-3-[1-(4-tert.Butoxycarbonylamino-phenylamino)-1-phenyl-methyliden]-2-indolinon werden in 15 ml Dichlormethan suspendiert und nach Zugabe von 35 ml Essigester/Chlorwasserstoff 18 Stunden bei Raumtemperatur und 2 Stunden bei 40°C gerührt. Nach dem Abkühlen wird mit Ether verdünnt und der Niederschlag abgesaugt. Der Rückstand wird zwischen Natriumcarbonatlösung und Methylenchlorid verteilt, die organischen Extrakte getrocknet und eingedampft.
Ausbeute: 1.0 g (77 % der Theorie,
Schmelzpunkt: 299-300°C

b) (Z)-3-[1-(4-Formylamino-phenylamino)-1-phenyl-methyliden]-2-indolinon

200 mg (0.6 mMol) (Z)-3-[1-(4-Aminophenylamino)-1-phenyl-methyliden]-2-indolinon und 5 ml Ameisensäureethylester werden in 2.5 ml DMF 60 Stunden bei 90°C gerührt. Nach Entfernen des Lösungsmittels im Vakuum wird mit Essigester versetzt und nochmals eingedampft. Der Rückstand wird mit Ether verrührt, abgesaugt und getrocknet.
Ausbeute: 73 % der Theorie.
Schmelzpunkt: 268-269°C
$C_{22}H_{17}N_3O_2$ (355.40)
Massenspektrum: $M^+$ = 355

Beispiel 30

(Z)-3-[1-(3-Formylamino-phenylamino)-1-phenyl-methyliden]-2-indolinon

Hergestellt analog Beispiel 29 aus (Z)-3-[1-(3-Aminophenylamino)-1-phenyl-methyliden]-2-indolinon und Ameisensäureethylester in DMF.
Ausbeute: 80 % der Theorie,
Schmelzpunkt: 231°C
$C_{22}H_{17}N_3O_2$ (355.40)
Massenspektrum: $M^+ = 355$
$C_{22}H_{17}N_3O_2 \times H_2O$ (373.41)

| | | | |
|---|---|---|---|
| Ber. | C 70.76 | H 5.13 | N 11.25 |
| Gef. | 70.66 | 4.77 | 11.03 |

Beispiel 31

(Z)-3-[1-(4-Acetylamino-phenylamino]-1-phenyl-methyliden]-2-indolinon

196 mg (0.6 mMol) (Z)-3-[1-(4-Aminophenylamino)-1-phenyl-methyliden]-2-indolinon werden in 5 ml Eisessig gelöst und nach Zugabe von 0.1 g (1 mMol) Acetanhydrid 3 Stunden bei Raumtemperatur gerührt. Anschließend werden 15 ml Wasser zugesetzt, das ausgefallene Produkt abgesaugt, mit Wasser gewaschen und getrocknet.
Ausbeute: 210 mg (95 % der Theorie),
Schmelzpunkt: 236-238°C
$C_{23}H_{19}N_3O_2$ (369.43)
Massenspektrum: $M^+ = 369$

| | | | |
|---|---|---|---|
| Ber. | C 74.78 | H 5.18 | N 11.37 |
| Gef. | 74.32 | 5.28 | 11.15 |

Beispiel 32

(Z)-3-[1-(3-Acetylamino-phenylamino)-1-phenyl-methyliden]-2-indolinon

Hergestellt analog Beispiel 31 aus (Z)-3-[1-(3-Aminophenylamino)-1-phenyl-methyliden]-2-indolinon und Acetanhydrid in Eisessig.
Ausbeute: 89 % der Theorie,
Schmelzpunkt: 285-288°C
$C_{23}H_{19}N_3O_2$ (369.43)
Massenspektrum: $M^+ = 369$

| | | | |
|---|---|---|---|
| Ber. | C 74.78 | H 5.18 | N 11.37 |
| Gef. | 74.53 | 5.37 | 11.37 |

Beispiel 33

(Z)-3-[1-(3-Trifluoracetylamino-phenylamino)-1-phenyl-methyliden]-2-indolinon

Hergestellt analog Beispiel 31 aus (Z)-3-[1-(3-Aminophenylamino)-1-phenyl-methyliden]-2-indolinon und Trifluoressigsäureanhydrid in Trifluoressigsäure.
Ausbeute: 79 % der Theorie,
Schmelzpunkt: 273-276°C
$C_{23}H_{16}F_3N_3O_2$ (423.40)
Massenspektrum: $M^+ = 423$

| | | | |
|---|---|---|---|
| Ber. | C 65.25 | H 3.81 | N 9.92 |

(fortgesetzt)

| | | | |
|---|---|---|---|
| Gef. | 65.48 | 3.85 | 9.96 |

Beispiel 34

(Z)-3-[1-(4-tert.Butoxycarbonylaminomethylcarbonylamino-phenylamino)-1-phenyl-methyliden]-2-indolinon

Hergestellt analog Beispiel 18 aus (Z)-3-[1-(4-Aminophenylamino)-1-phenyl-methyliden]-2-indolinon, N-tert. Butoxycarbonyl-glycin, TBTU, HOBt und N-Methylmorpholin in DMF.
Ausbeute: 31 % der Theorie,
Schmelzpunkt: 243-244°C (Zers.)
$C_{28}H_{28}N_4O_4$ (484.56)
Massenspektrum: $M^+$ = 484

| | | | |
|---|---|---|---|
| Ber. | C 69.41 | H 5.82 | N 11.56 |
| Gef. | 68.52 | 5.73 | 11.30 |

Beispiel 35

(Z)-3-[1-(4-Aminomethylcarbonylamino-phenylamino)-1-phenyl-methyliden]-2-indolinon-hydrochlorid

Hergestellt analog Beispiel 29a aus (Z)-3-[1-(4-tert.Butoxycarbonylaminomethylcarbonylamino-phenylamino)-1-phenyl-methyliden]-2-indolinon und Essigester/Chlorwasserstoff in Dichlormethan.
Ausbeute: 73 % der Theorie,
Schmelzpunkt: 289-290°C
$C_{23}H_{20}N_4O_2$ (384.44)
Massenspektrum: $M^+$ = 384
$C_{23}H_{20}N_4O_2$ x HCl x $H_2O$ (438.92)

| | | | |
|---|---|---|---|
| Ber. | C 62.94 | H 5.28 | N 12.76 |
| Gef. | 62.66 | 5.37 | 12.07 |

Beispiel 36

(Z)-3-{1-[3-(N-Trifluoracetyl-N-methyl-amino)-phenylamino]-1-phenyl-methyliden}-2-indolinon

636 mg (1.5 mMol) (Z)-3-[1-(3-Trifluoracetylamino-phenylamino)-1-phenyl-methyliden]-2-indolinon werden in 20 ml Aceton gelöst und nach Zugabe von 423 mg (3 mMol) Kaliumcarbonat und 0.25 g (3 mMol) Methyljodid 18 Stunden bei Raumtemperatur gerührt. Die Reaktionslösung wird nach Abfiltrieren von Unlöslichem vom Lösungsmittel im Vakuum befreit. Der Rückstand wird in Dichlormethan/Wasser verteilt, die organische Phase getrocknet und eingedampft. Der Rückstand wird mit Ether verrieben, abgesaugt und getrocknet.
Ausbeute: 550 mg (85 % der Theorie),
Schmelzpunkt: 224-227°C
$C_{24}H_{18}F_3N_3O_2$ (437.43)
Massenspektrum: $M^+$ = 437

| | | | |
|---|---|---|---|
| Ber. | C 65.90 | H 4.15 | N 9.61 |
| Gef. | 65.96 | 4.22 | 9.59 |

Beispiel 37

(Z)-3-[1-(3-Methylamino-phenylamino)-1-phenyl-methyliden]-2-indolinon

Hergestellt analog Beispiel 8 aus (Z)-3-{1-[3-(N-Trifluoracetyl-N-methyl-amino)-phenylamino]-1-phenyl-methyliden}-2-indolinon und Natronlauge in Methanol.

Ausbeute: 91 % der Theorie,
Schmelzpunkt: 247-248°C
$C_{22}H_{18}N_4O_3$ (386.41)
Massenspektrum: $M^+$ = 341

| | | | |
|---|---|---|---|
| Ber. | C 77.40 | H 5.61 | N 12.31 |
| Gef. | 76.65 | 5.60 | 12.09 |

Beispiel 38

(Z)-3-{1-[3-(N-Acetyl-N-methylamino)-phenylamino]-1-phenyl-methyliden}-2-indolinon

Hergestellt analog Beispiel 31 aus (Z)-3-[1-(3-Methylaminophenylamino)-1-phenyl-methyliden]-2-indolinon und Acetanhydrid in Eisessig.
Ausbeute: 73 % der Theorie,
Schmelzpunkt: 237-239°C
$C_{24}H_{21}N_3O_2$ (383.45)
Massenspektrum: $M^+$ = 383

| | | | |
|---|---|---|---|
| Ber. | C 75.18 | H 5.52 | N 10.96 |
| Gef. | 74.51 | 5.51 | 10.80 |

Beispiel 39

(Z)-3-[1-(4-Propionylamino-phenylamino)-1-phenyl-methyliden]-2-indolinon

a) 3-(1-Ethoxy-1-phenyl-methyliden)-2-indolinon

4.0 g (13.2 mMol) 1-Acetyl-3-(1-ethoxy-1-phenyl-methyliden)-2-indolinon werden in 50 ml Ethanol suspendiert und nach Zugabe von 10 ml 4N Natronlauge 90 Minuten bei Raumtemperatur gerührt. Die Lösung wird mit 150 ml Wasser verdünnt, das kristalline Produkt abgesaugt, gewaschen und getrocknet.
Ausbeute: 2.8 g (80 % der Theorie),
Schmelzpunkt: 168-169°C

b) N-Propionyl-4-nitroanilin

6.9 g (50 mMol) Nitroanilin werden in 50 ml Propionsäure suspendiert und mit 9.1 g (50 mMol) Propionsäureanhydrid versetzt. Man erwärmt 90 Minuten auf 50°C und rührt anschließend 16 Stunden bei Raumtemperatur. Danach werden 200 ml Wasser zugegeben. Der Niederschlag wird abgesaugt, gewaschen und getrocknet.
Ausbeute: 9.4 g (97 % der Theorie),
Schmelzpunkt: 192-195°C

c) 4-Propionylamino-anilin

250 mg (2 mMol) N-Propionyl-4-nitroanilin werden in 200 ml Methanol gelöst und mit 0.6 g 10%igem Palladium/Kohle versetzt. Man hydriert in einer Wasserstoffatmosphäre bei 2 bar für 30 Minuten. Danach wird der Katalysator abfiltriert und die Lösung im Vakuum vom Lösungsmittel befreit.
Ausbeute: 4.5 g (91 % der Theorie),
Schmelzpunkt: 82-84°C
$C_9H_{12}N_2O$ (164.21)

| | | | |
|---|---|---|---|
| Ber. | C 65.83 | H 7.37 | N 17.06 |
| Gef. | 65.99 | 7.36 | 17.02 |

d) (Z)-3-[1-(4-Propionylamino-phenylamino)-1-phenyl-methyliden]-2-indolinon

265 mg (1 mMol) 3-(1-Ethoxy-1-phenyl-methyliden)-2-indolinon werden in 5 ml DMF gelöst und nach Zugabe von 300 mg (1.8 mMol) 4-Propionylamino-anilin 8 Stunden bei 150°C gerührt. Nach dem Abkühlen wird mit Wasser verdünnt, das kristalline Produkt abgesaugt, gewaschen und getrocknet.
Ausbeute: 280 mg (68 % der Theorie),
Schmelzpunkt: 255-256°C
$C_{24}H_{21}N_3O_2$ (383.45)
Massenspektrum: $M^+ = 383$
$C_{24}H_{21}N_3O_2 \times H_2O$ (401.47)

| | | | |
|---|---|---|---|
| Ber. | C 71.80 | H 5.77 | N 10.47 |
| Gef. | 71.62 | 5.61 | 10.50 |

Beispiel 40

(Z)-3-[1-(4-Methoxymethylcarbonylamino-phenylamino)-1-phenylmethyliden]-2-indolinon

Hergestellt analog den Beispielen 1 und 39 aus 1-Acetyl-3-(1-ethoxy-1-phenyl-methyliden)-2-indolinon und 4-Methoxymethylcarbonylamino-anilin in DMF und anschließender Behandlung mit Natronlauge in Methanol.
Ausbeute: 80 % der Theorie,
Schmelzpunkt: 238-240°C
$C_{24}H_{21}N_3O_3$ (399.45)
Massenspektrum: $M^+ = 399$

| | | | |
|---|---|---|---|
| Ber. | C 72.17 | H 5.30 | N 10.52 |
| Gef. | 71.92 | 5.33 | 10.44 |

Beispiel 41

(Z)-3-[1-(4-Dimethylaminomethylcarbonylamino-phenylamino)-1-phenyl-methyliden]-2-indolinon

Hergestellt analog Beispiel 1 aus 1-Acetyl-3-(1-ethoxy-1-phenyl-methyliden)-2-indolinon und 4-Dimethylaminomethylcarbonylamino-anilin in DMF und anschließender Behandlung mit Natronlauge in Methanol.
Ausbeute: 68 % der Theorie,
Schmelzpunkt: 234-236°C
$C_{25}H_{24}N_4O_2$ (412.50)
Massenspektrum: $M^+ = 412$
$R_f$-Wert: 0.28 (Kieselgel; Essigester/Methanol = 19:1)

| | | | |
|---|---|---|---|
| Ber. | C 72.29 | H 5.86 | N 13.58 |
| Gef. | 72.35 | 5.83 | 13.37 |

Beispiel 42

(Z)-3-[1-(4-Diethylaminomethylcarbonylamino-phenylamino)-1-phenyl-methyliden]-2-indolinon-hydrochlorid

Hergestellt analog Beispiel 1 aus 1-Acetyl-3-(1-ethoxy-1-phenyl-methyliden)-2-indolinon und 4-Diethylaminomethylcarbonylamino-anilin in DMF und anschließender Behandlung mit Natronlauge in Methanol.
Ausbeute: 80 % der Theorie,
Schmelzpunkt: 267-269°C
$C_{27}H_{28}N_4O_2$ (440.55)
Massenspektrum: $M^+ = 440$
$R_f$-Wert: 0.32 (Kieselgel; Dichlormethan/Methanol = 19:1) $C_{27}H_{28}N_4O_2 \times HCl \times 1.5\ H_2O$ (504.03)

| | | | |
|---|---|---|---|
| Ber. | C 64.34 | H 6.40 | N 11.12 |

(fortgesetzt)

| | | | |
|---|---|---|---|
| Gef. | 64.72 | 6.69 | 11.16 |

Beispiel 43

(Z)-3-[1-(4-Morpholinomethylcarbonylamino-phenylamino)-1-phenyl-methyliden]-2-indolinon

a) N-Morpholinomethylcarbonyl-4-nitroanilin

2.6 g (30 mMol) Morpholin und 4.2 g (30 mMol) Kaliumcarbonat werden in 120 ml Aceton suspendiert. Man tropft über einen Zeitraum von 20 Minuten 5.3 g (20 mMol) N-Bromacetyl-4-nitroanilin, gelöst in 80 ml Aceton, zu und rührt anschließend 2 Stunden bei Raumtemperatur. Man filtiert den Niederschlag ab und entfernt das Lösungsmittel im Vakuum. Der Rückstand wird mit Wasser aufgeschlämmt. Der Niederschlag wird abgesaugt und im Trockenschrank getrocknet.
Ausbeute: 5.0 g (94 % der Theorie),
Schmelzpunkt: 148-149°C

b) 4-Morpholinomethylcarbonylamino-anilin

Hergestellt analog Beispiel 39c durch katalytische Hydrierung aus N-Morpholinomethylcarbonyl-4-nitroanilin.
Ausbeute: 92 % der Theorie,
Schmelzpunkt: 106-107°C
$C_{12}H_{17}N_3O_2$ (235.29)

| | | | |
|---|---|---|---|
| Ber. | C 61.26 | H 7.28 | N 17.86 |
| Gef. | 60.91 | 7.28 | 17.60 |

c) (Z)-3-[1-(4-Morpholinomethylcarbonylamino-phenylamino)-1-phenyl-methyliden]-2-indolinon

Hergestellt analog Beispiel 1 aus 1-Acetyl-3-(1-ethoxy-1-phenyl-methyliden)-2-indolinon und 4-Morpholinomethylcarbonylamino-anilin in DMF und anschließender Behandlung mit Natronlauge in Methanol.
Ausbeute: 97 % der Theorie,
Schmelzpunkt: 246-248°C
$C_{27}H_{26}N_4O_3$ (454.53)
Massenspektrum: $M^+$ = 454
$R_f$-Wert: 0.35 (Kieselgel; Essigester)
$C_{27}H_{26}N_4O_3$ x 0.5 $H_2O$ (463.54)

| | | | |
|---|---|---|---|
| Ber. | C 69.96 | H 5.87 | N 12.09 |
| Gef. | 70.36 | 5.90 | 12.08 |

Beispiel 44

(Z)-3-{1-[4-(4-Methylpiperazinomethylcarbonylamino-phenylamino]-1-phenyl-methyliden}-2-indolinon

Hergestellt analog Beispiel 43 aus 1-Acetyl-3-(1-ethoxy-1-phenyl-methyliden)-2-indolinon und 4- (4-Methylpiperazinomethylcarbonylamino)-anilin in DMF und anschließender Behandlung mit Natronlauge in Methanol.
Ausbeute: 86 % der Theorie,
Schmelzpunkt: 282-284°C
$C_{28}H_{29}N_5O_2$ (467.58)
Massenspektrum: $M^+$ = 467
$R_f$-Wert: 0.32 (Kieselgel; Dichlormethan/Methanol = 9:1) $C_{28}H_{29}N_5O_2$ x 0.5 $H_2O$ (476.58)

| | | | |
|---|---|---|---|
| Ber. | C 70.57 | H 6.34 | N 14.70 |
| Gef. | 70.88 | 6.29 | 14.54 |

Beispiel 45

(Z)-3-[1-(4-Ethylaminocarbonylamino-phenylamino)-1-phenyl-methyliden]-2-indolinon

196 mg (0.6 mMol) (Z)-3-[1-(4-Aminophenylamino)-1-phenyl-methyliden]-2-indolinon werden in 10 ml THF suspendiert und nach Zugabe von 70 mg (0.1 mMol) Ethylisocyanat 140 Stunden bei Raumtemperatur gerührt. Das ausgefallene Produkt wird abgesaugt, mit Ether gewaschen und getrocknet.
Ausbeute: 200 mg (84 % der Theorie),
Schmelzpunkt: 264-265°C
$C_{24}H_{22}N_4O_2$ (398.47)
Massenspektrum: $M^+$ = 398

| | | | |
|---|---|---|---|
| Ber. | C 72.34 | H 5.57 | N 14.06 |
| Gef. | 71.70 | 5.83 | 13.49 |

Beispiel 46

(Z)-3-[1-(4-Butylaminocarbonylamino-phenylamino)-1-phenyl-methyliden]-2-indolinon

Hergestellt analog Beispiel 45 aus (Z)-3-[1-(4-Aminophenylamino)-1-phenyl-methyliden]-2-indolinon und Butylisocyanat in THF.
Ausbeute: 72 % der Theorie,
Schmelzpunkt: 216-217°C
$C_{26}H_{26}N_4O_2$ (426.52)
Massenspektrum: $M^+$ = 426

| | | | |
|---|---|---|---|
| Ber. | C 73.22 | H 6.14 | N 13.14 |
| Gef. | 72.74 | 5.94 | 12.67 |

Beispiel 47

(Z)-3-{1-[4-(N-Acetyl-N-methyl-amino)-phenylamino]-1-phenylmethyliden}-2-indolinon

Hergestellt analog Beispiel 1 aus 1-Acetyl-3-(1-ethoxy-1-phenyl-methyliden)-2-indolinon und 4-(N-Acetyl-N-methyl-amino)-anilin in DMF und anschließender Behandlung mit Natronlauge in Methanol.
Ausbeute: 50 % der Theorie,
Schmelzpunkt: 287-288°C
$C_{24}H_{21}N_3O_2$ (383.45)
Massenspektrum: $M^+$ = 383

| | | | |
|---|---|---|---|
| Ber. | C 75.18 | H 5.52 | N 10.96 |
| Gef. | 75.18 | 5.62 | 10.89 |

Beispiel 48

(Z)-3-{1-[4-(N-Dimethylaminomethylcarbonyl-N-methyl-amino)-phenylamino]-1-phenyl-methyliden}-2-indolinon-hydrochlorid

Hergestellt analog Beispiel 43 aus 1-Acetyl-3-(1-ethoxy-1-phenyl-methyliden)-2-indolinon und 4-(N-Dimethylaminomethylcarbonyl-N-methyl-amino)-anilin in DMF und anschließender Behandlung mit Natronlauge in Methanol.
Ausbeute: 30 % der Theorie,
Schmelzpunkt: 290-292°C
$C_{26}H_{26}N_4O_2$ (426.52)
Massenspektrum: $M^+$ = 426
$C_{26}H_{26}N_4O_2$ x HCl x 2 $H_2O$ (499.00)

| Ber. | C 62.58 | H 6.26 | N 11.23 | Cl 7.10 |
|---|---|---|---|---|
| Gef. | 62.68 | 6.07 | 11.19 | 7.88 |

Beispiel 49

(Z)-3-{1-[4-(N-Diethylaminomethylcarbonyl-N-methyl-amino)-phenylamino]-1-phenyl-methyliden}-2-indolinon

Hergestellt analog Beispiel 43 aus 1-Acetyl-3-(1-ethoxy-1-phenyl-methyliden)-2-indolinon und 4-(N-Diethylaminomethylcarbonyl-N-methyl-amino)-anilin in DMF und anschließender Behandlung mit Natronlauge in Methanol.
Ausbeute: 64 % der Theorie,
Schmelzpunkt: 242-247°C
$C_{28}H_{30}N_4O_2$ (454.58)
Massenspektrum: $M^+$ = 454
$R_f$-Wert: 0.56 (Kieselgel; Dichlormethan/Methanol/$NH_4OH$ = 9:1:0.1)
$C_{28}H_{30}N_4O_2$ x 0.5 $H_2O$ (454.57)

| Ber. | C 72.55 | H 6.74 | N 12.09 |
|---|---|---|---|
| Gef. | 72.70 | 6.41 | 12.11 |

Beispiel 50

(Z)-3-{1-[4-(N-Piperidinomethylcarbonyl-N-methyl-amino)-phenylamino]-1-phenyl -methyliden}-2-indolinon

Hergestellt analog Beispiel 43 aus 1-Acetyl-3-(1-ethoxy-1-phenyl-methyliden)-2-indolinon und 4-(N-Piperidinomethylcarbonyl-N-methyl-amino)-anilin in DMF und anschließender Behandlung mit Natronlauge in Methanol.
Ausbeute: 43 % der Theorie,
Schmelzpunkt: 230-235°C (Zers.)
$C_{29}H_{30}N_4O_2$ (466.59)
Massenspektrum: $M^+$ = 466
$R_f$-Wert: 0.54 (Kieselgel; Dichlormethan/Methanol/$NH_4OH$ = 9:1:0.1)
$C_{29}H_{30}N_4O_2$ x 1.5 $H_2O$ (493.61)

| Ber. | C 70.57 | H 6.74 | N 11.35 |
|---|---|---|---|
| Gef. | 70.57 | 6.32 | 11.28 |

Beispiel 51

(Z)-3-{1-[4-(N-Morpholinomethylcarbonyl-N-methyl-amino)-phenylamino]-1-phenyl-methyliden}-2-indolinon

Hergestellt analog Beispiel 43 aus 1-Acetyl-3-(1-ethoxy-1-phenyl-methyliden)-2-indolinon und 4-(N-Morpholinomethylcarbonyl-N-methyl-amino)-anilin in DMF und anschließender Behandlung mit Natronlauge in Methanol.
Ausbeute: 99 % der Theorie,
Schmelzpunkt: 263-265°C
$C_{28}H_{28}N_4O_3$ (468.56)
Massenspektrum: $M^+$ = 468

| Ber. | C 71.78 | H 6.02 | N 11.96 |
|---|---|---|---|
| Gef. | 70.75 | 6.05 | 11.90 |

Beispiel 52

(Z)-3-{1-[4-(N-(4-Methylpiperazinomethylcarbonyl)-N-methylamino)-phenylamino]-1-phenyl-methyliden}-2-indolinon

Hergestellt analog Beispiel 43 aus 1-Acetyl-3-(1-ethoxy-1-phenyl-methyliden)-2-indolinon und 4- [N-(4-Methylpiperazinomethylcarbonyl)-N-methyl-amino]-anilin in DMF und anschließender Behandlung mit Natronlauge in Methanol.
Ausbeute: 73 % der Theorie,
Schmelzpunkt: 277-278°C
$C_{29}H_{31}N_5O_2$ (481.60)
Massenspektrum: $M^+$ = 481
$R_f$-Wert: 0.37 (Kieselgel; Dichlormethan/Methanol/$NH_4OH$ = 9:1:0.1)

Beispiel 53

(Z)-3-{1-[4-(N-(4-Benzylpiperazinomethylcarbonyl)-N-methylamino)-phenylamino]-1-phenyl -methyliden}-2-indolinon

Hergestellt analog Beispiel 43 aus 1-Acetyl-3-(1-ethoxy-1-phenyl-methyliden) -2-indolinon und 4-[N-(4-Benzylpiperazinomethylcarbonyl)-N-methylamino]-anilin in DMF und anschließender Behandlung mit Natronlauge in Methanol.
Ausbeute: 55 % der Theorie,
Schmelzpunkt: 157-158°C
$C_{35}H_{35}N_5O_2$ (557.70)
Massenspektrum: $M^+$ = 557
$R_f$-Wert: 0.62 (Kieselgel; Dichlormethan/Methanol/$NH_4OH$ = 9:1:0.1)
$C_{35}H_{35}N_5O_2$ x $H_2O$ (575.72)

| | | | |
|---|---|---|---|
| Ber. | C 73.02 | H 6.48 | N 12.16 |
| Gef. | 73.10 | 6.46 | 12.13 |

Beispiel 54

(Z)-3-{1-[4-(N-Piperazinomethylcarbonyl-N-methyl-amino)-pheylamino]-1-phenyl-methyliden}-2-indolinon-dihydrochlorid

390 mg (0.7 mMol) (Z)-3-{1-[4-(N-(N-Benzylpiperazinomethylcarbonyl)-N-methyl-amino)-phenylamino]-1-phenyl-methyliden}-2-indolinon werden in 20 ml Dichlormethan gelöst und nach Zugabe von 0.2 g (1.4 mMol) Chlorameisensäure-1-chlorethylester 30 Minuten bei Raumtemperatur und 60 Minuten zum Rückfluß erhitzt. Das Lösungsmittel wird eingedampft, der Rückstand mit 10 ml Methanol versetzt und 90 Minuten zum Rückfluß erhitzt. Nach 18 Stunden Rühren bei Raumtemperatur wird das Produkt abgesaugt, mit Methanol gewaschen und getrocknet.
Ausbeute: 200 mg (51 % der Theorie),
Schmelzpunkt: 255-258°C (Zers.)
$C_{28}H_{29}N_5O_2$ (467.58)
Massenspektrum: $M^+$ = 467
$C_{28}H_{29}N_5O_2$ x 2 HCl x $H_2O$ (558.52)

| | | | | |
|---|---|---|---|---|
| Ber. | C 60.22 | H 5.96 | N 12.54 | Cl 12.70 |
| Gef. | 60.06 | 5.91 | 12.53 | 12.75 |

Beispiel 55

(Z)-3-[1-(4-Aminomethyl-phenylamino)-1-phenyl-methyliden]-2-indolinon

a) (Z)-3-[1-(4-Cyanophenylamino)-1-phenyl-methyliden]-2-indolinon

Hergestellt analog Beispiel 1 aus 1-Acetyl-3-(1-ethoxy-1-phenyl-methyliden)-2-indolinon und 4-Aminobenzonitril in DMF und anschließender Behandlung mit Natronlauge.
Ausbeute: 44 % der Theorie,
Schmelzpunkt: 293-295°C
$C_{22}H_{15}N_3O$ (337.38)

| | | | |
|---|---|---|---|
| Ber. | C 78.32 | H 4.48 | N 12.45 |

(fortgesetzt)

| | | | |
|---|---|---|---|
| Gef. | 77.75 | 4.68 | 12.50 |

b) (Z)-3-[1-(4-Aminomethyl-phenylamino)-1-phenyl-methyliden]-2-indolinon

900 mg (2.7 mMol) (Z)-3-[1-(4-Cyanophenylamino)-1-phenyl-methyliden]-2-indolinon werden in 200 ml methanolischem Ammoniak 7 Stunden über 1.4 g Raney-Nickel bei einem Wasserstoffdruck von 3 bar hydriert. Der Katalysator wird abfiltriert, die Lösung eingedampft und der Rückstand in Wasser/Dichlormethan verteilt. Die organische Phase wird getrocknet, eingedampft, mit Ether verrieben, abgesaugt und getrocknet.
Ausbeute: 780 mg (83 % der Theorie),
Schmelzpunkt: 236-237°C
$C_{22}H_{19}N_3O$ (341.42)
Massenspektrum: $M^+ = 341$
$C_{22}H_{19}N_3O \times 0.5\ H_2O$ (350.42)

| | | | |
|---|---|---|---|
| Ber. | C 75.41 | H 5.75 | N 11.99 |
| Gef. | 75.08 | 5.62 | 11.81 |

Beispiel 56

(Z)-3-[1-(4-Acetylaminomethyl-phenylamino)-1-phenyl-methyliden]-2-indolinon

Hergestellt analog Beispiel 31 aus (Z)-3-[1-(4-Aminomethylphenylamino)-1-phenyl-methyliden]-2-indolinon, Eisessig und Acetanhydrid.
Ausbeute: 135 mg (88 % der Theorie),
Schmelzpunkt: 207-210°C
$C_{24}H_{21}N_3O_2$ (383.45)
Massenspektrum: $M^+ = 383$

| | | | |
|---|---|---|---|
| Ber. | C 75.18 | H 5.52 | N 10.96 |
| Gef. | 74.79 | 5.46 | 10.77 |

Beispiel 57

(Z)-3-[1-(4-tert.Butoxycarbonylaminomethylcarbonylaminomethylphenylamino)-1-phenyl-methyliden}-2-indolinon

Hergestellt analog Beispiel 18 aus (Z)-3-{1-[4-(Aminomethyl)-phenylamino]-1-phenyl-methyliden}-2-indolinon, N-tert.Butoxycarbonyl-glycin, TBTU, HOBt und N-Ethyl-N,N-diisopropylamin in DMF.
Ausbeute: 85 % der Theorie,
Schmelzpunkt: 218-220°C
$C_{29}H_{30}N_4O_4$ (498.59)
Massenspektrum: $M^+ = 498$

| | | | |
|---|---|---|---|
| Ber. | C 69.86 | H 6.06 | N 11.24 |
| Gef. | 69.40 | 6.20 | 11.18 |

Beispiel 58

(Z)-3-[1-(4-Aminomethylcarbonylaminomethyl-phenylamino)-1-phenyl-methyliden]-2-indolinon-hydrochlorid

Hergestellt analog Beispiel 29a aus (Z)-3-[1-(4-tert.Butoxycarbonylaminomethylcarbonylaminomethyl-phenylamino)-1-phenylmethyliden]-2-indolinon und Essigester/Chlorwasserstoff in Dichlormethan.
Ausbeute: 88 % der Theorie,
Schmelzpunkt: 190-195°C
$C_{24}H_{22}N_4O_2$ (398.47)

Massenspektrum: $M^+$ = 398
$C_{24}H_{22}N_4O_2$ x HCl x $H_2O$ (452.95)

| | | | |
|---|---|---|---|
| Ber. | C 63.64 | H 5.56 | N 12.37 |
| Gef. | 64.11 | 5.55 | 12.19 |

Beispiel 59

(Z)-3-[1-(4-Morpholinomethyl-phenylamino)-1-phenyl-methyliden]-2-indolinon

Hergestellt analog Beispiel 1 aus 1-Acetyl-3-(1-ethoxy-1-phenyl-methyliden)-2-indolinon und 4-Morpholinomethyl-anilin in DMF und anschließender Behandlung mit Natronlauge in Methanol.
Ausbeute: 66 % der Theorie,
Schmelzpunkt: 267-268°C
$C_{26}H_{25}N_3O_2$ (411.51)
Massenspektrum: $M^+$ = 411
$R_f$-Wert: 0.58 (Kieselgel; Essigester/Petrolether = 9:1)

| | | | |
|---|---|---|---|
| Ber. | C 75.89 | H 6.12 | N 10.21 |
| Gef. | 75.18 | 6.09 | 10.14 |

Beispiel 60

(Z)-3-[1-(4-Acetylphenylamino)-1-phenyl-methyliden]-2-indolinon

Hergestellt analog Beispiel 1 aus 1-Acetyl-3-(1-ethoxy-1-phenyl-methyliden)-2-indolinon und 4-Aminoacetophenon in DMF und anschließender Behandlung mit Natronlauge in Methanol.
Ausbeute: 20 % der Theorie,
Schmelzpunkt: 207-209°C
$C_{23}H_{18}N_2O_2$ (354.41)
Massenspektrum: $M^+$ = 354
$R_f$-Wert: 0.24 (Kieselgel; Dichlormethan/Methanol = 19:1)

Beispiel 61

3-{1-[N-(4-Cyanophenyl)-N-methyl-amino]-1-phenyl-methyliden}-2-indolinon

Hergestellt analog Beispiel 2 aus 1-Acetyl-3-(1-chlor-1-phenyl-methyliden)-2-indolinon und 4-Methylaminobenzonitril in THF und anschließender Behandlung mit Natronlauge in Methanol.
Ausbeute: 6 % der Theorie,
Schmelzpunkt: 239°C
$C_{23}H_{17}N_3O$ (702.82)
Massenspektrum: $M^+$ = 351

Beispiel 62

(Z)-3-{1-[N-(4-Amidinophenyl)-amino]-1-phenyl-methyliden}-2-indolinon-hydroacetat

1.0 g (2.8 mMol) (Z)-1-Benzoyl-3-[1-(4-Cyanophenylamino]-1-phenyl-methyliden]-2-indolinon werden in 20 ml gesättigter methanolischer Salzsäure gelöst und 18 Stunden bei Raumtemperatur gerührt. Das Solvens wird abdestilliert, der Rückstand in 20 ml absolutem Methanol gelöst und mit konz. Ammoniak auf pH 8 eingestellt. Der Niederschlag wird abgesaugt, in Methanol suspendiert und mit 0.4 g Ammoniumacetat 2 Stunden zum Rückfluß erhitzt. Das Produkt wird abgesaugt, mit Methanol gewaschen und getrocknet.
Ausbeute: 340 mg (34 % der Theorie),
Schmelzpunkt: >260°C (Zers.)
$C_{22}H_{18}N_4O$ (354.41)
Massenspektrum: $(M+H)^+$ = 355

$R_f$-Wert: 0.44 (Reversed Phase P8; Wasser/Acetonitril = 1:1 + 1% Trifuoressigsäure)

Beispiel 63

(Z)-3-[1-(3-Cyanophenylamino)-1-phenyl-methyliden]-2-indolinon

Hergestellt analog Beispiel 9 aus 1-Benzoyl-3-(1-chlor-1-phenyl-methyliden]-2-indolinon und 3-Aminobenzonitril in THF und anschließender Behandlung mit Natronlauge in Methanol.
Ausbeute: 70 % der Theorie,
Schmelzpunkt: 262-272°C
$C_{22}H_{15}N_3O$ (337.38)
Massenspektrum: $M^+$ = 337

Beispiel 64

(Z)-3-[1-(3-Amidinophenylamino)-1-phenyl-methyliden]-2-indolinon

Hergestellt analog Beispiel 62 aus (Z)-3-[1-(3-Cyanophenylamino)-1-phenyl-methyliden]-2-indolinon und methanolischer Salzsäure in Methanol und Ammoniumacetat.
Ausbeute: 26 % der Theorie,
Schmelzpunkt: 235-237°C
$C_{22}H_{18}N_4O$ (354.41)
Massenspektrum: $M^+$ = 354

Beispiel 65

(Z)-3-{1-[3-(N-Methylcarbamimidoyl)-phenylamino]-1-phenyl-methyliden}-2-indolinon

Hergestellt analog Beispiel 62 aus (Z)-3-[1-(3-Cyanophenylamino)-1-phenyl-methyliden]-2-indolinon, methanolischer Salzsäure und Methylamin in Methanol.
Ausbeute: 7 % der Theorie,
Schmelzpunkt: 248-250°C
$C_{23}H_{20}N_4O$ (368.44)
Massenspektrum: $(M+H)^+$ = 369
$R_f$-Wert: 0.23 (Reversed Phase P8; Methanol/5%ige Kochsalzlösung = 6:4)

Beispiel 66

(Z)-3-{1-[3-(N,N-Dimethylcarbamimidoyl)-phenylamino)-1-phenylmethyliden}-2-indolinon

Hergestellt analog Beispiel 62 aus (Z)-3-[1-(3-Cyanophenylamino)-1-phenyl-methyliden]-2-indolinon, methanolischer Salzsäure und Dimethylamin in Methanol.
Ausbeute: 30 % der Theorie,
Schmelzpunkt: 238-242°C
$C_{24}H_{22}N_4O$ (382.47)
Massenspektrum: $(M^+H)^+$ = 383
$R_f$-Wert: 0.27 (Reversed Phase P8; Methanol/5%ige Kochsalzlösung = 6:4)

Beispiel 67

(Z)-3-[1-(3-tert.Butoxycarbonylaminomethyl-phenylamino)-1-pheyl-methyliden]-2-indolinon

Hergestellt analog Beispiel 9 aus 1-Benzoyl-3-(1-chlor-1-phenyl-methyliden)-2-indolinon und 3-tert.Butoxycarbonylaminomethyl-anilin in Triethylamin.
Ausbeute: 7 % der Theorie,
Schmelzpunkt: 190-195°C
$C_{27}H_{27}N_3O_3$ (441.53)
Massenspektrum: $M^+$ = 441

$R_f$-Wert: 0.35 (Kieselgel; Essigester/Petrolether = 1:1)

Beispiel 68

(Z)-3-[1-(3-Aminomethyl-phenylamino)-1-phenyl-methyliden]-2-indolinon

Hergestellt analog Beispiel 57 aus (Z)-3-[1-(3-tert.Butoxycarbonylaminomethyl-phenylamino)-1-phenyl-methyliden]-2-indolinon und Trifluoressigsäure in Dichlormethan.
Ausbeute: 60 % der Theorie,
Schmelzpunkt: 175-185°C
$C_{22}H_{19}N_3O$ (341.42)
Massenspektrum: $M^+ = 341$
$R_f$-Wert: 0.44 (Kieselgel; Essigester/Methanol/$NH_4OH$ = 4:1:0.5)

Beispiel 69

(Z)-3-[1-(3-Aminophenylamino)-1-phenyl-methyliden]-2-indolinon

3.5 g (0.01 Mol) (Z)-3-[1-(3-Nitrophenylamino)-1-phenyl-methyliden}-2-indolinon werden in 200 ml THF gelöst und nach Zugabe von 0.5 g Palladium/Kohle mit Wasserstoff hydriert. Anschließend wird vom Katalysator abfiltriert und eingedampft.
Ausbeute: 3.4 g (99 % der Theorie),
Schmelzpunkt: 267-268°C
$C_{21}H_{17}N_3O$ (327.39)
Massenspektrum: $M^+ = 327$

Beispiel 70

(Z)-3-{1-[N-(4-Amino)-phenylamino]-1-phenyl-methyliden}-2-indolinon

Hergestellt analog Beispiel 69 aus (Z)-3-[1-(4-Nitrophenylamino)-1-phenyl-methyliden]-2-indolinon und Palladium/Kohle mit Wasserstoff in THF.
Ausbeute: 77 % der Theorie,
Schmelzpunkt: >290°C
$C_{21}H_{17}N_3O$ (327.39)
Massenspektrum: $M^+ = 327$
$R_f$-Wert: 0.51 (Kieselgel; Dichlorethan/Essigester/Eisessig = 80:17:3)

Beispiel 71

(Z)-3-[1-(3-Guanidinophenylamino)-1-phenyl-methyliden]-2-indolinon

2.0 g (6.1 mMol) (Z)-3-[1-(3-Aminophenylamino)-1-phenyl-methyliden]-2-indolinon und 1.0 g (23.7 mMol) Cyanamid werden in 100 ml Ethanol und 10 ml etherischer Salzsäure gelöst und 24 Stunden in einer Glasbombe bei 80°C erhitzt. Das Solvens wird abdestilliert. Chromatographie des Rückstands an Kieselgel (Essigester/Methanol/Eisessig/Wasser = 17:3:5:5) liefert das Produkt.
Ausbeute: 300 mg (13 % der Theorie),
$C_{22}H_{19}N_5O$ (369.43)
Massenspektrum: $(M^+H)^+ = 370$

Beispiel 72

(Z)-3-[1-(4-Guanidinophenylamino)-1-phenyl-methyliden]-2-indolinon

Hergestellt analog Beispiel 71 aus (Z)-3-[1-(4-Aminophenylamino)-1-phenyl-methyliden]-2-indolinon und Cyanamid in Dioxan/Chlorwasserstoff.
Ausbeute: 27 % der Theorie,
$C_{22}H_{19}N_5O$ (369.43)

Massenspektrum: $(M+H)^+ = 370$
$R_f$-Wert: 0.27 (Kieselgel; Methanol/Wasser/Eisessig = 17:3:0.55)

Beispiel 73

(Z)-1-Methyl-3-[1-(3-cyanophenylamino)-1-phenyl-methyliden]-2-indolinon

a) 1-Methyl-3-(1-hydroxy-1-1-phenyl-methyliden)-2-indolinon

4.15 g (41 mMol) Diisopropylamin werden in 50 ml THF vorgelegt, auf -70°C abgekühlt und mit einer Lösung von 14.4 ml (36 mMol) n-Butyllithiumlösung (2.5 Mol in Toluol) versetzt und 10 Minuten gerührt. Anschließend wird eine Lösung von 5.0 g (34 mMol) 1-Methyl-2-indolinon in 30 ml THF zugetropft und 45 Minuten bei -70°C gerührt. Danach werden 5.8 g (0.041 Mol) Benzoylchlorid zugetropft. Man beläßt die Reaktionslösung innerhalb 14 Stunden langsam erwärmen. Anschließend wird die Reaktionslösung auf Natriumchloridlösung gegossen und mit Essigester extrahiert. Die vereinigten organischen Extrakte werden getrocknet und eingedampft. Der Rückstand wird an Kieselgel (Dichlormethan/Methanol/Ammoniak = 200:8:1) chromatographiert.
Ausbeute: 7.1 g (84 % der Theorie),
Schmelzpunkt: 145-147°C

b) (Z)-1-Methyl-3-[1-(3-cyanophenylamino)-1-phenyl-methyliden]-2-indolinon

Hergestellt analog Beispiel 2 aus 1-Methyl-3-(1-hydroxy-1-phenyl-methyliden)-2-indolinon, Phosphorpentachlorid und 3-Aminobenzonitril.
Ausbeute: 15 % der Theorie,
Schmelzpunkt: 158-160°C
$C_{23}H_{17}N_3O$ (351.41)
Massenspektrum: $M^+ = 351$
$R_f$-Wert: 0.42 (Kieselgel; Dichlormethan/Essigester = 100:3)

| | | | |
|---|---|---|---|
| Ber. | C 78.61 | H 4.88 | N 11.96 |
| Gef. | 78.15 | 4.89 | 11.91 |

Beispiel 74

(Z)-3-[1-(4-Dimethylaminomethylcarbonylaminomethyl-phenylamino)-1-phenyl-methyliden}-2-indolinon

a) Isocyanatomethyl-Polystyrolharz

Man laßt 18.2 g (31.5 mMol) Aminomethyl-Polystyrolharz in 200 ml Toluol 45 Minuten bei Raumtemperatur quellen. Bei 5°C werden 16.6 ml (0.31 Mol) Phosgenlösung (20%ig in Toluol) zugegeben. Anschließend beläßt man die Reaktionslösung 100 Minuten im Ultraschallbad bei 20°C und erhitzt danach 4 Stunden zum Rückfluß. Nach 18 Stunden Stehen bei Raumtemperatur wird abgesaugt, mit Dichlormethan und Essigester gewaschen und getrocknet.
Ausbeute: 18.3 g (100 % der Theorie).

b) 1-Polystyrylmethylaminocarbonyl-2-indolinon

13.3 g (0.1 Mol) 2-Indolinon und 12.1 g (20.5 mMol) Isocyanatomethyl-Polystyrolharz werden in 400 ml Toluol 12 Stunden zum Rückfluß erhitzt. Danach wird abgekühlt, mit Toluol, Methylenchlorid und Methanol gewaschen und getrocknet.
Ausbeute: 13.4 g (100 % der Theorie).

c) 3-(1-Ethoxy-1-phenyl-methyliden)-1-polystyrylmethylaminocarbonyl-2-indolinon

13.4 g (20.5 mMol) 1-Polystyrylmethylaminocarbonyl-2-indolinon und 33.4 g (0.15 Mol) Orthobenzoesäuretriethylester werden in 200 ml Acetanhydrid 22 Stunden zum Rückfluß erhitzt. Danach wird abgekühlt, mit Essigester, Methylenchlorid und Methanol gewaschen und getrocknet.
Ausbeute: 14.3 g (100 % der Theorie).

d) 3-[1-(4-tert.Butoxycarbonylaminomethyl-phenylamino)-1-phenyl-methyliden]-1-polystyrylmethylaminocarbonyl-2-indolinon

710 mg (1 mMol) 3-(1-Ethoxy-1-phenyl-methyliden)-1-polystyrylmethylaminocarbonyl-2-indolinon werden in 15 ml DMF suspendiert und nach Zugabe von 1.1 g (5 mMol) 4-tert.Butoxycarbonylamino-anilin 11 Stunden auf 120°C erhitzt. Nach 14 Stunden bei Raumtemperatur wird abgesaugt, mit Dichlormethan und Methanol gewaschen und getrocknet.
Ausbeute: 770 mg (100 % der Theorie),

e) 3-[1-(4-Aminomethyl-phenylamino)-1-phenyl-methyliden]-1-polystyrylmethylaminocarbonyl-2-indolinon

770 mg (1 mMol) (3-[1-(4-tert.Butoxycarbonylaminomethyl-phenylamino)-1-phenyl-methyliden]-1-polystyrylmethylaminocarbonyl-2-indolinon werden in 10 ml Dichlormethan und 5 ml Trifluoressigsäure 2 Stunden im Ultraschallbad beschallt. Anschließend wird abgesaugt, mit Dichlormethan und Methanol gewaschen und getrocknet.
Ausbeute: 720 mg (100 % der Theorie),

f) 3-[1-(4-(Dimethylaminomethylcarbonylaminomethyl-phenylamino)-1-phenyl-methyliden]-1-polystyrylmethylaminocarbonyl-2-indolinon

680 mg (1.0 mMol) 3-[1-(4-Aminomethyl-phenylamino)-1-phenylmethyliden]-1-polystyrylmethylaminocarbonyl-2-indolinon, 1.6 g (5 mMol) TBTU, 770 mg (5 mMol) HOBt, 2.6 g (20 mMol) N-Ethyl-N,N-diisopropylamin und 515 mg (5 mMol) Dimethylglycin werden in 20 ml Dimethylformamid 6 Stunden im Ultraschallbad bei 35°C beschallt. Anschließend wird abgesaugt, mit Dichlormethan und Methanol gewaschen und getrocknet.
Ausbeute: 570 mg (100 % der Theorie),

g) (Z)-3-[1-(4-Dimethylaminomethylcarbonylaminomethyl-phenylamino)-1-phenyl-methyliden]-2-indolinon

560 mg (0.95 mMol) 3-[1-(4-(Dimethylaminomethylcarbonylaminomethyl-phenylamino)-1-phenyl-methyliden] -1-polystyrylmethylaminocarbonyl-2-indolinon werden in 20 ml Dioxan und 5 ml 1N Natronlauge 7 Stunden auf 90°C erwärmt. Danach wird abfiltriert und eingedampft. Der Rückstand wird in Dichlormethan/-Wasser verteilt, die organische Phase getrocknet und bis zur Trockene eingeengt. Das Rohprodukt wird mit Essigester und Ether verrieben, abgesaugt und getrocknet.
Ausbeute: 27 mg (7 % der Theorie),
Schmelzpunkt: 200-205°C
$C_{26}H_{26}N_4O_2$ (426.52)
Massenspektrum: $M^+$ = 426
$R_f$-Wert: 0.60 (Kieselgel; Dichlormethan/Methanol = 9:1)

Beispiel 75

(Z)-3-{1-[4-(2-Carboxy-ethylcarbonylaminomethyl)-phenylamino]-1-phenyl-methyliden}-2-indolinon

Hergestellt analog Beispiel 74 aus (Z)-3-{1-[4-(2-Carboxyethylcarbonylaminomethyl)-phenylamino]-1-phenyl-methyliden}-1-polystyrylmethylaminocarbonyl-2-indolinon und Natronlauge in Dioxan.
Ausbeute: 5 % der Theorie,
$C_{26}H_{23}N_3O_4$ (441.49)
Massenspektrum: $(M-H)^-$ = 440

Beispiel 76

(Z)-3-[1-(4-Methoxymethylcarbonylaminomethyl-phenylamino)-1-phenyl-methyliden]-2-indolinon

Hergestellt analog Beispiel 74 aus (Z)-3-[1-(4-Methoxymethylcarbonylaminomethyl-phenylamino)-1-phenyl-methyliden]-1-polystyrylmethylaminocarbonyl-2-indolinon und Natronlauge in Dioxan.
Ausbeute: 6 % der Theorie,
Schmelzpunkt: 178-180°C
$C_{25}H_{23}N_3O_3$ (413.48)
Massenspektrum: $M^+$ = 413

Beispiel 77

(Z)-3-[1-(4-Chlorphenylamino)-1-phenyl-methyliden]-5-nitro-2-indolinon

a) 1-Acetyl-5-nitro-2-indolinon

17.5 g (0.10 Mol) 1-Acetyl-2-indolinon werden in 100 ml konz. Schwefelsäure gelöst und bei -10°C portionsweise mit 8.8 g (0.11 Mol) Ammoniumnitrat versetzt und 15 Minuten gerührt. Die Reaktion wird auf Eiswasser gegossen, abgesaugt und mit Wasser gewaschen. Der Rückstand wird in Essigester/Wasser verteilt, die vereinigten organischen Extrakte getrocknet und eingedampft.
Ausbeute: 20.5 g (93 % der Theorie),
Schmelzpunkt: 154-156°C

b) 1-Acetyl-3-(1-methoxy-1-phenyl-methyliden)-5-nitro-2-indolinon

30.0 g (0.137 Mol) 1-Acetyl-5-nitro-2-indolinon werden in 200 ml Acetanhydrid gelöst und nach Zugabe von 50.0 g (0.274 Mol) Orthobenzoesäuretrimethylester 3 Stunden bei 100°C gerührt. Nach Abkühlung wird auf die Hälfte eingeengt, mit Ether/Petrolether verdünnt, der Niederschlag abgesaugt und getrocknet.
Ausbeute: 40.9 g (88 % der Theorie),
$R_f$-Wert : 0.61 (Kieselgel; Dichlormethan/Petrolether/Essigester = 4:5:1)

c) (Z)-3-[1-(4-Chlorphenylamino)-1-phenyl-methyliden]-5-nitro-2-indolinon

0.5 g (1.5 mMol) 1-Acetyl-3-(1-methoxy-1-phenyl-methyliden)-5-nitro-2-indolinon werden in 20 ml Dichlormethan gelöst und nach Zugabe von 0.57 g (4.5 mMol) 4-Chloranilin 72 Stunden bei Raumtemperatur gerührt. Anschließend werden 3 ml methanolisches Ammoniak zugesetzt und 48 Stunden gerührt. Nach Entfernen des Lösungsmittels im Vakuum wird der Rückstand mit Ether verrieben, abgesaugt und getrocknet.
Ausbeute: 150 mg (26 % der Theorie),
$C_{21}H_{14}ClN_3O_3$ (391.82)
Massenspektrum: $M^+$ = 393/391
$R_f$-Wert: 0.68 (Kieselgel; Dichlormethan/Methanol = 9:1)

Beispiel 78

(Z)-3-[1-(4-Methoxyphenylamino)-1-phenyl-methyliden]-5-nitro-2-indolinon

Hergestellt analog Beispiel 77 aus 1-Acetyl-3-(1-methoxy-1-phenyl-methyliden)-5-nitro-2-indolinon und 4-Methoxyanilin in Dichlormethan und methanolischem Ammoniak.
Ausbeute: 87 % der Theorie,
$C_{22}H_{17}N_3O_4$ (387.40)
Massenspektrum: $M^+$ = 387
$R_f$-Wert: 0.66 (Kieselgel; Dichlormethan/Methanol = 9:1)

Beispiel 79

(Z)-3-[1-(4-Triflourmethyl-phenylamino)-1-phenyl-methyliden]-5-nitro-2-indolinon

Hergestellt analog Beispiel 77 aus 1-Acetyl-3-(1-methoxy-1-phenyl-methyliden)-5-nitro-2-indolinon und Trifluormethylanisidin in Dichlormethan und anschließender Behandlung mit methanolischem Ammoniak.
Ausbeute: 62 % der Theorie,
$C_{22}H_{14}F_3N_3O_3$ (425.37)
Massenspektrum: $M^+$ = 425
$R_f$-Wert: 0.23 (Kieselgel; Dichlormethan)

Beispiel 80

(Z)-3-[1-(4-Morpholinophenylamino)-1-phenyl-methyliden]-5-nitro-2-indolinon

Hergestellt analog Beispiel 77 aus 1-Acetyl-3-(1-methoxy-1-phenyl-methyliden)-5-nitro-2-indolinon und 4-Morpholinoanilin in Dichlormethan und anschließender Behandlung mit methanolischem Ammoniak.
Ausbeute: 68 % der Theorie,
Schmelzpunkt: >300°C
$C_{25}H_{22}N_4O_4$ (442.48)
Massenspektrum: $M^+$ = 442
$R_f$-Wert: 0.56 (Kieselgel; Essigester/Cyclohexan/Methanol = 1:1:0.2)

Beispiel 81

(Z)-3-[1-(4-Nitrophenylamino)-1-phenyl-methyliden]-5-nitro-2-indolinon

Hergestellt analog Beispiel 77 aus 1-Acetyl-3-(1-methoxy-1-phenyl-methyliden)-5-nitro-2-indolinon und 4-Nitroanilin in DMF und anschließender Behandlung mit methanolischem Ammoniak.
Ausbeute: 38 % der Theorie,
$C_{21}H_{14}N_4O_5$ (402.37)
Massenspektrum: $M^+$ = 402
$R_f$-Wert: 0.65 (Kieselgel; Dichlormethan/Methanol 9:1)

Beispiel 82

(Z)-3-[1-(4-Bromphenylamino)-1-phenyl-methyliden]-5-nitro-2-indolinon

a) 1-Acetyl-3-(1-ethoxy-1-phenyl-methyliden)-5-nitro-2-indolinon

5.07 g (23 mMol) 5-Nitro-2-indolinon werden zusammen mit 15.5 g (69 mMol) Orthobenzoesäuretrietylester in 50 ml Acetanhydrid 2.5 Stunden bei 100°C gerührt. Nach dem Abkühlen werden 100 ml Ether/Petrolether (1:1) zugegeben. Der dabei ausfallende Niederschlag wird abgesaugt mit Ether/Petrolether (1:1) gewaschen und getrocknet.
Ausbeute: 6.6 g (81 % der Theorie),
Schmelzpunkt: 233-234°C

b) (Z)-3-[1-(4-Bromphenylamino)-1-phenyl-methyliden]-5-nitro-2-indolinon

Hergestellt analog Beispiel 77 aus 1-Acetyl-3-(1-ethoxy-1-phenyl-methyliden)-5-nitro-2-indolinon und 4-Bromanilin in DMF unter Erhitzen und anschließender Behandlung mit Piperidin.
Ausbeute: 92 % der Theorie,
Schmelzpunkt: 300-305°C
$C_{21}H_{14}BrN_3O_3$ (436.27)
Massenspektrum: $M^+$ = 437/435
$R_f$-Wert: 0.33 (Kieselgel; Dichlormethan/Methanol = 20:1)

| | | | | |
|---|---|---|---|---|
| Ber. | C 57.82 | H 3.23 | N 9.63 | Br 18.32 |
| Gef. | 57.81 | 3.20 | 9.65 | 18.22 |

Beispiel 83

(Z)-3-[1-(4-Cyanophenylamino)-1-phenyl-methyliden]-5-nitro-2-indolinon

Hergestellt analog Beispiel 77 aus 1-Benzoyl-3-(1-methoxy-1-phenyl-methyliden)-5-nitro-2-indolinon und 4-Aminobenzonitril in DMF und anschließende Behandlung mit methanolischem Ammoniak.
Ausbeute: 33 % der Theorie,
$C_{22}H_{14}N_4O_3$ (382.38)
Massenspektrum: $M^+$ = 382

$R_f$-Wert: 0.58 (Kieselgel; Dichlormethan/Methanol = 9:1)

Beispiel 84

(Z)-3-[1-(4-Amidinophenylamino)-1-phenyl-methyliden]-5-nitro-2-indolinon-hydrochlorid

Hergestellt analog Beispiel 77 aus 1-Acetyl-3-(1-methoxy-1-phenyl-methyliden)-5-nitro-2-indolinon und 4-Aminobenzamidin in DMF.
Ausbeute: 20 % der Theorie,
$C_{22}H_{17}N_5O_3$ (399.41)
Massenspektrum: $(M+H)^+$ = 400
$R_f$-Wert: 0.07 (Kieselgel; Dichlormethan/Methanol = 9:1)

Beispiel 85

(Z)-3-[1-(3-Cyanophenylamino)-1-phenyl-methyliden]-5-nitro-2-indolinon

2 g (5.2 mMol) 1-Benzoyl-3-(1-hydroxy-1-phenyl-methyliden)-5-nitro-2-indolinon und 1.8 g (16 mMol) 3-Aminobenzonitril werden 70 Stunden bei Raumtemperatur in DMF gerührt. Danach extrahiert man die Reaktionslösung mit Ether, wäscht die organische Phase mit Wasser und trocknet über Natriumsulfat. Nach Entfernen des Lösungsmittels im Vakuum wird der Rückstand an Kieselgel (Dichlormetan/Methanol = 50:1) chromatographiert.
Ausbeute: 580 mg (23 % der Theorie),
$C_{22}H_{14}N_4O_3$ (382.38)
Massenspektrum: $M^+$ = 382
$R_f$-Wert: 0.32 (Kieselgel; Dichlormethan/Methanol = 50:1)

Beispiel 86

(Z)-3-[1-(3-Amidinophenylamino)-1-phenyl-methyliden]-5-nitro-2-indolinon-hydrochlorid

Hergestellt analog Beispiel 77 aus 1-Acetyl-3-(1-methoxy-1-phenyl-methyliden)-5-nitro-2-indolinon und 3-Aminobenzamidin in DMF.
Ausbeute: 22 % der Theorie,
$C_{22}H_{17}N_5O_3$ (399.41)
Massenspektrum: $(M+H)^+$ = 400
$R_f$-Wert: 0.17 (Kieselgel; Dichlormethan/Methanol = 4:1)

Beispiel 87

(Z)-3-[1-(4-Methoxycarbonyl-phenylamino)-1-phenyl-methyliden]-5-nitro-2-indolinon

Hergestellt analog Beispiel 77 aus 1-Acetyl-3-(1-methoxy-1-phenyl-methyliden)-5-nitro-2-indolinon und 4-Aminobenzoesäuremethylester in Dichlormethan und anschließende Behandlung mit methanolischem Ammoniak.
Ausbeute: 10 % der Theorie,
$C_{23}H_{17}N_3O_5$ (415.41)
Massenspektrum: $M^+$ = 415
$R_f$-Wert: 0.23 (Kieselgel; Dichlormethan/Methanol = 50:1)

Beispiel 88

(Z)-3-[1-(4-Carboxy-phenylamino)-1-phenyl-methyliden]-5-nitro-2-indolinon

Hergestellt analog Beispiel 8 aus (Z)-3-[1-(4-Methoxycarbonylphenylamino)-1-phenyl-methyliden]-5-nitro-2-indolinon und Natronlauge in Methanol.
Ausbeute: 88 % der Theorie,
$C_{22}H_{15}N_3O_5$ (401.38)
Massenspektrum: $M^+$ = 401
$R_f$-Wert: 0.52 (Kieselgel; Dichlormethan/Methanol = 9:1)

Beispiel 89

(Z)-3-[1-(3-Acetylamino-phenylamino)-1-phenyl-methyliden]-5-nitro-2-indolinon

a) 3-(1-Ethoxy-1-phenyl-methyliden)-5-nitro-2-indolinon

17.6 g (50 mMol) 1-Acetyl-3-(1-ethoxy-1-phenyl-methyliden)-5-nitro-2-indolinon werden in 200 ml Dichlormethan und 150 ml Ethanol suspendiert. Man gibt 75 ml 1N Natronlauge bei 0°C zu und rührt anschließend noch 30 Minuten bei Raumtemperatur. Man engt die Reaktionslösung auf die Hälfte ein und setzt anschließend 200 ml Wasser zu. Das ausgefallene Produkt wird abgesaugt, mit Wasser, Isopropanol und Ether gewaschen und getrocknet.
Ausbeute: 13.3 g (86 % der Theorie),
Schmelzpunkt: 239-240°C

b) (Z)-3-[1-(3-Acetylamino-phenylamino)-1-phenyl-methyliden]-5-nitro-2-indolinon

Hergestellt analog Beispiel 82 aus 3-(1-Ethoxy-1-phenyl-methyliden)-5-nitro-2-indolinon und 3-Acetylamino-anilin in DMF.
Ausbeute: 72 % der Theorie,
Schmelzpunkt: 318-320°C (Zers.)
$C_{23}H_{18}N_4O_4$ (414.42)
Massenspektrum: $M^+$ = 414

| | | | |
|---|---|---|---|
| Ber. | C 66.66 | H 4.38 | N 13.52 |
| Gef. | 66.42 | 4.46 | 13.45 |

Beispiel 90

(Z)-3-[1-(4-tert.Butoxycarbonylamino-phenylamino)-1-phenyl-methyliden]-5-nitro-2-indolinon

Hergestellt analog Beispiel 82 aus 1-Acetyl-3-(1-methoxy-1-phenyl-methyliden)-5-nitro-2-indolinon und 4-tert. Butoxycarbonylamino-anilin in DMF und anschließende Behandlung mit Natronlauge in Methanol.
Ausbeute: 56 % der Theorie,
Schmelzpunkt: 235-237°C (Zers.)
$C_{26}H_{24}N_4O_5$ (472.51)
Massenspektrum: $M^+$ = 472

| | | | |
|---|---|---|---|
| Ber. | C 66.09 | H 5.12 | N 11.86 |
| Gef. | 66.35 | 5.19 | 11.80 |

Beispiel 91

(Z)-3-[1-(4-Aminophenylamino)-1-phenyl-methyliden]-5-nitro-2-indolinon

Hergestellt analog Beispiel 29a aus (Z)-3-[1-(4-tert.Butoxycarbonylamino-phenylamino)-1-phenyl-methyliden]-5-nitro-2-indolinon und Essigester/Chlorwasserstoff in Dichlormethan.
Ausbeute: 74 % der Theorie,
Schmelzpunkt: 269°C
$C_{21}H_{16}N_4O_3$ (372.39)
Massenspektrum: $M^+$ = 372

| | | | |
|---|---|---|---|
| Ber. | C 67.73 | H 4.33 | N 15.05 |
| Gef. | 67.70 | 4.48 | 14.83 |

Beispiel 92

(Z)-3-[1-(4-Formylamino-phenylamino)-1-phenyl-methyliden]-5-nitro-2-indolinon

Hergestellt analog Beispiel 29b aus (Z)-3-[1-(4-Aminophenylamino)-1-phenyl-methyliden}-5-nitro-2-indolinon und Ameisensäureethylester in DMF.
Ausbeute: 89 % der Theorie,
Schmelzpunkt: 355-356°C (Zers.)
$C_{22}H_{16}N_4O_4$ (400.40)
Massenspektrum: $M^+ = 400$

| | | | |
|---|---|---|---|
| Ber. | C 66.00 | H 4.03 | N 13.99 |
| Gef. | 65.59 | 4.13 | 13.85 |

Beispiel 93

(Z)-3-[1-(4-Acetylamino-phenylamino)-1-phenyl-methyliden]-5-nitro-2-indolinon

Hergestellt analog Beispiel 31 aus (Z)-3-[1-(4-Aminophenylamino)-1-phenyl-methyliden]-5-nitro-2-indolinon und Acetanhydrid in Eisessig.
Ausbeute: 93 % der Theorie,
Schmelzpunkt: 328-330°C
$C_{23}H_{18}N_4O_4$ (414.42)
Massenspektrum: $M^+ = 414$
$C_{23}H_{18}N_4O_4 \times H_2O$ (432.44)

| | | | |
|---|---|---|---|
| Ber. | C 63.88 | H 4.66 | N 12.96 |
| Gef. | 64.09 | 4.68 | 12.34 |

Beispiel 94

(Z)-3-[1-(4-Dimethylaminomethylcarbonylamino-phenylamino)-1-phenyl-methyliden]-5-nitro-2-indolinon

Hergestellt analog Beispiel 82 aus 1-Acetyl-3-(1-ethoxy-1-phenyl-methyliden)-5-nitro-2-indolinon und 4-Dimethylaminomethylcarbonylamino-anilin in DMF und anschließende Behandlung mit Natronlauge in Methanol.
Ausbeute: 63 % der Theorie,
Schmelzpunkt: 254-257°C
$C_{25}H_{23}N_5O_4$ (457.49)
Massenspektrum: $M^+ = 457$

| | | | |
|---|---|---|---|
| Ber. | C 65.64 | H 5.07 | N 15.31 |
| Gef. | 65.20 | 5.16 | 14.99 |

Beispiel 95

(Z)-3-[1-(4-Diethylaminomethylcarbonylamino-phenylamino)-1-phenyl-methyliden]-5-nitro-2-indolinon-hydrochlorid

Hergestellt analog Beispiel 82 aus 1-Acetyl-3-(1-ethoxy-1-phenyl-methyliden)-5-nitro-2-indolinon und 4-Diethylaminomethylcarbonylamino-anilin in DMF und anschließende Behandlung mit Natronlauge in Methanol.
Ausbeute: 54 % der Theorie,
Schmelzpunkt: 287-288
$C_{27}H_{27}N_5O_4$ (485.55)
Massenspektrum: $M^+ = 485$

Beispiel 96

(Z)-3-[1-(4-Morpholinomethylcarbonylamino-phenylamino)-1-phenyl-methyliden]-5-nitro-2-indolinon

Hergestellt analog Beispiel 82 aus 1-Acetyl-3-(1-ethoxy-1-phenyl-methyliden)-5-nitro-2-indolinon und 4-Morpholinomethylcarbonylamino-anilin in DMF und anschließende Behandlung mit Natronlauge in Methanol.
Ausbeute: 88 % der Theorie,
Schmelzpunkt: 265-267°C
$C_{27}H_{25}N_5O_5$ (499.53)
Massenspektrum: $M^+$ = 499
$C_{27}H_{25}N_5O_5 \times H_2O$ (517.55)

| | | | |
|---|---|---|---|
| Ber. | C 62.60 | H 5.26 | N 13.53 |
| Gef. | 62.68 | 5.15 | 13.57 |

Beispiel 97

(Z)-3-{1-[4-(4-Methylpiperazinomethylcarbonylamino)-phenylamino]-1-phenyl-methyliden}-5-nitro-2-indolinon

Hergestellt analog Beispiel 82 aus 1-Acetyl-3-(1-ethoxy-1-phenyl-methyliden)-5-nitro-2-indolinon und 4-(4-Methylpiperazinomethylcarbonylamino)-anilin in DMF und anschließende Behandlung mit Natronlauge in Methanol.
Ausbeute: 74 % der Theorie,
Schmelzpunkt: 232-233°C
$C_{28}H_{28}N_6O_4$ (512.57)
Massenspektrum: $M^+$ = 512

Beispiel 98

(Z)-3-{1-[4-(N-Acetyl-N-methyl-amino)-phenylamino]-1-phenylmethyliden}-5-nitro-2-indolinon

Hergestellt analog Beispiel 82 aus 1-Acetyl-3-(1-ethoxy-1-phenyl-methyliden)-5-nitro-2-indolinon und 4-(N-Acetyl-N-methylamino)-anilin in DMF und anschließende Behandlung mit Natronlauge in Methanol.
Ausbeute: 82 % der Theorie,
Schmelzpunkt: 305-307°C
$C_{24}H_{20}N_4O_4$ (428.45)
Massenspektrum: $M^+$ = 428

| | | | |
|---|---|---|---|
| Ber. | C 67.28 | H 4.71 | N 13.08 |
| Gef. | 67.05 | 4.76 | 12.94 |

Beispiel 99

(Z)-3-{1-[4-(N-Dimethylaminomethylcarbonyl-N-methyl-amino)-1-phenylamino]-1-phenyl-methyliden}-5-nitro-2-indolinon

Hergestellt analog Beispiel 82 aus 1-Acetyl-3-(1-ethoxy-1-phenyl-methyliden)-5-nitro-2-indolinon und 4-(N-Dimethylaminomethylcarbonyl-N-methyl-amino)-anilin in DMF und anschließende Behandlung mit Natronlauge in Methanol.
Ausbeute: 91 % der Theorie,
Schmelzpunkt: 295-297°C
$C_{26}H_{25}N_5O_4$ (471.52)
Massenspektrum: $M^+$ = 471
$C_{26}H_{25}N_5O_4 \times 0.5\ H_2O$ (480.5)

| | | | |
|---|---|---|---|
| Ber. | C 64.99 | H 5.45 | N 14.57 |

(fortgesetzt)

| | | | |
|---|---|---|---|
| Gef. | 64.49 | 5.51 | 14.45 |

Beispiel 100

(Z)-3-{[4-(N-Diethylaminomethylcarbonyl-N-methyl-amino)-1-phenylamino]-1-phenyl-methyliden}-5-nitro-2-indolinon

Hergestellt analog Beispiel 82 aus 1-Acetyl-3-(1-ethoxy-1-phenyl-methyliden)-5-nitro-2-indolinon und 4-(N-Diethylaminomethylcarbonyl-N-methyl-amino)-anilin in DMF und anschließende Behandlung mit Natronlauge in Methanol.
Ausbeute: 40 % der Theorie,
Schmelzpunkt: 225°C
$C_{28}H_{29}N_5O_4$ (499.57)
Massenspektrum: $M^+$ = 499

| | | | |
|---|---|---|---|
| Ber. | C 67.37 | H 5.85 | N 14.02 |
| Gef. | 66.99 | 5.88 | 13.98 |

Beispiel 101

(Z)-3-{1-[4-(N-Piperidinomethylcarbonyl-N-methyl-amino)-1-phenylamino]-1-phenyl-methyliden}-5-nitro-2-indolinon

Hergestellt analog Beispiel 82 aus 1-Acetyl-3-(1-ethoxy-1-phenyl-methyliden)-5-nitro-2-indolinon und 4-(N-Piperidinomethylcarbonyl-N-methyl-amino)-anilin in DMF und anschließende Behandlung mit Natronlauge in Methanol.
Ausbeute: 80 % der Theorie,
Schmelzpunkt: 267-269°C
$C_{29}H_{29}N_5O_4$ (511.59)
Massenspektrum: $M^+$ = 511
$R_f$-Wert: 0.55 (Kieselgel; Dichlormethan/Methanol/$NH_4OH$ = 9:1:0.1)

| | | | |
|---|---|---|---|
| Ber. | C 68.09 | H 5.71 | N 13.69 |
| Gef. | 67.29 | 5.58 | 13.50 |

Beispiel 102

(Z)-3-{1-[4-(N-Morpholinomethylcarbonyl-N-methyl-amino)-phenylamino]-1-phenyl-methyliden}-5-nitro-2-indolinon

Hergestellt analog Beispiel 82 aus 1-Acetyl-3-(1-ethoxy-1-phenyl-methyliden)-5-nitro-2-indolinon und 4-(N-Morpholinomethylcarbonyl-N-methyl-amino)-anilin in DMF und anschließende Behandlung mit Natronlauge in Methanol.
Ausbeute: 58 % der Theorie,
Schmelzpunkt: 293-295°C
$C_{28}H_{27}N_5O_5$ (513.56)
Massenspektrum: $M^+$ = 513

| | | | |
|---|---|---|---|
| Ber. | C 64.49 | H 5.30 | N 13.64 |
| Gef. | 64.54 | 5.25 | 13.50 |

Beispiel 103

(Z)-3-{1-[4-(N-(N-Methylpiperazinomethylcarbonyl)-N-methylamino)-phenylamino]-1-phenyl-methyliden}-5-nitro-2-indolinon

Hergestellt analog Beispiel 82 aus 1-Acetyl-3-(1-ethoxy-1-phenyl-methyliden)-5-nitro-2-indolinon und

4-[N-(N-Methylpiperazinomethylcarbonyl)-N-methyl-amino]-anilin in DMF und anschließende Behandlung mit Natronlauge in Methanol.
Ausbeute: 76 % der Theorie,
Schmelzpunkt: 239-241°C
$C_{29}H_{30}N_6O_4$ (526.60)
Massenspektrum: $M^+$ = 526
$R_f$-Wert : 0.36 (Kieselgel; Dichlormethan/Methanol/$NH_4OH$ 9:1:0.1) $C_{29}H_{30}N_6O_4$ x $H_2O$ (544.61)

| | | | |
|---|---|---|---|
| Ber. | C 63.96 | H 5.92 | N 15.43 |
| Gef. | 63.81 | 5.95 | 15.35 |

Beispiel 104

(Z)-3-{1-[4-(N-(4-Benzylpiperazinomethylcarbonyl)-N-methylamino)-phenylamino]-1-phenyl-methyliden}-5-nitro-2-indolinon

Hergestellt analog Beispiel 82 aus 1-Acetyl-3-(1-ethoxy-1-phenyl-methyliden)-5-nitro-2-indolinon und 4-[N-(4-Benzylpiperazinomethylcarbonyl)-N-methyl-amino)-anilin in DMF und anschließende Behandlung mit Natronlauge in Methanol.
Ausbeute: 78 % der Theorie,
Schmelzpunkt: 201-203°C
$C_{35}H_{34}N_6O_4$ (602.70)
Massenspektrum: $M^+$ = 602
$R_f$-Wert: 0.6 (Kieselgel; Dichlormethan/Methanol/$NH_4OH$ = 9:1:0.1)
$C_{35}H_{34}N_6O_4$ x 0.5 $H_2O$ (611.70)

| | | | |
|---|---|---|---|
| Ber. | C 69.75 | H 5.69 | N 13.94 |
| Gef. | 68.73 | 5.69 | 13.52 |

Beispiel 105

(Z)-3-{1-[4-(N-Piperazinomethylcarbonyl-N-methyl-amino)phenylamino]-1-phenyl-methyliden}-5-nitro-2-indolinon-dihydrochlorid

Hergestellt analog Beispiel 54 aus (Z)-3-{1-[4-(N-(4-Benzylpiperazinomethylcarbonyl)-N-methyl-amino)-phenylamino]-1-phenylmethyliden}-5-nitro-2-indolinon und Chlorameisensäure-1-chlorethylester in Dichlormethan.
Ausbeute: 68 % der Theorie,
Schmelzpunkt: 246-248°C
$C_{28}H_{28}N_6O_4$ (512.57)
Massenspektrum: $M^+$ = 512
$C_{28}H_{28}N_6O_4$ x 2 HCl (585.50)

| | | | |
|---|---|---|---|
| Ber. | C 57.44 | H 5.16 | N 14.35 |
| Gef. | 57.00 | 4.87 | 14.09 |

Beispiel 106

(Z)-3-[1-(3-Dimethylaminomethylcarbonylaminomethyl-phenylamino)-1-phenyl-methyliden]-5-nitro-2-indolinon

Hergestellt analog Beispiel 82 aus 1-Acetyl-3-(1-ethoxy-1-phenyl-methyliden)-5-nitro-2-indolinon und 3-Dimethylaminomethylcarbonylaminomethyl-anilin in DMF und anschließende Behandlung mit Natronlauge in Methanol.
Ausbeute: 64 % der Theorie,
Schmelzpunkt: 171-173°C
$C_{26}H_{25}N_5O_4$ (471.52)
Massenspektrum: $M^+$ = 471

| | | | |
|---|---|---|---|
| Ber. | C 66.23 | H 5.34 | N 14.85 |
| Gef. | 65.97 | 5.18 | 14.79 |

Beispiel 107

(Z)-3-[1-(3-Dimethylaminomethyl-phenylamino)-1-phenyl-methyliden]-5-nitro-2-indolinon

Hergestellt analog Beispiel 82 aus 1-Acetyl-3-(1-ethoxy-1-phenyl-methyliden)-5-nitro-2-indolinon und 3-Dimethylaminomethyl-anilin in DMF und anschließende Behandlung mit Natronlauge in Methanol.
Ausbeute: 85 % der Theorie,
Schmelzpunkt: 214-217°C
$C_{24}H_{22}N_4O_3$ (414.47)
Massenspektrum: $M^+ = 414$
$R_f$-Wert: 0.48 (Kieselgel; Dichlormethan/Methanol/$NH_4OH$ = 9:1:0.1)

| | | | |
|---|---|---|---|
| Ber. | C 69.55 | H 5.35 | N 13.52 |
| Gef. | 69.55 | 5.45 | 13.38 |

Beispiel 108

(Z)-3-[1-(3-Piperidinomethyl-phenylamino)-1-phenyl-methyliden]-5-nitro-2-indolinon

Hergestellt analog Beispiel 82 aus 1-Acetyl-3-(1-ethoxy-1-phenyl-methyliden)-5-nitro-2-indolinon und 3-Piperidinomethyl-anilin in DMF und anschließende Behandlung mit Natronlauge in Methanol.
Ausbeute: 95 % der Theorie,
Schmelzpunkt: 214-215°C
$C_{27}H_{26}N_4O_3$ (454.53)
Massenspektrum: $M^+ = 454$

| | | | |
|---|---|---|---|
| Ber. | C 71.35 | H 5.77 | N 12.33 |
| Gef. | 70.85 | 5.79 | 12.28 |

Beispiel 109

(Z)-3-[1-(3-Morpholinomethyl-phenylamino)-1-phenyl-methyliden]-5-nitro-2-indolinon

Hergestellt analog Beispiel 82 aus 1-Acetyl-3-(1-ethoxy-1-phenyl-methyliden)-5-nitro-2-indolinon und 3-Morpholinomethyl-anilin in DMF und anschließende Behandlung mit Natronlauge in Methanol.
Ausbeute: 88 % der Theorie,
Schmelzpunkt: 272-275°C
$C_{26}H_{24}N_4O_4$ (456.51)
Massenspektrum: $M^+ = 456$

| | | | |
|---|---|---|---|
| Ber. | C 68.41 | H 5.30 | N 12.27 |
| Gef. | 68.05 | 5.21 | 12.23 |

Beispiel 110

(Z)-3-{1-[3-(4-Methylpiperazinomethyl)-phenylamino]-1-phenylmethyliden}-5-nitro-2-indolinon

Hergestellt analog Beispiel 82 aus 1-Acetyl-3-(1-ethoxy-1-phenyl-methyliden)-5-nitro-2-indolinon und 3-(4-Methylpiperazinomethyl)-anilin in DMF und anschließende Behandlung mit Natronlauge in Methanol.
Ausbeute: 92 % der Theorie,
Schmelzpunkt: 256-258°C
$C_{27}H_{27}N_5O_3$ (469.55)

Massenspektrum: $M^+$ = 469
$R_f$-Wert: 0.59 (Kieselgel; Dichlormethan/Methanol/$NH_4OH$ = 9:1:0.1)

| | | | |
|---|---|---|---|
| Ber. | C 69.07 | H 5.80 | N 14.92 |
| Gef. | 68.86 | 5.78 | 14.96 |

Beispiel 111

(Z)-3-[1-(3-Ethoxycarbonylmethylaminomethyl-phenylamino)-1-phenyl-methyliden]-5-nitro-2-indolinon

Hergestellt analog Beispiel 89 aus 3-(1-Ethoxy-1-phenyl-methyliden)-5-nitro-2-indolinon und 3-Ethoxycarbonylmethylaminomethyl-anilin in DMF.
Ausbeute: 38 % der Theorie,
Schmelzpunkt: 130-133°C
$C_{26}H_{24}N_4O_5$ (472.51)
Massenspektrum: $M^+$ = 476

| | | | |
|---|---|---|---|
| Ber. | C 66.09 | H 5.12 | N 11.86 |
| Gef. | 66.46 | 5.32 | 11.80 |

Beispiel 112

(Z)-3-{1-[3-(2-Ethoxycarbonyl-ethylaminomethyl)-phenylamino]-1-phenyl-methyliden}-5-nitro-2-indolinon

Hergestellt analog Beispiel 89 aus 3-(1-Ethoxy-1-phenyl-methyliden)-5-nitro-2-indolinon und 3-(2-Ethoxycarbonyl-ethylaminomethyl) -anilin in DMF.
Ausbeute: 70 % der Theorie,
Schmelzpunkt: 142-145°C
$C_{27}H_{26}N_4O_5$ (486.53)
Massenspektrum: $M^+$ = 486

| | | | |
|---|---|---|---|
| Ber. | C 66.66 | H 5.39 | N 11.52 |
| Gef. | 66.44 | 5.49 | 11.43 |

Beispiel 113

(Z)-3-[1-(4-tert.Butoxycarbonylaminomethyl-phenylamino)-1-phenyl-methyliden]-5-nitro-2-indolinon

Hergestellt analog Beispiel 82 aus 1-Acetyl-3-(1-ethoxy-1-phenyl-methyliden)-5-nitro-2-indolinon und 4-tert. Butoxycarbonylaminomethyl-anilin in DMF und anschließende Behandlung mit Natronlauge in Methanol.
Ausbeute: 89 % der Theorie,
Schmelzpunkt: 234-236°C (Zers.)
$C_{27}H_{26}N_4O_5$ (486.53)
Massenspektrum: $M^+$ = 486

| | | | |
|---|---|---|---|
| Ber. | C 66.66 | H 5.39 | N 11.52 |
| Gef. | 66.98 | 5.44 | 11.42 |

Beispiel 114

(Z)-3-[1-(4-Aminomethyl-phenylamino]-1-phenyl-methyliden]-5-nitro-2-indolinon-hydrochlorid

Hergestellt analog Beispiel 29a aus (Z)-3-[1-(4-tert.Butoxycarbonylaminomethyl-phenylamino)-1-phenyl-methyliden]-5-nitro-2-indolinon und Essigester/Chlorwasserstoff.
Ausbeute: 86 % der Theorie,
Schmelzpunkt: >370°C

$C_{22}H_{18}N_4O_3$ (386.41)
Massenspektrum: $M^+$ = 386
$C_{22}H_{18}N_4O_3$ x HCl x $H_2O$ (440.89)

| | | | |
|---|---|---|---|
| Ber. | C 59.93 | H 4.80 | N 12.71 |
| Gef. | 60.81 | 4.66 | 12.80 |

Beispiel 115

(Z)-3-[1-(4-Aminomethylcarbonylaminomethyl-phenylamino]-1-phenyl-methyliden}-5-nitro-2-indolinon-hydrochlorid

Hergestellt analog Beispiel 29a aus (Z)-3-[1-(4-tert.Butoxycarbonylaminomethylcarbonylaminomethyl-phenylamino)-1-phenylmethyliden]-5-nitro-2-indolinon und Essigester/Chlorwasserstoff.
Ausbeute: 76 % der Theorie,
Schmelzpunkt: 225-228°C
$C_{24}H_{21}N_5O_4$ (443.47)
Massenspektrum: $M^+$ = 443
$C_{24}H_{21}N_5O_4$ x HCl x 1.5 $H_2O$ (506.95)

| | | | |
|---|---|---|---|
| Ber. | C 56.86 | H 4.97 | N 13.81 |
| Gef. | 56.71 | 4.91 | 13.57 |

Beispiel 116

(Z)-3-[1-(4-Methylaminomethylcarbonylaminomethyl-phenylamino]-1-phenyl-methyliden}-5-nitro-2-indolinon-hydrochlorid

Hergestellt analog Beispiel 29a aus (Z)-3-{1-[4-(N-tert.Butoxycarbonyl-N-methyl-amino)methylcarbonylaminomethyl-phenylamino]-1-phenyl-methyliden}-5-nitro-2-indolinon und Essigester/Chlorwasserstoff.
Ausbeute: 76 % der Theorie,
Schmelzpunkt: 195-198°C
$C_{25}H_{23}N_5O_4$ (457.49)
Massenspektrum: $M^+$ = 457
$C_{25}H_{23}N_5O_4$ x HCl x $H_2O$ (511.97)

| | | | |
|---|---|---|---|
| Ber. | C 58.65 | H 5.12 | N 13.68 |
| Gef. | 58.19 | 4.96 | 13.49 |

Beispiel 117

(Z)-3-[1-(4-Dimethylaminomethyl-phenylamino)-1-phenyl-methyliden]-5-nitro-2-indolinon

Hergestellt analog Beispiel 82 aus 1-Acetyl-3-(1-ethoxy-1-phenyl-methyliden)-5-nitro-2-indolinon und 4-Dimethylaminomethyl-anilin in DMF und anschließende Behandlung mit Natronlauge in Methanol.
Ausbeute: 73 % der Theorie,
Schmelzpunkt: 264-265°C
$C_{24}H_{22}N_4O_3$ (414.47)
Massenspektrum: $M^+$ = 414

| | | | |
|---|---|---|---|
| Ber. | C 69.55 | H 5.35 | N 13.52 |
| Gef. | 69.29 | 5.31 | 13.33 |

Beispiel 118

(Z)-3-[1-(4-Morpholinomethyl-phenylamino)-1-phenyl-methyliden]-5-nitro-2-indolinon

Hergestellt analog Beispiel 82 aus 1-Acetyl-3-(1-ethoxy-1-phenyl-methyliden)-5-nitro-2-indolinon und 4-Morpholinomethyl-anilin in DMF und anschließende Behandlung mit Natronlauge in Methanol.
Ausbeute: 57 % der Theorie,
Schmelzpunkt: 273°C
$C_{26}H_{24}N_4O_4$ (456.51)
Massenspektrum: $M^+ = 456$
$R_f$-Wert: 0.43 (Kieselgel; Essigester/Methanol = 9:1)
$C_{26}H_{24}N_4O_4 \times H_2O$ (474.52)

| | | | |
|---|---|---|---|
| Ber. | C 65.81 | H 5.52 | N 11.81 |
| Gef. | 65.24 | 5.44 | 11.62 |

Beispiel 119

(Z)-3-[1-(4-Hexamethyleniminomethyl-phenylamino)-1-phenyl-methyliden]-5-nitro-2-indolinon

Hergestellt analog Beispiel 89 aus 3-(1-Ethoxy-1-phenyl-methyliden)-5-nitro-2-indolinon und 4-Hexamethyleniminomethylanilin in DMF.
Ausbeute: 64 % der Theorie,
Schmelzpunkt: 220°C
$C_{28}H_{28}N_4O_3$ (468.56)
Massenspektrum: $M^+ = 468$
$R_f$-Wert: 0.25 (Kieselgel; Essigester/Methanol = 8:2)

| | | | |
|---|---|---|---|
| Ber. | C 71.78 | H 6.02 | N 11.96 |
| Gef. | 71.57 | 6.12 | 11.71 |

Beispiel 120

(Z)-3-{1-[4-(N-tert.Butoxycarbonyl-N-methyl-aminomethyl)-phenylamino]-1-phenyl-methyliden}-5-nitro-2-indolinon

Hergestellt analog Beispiel 89 aus 3-(1-Ethoxy-1-phenyl-methyliden)-5-nitro-2-indolinon und 4-(N-tert.Butoxycarbonyl-N-methyl-amino)methyl-anilin in DMF.
Ausbeute: 60 % der Theorie,
Schmelzpunkt: 235
$C_{28}H_{28}N_4O_5$ (500.56)
Massenspektrum: $M^+ = 500$
$R_f$-Wert: 0.50 (Kieselgel; Dichlormethan/Essigester = 7:3)

| | | | |
|---|---|---|---|
| Ber. | C 67.19 | H 5.64 | N 11.19 |
| Gef.: | 66.95 | 5.68 | 11.00 |

Beispiel 121

(Z)-3-[1-(4-Methylaminomethyl-phenylamino)-1-phenyl-methyliden]-5-nitro-2-indolinon-hydrochlorid

Hergestellt analog Beispiel 29a aus (Z)-3-{1-[4-(N-tert.Butoxycarbonyl-N-methyl-amino)methyl-phenylamino]-1-phenyl-methyliden}-5-nitro-2-indolinon und Essigester/Chlorwasserstoff.
Ausbeute: 99 % der Theorie,
Schmelzpunkt: 351°C
$C_{23}H_{20}N_4O_3$ (400.44)
Massenspektrum: $M^+ = 400$

$R_f$-Wert: 0.36 (Kieselgel; Dichlormethan/Methanol/$NH_4OH$ = 9:1:0.1)
$C_{23}H_{20}N_4O_3$ x HCl (436.91)

| | | | |
|---|---|---|---|
| Ber. | C 63.23 | H 4.84 | N 12.82 |
| Gef. | 62.37 | 4.78 | 12.47 |

Beispiel 122

(Z) -3-{1-[4-(N-Acetyl-N-methyl-aminomethyl)-phenylamino]-1-phenyl-methyliden}-5-nitro-2-indolinon

Hergestellt analog Beispiel 31 aus (Z)-3-[1-(4-Methylaminomethyl-phenylamino)-1-phenyl-methyliden]-5-nitro-2-indolinon und Acetanhydrid in Eisessig.
Ausbeute: 79 % der Theorie,
Schmelzpunkt: 307°C
$C_{25}H_{22}N_4O_4$ (442.48)
Massenspektrum: $M^+$ = 442
$R_f$-Wert: 0.46 (Kieselgel; Dichlormethan/Methanol = 9:1)

Beispiel 123

(Z,S)-3-{1-[4-(1-tert.Butoxycarbonylamino-ethyl)-phenylamino]-1-phenyl-methyliden}-5-nitro-2-indolinon

Hergestellt analog Beispiel 89 aus 3-(1-Ethoxy-1-phenyl-methyliden)-5-nitro-2-indolinon und (S)-4-(1-tert.butoxycarbonylamino-ethyl)-anilin in DMF.
Ausbeute: 66 % der Theorie,
Schmelzpunkt: 247-249°C (Zers.)
$C_{28}H_{28}N_4O_5$ (500.56)
Massenspektrum: $M^+$ = 500

| | | | |
|---|---|---|---|
| Ber. | C 67.19 | H 5.64 | N 11.19 |
| Gef. | 67.23 | 5.56 | 11.28 |

Beispiel 124

(Z,S)-3-{1-[4-(1-Aminoethyl)-phenylamino]-1-phenyl-methyliden}-5-nitro-2-indolinon-hydrochlorid

Hergestellt analog Beispiel 29a aus (Z,S)-3-{1-[4-(1-tert.Butoxycarbonylamino-ethyl)-phenylamino]-1-phenyl-methyliden}-5-nitro-2-indolinon und Essigester/Chlorwasserstoff.
Ausbeute: 88 % der Theorie,
Schmelzpunkt: 230-235°C
$C_{23}H_{20}N_4O_3$ (400.44)
Massenspektrum: $M^+$ = 400
$C_{23}H_{20}N_4O_3$ x HCl x $H_2O$ (454.92)

| | | | |
|---|---|---|---|
| Ber. | C 60.73 | H 5.10 | N 12.32 |
| Gef. | 60.50 | 5.09 | 12.26 |

Beispiel 125

(Z,R)-3-{1-[4-(1-tert.Butoxycarbonylamino-ethyl)-phenylamino]-1-phenyl-methyliden}-5-nitro-2-indolinon

Hergestellt analog Beispiel 89 aus 3-(1-Ethoxy-1-phenyl-methyliden)-5-nitro-2-indolinon und (R)-4-(1-tert.Butoxycarbonylamino-ethyl)-anilin in DMF.
Ausbeute: 88 % der Theorie,
Schmelzpunkt: 247-249°C
$C_{28}H_{28}N_4O_5$ (500.56)

Massenspektrum: $M^+$ = 500

| | | | |
|---|---|---|---|
| Ber. | C 67.19 | H 5.64 | N 11.19 |
| Gef. | 67.38 | 5.69 | 11.25 |

Beispiel 126

(Z,R)-3-{1-[4-(1-Aminoethyl)-phenylamino]-1-phenyl-methyliden}-5-nitro-2-indolinon-hydrochlorid

Hergestellt analog Beispiel 29a aus (Z,R)-3-{1-[4-(1-tert.Butoxycarbonylamino-ethyl)-phenylamino]-1-phenyl-methyliden}-5-nitro-2-indolinon und Essigester/Chlorwasserstoff.
Ausbeute: 91 % der Theorie,
Schmelzpunkt: 230-235°C
$C_{23}H_{20}N_4O_3$ (400.44)
Massenspektrum: $M^+$ = 400
$C_{23}H_{20}N_4O_3$ x HCl x $H_2O$ (454.92)

| | | | |
|---|---|---|---|
| Ber. | C 60.73 | H 5.10 | N 12.32 |
| Gef. | 60.87 | 5.12 | 12.35 |

Beispiel 127

(Z)-3-{1-[4-(2-tert.Butoxycarbonylamino-ethyl)-phenylamino]-1-phenyl-methyliden}-5-nitro-2-indolinon

Hergestellt analog Beispiel 82 aus 1-Acetyl-3-(1-ethoxy-1-phenyl-methyliden)-5-nitro-2-indolinon und 4-(1-tert.Butoxycarbonylamino-ethyl)-anilin in DMF und anschließende Behandlung mit Natronlauge in Methanol.
Ausbeute: 92 % der Theorie,
Schmelzpunkt: 213-214°C
$C_{26}H_{28}N_4O_5$ (500.56)
Massenspektrum: $M^+$ = 500

| | | | |
|---|---|---|---|
| Ber. | C 67.19 | H 5.64 | N 11.19 |
| Gef. | 66.46 | 5.79 | 11.02 |

Beispiel 128

(Z)-3-{1-[4-(2-Aminoethyl)-phenylamino]-1-phenyl-methyliden}-5-nitro-2-indolinon-hydrochlorid

Hergestellt analog Beispiel 29a aus (Z)-3-{1-[4-(2-tert.Butoxycarbonylamino-ethyl)-phenylamino]-1-phenyl-methyliden}-5-nitro-2-indolinon und Essigester/Clorwasserstoff.
Ausbeute: 90 % der Theorie,
Schmelzpunkt: 335-340°C (Zers.)
$C_{23}H_{20}N_4O_3$ (400.44)
Massenspektrum: $M^+$ = 400
$C_{23}H_{20}N_4O_3$ x HCl (436.91)

| | | | |
|---|---|---|---|
| Ber. | C 61.95 | H 4.97 | N 12.56 |
| Gef. | 61.68 | 5.00 | 12.50 |

Beispiel 129

(Z)-3-{1-[4-(2-Acetylamino-ethyl)-phenylamino]-1-phenyl-methyliden}-5-nitro-2-indolinon

Hergestellt analog Beispiel 31 aus (Z)-3-{1-[4-(2-Aminoethyl)-phenylamino]-1-phenyl-methyliden}-5-nitro-2-indolinon und Acetanhydrid in Eisessig.
Ausbeute: 88 % der Theorie,

Schmelzpunkt: 306-307°C
$C_{25}H_{22}N_4O_4$ (442.48)
Massenspektrum: $M^+$ = 442
$C_{25}H_{22}N_4O_4$ x 0.5 $H_2O$ (451.48)

| | | | |
|---|---|---|---|
| Ber. | C 66.51 | H 5.13 | N 12.41 |
| Gef. | 66.71 | 5.00 | 12.23 |

Beispiel 130

(Z)-3-{1-[4-(2-Diethylamino-ethyl)-phenylamino]-1-phenyl-methyliden}-5-nitro-2-indolinon

Hergestellt analog Beispiel 82 aus 1-Acetyl-3-(1-ethoxy-1-phenyl-methyliden)-5-nitro-2-indolinon und 4-(2-Diethylaminoethyl)-anilin in DMF und anschließende Behandlung mit Natronlauge in Methanol.
Ausbeute: 75 % der Theorie,
Schmelzpunkt: 167-168°C
$C_{27}H_{28}N_4O_3$ (456.55)
Massenspektrum: $(M+H)^+$ = 457

| | | | |
|---|---|---|---|
| Ber. | C 71.03 | H 6.18 | N 12.27 |
| Gef. | 70.83 | 6.10 | 12.14 |

Beispiel 131

(Z)-3-{1-[4-(2-(N-(2-Hydroxyethyl)-N-ethyl-amino)-ethyl)-phenylamino]-1-phenyl-methyliden}-5-nitro-2-indolinon

Hergestellt analog Beispiel 89 aus 3-(1-ethoxy-1-phenyl-methyliden)-5-nitro-2-indolinon und {4-[2-(N-(2-Hydroxyethyl)-N-ethyl-amino)-ethyl]-phenylamino}-anilin in DMF.
Ausbeute: 68 % der Theorie,
Schmelzpunkt: 165-166°C
$C_{27}H_{28}N_4O_4$ (472.55)
Massenspektrum: $M^+$ = 472
$R_f$-Wert : 0.42 (Kieselgel; Dichlormethan/Methanol/$NH_4OH$ = 9:1:0.1)

| | | | |
|---|---|---|---|
| Ber. | C 68.63 | H 5.97 | N 11.86 |
| Gef. | 68.63 | 5.99 | 11.74 |

Beispiel 132

(Z) -3-{1-[4-(2-Piperidinoethyl)-phenylamino]-1-phenyl-methyliden}-5-nitro-2-indolinon

Hergestellt analog Beispiel 82 aus 1-Acetyl-3-(1-ethoxy-1-phenyl-methyliden)-5-nitro-2-indolinon und 4-(2-Piperidinoethyl)-anilin in DMF und anschließende Behandlung mit Natronlauge in Methanol.
Ausbeute: 68 % der Theorie,
Schmelzpunkt: 236-237°C
$C_{28}H_{28}N_4O_3$ (468.56)
Massenspektrum: $M^+$ = 468
$R_f$-Wert: 0.62 (Kieselgel; Dichlormethan/Methanol/$NH_4OH$ = 4:1:0.2)
$C_{27}H_{26}N_4O_4$ x 0.5 $H_2O$ (477.56)

| | | | |
|---|---|---|---|
| Ber. | C 70.42 | H 6.12 | N 11.73 |
| Gef. | 70.97 | 6.08 | 11.70 |

Beispiel 133

(Z)-3-{1-[4-(2-Morpholinoethyl)-phenylamino]-1-phenyl-methyliden}-5-nitro-2-indolinon

Hergestellt analog Beispiel 82 aus 1-Acetyl-3-(1-ethoxy-1-phenyl-methyliden)-5-nitro-2-indolinon und 4-(2-Morpholinoethyl)-anilin in DMF und anschließende Behandlung mit Natronlauge in Methanol.
Ausbeute: 87 % der Theorie,
Schmelzpunkt: 304-306°C
$C_{27}H_{26}N_4O_4$ (470.53)
Massenspektrum: $M^+ = 470$
$R_f$-Wert: 0.3 (Kieselgel; Dichlormethan/Methanol = 19:1)

| | | | |
|---|---|---|---|
| Ber. | C 68.92 | H 5.57 | N 11.91 |
| Gef. | 68.68 | 5.55 | 11.90 |

Beispiel 134

(Z)-3-{1-[4-(2-Dimethylamino-ethyl)-phenylamino]-1-phenylmethyliden}-5-nitro-2-indolinon

Hergestellt analog Beispiel 82 aus 1-Acetyl-3-(1-ethoxy-1-phenyl-methyliden)-5-nitro-2-indolinon und 4-(2-Dimethylaminoethyl)-anilin in DMF und anschließende Behandlung mit Natronlauge in Methanol.
Ausbeute: 77 % der Theorie,
Schmelzpunkt: 238-240°C
$C_{25}H_{24}N_4O_3$ (428.50)
Massenspektrum: $M^+ = 428$
$R_f$-Wert: 0.4 (Kieselgel; Dichlormethan/Methanol = 9:1)

| | | | |
|---|---|---|---|
| Ber. | C 70.08 | H 5.65 | N 13.08 |
| Gef. | 69.87 | 5.64 | 12.99 |

Beispiel 135

(Z)-3-{1-[4-(2-(4-Methylpiperazino)-ethyl)-phenylamino]-1-phenyl-methyliden}-5-nitro-2-indolinon

Hergestellt analog Beispiel 82 aus 1-Acetyl-3-(1-ethoxy-1-phenyl-methyliden)-5-nitro-2-indolinon und 4-[2-(N-Methylpiperazino)-ethyl]-anilin in DMF und anschließende Behandlung mit Natronlauge in Methanol.
Ausbeute: 90 % der Theorie,
Schmelzpunkt: 238-240°C
$C_{28}H_{29}N_5O_3$ (483.58)
Massenspektrum: $(M+H)^+ = 484$
$R_f$-Wert: 0.44 (Kieselgel; Dichlormethan/Methanol = 9:1) $C_{28}H_{29}N_5O_3 \times 0.5\ H_2O$ (492.58)

| | | | |
|---|---|---|---|
| Ber. | C 68.27 | H 6.14 | N 14.22 |
| Gef. | 67.87 | 6.15 | 14.14 |

Beispiel 136

(Z)-3-[1-(3-tert.Butoxycarbonylaminomethylcarbonylaminomethylphenylamino)-1-phenyl-methyliden]-5-nitro-2-indolinon

Hergestellt analog Beispiel 82 aus 1-Acetyl-3-(1-ethoxy-1-phenyl-methyliden)-5-nitro-2-indolinon und 3-tert. Butoxycarbonylaminomethylcarbonylaminomethyl-anilin in DMF und anschließende Behandlung mit Natronlauge in Methanol.
Ausbeute: 78 % der Theorie,
Schmelzpunkt: 228°C
$C_{29}H_{29}N_5O_6$ (543.58)

Massenspektrum: $M^+$ = 543

| | | | |
|---|---|---|---|
| Ber. | C 64.08 | H 5.38 | N 12.88 |
| Gef. | 63.72 | 5.45 | 12.73 |

Beispiel 137

(Z)-3-[1-(3-Aminomethylcarbonylaminomethyl-phenylamino)-1-phenyl-methyliden]-5-nitro-2-indolinon-hydrochlorid

Hergestellt analog Beispiel 29a aus (Z)-3-[1-(3-tert.Butoxycarbonylaminomethylcarbonylaminomethyl-phenylamino)-1-phenylmethyliden]-5-nitro-2-indolinon und Essigester/Chlorwasserstoff.
Ausbeute: 99 % der Theorie,
Schmelzpunkt: 309°C
$C_{24}H_{21}N_5O_4$ (443.47)
Massenspektrum: $M^+$ = 443
$C_{24}H_{21}N_5O_4$ x HCl x 0.5 $H_2O$ (488.94)

| | | | |
|---|---|---|---|
| Ber. | C 58.96 | H 4.74 | N 14.32 |
| Gef. | 58.40 | 4.74 | 14.01 |

Beispiel 138

(Z)-3-[1-(3-Acetylaminomethyl-phenylamino)-1-phenyl-methylien]-5-nitro-2-indolinon

Hergestellt analog Beispiel 89 aus 3-(1-Ethoxy-1-phenyl-methyliden)-5-nitro-2-indolinon und 3-Acetylaminomethyl-anilin in DMF.
Ausbeute: 57 % der Theorie,
Schmelzpunkt: 238°C
$C_{24}H_{20}N_4O_4$ (428.45)
Massenspektrum: $M^+$ = 428
$C_{24}H_{20}N_4O_4$ x 0.5 $H_2O$ (437.46)

| | | | |
|---|---|---|---|
| Ber. | C 65.90 | H 4.84 | N 12.81 |
| Gef. | 66.29 | 4.80 | 12.76 |

Beispiel 139

(Z) -3-{1-[4-(N-Aminomethylcarbonyl-N-methyl-amino)-phenylamino]-1-phenyl-methyliden}-5-nitro-2-indolinon

a) (Z)-3-{1-[4-(N-Phthalimidomethylcarbonyl-N-methyl-amino)-phenylamino]-1-phenyl-methyliden}-5-nitro-2-indolinon

Hergestellt analog Beispiel 89 aus 3-(1-Ethoxy-1-phenyl-methyliden)-5-nitro-2-indolinon und 4-(N-Phthalimidomethylcarbonyl-N-methyl-amino)-anilin in DMF.
Ausbeute: 99 % der Theorie,
Schmelzpunkt: 303-305°C
$C_{32}H_{23}N_5O_6$ (573.57)
Massenspektrum: $M^+$ = 573
$C_{32}H_{23}N_5O_6$ x $H_2O$ (591.59)

| | | | |
|---|---|---|---|
| Ber. | C 64.97 | H 4.26 | N 11.84 |
| Gef. | 64.74 | 4.41 | 11.59 |

b) (Z)-3-{1-[4-(N-Aminomethylcarbonyl-N-methyl-amino)phenylamino]-1-phenyl-methyliden}-5-nitro-2-indolinon

287 mg (0.5 mMol) (Z)-3-{1-[4-(N-Phthalimidomethylcarbonyl-N-methyl-amino)phenylamino]-1-phenyl-methyliden}-5-nitro-2-indolinon werden in 20 ml Ethanol und 20 ml Dichlormethan suspendiert und nach Zugabe von 0.3

ml 80%iger Hydrazinhydratlösung 18 Stunden bei 50°C gerührt. Anschließend wird auf Raumtemperatur abgekühlt, von Unlöslichem abgesaugt und die Mutterlauge eingedampft. Der Rückstand wird an Kieselgel (Dichlormethan/Methanol/Ammoniak = 92:8:0.8) chromatographiert und das Produkt nochmals mit Methanol verrieben, abgesaugt und getrocknet.
Ausbeute: 220 mg (99 % der Theorie),
Schmelzpunkt: 255-256°C
$C_{24}H_{21}N_5O_4$ (443.47)
Massenspektrum: $M^+$ = 443

| | | | |
|---|---|---|---|
| Ber. | C 65.00 | H 4.77 | N 15.79 |
| Gef. | 64.73 | 4.91 | 15.66 |

Beispiel 140

(Z)-3-{1-[4-(N-Acetylaminomethylcarbonyl-N-methyl-amino)-phenylaminol-1-phenyl-methyliden}-5-nitro-2-indolinon

Hergestellt ananlog Beispiel 31 aus (Z)-3-{1-[4-(N-Aminomethylcarbonyl-N-methyl-amino)-phenylamino]-1-phenyl-methyliden}-5-nitro-2-indolinon und Essigsäureanhydrid in Eisessig. Ausbeute: 83 % der Theorie,
Schmelzpunkt: 277-278°C
C26H23N5O5 (485.50)
Massenspektrum: $M^+$ = 485
$R_f$-Wert: 0.6 (Kieselgel; Essigester/Methanol/$NH_4OH$ = 8:2:0.1)
$C_{26}H_{23}N_4O_5$ x $H_2O$ (503.52)

| | | | |
|---|---|---|---|
| Ber. | C 62.02 | H 5.00 | N 13.91 |
| Gef. | 61.77 | 5.01 | 13.79 |

Beispiel 141

(Z)-3-[1-(4-Morpholinomethylcarbonylaminomethyl-phenylamino)-1-phenyl-methyliden]-5-nitro-2-indolinon

Hergestellt analog Beispiel 74 aus (Z)-1-Polystyrylmethylaminocarbonyl-3-{1-[4-(morpholinomethylcarbonylaminomethyl)-phenylamino]-1-phenyl-methyliden}-5-nitro-2-indolinon und Natronlauge in Dioxan.
Ausbeute: 33 % der Theorie,
Schmelzpunkt: 290-295°C
$C_{28}H_{27}N_5O_5$ (513.56)
Massenspektrum: $M^+$ = 513

| | | | |
|---|---|---|---|
| Ber. | C 65.49 | H 5.30 | N 13.64 |
| Gef. | 65.09 | 5.32 | 13.46 |

Beispiel 142

(Z)-3-[1-(4-Dimethylaminomethylcarbonylaminomethyl-phenylamino)-1-phenyl-methyliden]-5-nitro-2-indolinon

Hergestellt analog Beispiel 74 aus (Z)-1-Polystyrylmethylaminocarbonyl-3-{1-[4-(dimethylaminomethylcarbonyl-aminomethyl)-phenylamino]-1-phenyl-methyliden}-5-nitro-2-indolinon und Natronlauge in Dioxan.
Ausbeute: 32 % der Theorie,
Schmelzpunkt: 272-273°C
$C_{26}H_{25}N_5O_4$ (471.52)
Massenspektrum: $M^+$ = 471
$R_f$-Wert: 0.55 (Kieselgel; Dichlormethan/Methanol/$NH_4OH$ = 9:1:0.1)

| | | | |
|---|---|---|---|
| Ber. | C 66.23 | H 5.34 | N 14.85 |
| Gef. | 66.10 | 5.35 | 14.70 |

Beispiel 143

(Z)-3-[1-(4-Acetylaminomethyl-phenylamino)-1-phenyl-methyliden]-5-nitro-2-indolinon

Hergestellt analog Beispiel 74 aus (Z)-1-Polystyrylmethylamino-carbonyl-3-[1-(4-acetylaminomethyl-phenylamino)-1-phenyl-methyliden]-5-nitro-2-indolinon und Natronlauge in Dioxan. Ausbeute: 37 % der Theorie,
Schmelzpunkt: 345-346°C
$C_{24}H_{20}N_4O_4$ (428.45)
Massenspektrum: $M^+$ = 428

| | | | |
|---|---|---|---|
| Ber. | C 67.94 | H 4.79 | N 12.73 |
| Gef. | 66.46 | 4.87 | 12.80 |

Beispiel 144

(Z)-3-[1-(4-tert.Butoxycarbonylaminomethylcarbonylaminomethylphenylamino)-1-phenyl-methyliden]-5-nitro-2-indolinon

Hergestellt analog Beispiel 74 aus (Z)-1-Polystyrylmethylaminocarbonyl-3-{1-[4-(tert.butoxycarbonylaminomethylcarbonylaminomethyl)-phenylamino]-1-phenyl-methyliden}-5-nitro-2-indolinon und Natronlauge in Dioxan.
Ausbeute: 24 % der Theorie,
Schmelzpunkt: 219-221°C (Zers.)
$C_{29}H_{29}N_5O_6$ (543.58)
Massenspektrum: $M^+$ = 543
$C_{29}H_{29}N_5O_6$ x 0.5 $H_2O$ (552.59)

| | | | |
|---|---|---|---|
| Ber. | C 63.03 | H 5.47 | N 12.67 |
| Gef. | 63.20 | 5.35 | 12.61 |

Beispiel 145

(Z)-3-{1-[4-((N-tert.Butoxycarbonyl-N-methyl-amino)-methylcarbonylaminomethyl)-phenylamino]-1-phenyl-methyliden}-5-nitro-2-indolinon

Hergestellt analog Beispiel 74 aus (Z)-1-Polystyrolmethylaminocarbonyl-3-{1-[4-((N-tert.butoxycarbonyl-N-methyl-amino)-methylcarbonylaminomethyl)-phenylamino]-1-phenyl-methyliden}-5-nitro-2-indolinon und Natronlauge in Dioxan.
Ausbeute: 31 % der Theorie,
Schmelzpunkt: 225-227°C (Zers.)
$C_{30}H_{31}N_5O_6$ (557.61)
Massenspektrum: $M^+$ = 557
$C_{30}H_{31}N_5O_6$ x 0.5 $H_2O$ (566.62)

| | | | |
|---|---|---|---|
| Ber. | C 63.59 | H 5.69 | N 12.36 |
| Gef. | 63.75 | 5.31 | 12.22 |

Beispiel 146

(Z)-3-[1-(4-tert.Butoxycarbonylaminomethyl-phenylamino)-1-(4-phthalimidomethyl-phenyl)-methyliden]-1-acetyl-5-nitro-2-indolinon

a) 4-Phthalimidomethyl-benzoesäure-tert.butylester

18.5 g (0.1 Mol) Phthalimid-Kalium werden in 80 ml DMF suspendiert und mit 22.5 g (0.09 Mol) 4-Brommethylbenzoesäuretert.butylester versetzt. Die Reaktionslösung wird 16 Stunden bei Raumtemperatur gerührt und anschließend in 40 ml Wasser eingerührt, mit Essigester extrahiert und an Kieselgel (Toluol) chromatographiert.

Ausbeute: 17.9 g (60 % der Theorie),
Schmelzpunkt: 144-145°C
$C_{20}H_{19}NO_4$ x 0.25 $H_2O$ (341.88)

| | | | |
|---|---|---|---|
| Ber. | C 70.26 | H 5.75 | N 4.10 |
| Gef. | 70.10 | 5.73 | 4.11 |

b) 4-Phthalimidomethyl-benzoesäure

337 mg (1.0 mMol) 4-Phthalimidomethyl-benzoesäure-tert.butylester werden in 3 ml Triflouressigsäure 45 Minuten bei Raumtemperatur gerührt. Anschließend wird das Lösungsmittel im Vakuum entfernt.
Ausbeute: 96 % der Theorie,
Schmelzpunkt: 260-262°C
$C_{16}H_{11}NO_4$ (281.3)
Massenspektrum: $M^+$ = 281

c) 3-[1-Hydroxy-1-(4-phthalimidomethyl-phenyl)-methyliden]-1-acetyl-5-nitro-2-indolinon

Hergestellt analog Beispiel 2a aus 1-Acetyl-5-nitro-2-indolinon und 4-Phthalimidomethyl-benzoesäure, TBTU, HOBt und N-Ethyl-N,N-diisopropyl-amin in DMF.
Ausbeute: 75 % der Theorie,
Schmelzpunkt: 246-248°C (Zers.)
$R_f$-Wert: 0.55 (Kieselgel; Dichlormathan/Methanol = 10:1)

d) 3- [1-Chlor-1- (4-phthalimidomethyl-phenyl)-methyliden]-1-acetyl-5-nitro-2-indolinon

Hergestellt analog Beispiel 2b aus 3-[1-Hydroxy-1-(4-phthalimidomethyl-phenyl)-methyliden]-1-acetyl-5-nitro-2-indolinon und Phosphorpentachlorid in Toluol.
Ausbeute: 65 % der Theorie,
Schmelzpunkt: 234-236°C (Zers.)
$C_{26}H_{16}ClN_3O_6$ (501.9)

| | | | | |
|---|---|---|---|---|
| Ber. | C 62.22 | H 3.21 | N 8.37 | Cl 7.06 |
| Gef. | 62.25 | 3.31 | 8.27 | 7.20 |

e) (Z)-3-[1-(4-tert.Butoxycarbonylaminomethyl-phenylamino)-1-(4-phthalimidomethyl-phenyl)-methyliden]-1-acetyl-5-nitro-2-indolinon

Hergestellt analog Beispiel 2c aus 3-[1-Chlor-1-(4-phthalimidomethyl-phenyl)-methyliden]-1-acetyl-5-nitro-2-indolinon, 4-tert.Butoxycarbonylaminomethyl-anilin und Triethylamin in Dichlormethan.
Ausbeute: 47 % der Theorie,
Schmelzpunkt: 125°C (Zers.)
$C_{38}H_{33}N_5O_8$ (687.71)
Massenspektrum: $M^+$ = 687

Beispiel 147

(Z)-3-{1-[4-tert.Butoxycarbonylaminomethyl-phenylamino]-1-[4-(2-carboxyphenyl)-carbonylaminomethyl-phenyl]-methyliden}-5-nitro-2-indolinon

Hergestellt analog Beispiel 1 aus (Z)-3-[1-(4-tert.Butyloxycarbonylaminomethyl-phenylamino)-1-(4-phthalimidomethyl-phenyl)-methyliden]-1-acetyl-5-nitro-2-indolinon und Natronlauge in Methanol.
Ausbeute: 88 % der Theorie,
Schmelzpunkt: 138°C (Zers.)
$C_{36}H_{33}N_5O_8$ (663.69)
Massenspektrum: $(M+H)^+$ = 664
$R_f$-Wert: 0.31 (Kieselgel; Dichlormethan/Methanol = 10:1)

Beispiel 148

(Z)-3-[1-(4-tert.Butoxycarbonylaminomethyl-phenylamino)-1-(4-aminomethyl-phenyl)-methyliden]-5-nitro-2-indolinon

Hergestellt analog Beispiel 139b aus (Z)-3-[1-(4-tert.Butoxycarbonylaminomethyl-phenylamino)-1-(4-phthalimidomethyl-phenyl)-methyliden]-1-acetyl-5-nitro-2-indolinon und Hydrazinhydratlösung in Ethanol.
Ausbeute: 42 % der Theorie,
Schmelzpunkt: 220-223°C
$C_{28}H_{29}N_5O_5$ (515.57)
Massenspektrum: $M^+$ = 515
$R_f$-Wert: 0.61 (Kieselgel; Dichlormethan/Methanol = 9:1)

Beispiel 149

(Z)-3- [1- (4-Aminomethyl-phenylamino] -1- (4-aminomethyl-phenyl)-methyliden]-5-nitro-2-indolinon-dihydrotrifluoracetat

Hergestellt analog Beispiel 29a aus (Z)-3-[1-(4-tert.Butoxycarbonylaminomethyl-phenylamino)-1-(4-aminomethyl-phenyl)-methyliden]-5-nitro-2-indolinon und Triflouressigsäure in Dichlormethan.
Ausbeute: 54 % der Theorie,
Schmelzpunkt: 265°C
$C_{23}H_{21}N_5O_3$ (415.46)
Massenspektrum: $M^+$ = 415
$R_f$-Wert: 0.50 (Reversed Phase P8; Methanol/5%ige Kochsalzlösung = 6:4)
$C_{23}H_{21}N_5O_3$ x 2 $C_2HF_3O_2$ x 2 $H_2O$ (679.53)

| | | | |
|---|---|---|---|
| Ber. | C 47.72 | H 4.00 | N 10.30 |
| Gef. | 47.69 | 3.96 | 10.39 |

Beispiel 150

(Z)-3-[1-(4-tert.Butoxycarbonylaminomethyl-phenylamino)-1-(4-acetylaminomethyl-phenyl)-methyliden]-5-nitro-2-indolinon

Hergestellt analog Beispiel 31 aus (Z)-3-[1-(4-tert.Butoxycarbonylaminomethyl-phenylamino)-1-(4-aminomethyl-phenyl)-methyliden]-5-nitro-2-indolinon und Acetanhydrid in Dioxan.
Ausbeute: 61 % der Theorie,
Schmelzpunkt: 234°C (Zers.)
$C_{30}H_{31}N_5O_6$ (557.61)
Massenspektrum: $M^+$ = 557
$R_f$-Wert: 0.60 (Kieselgel; Dichlormethan/Methanol = 20:1) $C_{30}H_{31}N_5O_6$ x 0.25 $H_2O$ (562.12)

| | | | |
|---|---|---|---|
| Ber. | C 64.07 | H 5.70 | N 12.46 |
| Gef. | 64.01 | 5.70 | 12.13 |

Beispiel 151

(Z)-3-[1-(4-Aminomethyl-phenylamino)-1-(4-acetylaminomethylphenyl)-methyliden]-5-nitro-2-indolinon-dihydrotrifluoracetat

Hergestellt analog Beispiel 29a aus (Z)-3-[1-(4-tert.Butoxycarbonylaminomethyl-phenylamino)-1-(4-acetylaminomethyl-phenyl)-methyliden]-5-nitro-2-indolinon und Trifluoressigsäure in Dichlormethan.
Ausbeute: 92 % der Theorie,
Schmelzpunkt: 239-241°C (Zers.)
$C_{25}H_{23}N_5O_4$ (457.49)
Massenspektrum: $M^+$ = 457
$C_{25}H_{23}N_5O_4$ x 2 $C_2HF_3O_2$ x 0.5 $H_2O$ (694.55)

| | | | |
|---|---|---|---|
| Ber. | C 50.80 | H 3.67 | N 10.21 |
| Gef. | 50.14 | 3.77 | 10.08 |

Beispiel 152

(Z)-3-[1-(4-Acetylaminomethyl-phenylamino)-1-(4-acetylaminomethyl-phenyl)-methyliden]-5-nitro-2-indolinon-hydrotrifluoracetat

Hergestellt analog Beispiel 31 aus (Z)-3-[1-(4-Aminomethylphenylamino)-1-(4-acetylaminomethyl-phenyl)-methyliden]-5-nitro-2-indolinon und Acetanhydrid in Dioxan.
Ausbeute: 99 % der Theorie,
Schmelzpunkt: 126°C (Zers.)
$C_{27}H_{25}N_5O_5$ (499.53)
Massenspektrum: $M^+$ = 499
$R_f$-Wert: 0.42 (Kieselgel; Dichlormethan/Methanol/$NH_4OH$ = 10:1:0.1)

Beispiel 153

(Z)-3-[1-Phenylamino-1-(4-phthalimidomethyl-phenyl)-methyliden]-5-nitro-2-indolinon

Hergestellt analog Beispiel 146 aus 1-Acetyl-3-[1-chlor-1-(4-phthalimidomethyl-phenyl)-methyliden]-5-nitro-2-indolinon, Anilin, N-Ethyl-N,N-diisopropyl-amin und DMF.
Ausbeute: 18 % der Theorie,
Schmelzpunkt: 334-336°C (Zers.)
$C_{30}H_{20}N_4O_5$ (516.52)
Massenspektrum: 516
$R_f$-Wert: 0.30 (Kieselgel; Toluol/Aceton = 4:1)

Beispiel 154

(Z)-3-[1-Phenylamino-1-(4-aminomethyl-phenyl)-methyliden]-5-nitro-2-indolinon

Hergestellt analog Beispiel 140 aus (Z)-3-[1-Phenylamino-1-(4-phthalimidomethyl-phenyl)-methyliden]-5-nitro-2-indolinon und Hydrazinhydratlösung in Ethanol.
Ausbeute: 66 % der Theorie,
Schmelzpunkt: 332°C (Zers.)
$C_{22}H_{18}N_4O_3$ (386.41)
Massenspektrum: $(M+H)^+$ = 387
$R_f$-Wert: 0.38 (Kieselgel; Dichlormethan/Methanol/$NH_4OH$ = 10:1:0.1)

Beispiel 155

(Z)-3-{1-[4-(2-tert.Butoxycarbonylamino-ethyl)-phenylamino]-1-(4-aminomethyl-phenyl)-methyliden}-5-nitro-2-indolinon

Hergestellt analog Beispiel 140 aus (Z)-3-{1-[4-(2-tert.Butoxycarbonylamino-ethyl)-phenylamino]-1-(4-phthalimidomethylphenyl)-methyliden}-1-acetyl-5-nitro-2-indolinon und Hydrazinhydratlösung in Ethanol.
Ausbeute: 65 % der Theorie,
Schmelzpunkt: 215-217°C (Zers.)
$C_{29}H_{31}N_5O_5$ (529.60)
Massenspektrum: $M^+$ = 529
$R_f$-Wert: 0.33 (Kieselgel; Dichlormethan/Methanol = 10:1) $C_{29}H_{31}N_5O_5$ x $H_2O$ x $C_8H_6N_2O_2$ (628.70)

| | | | |
|---|---|---|---|
| Ber. | C 63.05 | H 5.77 | N 13.37 |
| Gef. | 63.16 | 5.73 | 13.50 |

Beispiel 156

(Z)-3-{1-[4-(2-Aminoethyl)-phenylamino]-1-(4-aminomethyl-phenyl)-methyliden}-5-nitro-2-indolinon-dihydrotrifluoracetat

Hergestellt analog Beispiel 29a aus (Z)-3-{1-[4-(2-tert.Butoxycarbonylamino-ethyl)-phenylamino]-1-(4-aminomethyl-phenyl)-methyliden}-5-nitro-2-indolinon und Trifluoressigsäure in Dichlormethan.
Ausbeute: 96 % der Theorie,
Schmelzpunkt: 230-232°C (Zers.)
$C_{24}H_{23}N_5O_3$ (429.48)
Massenspektrum: 429
$R_f$-Wert: 0.27 (Kieselgel; Dichlormethan/Methanol/$NH_4OH$ = 4:1:0.1)
$C_{24}H_{23}N_5O_3$ x 2 $C_2HF_3O_2$ (657.53)

| | | | |
|---|---|---|---|
| Ber. | C 51.14 | H 3.83 | N 10.65 |
| Gef. | 51.53 | 4.05 | 11.05 |

Beispiel 157

(Z)-3-{1-[4-(2-tert.Butoxycarbonylamino-ethyl)-phenylamino]-1-(4-acetylaminomethyl-phenyl)-methyliden}-5-nitro-2-indolinon

Hergestellt analog Beispiel 31 aus (Z)-3-{1-[4-(2-tert.Butoxycarbonylamino-ethyl)-phenylamino]-1-(4-aminomethyl-phenyl)-methyliden}-5-nitro-2-indolinon und Acetanhydrid in Dioxan.
Ausbeute: 53 % der Theorie,
Schmelzpunkt: 94°C (Zers.)
$C_{31}H_{33\,5}O_6$ (571.64)
Massenspektrum: $(M-H)^-$ = 570
$R_f$-Wert: 0.52 (Kieselgel; Dichlormethan/Methanol = 25:1)
$C_{31}H_{33}N_5O_6$ x $H_2O$ (589.65)

Beispiel 158

(Z)-3-{1-[4-(2-Aminoethyl)-phenylamino]-1-(4-acetylaminomethyl-phenyl)-methyliden}-5-nitro-2-indolinon-dihydrotrifluoracetat

Hergestellt analog Beispiel 29a aus (Z)-3-{1-[4-(2-tert.Butoxycarbonylamino-ethyl)-phenylamino]-1-(4-acetylaminomethylphenyl)-methyliden}-5-nitro-2-indolinon und Trifluoressigsäure in Dichlormethan.
Ausbeute: 67 % der Theorie,
Schmelzpunkt: 229°C (Zers.)
$C_{26}H_{25}N_5O_4$ (471.52)
Massenspektrum: 471
$R_f$-Wert: 0.33 (Kieselgel; Dichlormethan/Methanol/$NH_4OH$ = 4:1:0.1)
$C_{26}H_{25}N_5O_4$ x $C_2HF_3O_2$ x 0.5 $H_2O$ (594.55)

| | | | |
|---|---|---|---|
| Ber. | C 56.56 | H 4.58 | N 11.78 |
| Gef. | 56.33 | 4.54 | 11.62 |

Beispiel 159

(Z)-3-[1-(4-Diethylaminomethyl-phenylamino)-1-phenyl-methyliden]-5-nitro-2-indolinon

846 mg (2.0 mMol) (Z)-[(4-Aminomethyl-phenylamino)-1-phenylmethyliden]-5-nitro-2-indolinon-hydrochlorid werden in 20 ml Methanol suspendiert und mit 0.1 ml (2.5 mMol) Acetaldehyd versetzt. Nach 15 Minuten Rühren bei Raumtemperatur werden 157 mg (2.5 mMol) Natriumcyanoborhydrid zugegeben. Man rührt 16 Stunden bei Raumtemperatur und gibt anschließend nochmals 0.1 ml (2.5 mMol) Acetaldehyd und 157 mg (2.5 mMol) Natriumcyanoborhydrid zu. Nach 22 Stunden Rühren bei Raumtemperatur wird die Reaktionsmischung eingedampft und der Rückstand

in Wasser/Dichlormethan aufgenommen. Extraktion mit Dichlormethan und Chromatographie an Kieselgel (Dichlormethan/Methanol/$NH_4OH$ = 93:7:0.7) liefern das Produkt.
Ausbeute: 340 mg (38 % der Theorie),
Schmelzpunkt: 173-174°C
$C_{26}H_{26}N_4O_3$ (442.52)
Massenspektrum: $M^+$ = 442
$R_f$-Wert: 0.4 (Kieselgel; Dichlormethan/Methanol/$NH_4OH$ = 9:1:0.1)

| | | | |
|---|---|---|---|
| Ber. | C 70.57 | H 5.92 | N 12.66 |
| Gef. | 70.27 | 5.90 | 12.57 |

Beispiel 160

(Z)-3-[1-(4-Ethylaminomethyl-phenylamino)-1-phenyl-methyliden]-5-nitro-2-indolinon

Hergestellt analog Beispiel 159 aus (Z)-3-[1-(4-Aminomethylphenylamino)-1-phenyl-methyliden]-5-nitro-2-indolinon-hydrochlorid, Acetaldehyd und Natriumcyanoborhydrid in Methanol.
Ausbeute: 17 % der Theorie,
Schmelzpunkt: 220-223°C
$C_{24}H_{22}N_4O_3$ (414.47)
Massenspektrum: $M^+$ = 414
$R_f$-Wert: 0.2 (Kieselgel; Essigester/Methanol/$NH_4OH$ = 8:2:0.1) $C_{24}H_{22}N_4O_3$ x 0.5 $H_2O$ (423.47)

| | | | |
|---|---|---|---|
| Ber. | C 68.07 | H 5.47 | N 13.23 |
| Gef. | 68.55 | 5.41 | 13.15 |

Beispiel 161

(Z)-3-[1-(4-Dipropylaminomethyl-phenylamino)-1-phenyl-methyliden]-5-nitro-2-indolinon

Hergestellt analog Beispiel 159 aus (Z)-3-[1-(4-Aminomethylphenylamino)-1-phenyl-methyliden]-5-nitro-2-indolinon-hydrochlorid, Propionaldehyd und Natriumcyanoborhydrid in Methanol.
Ausbeute: 29 % der Theorie,
Schmelzpunkt: 160-162°C
$C_{24}H_{22}N_4O_3$ (414.47)
Massenspektrum: $M^+$ = 470
$R_f$-Wert: 0.6 (Kieselgel; Dichlormethan/Methanol/$NH_4OH$ = 9:1:0.1)
$C_{28}H_{30}N_4O_3$ x 0.5 $H_2O$ (479.58)

| | | | |
|---|---|---|---|
| Ber. | C 70.13 | H 6.52 | N 11.68 |
| Gef. | 69.80 | 6.61 | 11.65 |

Beispiel 162

(Z)-3-[1-(4-Propylaminomethyl-phenylamino)-1-phenyl-methyliden]-5-nitro-2-indolinon

Hergestellt analog Beispiel 159 aus (Z)-3-[1-(4-Aminomethylphenylamino)-1-phenyl-methyliden]-5-nitro-2-indolinon-hydrochlorid, Propionaldehyd und Natriumcyanoborhydrid in Methanol.
Ausbeute: 12 % der Theorie,
Schmelzpunkt: 201-202°C
$C_{25}H_{24}N_4O_3$ (428.50)
Massenspektrum: $M^+$ = 428
$R_f$-Wert: 0.4 (Kieselgel; Dichlormethan/Methanol/$NH_4OH$ = 9:1:0.1)
$C_{25}H_{24}N_4O_3$ x 0.5 $H_2O$ (437.50)

| | | | |
|---|---|---|---|
| Ber. | C 68.63 | H 5.76 | N 12.81 |

(fortgesetzt)

| | | | |
|---|---|---|---|
| Gef. | 68.81 | 5.87 | 12.83 |

Beispiel 163

(Z)-3-[1-(4-Diisobutylaminomethyl-phenylamino)-1-phenyl-methyliden]-5-nitro-2-indolinon

Hergestellt analog Beispiel 159 aus (Z)-3-[1-(4-Aminomethylphenylamino)-1-phenyl-methyliden]-5-nitro-2-indolinon-hydrochlorid, Isobutyraldehyd und Natriumcyanoborhydrid in Methanol.
Ausbeute: 3 % der Theorie,
Schmelzpunkt: 204-207°C
$C_{30}H_{34}N_4O_3$ (498.63)
Massenspektrum: $M^+$ = 498
$R_f$-Wert: 0.95 (Kieselgel; Essigester/Methanol/$NH_4OH$ = 8:2:0.1)

Beispiel 164

(Z)-3-[1-(4-Isobutylaminomethyl-phenylamino)-1-phenyl-methyliden]-5-nitro-2-indolinon

Hergestellt analog Beispiel 159 aus (Z)-3-[1-(4-Aminomethylphenylamino)-1-phenyl-methyliden]-5-nitro-2-indolinon-hydrochlorid, Isobutyraldehyd und Natriumcyanoborhydrid in Methanol.
Ausbeute: 44 % der Theorie,
Schmelzpunkt: 208°C
$C_{26}H_{26}N_4O_3$ (442.52)
Massenspektrum: $M^+$ = 442
$R_f$-Wert: 0.4 (Kieselgel; Essigester/Methanol/$NH_4OH$ = 8:2:0.1)

| | | | |
|---|---|---|---|
| Ber. | C 70.57 | H 5.92 | N 12.66 |
| Gef. | 70.03 | 6.00 | 12.42 |

Beispiel 165

(Z)-3-[1-(4-Dibutylaminomethyl-phenylamino)-1-phenyl-methyliden]-5-nitro-2-indolinon

Hergestellt analog Beispiel 159 aus (Z)-3-[1-(4-Aminomethylphenylamino)-1-phenyl-methyliden]-5-nitro-2-indolinon-hydrochlorid, Butyraldehyd und Natriumcyanoborhydrid in Methanol.
Ausbeute: 12 % der Theorie,
Schmelzpunkt: 175°C
$C_{30}H_{34}N_4O_3$ (498.63)
Massenspektrum: $M^+$ = 498

| | | | |
|---|---|---|---|
| Ber. | C 72.26 | H 6.87 | N 11.24 |
| Gef. | 71.79 | 6.91 | 11.35 |

Beispiel 166

(Z)-3-[1-(4-Butylaminomethyl-phenylamino)-1-phenyl-methyliden]-5-nitro-2-indolinon

Hergestellt analog Beispiel 159 aus (Z)-3-[1-(4-Aminomethylphenylamino)-1-phenyl-methyliden]-5-nitro-2-indolinon-hydrochlorid, Butyraldehyd und Natriumcyanoborhydrid in Methanol.
Ausbeute: 14 % der Theorie,
Schmelzpunkt: 183°C
$C_{26}H_{26}N_4O_3$ (442.52)
Massenspektrum: $M^+$ = 442

| | | | |
|---|---|---|---|
| Ber. | C 70.57 | H 5.97 | N 12.66 |
| Gef. | 70.33 | 6.04 | 12.44 |

Beispiel 167

(Z)-3-[1-(4-Methylsulfonylaminomethyl-phenylamino)-1-phenylmethyliden]-5-nitro-2-indolinon

Hergestellt analog Beispiel 74 aus (Z)-3-[1-(4-Methylsulfonylaminomethyl-phenylamino)-1-phenyl-methyliden]-5-nitro-1-polystyrylmethylaminocarbonyl-2-indolinon und Natronlauge in Dioxan.
Ausbeute: 16 % der Theorie,
Schmelzpunkt: 294-296°C
$C_{23}H_{20}N_4O_5S$ (464.50)
Massenspektrum: $M^+$ = 464
$C_{23}H_{20}N_4O_5S$ x $H_2O$ (482.52)

| | | | |
|---|---|---|---|
| Ber. | C 57.25 | H 4.60 | N 11.61 |
| Gef. | 57.56 | 4.67 | 11.70 |

Beispiel 168

(Z)-3-{1-[4-(4-Hydroxypiperidinomethyl)-phenylamino]-1-phenylmethyliden}-5-nitro-2-indolinon

Hergestellt analog Beispiel 89 aus 3-(1-Ethoxy-1-phenyl-methyliden)-5-nitro-2-indolinon und 4-(4-Hydroxypiperidinomethyl)-anilin in DMF.
Ausbeute: 43 % der Theorie,
Schmelzpunkt: 155°C
$C_{27}H_{26}N_4O_4$ (470.53)
Massenspektrum: $M^+$ = 470
$R_f$-Wert: 0.45 (Kieselgel; Essigester/Methanol/$NH_4OH$ = 19:1:0.1) $C_{27}H_{26}N_4O_4$ x 0.5 $H_2O$ (479.54)

| | | | |
|---|---|---|---|
| Ber. | C 67.63 | H 5.67 | N 11.68 |
| Gef. | C 67.63 | H 5.63 | N 11.59 |

Beispiel 169

(Z)-3-{1-[4-(4-Methylpiperidinomethyl)-phenylamino]-1-phenylmethyliden}-5-nitro-2-indolinon

Hergestellt analog Beispiel 89 aus 3-(1-Ethoxy-1-phenyl-methyliden)-5-nitro-2-indolinon und 4-(4-Methylpiperidinomethyl)-anilin in DMF.
Ausbeute: 92 % der Theorie,
Schmelzpunkt: 161°C
$C_{28}H_{28}N_4O_3$ (468.56)
Massenspektrum: $M^+$ = 468
$R_f$-Wert: 0.3 (Kieselgel; Essigester/Methanol = 9:1)
$C_{28}H_{28}N_4O_3$ x 0.5 $H_2O$ (477.57)

| | | | |
|---|---|---|---|
| Ber. | C 70.42 | H 6.12 | N 11.73 |
| Gef. | 70.58 | 6.25 | 11.68 |

Beispiel 170

(Z)-3-[1-(4-Piperidinomethyl-phenylamino)-1-phenyl-methyliden]-5-nitro-2-indolinon

Hergestellt analog Beispiel 89 aus 3-(1-Ethoxy-1-phenyl-methyliden)-5-nitro-2-indolinon und 4-Piperidinomethyl-anilin in DMF.

Ausbeute: 77 % der Theorie,
Schmelzpunkt: 242-243°C
$C_{27}H_{26}N_4O_3$ (454.53)
Massenspektrum: $M^+$ = 454
$R_f$-Wert: 0.3 (Kieselgel; Dichlormethan/Methanol/$NH_4OH$ = 9:1:0.1)

| | | | |
|---|---|---|---|
| Ber. | C 71.35 | H 5.77 | N 12.33 |
| Gef. | 71.40 | 6.00 | 12.37 |

Beispiel 171

(Z)-3-{1-[4-(4-Methoxypiperidinomethyl)-phenylamino]-1-phenylmethyliden}-5-nitro-2-indolinon

Hergestellt analog Beispiel 89 aus 3-(1-Ethoxy-1-phenyl-methyliden)-5-nitro-2-indolinon und 4-(4-Methoxypiperidinomethyl)-anilin in DMF.
Ausbeute: 48 % der Theorie,
Schmelzpunkt: 204-206°C
$C_{28}H_{28}N_4O_4$ (484.56)
Massenspektrum: $M^+$ = 484
$R_f$-Wert: 0.5 (Kieselgel; Dichlormethan/Methanol/$NH_4OH$ = 9:1:0.1)

| | | | |
|---|---|---|---|
| Ber. | C 69.41 | H 5.82 | N 11.56 |
| Gef. | 69.11 | 5.83 | 11.47 |

Beispiel 172

(Z)-3-{1-[4-(4-Phenylmethyl-piperidinomethyl)-phenylamino]-1-phenyl-methyliden}-5-nitro-2-indolinon

Hergestellt analog Beispiel 89 aus 3-(1-Ethoxy-1-phenyl-methyliden)-5-nitro-2-indolinon und 4-(4-Phenylmethyl-piperidino)methyl-anilin in DMF.
Ausbeute: 48 % der Theorie,
Schmelzpunkt: 252°C
$C_{34}H_{32}N_4O_3$ (544.66)
Massenspektrum: $M^+$ = 544

| | | | |
|---|---|---|---|
| Ber. | C 74.98 | H 5.92 | N 10.29 |
| Gef. | 74.52 | 5.81 | 10.23 |

Beispiel 173

(Z)-3-{1-[4-(4-Hydroxy-4-phenyl-piperidinomethyl)-phenylaminol-1-phenyl-methyliden}-5-nitro-2-indolinon

Hergestellt analog Beispiel 89 aus 3-(1-Ethoxy-1-phenyl-methyliden)-5-nitro-2-indolinon und 4-(4-Hydroxy-4-phenyl-piperidinomethyl)-anilin in DMF.
Ausbeute: 68 % der Theorie,
Schmelzpunkt: 191-194°C
$C_{33}H_{30}N_4O_4$ (546.63)
Massenspektrum: $M^+$ = 546
$R_f$-Wert: 0.4 (Kieselgel; Essigester/Methanol/$NH_4OH$ = 95:5:0.5)

| | | | |
|---|---|---|---|
| Ber. | C 72.51 | H 5.53 | N 10.25 |
| Gef. | 72.04 | 5.50 | 10.30 |

Beispiel 174

(Z)-3-{1-[4-(2-Methoxyethylaminomethyl)-phenylamino]-1-phenylmethyliden}-5-nitro-2-indolinon

Hergestellt analog Beispiel 89 aus 3-(1-Ethoxy-1-phenyl-methyliden)-5-nitro-2-indolinon und 4-(2-Methoxyethylaminomethyl)-anilin in DMF.
Ausbeute: 76 % der Theorie,
Schmelzpunkt: 184-185°C
$C_{25}H_{24}N_4O_4$ (444.49)
Massenspektrum: $(M+H)^+ = 445$
$R_f$-Wert: 0.3 (Kieselgel; Essigester/Methanol/$NH_4OH$ = 8:2:0.1)

| | | | |
|---|---|---|---|
| Ber. | C 67.56 | H 5.44 | N 12.60 |
| Gef. | 67.10 | 5.68 | 12.31 |

Beispiel 175

(2)-3-{-[4-(4-Ethylpiperidinomethyl)-phenylamino]-1-phenylmethyliden}-5-nitro-2-indolinon

Hergestellt analog Beispiel 89 aus 3-(1-Ethoxy-1-phenyl-methyliden)-5-nitro-2-indolinon und 4-(4-Ethylpiperidinomethyl)-anilin in DMF.
Ausbeute: 37 % der Theorie,
Schmelzpunkt: 225-227°C
$C_{29}H_{30}N_4O_3$ (482.59)
Massenspektrum: $[M^+H]^+ = 483$
$R_f$-Wert: 0.5 (Kieselgel; Essigester/Methanol/$NH_4OH$ = 95:5:0.5) $C_{29}H_{30}N_4O_3$ x 0.5 $H_2O$ (491.60)

| | | | |
|---|---|---|---|
| Ber. | C 70.86 | H 6.36 | N 11.40 |
| Gef. | 71.09 | 6.45 | 11.32 |

Beispiel 176

(Z)-3-{1-[4-(4-Ethoxycarbonyl-piperidinomethyl)-phenylamino]-1-phenyl-methyliden}-5-nitro-2-indolinon

Hergestellt analog Beispiel 89 aus 3-(1-Ethoxy-1-phenyl-methyliden)-5-nitro-2-indolinon und 4-(4-Ethoxycarbonyl-piperidinomethyl)-anilin in DMF.
Ausbeute: 63 % der Theorie,
Schmelzpunkt: 194°C
$C_{30}H_{30}N_4O_5$ (526.60)
Massenspektrum: $M^+ = 526$

| | | | |
|---|---|---|---|
| Ber. | C 68.43 | H 5.74 | N 10.64 |
| Gef. | 68.19 | 5.86 | 10.49 |

Beispiel 177

(Z)-3-{1-[4-(4-Carboxypiperidinomethyl)-phenylamino]-1-phenylmethyliden}-5-nitro-2-indolinon

Hergestellt analog Beispiel 8 durch Verseifung von (Z)-3-{1-[4-(4-Ethoxycarbonyl-piperidinomethyl)-phenylamino]-1-phenylmethyliden}-5-nitro-2-indolinon mit Natronlauge in Ethanol.
Ausbeute: 80 % der Theorie,
Schmelzpunkt: 207°C
$C_{28}H_{26}N_4O_5$ (498.54)
Massenspektrum: $M^+ = 498$
$C_{28}H_{26}N_4O_5$ x 0.5 $H_2O$ (507.55)

| | C | H | N |
|---|---|---|---|
| Ber. | C 66.26 | H 5.36 | N 11.04 |
| Gef. | 66.14 | 5.38 | 11.03 |

Beispiel 178

(Z)-3-{1-[4-(2-Ethoxycarbonylmethylamino-ethyl)-phenylamino]-1-phenyl-methyliden}-5-nitro-2-indolinon

Hergestellt analog Beispielen 43 und 89 aus 3-(1-Ethoxy-1-phenyl-methyliden)-5-nitro-2-indolinon und 4-(2-Ethoxycarbonylmethylamino-ethyl)-anilin in DMF.
Ausbeute: 57 % der Theorie,
Schmelzpunkt: 139-140°C
$C_{27}H_{26}N_4O_5$ (486.53)
Massenspektrum: $M^+$ = 486
$R_f$-Wert: 0.5 (Kieselgel; Essigester/Methanol = 9:1)

| | C | H | N |
|---|---|---|---|
| Ber. | C 66.66 | H 5.39 | N 11.52 |
| Gef. | 66.74 | 5.10 | 11.55 |

Beispiel 179

(Z)-3-[1-(4-Cyanomethyl-phenylamino)-1-phenyl-methyliden]-5-nitro-2-indolinon

Hergestellt analog Beispiel 82 aus 1-Acetyl-3-(1-ethoxy-1-phenyl-methyliden) -5-nitro-2-indolinon, (4-Aminophenyl)acetonitril in DMF und anschließende Behandlung mit Piperidin.
Ausbeute: 97 % der Theorie,
Schmelzpunkt: 329°C
$R_f$-Wert: 0.3 (Kieselgel; Dichlormethan/Methanol = 25:1) $C_{23}H_{16}N_4O_3$ x 0.3 $H_2O$ (401.81)

| | C | H | N |
|---|---|---|---|
| Ber. | C 68.75 | H 4.16 | N 13.94 |
| Gef. | 68.84 | 4.13 | 14.12 |

Beispiel 180

(Z)-3-[1-(4-Methoxycarbonylmethyl-phenylamino)-1-phenylmethyliden]-5-nitro-2-indolinon

Hergestellt bei der Umsetzung analog Beispiel 62 aus (Z)-3-[1-(4-Cyanomethyl-phenylamino)-1-phenyl-methyliden]-5-nitro-2-indolinon mit methanolischer Salzsäure und 1,2-Ethylendiamin.
Ausbeute: 43 % der Theorie,
Schmelzpunkt: 238-240°C
$C_{24}H_{19}$N3 O5 (429.44)
Massenspektrum: $(M+Na)^+$ = 452
$R_f$-Wert: 0.8 (Kieselgel; Dichlormethan/Methanol/$NH_4OH$ = 4:1:0.1)

Beispiel 181

(Z)-3-[1-(4-Phenylsulfonylaminomethyl-phenylamino)-1-phenylmethyliden]-2-indolinon

Hergestellt analog Beispiel 74 aus (Z)-3-[1-(4-Phenylsulfonylaminomethyl-phenylamino)-1-phenyl-methyliden]-1-polystyrylmethylaminocarbonyl-2-indolinon und Natronlauge in Dioxan.
Ausbeute: 3 % der Theorie,
$C_{28}H_{23}N_3O_3$ S (481.58)
Massenspektrum: $M^+$ = 481

Beispiel 182

(Z)-3-[1-(4-Methylsulfonylaminomethyl-phenylamino)-1-phenylmethyliden]-2-indolinon

Hergestellt analog Beispiel 74 aus (Z)-3-[1-(4-Methylsulfonylaminomethyl-phenylamino)-1-phenyl-methyliden]-1-polystyrylmethylaminocarbonyl-2-indolinon und Natronlauge in Dioxan.
Ausbeute: 8 % der Theorie,
$C_{23}H_{21}N_3O_3$ S (419.51)
Massenspektrum: $M^+ = 419$

Beispiel 183

(Z)-3-[1-(3-Methylsulfonylamino-phenylamino)-1-phenyl-methyliden]-2-indolinon

Hergestellt analog Beispiel 1 aus 1-Acetyl-3-(1-ethoxy-1-phenyl-methyliden)-2-indolinon und 3-Methylsulfonylamino-anilin in DMF und anschließende Behandlung mit Natronlauge in Methanol.
Ausbeute: 62 % der Theorie,
Schmelzpunkt: 275°C
$C_{22}H_{19}N_3O_3$ S (405.48)
Massenspektrum: $M^+ = 405$

| | | | |
|---|---|---|---|
| Ber. | C 65.18 | H 4.72 | N 10.36 |
| Gef. | 65.02 | 4.95 | 9.95 |

Beispiel 184

(Z)-3-{1-[3-(N-Methyl-N-methylsulfonyl-amino)-phenylamino]-1-phenyl-methyliden}-2-indolinon

Hergestellt analog Beispiel 36 aus (Z)-3-[1-(3-Methylsulfonylamino-phenylamino)-1-phenyl-methyliden]-2-indolinon, Methyliodid und Kaliumcarbonat in Aceten.
Ausbeute: 96 % der Theorie,
Schmelzpunkt: 261°C
$C_{23}H_{21}N_3O_3$ S (419.51)
Massenspektrum: $M^+ = 419$

Beispiel 185

(Z)-3-[1-(4-Methylsulfonylamino-phenylamino)-1-phenyl-methyliden]-2-indolinon

Hergestellt analog Beispiel 1 aus 1-Acetyl-3-(1-ethoxy-1-phenyl-methyliden)-2-indolinon und 4-Methylsulfonylamino-anilin in DMF und anschließende Behandlung mit Natronlauge in Methanol.
Ausbeute: 4 % der Theorie,
Schmelzpunkt: 299-301°C
$C_{22}H_{19}N_3O_3$ S (405.48)
Massenspektrum: $M^+ = 405$
$R_f$-Wert: 0.27 (Kieselgel; Dichlormethan/Essigester = 7:3)

Beispiel 186

(Z)-3-{1-[4-(N-Methyl-N-methylsulfonyl-amino)-phenylamino]-1-phenyl-methyliden}-2-indolinon

Hergestellt analog Beispiel 1 aus 1-Acetyl-3-(1-ethoxy-1-phenyl-methyliden)-2-indolinon und 4-(N-Methyl-N-methylsulfonylamino)-anilin in DMF und anschließende Behandlung mit Natronlauge in Methanol.
Ausbeute: 35 % der Theorie,
Schmelzpunkt: 269°C
$C_{26}H_{28}N_4O_3S$ (476.60)
Massenspektrum: $M^+ = 419$

$C_{23}H_{21}N_3O_3S$ x 0.3 $H_2O$ (424.91)

| | | | |
|---|---|---|---|
| Ber. | C 65.02 | H 5.12 | N 9.89 |
| Gef. | 65.15 | 5.07 | 9.84 |

Beispiel 187

(Z)-3-{1-[4-(N-Cyanomethyl-N-methylsulfonyl-amino)-phenylamino]-1-phenyl-methyliden}-2-indolinon

a) N-Cyanomethyl-N-methylsulfonyl-4-nitroanilin

3.24 g (15 mMol) N-Methylsulfonyl-4-nitroanilin werden in 25 ml DMSO gelöst und portionsweise mit insgesamt 2.0 g (18 mMol) Kalium-tert.butylat versetzt. Nach 1 Stunde Rühren bei Raumtemperatur werden 2.7 g (23 mMol) Bromacetonitril zugetropft. Nach 3 Stunden Rühren bei Raumtemperatur wird auf Eiswasser gegossen und die Reaktionsmischung mit Essigester extrahiert. Die organische Phase wird mit Wasser gewaschen und vom Lösungsmittel im Vakuum befreit. Der so erhaltene Rückstand wird aus Ethanol umkristallisiert.
Ausbeute: 2.3 g (60 % der Theorie),
Schmelzpunkt: 116-118°C

b) 4-(N-Cyanomethyl-N-methylsulfonyl-amino)-anilin

Hergestellt analog Beispiel 39c durch katalytische Hydrierung von N-Cyanomethyl-N-methylsulfonyl-4-nitroanilin in DMF.
Ausbeute: 62 % der Theorie,
Schmelzpunkt: 152-154°C

c) (Z)-3-{1-[4-(N-Cyanomethyl-N-methylsulfonyl-amino)-phenylamino]-1-phenyl-methyliden}-2-indolinon

Hergestellt analog Beispiel 11 aus 3-(1-Ethoxy-1-phenyl-methyliden)-2-indolinon und 4-(N-Cyanomethyl-N-methylsulfonylamino)-anilin in DMF.
Ausbeute: 74 % der Theorie,
Schmelzpunkt: 266-268°C
$C_{24}H_{20}N_4O_3$ S (444.52)
Massenspektrum: $M^+$ = 444

| | | | |
|---|---|---|---|
| Ber. | C 64.85 | H 4.53 | N 12.60 |
| Gef. | 64.82 | 4.25 | 12.43 |

Beispiel 188

(Z)-3-{1-[4-(N-(2-Dimethylamino-ethyl)-N-methylsulfonylamino)-phenylamino]-1-phenyl-methyliden}-2-indolinon

Hergestellt analog Beispiel 1 und 187 aus 1-Acetyl-3-(1-ethoxy-1-phenyl-methyliden)-2-indolinon und 4-[N-(2-Dimethylamino-ethyl)-N-methylsulfonyl-amino]-anilin in DMF und anschließender Behandlung mit Natronlauge in Methanol.
Ausbeute: 42 % der Theorie,
Schmelzpunkt: 234-235°C
$C_{26}H_{28}N_4O_3$ S (476.60)
Massenspektrum: $M^+$ = 476

| | | | |
|---|---|---|---|
| Ber. | C 65.52 | H 5.92 | N 11.76 |
| Gef. | 65.43 | 5.96 | 11.78 |

Beispiel 189

(Z)-3-{1-[4-(N-(2-Morpholinoethyl)-N-methylsulfonyl-amino)-phenylamino]-1-phenyl-methyliden}-2-indolinon

Hergestellt analog den Beispielen 1 und 187 aus 1-Acetyl-3-(1-ethoxy-1-phenyl-methyliden)-2-indolinon und 4-[N-(2-Morpholinoethyl)-N-methylsulfonyl-amino]-anilin in DMF und anschließender Behandlung mit Piperidin in Methanol.
Ausbeute: 60 % der Theorie,
Schmelzpunkt: 249-250°C
$C_{28}H_{30}N_4O_4S$ (518.64)
Massenspektrum: $M^+$ = 518
$C_{28}H_{30}N_4O_4S$ x 0.5 $H_2O$ (527.65)

| | | | |
|---|---|---|---|
| Ber. | C 63.74 | H 5.92 | N 10.62 |
| Gef. | 63.89 | 5.82 | 10.55 |

Beispiel 190

(Z)-3-{1-[4-(N-Carboxymethyl-N-methylsulfonyl-amino)-phenylamino]-1-phenyl-methyliden}-2-indolinon

Hergestellt analog den Beispielen 1 und 187 aus 1-Acetyl-3-(1-ethoxy-1-phenyl-methyliden)-2-indolinon und 4-(N-Ethoxycarbonylmethyl-N-methylsulfonyl-amino)-anilin in DMF und anschließender Behandlung mit Natronlauge in Methanol.
Ausbeute: 60 % der Theorie,
Schmelzpunkt: 247-250°C
$C_{24}H_{21}N_3$ O5 S (463.52)
Massenspektrum: $M^+$ = 463

| | | | |
|---|---|---|---|
| Ber. | C 62.19 | H 4.57 | N 9.07 |
| Gef. | 62.13 | 4.64 | 8.98 |

Beispiel 191

(Z)-3-{1-[4-(N-Aminocarbonylmethyl-N-methylsulfonyl-amino)-phenylamino]-1-phenyl-methyliden}-2-indolinon

Hergestellt analog Beispiel 18 aus (Z)-3-{1-[4-(N-Carboxymethyl-N-methylsulfonyl-amino)-phenylamino]-1-phenyl-methyliden}-2-indolinon, N-Hydroxysuccinimid-ammoniumsalz, TBTU und Triethylamin in DMF.
Ausbeute: 48 % der Theorie,
Schmelzpunkt: 276-278°C
$C_{24}H_{22}N_4O_4S$ (462.53)
Massenspektrum: $M^+$ = 462
$R_f$-Wert: 0.5 (Kieselgel; Dichlormethan/Methanol = 9:1) $C_{24}H_{22}N_4O_4S$ x 0.5 $H_2O$ (471.54)

| | | | |
|---|---|---|---|
| Ber. | C 61.13 | H 4.92 | N 11.88 |
| Gef. | 61.26 | 4.93 | 11.47 |

Beispiel 192

(Z)-3-{1-[4-(N-Methylaminocarbonylmethyl-N-methylsulfonyl-amino)-phenylamino]-1-phenyl-methyliden}-2-indolinon

Hergestellt analog Beispiel 18 aus (Z)-3-{1-[4-(N-Carboxymethyl-N-methylsulfonyl-amino)-phenylamino]-1-phenyl-methyliden}-2-indolinon, Methylammoniumchlorid, HOBt, TBTU und N-Ethyl-N,N-diisopropylamin in DMF.
Ausbeute: 77 % der Theorie,
Schmelzpunkt: 268-270°C
$C_{25}H_{24}N_4O_4S$ (476.56)
Massenspektrum: $M^+$ = 476

| | | | |
|---|---|---|---|
| Ber. | C 63.01 | H 5.08 | N 11.76 |
| Gef. | 62.83 | 5.12 | 11.60 |

Beispiel 193

(Z)-3-{1-[4-(N-Dimethylaminocarbonylmethyl-N-methylsulfonylamino)-phenylamino]-1-phenyl-methyliden}-2-indolinon

Hergestellt analog Beispiel 18 aus (Z)-3-{1-[4-(N-Carboxymethyl-N-methyl-sulfonylamino)-phenylamino]-1-phenyl-methyliden}-2-indolinon, Dimethylammoniumchlorid, HOBt, TBTU und N-Ethyl-N,N-diisopropylamin in DMF.
Ausbeute: 85 % der Theorie,
Schmelzpunkt: 260-262°C
$C_{26}H_{26}N_4O_4S$ (490.59)
Massenspektrum: $M^+ = 490$

| | | | |
|---|---|---|---|
| Ber. | C 63.66 | H 5.34 | N 11.42 |
| Gef. | 63.52 | 5.34 | 11.37 |

Beispiel 194

(Z)-3-{1-[4-(N-(2-Dimethylamino-ethylaminocarbonylmethyl)-N-methylsulfonyl-amino)-phenylamino]-1-phenyl-methyliden}-2-indolinon

Hergestellt analog Beispiel 18 aus (Z)-3-{1-[4-(N-Carboxymethyl-N-methylsulfonyl-amino)-phenylamino]-1-phenyl-methyliden}-2-indolinon, 2-Dimethylamino-ethylamin, HOBt, TBTU und N-Ethyl-N,N-diisopropylamin in DMF.
Ausbeute: 88 % der Theorie,
Schmelzpunkt: 214-216°C
$C_{28}H_{31}N_5O_4S$ (533.65)
Massenspektrum: $M^+ = 533$

| | | | |
|---|---|---|---|
| Ber. | C 63.02 | H 5.85 | N 13.12 |
| Gef. | 62.85 | 5.89 | 12.96 |

Beispiel 195

(Z)-3-{1-[4-(N-(3-Ethoxycarbonyl-propyl)-N-methylsulfonyl-amino)-phenylamino]-1-phenyl-methyliden}-2-indolinon

Hergestellt analog Beispiel 187 aus 3-(1-Ethoxy-1-phenyl-methyliden)-2-indolinon und 4-[N-(3-Ethoxycarbonyl-propyl)-N-methyl-sulfonylamino]-anilin in DMF.
Ausbeute: 60 % der Theorie,
Schmelzpunkt: 265-268°C
$C_{28}H_{29}N_3O_5S$ (519.62)
Massenspektrum: $M^+ = 519$

| | | | |
|---|---|---|---|
| Ber. | C 64.72 | H 5.63 | N 8.09 |
| Gef. | 64.82 | 5.68 | 8.01 |

Beispiel 196

(Z)-3-[(4-Methylsulfonylamino-phenylamino)-1-phenyl-methyliden]-5-nitro-2-indolinon

Hergestellt analog Beispiel 82 aus 1-Acetyl-3-(1-ethoxy-1-phenyl-methyliden)-5-nitro-2-indolinon und 4-Methylsulfonylamino-anilin in DMF und anschließender Behandlung mit Natronlauge in Methanol.
Ausbeute: 74 % der Theorie,
Schmelzpunkt: 344-346°C

$C_{22}H_{18}N_4O_5S$ (450.48)
Massenspektrum: $M^+$ = 450

| | | | |
|---|---|---|---|
| Ber. | C 58.66 | H 4.03 | N 12.44 |
| Gef. | 58.22 | 4.18 | 12.44 |

Beispiel 197

(Z)-3-{1-[4-(N-Methyl-N-methylsulfonyl-amino)-phenylamino]-1-phenyl-methyliden}-5-nitro-2-indolinon

Hergestellt analog Beispiel 82 aus 1-Acetyl-3-(1-ethoxy-1-phenyl-methyliden)-5-nitro-2-indolinon und 4-(N-Methyl-N-methylsulfonylamino)-anilin in DMF und anschließender Behandlung mit Natronlauge in Methanol.
Ausbeute: 91 % der Theorie,
Schmelzpunkt: 306-308°C
$C_{23}H_{20}N_4O_5S$ (464.50)
Massenspektrum: $M^+$ = 464

| | | | |
|---|---|---|---|
| Ber. | C 59.47 | H 4.34 | N 12.06 |
| Gef. | 59.45 | 4.52 | 12.10 |

Beispiel 198

(Z)-3-{1-[4-(N-Ethoxycarbonylmethyl-N-methylsulfonyl-amino)-phenylamino]-1-phenyl-methyliden}-5-nitro-2-indolinon

Hergestellt analog den Beispielen 89 und 187 aus 3-(1-Ethoxy-1-phenyl-methyliden)-5-nitro-2-indolinon und 4-(N-Ethoxycarbonylmethyl-N-methylsulfonyl-amino)-anilin in DMF.
Ausbeute: 86 % der Theorie,
Schmelzpunkt: 236-238°C
$C_{26}H_{24}N_4O_7S$ (536.57)
Massenspektrum: $M^+$ = 536

| | | | |
|---|---|---|---|
| Ber. | C 58.20 | H 4.51 | N 10.44 |
| Gef. | 58.16 | 4.69 | 10.45 |

Beispiel 199

(Z)-3-{1-[4-(N-Carboxymethyl-N-methylsulfonyl-amino)-phenylamino]-1-phenyl-methyliden}-5-nitro-2-indolinon

Hergestellt analog Beispiel 8 durch Verseifung von (Z)-3-{1-[4-(N-Ethoxycarbonylmethyl-N-methylsulfonyl-amino)-phenylamino]-1-phenyl-methyliden}-5-nitro-2-indolinon mit Natronlauge in Dioxan.
Ausbeute: 89 % der Theorie,
Schmelzpunkt: 180-183°C
$C_{24}H_{20}N_4O_7S$ (508.51)
Massenspektrum: $M^+$ = 508
$C_{24}H_{20}N_4O_7S$ x 0.5 $C_4H_8O_2$ (552.56)

| | | | |
|---|---|---|---|
| Ber. | C 56.52 | H 4.38 | N 10.14 |
| Gef. | 56.52 | 4.56 | 9.96 |

Beispiel 200

(Z)-3-{1-[4-(N-Methylaminocarbonylmethyl-N-methylsulfonyl-amino)-phenylamino]-1-phenyl-methyliden}-5-nitro-2-indolinon

Hergestellt analog Beispiel 18 aus (Z)-3-{1-[4-(N-Carboxymethyl-N-methylsulfonyl-amino)-phenylamino]-1-phenyl-methyliden}-5-nitro-2-indolinon, Methylammoniumchlorid, HOBt, TBTU und N-Ethyl-diisopropylamin in DMF.
Ausbeute: 47 % der Theorie
Schmelzpunkt: 267-268°C
$C_{25}H_{23}N_5O_6S$ (521.56)
Massenspektrum: $M^+$ = 521

| | | | |
|---|---|---|---|
| Ber. | C 57.57 | H 4.44 | N 13.43 |
| Gef. | 57.44 | 4.69 | 13.02 |

Beispiel 201

(Z)-3-{1-[4-(N-Dimethylaminocarbonylmethyl-N-methylsulfonyl-amino)-phenylamino]-1-phenyl-methyliden}-5-nitro-2-indolinon

Hergestellt analog Beispiel 18 aus (Z)-3-{1-[4-(N-Carboxymethyl-N-methylsulfonyl-amino)-phenylamino]-1-phenyl-methyliden}-5-nitro-2-indolinon, Dimethylammoniumchlorid, HOBt, TBTU und N-Ethyl-N,N-diisopropylamin in DMF.
Ausbeute: 80 % der Theorie,
Schmelzpunkt: 277-280°C
$C_{26}H_{25}N_5O_6S$ (535.58)
Massenspektrum: $(M+H)^+$ = 536

Beispiel 202

(Z)-3-{1-[4-(N-(2-Dimethylamino-ethyl)-N-methylsulfonyl-amino)-phenylamino]-1-phenyl-methyliden}-5-nitro-2-indolinon

Hergestellt analog den Beispielen 1 und 187 aus 1-Acetyl-3-(1-ethoxy-1-phenyl-methyliden)-5-nitro-2-indolinon und 4-[N-(2-Dimethylamino-ethyl)-N-methylsulfonyl-amino]-anilin in DMF und anschließender Behandlung mit Natronlauge in Methanol.
Ausbeute: 86 % der Theorie,
Schmelzpunkt: 276-277°C
$C_{26}H_{27}N_5O_5S$ (521.60)
Massenspektrum: $M^+$ = 521

| | | | |
|---|---|---|---|
| Ber. | C 59.87 | H 5.22 | N 13.43 |
| Gef. | 60.03 | 5.19 | 13.39 |

Beispiel 203

(Z)-3-{1-[4-(N-(2-Morpholinoethyl)-N-methylsulfonyl-amino)-phenylamino]-1-phenyl-methyliden}-5-nitro-2-indolinon

Hergestellt analog den Beispielen 1 und 187 aus 1-Acetyl-3-(1-ethoxy-1-phenyl-methyliden)-5-nitro-2-indolinon und 4-[N-(2-Morpholinoethyl)-N-methylsulfonyl-amino]-anilin in DMF und anschließender Behandlung mit Piperidin in Methanol.
Ausbeute: 62 % der Theorie,
Schmelzpunkt: 255-257°C
$C_{28}H_{29}N_5O_6S$ (563.64)
Massenspektrum: $M^+$ = 563

| | | | |
|---|---|---|---|
| Ber. | C 59.67 | H 5.19 | N 12.43 |
| Gef. | 59.20 | 5.30 | 12.18 |

Beispiel 204

(Z)-3-{1-[4-(N-Dimethylaminocarbonylmethyl-N-ethylsulfonylamino)-phenylamino]-1-phenyl-methyliden}-2-indolinon

Hergestellt analog den Beispielen 1 und 187 aus (Z)-1-Acetyl-3-[1-(4-ethylsulfonylamino-phenylamino)-1-phenyl-methyliden]-2-indolinon, Bromessigsäure-N,N-dimethylamid und Kalium-tert.butylat in DMSO und anschließende Behandlung mit Natronlauge in Methanol.
Ausbeute: 30 % der Theorie,
Schmelzpunkt: 206-208°C
$C_{27}H_{28}N_4O_4S$ (504.61)
Massenspektrum: $M^+$ = 504
$C_{27}H_{28}N_4O_4S$ x 0.5 $H_2O$ (513.62)

| | | | |
|---|---|---|---|
| Ber. | C 63.14 | H 5.69 | N 10.91 |
| Gef. | 63.25 | 5.62 | 10.93 |

Beispiel 205

(Z)-3-{1-[4-(N-Dimethylaminocarbonylmethyl-N-phenylsulfonylamino)-phenylamino]-1-phenyl-methyliden}-2-indolinon

Hergestellt analog den Beispielen 1 und 187 aus (Z)-1-Acetyl-3-[1-(4-phenylsulfonylamino-phenylamino)-1-phenyl-methyliden]-2-indolinon, Bromessigsäure-N,N-dimethylamid und Kalium-tert.-butylat in DMSO und anschließende Behandlung mit Natronlauge in Methanol.
Ausbeute: 36 % der Theorie,
Schmelzpunkt: 255-258°C
$C_{31}H_{28}N_4O_4S$ (552.66)
Massenspektrum: $M^+$ = 552

Beispiel 206

(Z)-3-{1-[4-(N-Dimethylaminocarbonylmethyl-N-(p-tolylsulfonyl)-amino)-phenylamino]-1-phenyl-methyliden}-2-indolinon

Hergestellt analog den Beispielen 1 und 187 aus (Z)-1-Acetyl-3-{1-[4-(p-tolylsulfonylamino)-phenylamino]-1-phenyl-methyliden}-2-indolinon, Bromessigsäure-N,N-dimethylamid und Kaliumtert.butylat in DMSO und anschließender Behandlung mit Natronlauge in Methanol.
Ausbeute: 40 % der Theorie,
Schmelzpunkt: 223-226°C
$C_{32}H_{30}N_4O_4S$ (566.68)
Massenspektrum: $M^+$ = 566
$C_{32}H_{30}N_4O_4S$ x 0.5 $H_2O$ (575.68)

| | | | |
|---|---|---|---|
| Ber. | C 66.76 | H 5.43 | N 9.73 |
| Gef. | 66.54 | 5.49 | 9.81 |

Beispiel 207

(Z)-3-{1-[4-(N-Dimethylaminocarbonylmethyl-N-benzylsulfonylamino)-phenylamino]-1-phenyl-methyliden}-2-indolinon

Hergestellt analog den Beispielen 1 und 187 aus (Z)-1-Acetyl-3-[1-(4-benzylsulfonylamino-phenylamino)-1-phenyl-methyliden]-2-indolinon, Bromessigsäure-N,N-dimethylamid und Kalium-tert.-butylat in DMSO und anschließender Behandlung mit Natronlauge in Methanol.
Ausbeute: 77 % der Theorie,
Schmelzpunkt.: 133-135°C
$C_{32}H_{30}N_4O_4S$ (566.68)

Massenspektrum: $M^+$ = 566
$C_{32}H_{30}N_4O_4S$ x $H_2O$ (584.69)

| | | | |
|---|---|---|---|
| Ber. | C 65.74 | H 5.52 | N 9.58 |
| Gef. | 65.62 | 5.59 | 9.53 |

Beispiel 208

(Z)-3-{1-[4-(N-Dimethylaminocarbonylmethyl-N-ethylsulfonylamino)-phenylamino]-1-phenyl-methyliden}-5-nitro-2-indolinon

Hergestellt analog den Beispielen 82 und 187 aus (Z)-1-Acetyl-3-[1-(4-ethylsulfonylamino-phenylamino)-1-phenyl-methyliden]-5-nitro-2-indolinon, Bromessigsäure-N,N-dimethylamid und Kalium-tert.butylat in DMSO und anschließende Behandlung mit Natronlauge in Methanol.
Ausbeute: 27 % der Theorie,
Schmelzpunkt: 145-148°C
$C_{27}H_{27}N_5O_6S$ (549.61)
Massenspektrum: $M^+$ = 549
$R_f$-Wert : 0.42 (Kieselgel; Dichlormethan/Methanol = 19:1)

| | | | |
|---|---|---|---|
| Ber. | C 59.01 | H 4.95 | N 12.74 |
| Gef. | 59.20 | 4.96 | 12.26 |

Beispiel 209

(Z)-3-{1-[4-(N-Dimethylaminocarbonylmethyl-N-phenylsulfonylamino)-phenylamino]-1-phenyl-methyliden}-5-nitro-2-indolinon

Hergestellt analog den Beispielen 82 und 187 aus (Z)-1-Acetyl-3-[1-(4-phenylsulfonylamino-phenylamino)-1-phenyl-methyliden]-5-nitro-2-indolinon, Bromessigsäure-N,N-dimethylamid und Kalium-tert.butylat in DMSO und anschließende Behandlung mit Natronlauge in Methanol.
Ausbeute: 13 % der Theorie,
Schmelzpunkt: 160-162°C
$C_{31}H_{27}N_5O_6S$ (597.65)
Massenspektrum: $M^+$ = 597

Beispiel 210

(Z)-3-{1-[4-(N-Dimethylaminocarbonylmethyl-N-(p-tolylsulfonyl)-amino)-phenylamino]-1-phenyl-methyliden}-5-nitro-2-indolinon

Hergestellt analog den Beispielen 82 und 187 aus (Z)-1-Acetyl-3-{1-[4-(p-tolylsulfonylamino)-phenylamino]-1-phenyl-methyliden}-5-nitro-2-indolinon, Bromessigsäure-N,N-dimethylamid und Kalium-tert.butylat in DMSO und anschließender Behandlung mit Natronlauge in Methanol.
Ausbeute: 40 % der Theorie,
Schmelzpunkt: 198-200°C
$C_{32}H_{29}N_5O_6S$ (611.68)
Massenspektrum: $M^+$ = 611
$C_{32}H_{29}N_5O_6S$ x $H_2O$ (629.69)

| | | | |
|---|---|---|---|
| Ber. | C 61.04 | H 4.96 | N 11.12 |
| Gef. | 59.92 | 4.53 | 10.87 |

Beispiel 211

(Z)-3-[1-(4-Dimethylaminomethyl-phenylamino)-1-(p-tolyl)-methyliden]-2-indolinon

Hergestellt analog Beispiel 2 aus 1-Acetyl-3-[1-chlor-1-(p-tolyl)-methyliden)-2-indolinon und 4-Dimethylaminomethyl-anilin in DMF und anschließende Behandlung mit Natronlauge in Methanol.
Ausbeute: 27 % der Theorie,
Schmelzpunkt: 208-209°C
$C_{25}H_{25}N_3O$ (383.50)
Massenspektrum: $M^+$ = 383
$R_f$-Wert: 0.35 (Kieselgel; Essigester/Methanol/$NH_4OH$ = 8:2:0.1) $C_{25}H_{25}N_3O$ x 0.3 $H_2O$ (388.89)

| | | | |
|---|---|---|---|
| Ber. | C 77.21 | H 6.63 | N 10.80 |
| Gef. | 77.45 | 6.39 | 10.70 |

Beispiel 212

(Z)-3-[1-(4-Dimethylaminomethyl-phenylamino)-1-(p-tolyl)-methyliden]-5-nitro-2-indolinon

Hergestellt analog Beispiel 2 aus 1-Acetyl-3-[1-chlor-1-(p-tolyl)-methyliden)-5-nitro-2-indolinon und 4-Dimethylaminomethyl-anilin in DMF und anschließende Behandlung mit Natronlauge in Methanol.
Ausbeute: 84 % der Theorie,
Schmelzpunkt: 274-276°C
$C_{25}H_{24}N_4O_3$ (428.49)
Massenspektrum: $(M+H)^+$ = 429; $(M-H)^-$ = 427; $M^+$ = 428
$R_f$-Wert: 0.5 (Kieselgel; Dichlormethan/Methanol/$NH_4OH$ = 9:1:0.1)

| | | | |
|---|---|---|---|
| Ber. | C 70.08 | H 5.65 | N 13.07 |
| Gef. | 70.17 | 5.50 | 12.86 |

Beispiel 213

(Z)-3-[1-(4-Dimethylaminomethyl-phenylamino)-1-(m-tolyl)-methyliden]-2-indolinon

Hergestellt analog Beispiel 2 aus 1-Acetyl-3-[1-chlor-1-(m-tolyl)-methyliden)-2-indolinon und 4-Dimethylaminomethyl-anilin in DMF und anschließende Behandlung mit Natronlauge in Methanol.
Ausbeute: 36 % der Theorie,
Schmelzpunkt: 224-226°C
$C_{25}H_{25}N_3O$ (383.50)
Massenspektrum: $M^+$ = 383
$R_f$-Wert: 0.25 (Kieselgel; Essigester/Methanol/$NH_4OH$ = 8:2:0.1)

| | | | |
|---|---|---|---|
| Ber. | C 77.30 | H 6.57 | N 10.96 |
| Gef. | 77.27 | 6.74 | 10.74 |

Beispiel 214

(Z)-3-[1-(4-Dimethylaminomethyl-phenylamino)-1-(m-tolyl)-methyliden]-5-nitro-2-indolinon

Hergestellt analog Beispiel 2 aus 1-Acetyl-3-[1-chlor-1-(m-tolyl)-methyliden)-5-nitro-2-indolinon und 4-Dimethylaminomethyl-anilin in DMF und anschließende Behandlung mit Natronlauge in Methanol.
Ausbeute: 20 % der Theorie,
Schmelzpunkt: 210°C
$C_{25}H_{24}N_4O_3$ (428.49)
Massenspektrum: $M^+$ = 428

| | | | |
|---|---|---|---|
| Ber. | C 70.08 | H 5.65 | N 13.08 |
| Gef. | 69.63 | 5.94 | 12.89 |

Beispiel 215

(Z)-3-[1-(4-Dimethylaminomethyl-phenylamino)-1-(4-methoxyphenyl)-methyliden]-2-indolinon

Hergestellt analog Beispiel 2 aus 1-Acetyl-3-[1-chlor-1-(4-methoxyphenyl)-methyliden)-2-indolinon und 4-Dimethylaminomethyl-anilin in DMF und anschließende Behandlung mit Natronlauge in Methanol.
Ausbeute: 46 % der Theorie,
Schmelzpunkt: 206-207°C
$C_{25}H_{25}N_3O_2$ (399.50)
Massenspektrum: $M^+ = 399$
$R_f$-Wert: 0.3 (Kieselgel; Essigester/Methanol/$NH_4OH$ = 8:2:0.1) $C_{25}H_{25}N_3O_2$ x 0.5 $H_2O$ (408.50)

| | | | |
|---|---|---|---|
| Ber. | C 73.51 | H 6.42 | N 10.29 |
| Gef. | 73.81 | 6.58 | 10.15 |

Beispiel 216

(Z)-3-[1-(4-Dimethylaminomethyl-phenylamino)-1-(4-methoxyphenyl)-methyliden]-5-nitro-2-indolinon

Hergestellt analog Beispiel 2 aus 1-Acetyl-3-[1-chlor-1-(4-methoxyphenyl)-methyliden)-5-nitro-2-indolinon und 4-Dimethylaminomethyl-anilin in DMF und anschließende Behandlung mit Natronlauge in Methanol.
Ausbeute: 76 % der Theorie,
Schmelzpunkt: 259-262°C
$C_{25}H_{24}N_4O_4$ (444.49)
Massenspektrum: $M^+ = 444$
$R_f$-Wert: 0.6 (Kieselgel; Dichlormethan/Methanol = 9:1)

| | | | |
|---|---|---|---|
| Ber. | C 67.56 | H 5.44 | N 12.60 |
| Gef. | 67.49 | 5.48 | 12.39 |

Beispiel 217

(Z)-3-[1-(4-Dimethylaminomethyl-phenylamino)-1-(3-methoxyphenyl)-methyliden]-2-indolinon

Hergestellt analog Beispiel 2 aus 1-Acetyl-3-[1-chlor-1-(3-methoxyphenyl)-methyliden)-2-indolinon und 4-Dimethylaminomethyl-anilin in DMF und anschließende Behandlung mit Natronlauge in Methanol.
Ausbeute: 49 % der Theorie,
Schmelzpunkt: 193-194°C
$C_{25}H_{25}N_3O_2$ (399.50)
Massenspektrum: $M^+ = 399$
$R_f$-Wert: 0.3 (Kieselgel; Essigester/Methanol/$NH_4OH$ 8:2:0.1)

| | | | |
|---|---|---|---|
| Ber. | C 75.16 | H 6.31 | N 10.52 |
| Gef. | 75.16 | 6.32 | 10.59 |

Beispiel 218

(Z)-3-[1-(4-Dimethylaminomethyl-phenylamino)-1-(3-methoxyphenyl)-methyliden]-5-nitro-2-indolinon

Hergestellt analog Beispiel 2 aus 1-Acetyl-3-[1-chlor-1-(3-methoxyphenyl)-methyliden)-5-nitro-2-indolinon und 4-Dimethylaminomethyl-anilin in DMF und anschließende Behandlung mit Natronlauge in Methanol.
Ausbeute: 38 % der Theorie,.

Schmelzpunkt: 206-208°C
$C_{25}H_{24}N_4O_4$ (444.49)
Massenspektrum: $M^+$ = 444
$R_f$-Wert: 0.5 (Kieselgel; Dichlormethan/Methanol/$NH_4OH$ = 9:1:0.1)

| | | | |
|---|---|---|---|
| Ber. | C 67.56 | H 5.44 | N 12.60 |
| Gef. | 67.12 | 5.38 | 12.33 |

Beispiel 219

(Z)-3-[1-(4-Dimethylaminomethyl-phenylamino)-1-(4-nitrophenyl)-methyliden]-2-indolinon

Hergestellt analog Beispiel 2 aus 1-Acetyl-3-[1-chlor-1-(4-nitrophenyl)-methyliden)-2-indolinon und 4-Dimethylaminomethyl-anilin in DMF und anschließende Behandlung mit Natronlauge in Methanol.
Ausbeute: 39 % der Theorie,
Schmelzpunkt: 235-235°C
$C_{24}H_{22}N_4O_3$ (414.47)
Massenspektrum: $M^+$ = 414
$R_f$-Wert: 0.5 (Kieselgel; Dichlormethan/Methanol/$NH_4OH$ = 9:1:0.1)

| | | | |
|---|---|---|---|
| Ber. | C 69.55 | H 5.35 | N 13.52 |
| Gef. | 69.52 | 5.58 | 13.42 |

Beispiel 220

(Z)-3-[1-(4-Dimethylaminomethyl-phenylamino)-1-(4-nitrophenyl)-methyliden]-5-nitro-2-indolinon

Hergestellt analog Beispiel 2 aus 1-Acetyl-3-[1-chlor-1-(4-nitrophenyl)-methyliden)-5-nitro-2-indolinon und 4-Dimethylaminomethyl-anilin in DMF und anschließende Behandlung mit Natronlauge in Methanol.
Ausbeute: 22 % der Theorie,
Schmelzpunkt: 233°C
$C_{24}H_{21}N_5O_5$ (459.47)
Massenspektrum: $M^+$ = 459

| | | | |
|---|---|---|---|
| Ber. | C 62.74 | H 4.61 | N 15.24 |
| Gef. | 62.60 | 4.91 | 15.33 |

Beispiel 221

(Z)-3-[1-(4-Dimethylaminomethyl-phenylamino)-1-(4-chlorphenyl)-methyliden]-2-indolinon

Hergestellt analog Beispiel 2 aus 1-Acetyl-3-[1-chlor-1-(4-chlorphenyl)-methyliden)-2-indolinon und 4-Dimethylaminomethyl-anilin in DMF und anschließende Behandlung mit Natronlauge in Methanol.
Ausbeute: 46 % der Theorie,
Schmelzpunkt: 213°C
$C_{24}H_{22}ClN_3O$ (403.92)
Massenspektrum: $M^+$ = 405/403
$R_f$-Wert: 0.4 (Kieselgel; Essigester/Methanol/$NH_4OH$ = 8:2:0.1) $C_{24}H_{22}ClN_3O$ x 0.5 $H_2O$ (412.92)

| | | | |
|---|---|---|---|
| Ber. | C 69.81 | H 5.61 | N 10.18 |
| Gef. | 70.06 | 5.87 | 10.13 |

Beispiel 222

(Z)-3-[1-(4-Dimethylaminomethyl-phenylamino)-1-(4-chlorphenyl)-methyliden]-5-nitro-2-indolinon

Hergestellt analog Beispiel 2 aus 1-Acetyl-3-[1-chlor-1-(4-chlorphenyl)-methyliden)-5-nitro-2-indolinon und 4-Dimethylaminomethyl-anilin in DMF und anschließende Behandlung mit Natronlauge in Methanol.
Ausbeute: 36 % der Theorie,
Schmelzpunkt: 311°C
$C_{24}H_{21}ClN_4O_3$ (448.91)
Massenspektrum: $M^+$ = 450/448
$R_f$-Wert: 0.85 (Kieselgel; Dichlormethan/Methanol = 8:2)

| | | | |
|---|---|---|---|
| Ber. | C 64.21 | H 4.71 | N 12.48 |
| Gef. | 64.13 | 4.73 | 12.20 |

Beispiel 223

(Z)-3-[1-(4-Dimethylaminomethyl-phenylamino)-1-(3-chlorphenyl)-methyliden]-2-indolinon

Hergestellt analog Beispiel 2 aus 1-Acetyl-3-[1-chlor-1-(3-chlorphenyl)-methyliden)-2-indolinon und 4-Dimethylaminomethyl-anilin in DMF und anschließende Behandlung mit Natronlauge in Methanol.
Ausbeute: 14 % der Theorie,
Schmelzpunkt: 197-198°C
$C_{24}H_{22}ClN_3O$ (403.92)
Massenspektrum: $M^+$ = 405/403
$C_{24}H_{22}ClN_3O \times 0.5\ H_2O$ (412.92)

| | | | |
|---|---|---|---|
| Ber. | C 69.81 | H 5.61 | N 10.18 |
| Gef. | 69.74 | 5.63 | 10.07 |

Beispiel 224

(Z)-3-[1-(4-Dimethylaminomethyl-phenylamino)-1-(3-chlorphenyl)-methyliden]-5-nitro-2-indolinon

Hergestellt analog Beispiel 2 aus 1-Acetyl-3-[1-chlor-1-(3-chlorphenyl)-methyliden)-5-nitro-2-indolinon und 4-Dimethylaminomethyl-anilin in DMF und anschließende Behandlung mit Natronlauge in Methanol.
Ausbeute: 20 % der Theorie,
Schmelzpunkt: 274°C
$C_{24}H_{21}ClN_4O_3$ (448.91)
Massenspektrum: $M^+$ = 450/448
$C_{24}H_{21}ClN_4O_3 \times 0.5\ H_2O$ (457.92)

| | | | |
|---|---|---|---|
| Ber. | C 62.95 | H 4.84 | N 12.24 |
| Gef. | 62.97 | 4.81 | 12.29 |

Beispiel 225

(Z)-3-{1-[4-(2-tert.Butoxycarbonylamino-2-methoxycarbonylethyl)-phenylamino]-1-phenyl-methyliden}-5-nitro-2-indolinon

Hergestellt analog Beispiel 89.
Schmelzpunkt: 139°C
$C_{30}H_{30}N_4O_7$ (558.60)
Massenspektrum: $M^+$ = 558

| | | | |
|---|---|---|---|
| Ber. | C 64.51 | H 5.41 | N 10.03 |

(fortgesetzt)

| | | | |
|---|---|---|---|
| Gef. | 64.02 | 5.56 | 9.98 |

Beispiel 226

(Z)-3-{1-[4-(2-tert.Butoxycarbonylamino-2-carboxy-ethyl)-phenylamino]-1-phenyl-methyliden}-5-nitro-2-indolinon

Hergestellt analog Beispiel 8.
Schmelzpunkt: 235°C (Zers.)
$C_{29}H_{28}N_4O_7$ (544.57)
Massenspektrum: $M^+ = 544$
$C_{29}H_{28}N_4O_7 \times H_2O$ (562.59)

| | | | |
|---|---|---|---|
| Ber. | C 61.01 | H 5.37 | N 9.96 |
| Gef. | 62.45 | 5.40 | 10.06 |

Beispiel 227

(Z)-3-{1-[4-(2-Amino-2-methoxycarbonyl-ethyl)-phenylamino]-1-phenyl-methyliden}-5-nitro-2-indolinon-hydrochlorid

Hergestellt analog Beispiel 29a.
Schmelzpunkt: 215°C (Zers.)
$C_{25}H_{22}N_4O_5$ (458.48)
Massenspektrum: $M^+ = 458$
$C_{25}H_{22}N_4O_5 \times HCl \times H_2O$ (521.96)

| | | | |
|---|---|---|---|
| Ber | C 57.53 | H 5.02 | N 10.73 |
| Gef | 57.54 | 5.13 | 10.59 |

Beispiel 228

(Z)-3-{1-[4-(2-Amino-2-carboxy-ethyl)-phenylamino]-1-phenylmethyliden}-5-nitro-2-indolinon-hydrochlorid

Hergestellt analog Beispiel 29a.
Schmelzpunkt: 225°C (Zers.)
$C_{24}H_{20}N_4O_5$ (444.45)
Massenspektrum: $[M-CO_2]^+ = 400$
$C_{24}H_{20}N_4O_5 \times HCl \times 2\ H_2O$ (516.94)

| | | | |
|---|---|---|---|
| Ber | C 55.76 | H 4.87 | N 10.84 |
| Gef | 55.81 | 5.15 | 10.82 |

Beispiel 229

(Z)-3-[1-(4-Thiomorpholinomethyl-phenylamino)-1-phenyl-methyliden]-5-nitro-2-indolinon

Hergestellt analog Beispiel 89.
Schmelzpunkt: 276-277°C
$C_{26}H_{24}N_4O_3S$ (472.57)
Massenspektrum: $M^+ = 472$

| | | | |
|---|---|---|---|
| Ber. | C 66.08 | H 5.12 | N 11.86 |
| Gef. | 65.89 | 5.24 | 11.84 |

Beispiel 230

(Z)-3-{1-[4-((1-Oxothiomorpholino)-methyl)-phenylamino]-1-phenyl-methyliden}-5-nitro-2-indolinon

a) 4-(4-Nitrophenylmethyl)-thiomorpholin-1-oxid

Zu einer Lösung von 11.5 g (48 mMol) 4-(4-Nitrophenylmethyl)-thiomorpholin in 100 ml Dichlormethan werden bei Raumtemperatur 11.7 g (58 mMol) m-Chlorperbenzoesäure gegeben. Man rührt 4 Stunden bei Raumtemperatur und wäscht die Reaktionslösung anschließend mit 1N Natronlauge und Wasser und engt bis zur Trockene ein. Chromatographie an Kieselgel (Dichlormethan/Methanol = 9:1) liefert das Produkt.
Ausbeute: 3.9 g (32 % der Theorie),
$C_{11}H_{14}N_2O_3S$ (254.31)
Massenspektrum: $M^+$ = 254

b) 4-(4-Aminophenylmethyl)-thiomorpholin-1-oxid

Zu einer Lösung von 3.9 g (15 mMol) 4-(4-Nitrophenylmethyl)-thiomorpholin-1-oxid in 10 ml Dichlormethan und 40 ml Methanol werden 1.2 g Raney-Nickel gegeben. Man hydriert unter einer Wasserstoffatmosphäre. Chromatographie an Kieselgel (Dichlormethan/Methanol = 9:1) liefert das Produkt.
Ausbeute: 1.8 g (51 % der Theorie).

c) (Z)-3-{1-[4-((1-Oxo-thiomorpholino)-methyl)-phenylamino]-1-phenyl-methyliden}-5-nitro-2-indolinon

Hergestellt analog Beispiel 89.
Schmelzpunkt: 289-290°C
$C_{26}H_{24}N_4O_4S$ (488.57)
Massenspektrum: $M^+$ = 488

| | | | |
|---|---|---|---|
| Ber. | C 63.92 | H 4.95 | N 11.47 |
| Gef. | 63.90 | 5.09 | 11.41 |

Beispiel 231

(Z)-3-{1-[4-((1,1-Dioxothiomorpholino)-methyl)-phenylamino]-1-phenyl-methyliden}-5-nitro-2-indolinon

a) 4-(4-Nitrophenylmethyl)-thiomorpholin-1,1-dioxid

In 100 ml Aceton werden 8.6 g (40 mMol) 4-Nitrobenzylbromid gelöst. Man gibt 6.9 g (50 mMol) Kaliumcarbonat und 5.4 g (40 mMol) Thiomorpholin-1,1-dioxid zu. Die Reaktionslösung wird 7 Stunden bei Raumtemperatur gerührt. Nach Filtrieren von nicht gelöstem Feststoff wird die Lösung eingedampft. Der Rückstand wird zwischen Essigester und Wasser verteilt. Die organischen Phasen werden vom Lösungsmittel in Vakuum befreit. Das Produkt wird mit Ether verrieben und getrocknet.
Ausbeute: 7.2 g (67 % der Theorie),
Schmelzpunkt: 181-182°C

b) 4-(4-Aminophenylmethyl)-thiomorpholin-1,1-dioxid

Hergestellt analog Beispiel 230b.
Schmelzpunkt: 171-172°C

c) (Z)-3-{1-[4-((1,1-Dioxothiomorpholino)-methyl)-phenylamino]-1-phenyl-methyliden}-5-nitro-2-indolinon

Hergestellt analog Beispiel 89.
Schmelzpunkt: 328-329°C (Zers.)
$C_{26}H_{24}N_4O_5S$ (504.57)
Massenspektrum: $M^+$ = 504

| | | | |
|---|---|---|---|
| Ber. | C 61.89 | H 4.79 | N 11.10 |
| Gef. | 61.90 | 5.03 | 11.10 |

Beispiel 232

(Z) -3-{1-[4-(N-Cyclohexyl-N-methyl-aminomethyl)-phenylamino]-1-phenyl-methyliden}-5-nitro-2-indolinon

Hergestellt analog Beispiel 231.
Ausbeute: 44 % der Theorie,
Schmelzpunkt: 215°C
$C_{29}H_{30}N_4O_3$ (482.59)
Massenspektrum: $M^+ = 482$

Beispiel 233

(Z)-3-{1-[4-(Phenylaminomethyl)-phenylamino]-1-phenyl-methyliden}-5-nitro-2-indolinon

Hergestellt analog Beispiel 231.
Schmelzpunkt: 274-277°C
$C_{28}H_{22}N_4O_3$ (462.51)
Massenspektrum: $M^+ = 462$

| | | | |
|---|---|---|---|
| Ber. | C 72.71 | H 4.79 | N 12.11 |
| Gef. | 72.61 | 4.91 | 12.09 |

Beispiel 234

(Z)-3-{1-[4-(N-Methyl-N-phenyl-aminomethyl)-phenylamino]-1-phenyl-methyliden}-5-nitro-2-indolinon

Hergestellt analog Beispiel 231.
Schmelzpunkt: 228-230°C
$C_{29}H_{24}N_4O_3$ (476.54)
Massenspektrum: $M^+ = 476$

| | | | |
|---|---|---|---|
| Ber. | C 73.09 | H 5.08 | N 11.76 |
| Gef. | 72.79 | 5.25 | 11.56 |

Beispiel 235

(Z)-3-{1-[4-(N-Methyl-N-(2-pyridyl)-aminomethyl)-phenylamino]-1-phenyl-methyliden}-5-nitro-2-indolinon

Hergestellt analog Beispiel 231.
Schmelzpunkt: 213°-216°C
$C_{28}H_{23}N_5O_3$ (477.53)
Massenspektrum: $M^+ = 477$

Beispiel 236

(Z)-3-{1-[4-(N-Benzyl-N-tert.butoxycarbonyl-aminomethyl)-phenylamino]-1-phenyl-methyliden}-5-nitro-2-indolinon

Hergestellt analog Beispiel 231.
Schmelzpunkt: 202-203°C (Zers.)
$C_{34}H_{32}N_4O_5$ (576.66)
Massenspektrum: $M^+ = 576$

| | | | |
|---|---|---|---|
| Ber. | C 70.82 | H 5.59 | N 9.72 |
| Gef. | 70.81 | H 5.74 | N 9.65 |

Beispiel 237

(Z)-3-{1-[4-(Benzylaminomethyl)-phenylamino]-1-phenyl-methyliden}-5-nitro-2-indolinon-hydrochlorid

Hergestellt analog Beispiele 236 und 29a.
Schmelzpunkt: 298-300°C
$C_{29}H_{24}N_4O_3$ (476.534)
Massenspektrum: $M^+$ = 476
$C_{29}H_{24}N_4O_3$ x HCl x 1.5 $H_2O$ (540.02)

| | | | |
|---|---|---|---|
| Ber. | C 64.50 | H 5.23 | N 10.37 |
| Gef. | 64.79 | 5.08 | 10.38 |

Beispiel 238

(Z)-3-{1-[4-(N-Benzyl-N-methyl-aminomethyl)-phenylamino]-1-phenyl-methyliden}-5-nitro-2-indolinon

Hergestellt analog Beispiel 231.
Schmelzpunkt: 200-201°C
$C_{30}H_{26}N_4O_3$ (490.57)
Massenspektrum: $M^+$ = 490

| | | | |
|---|---|---|---|
| Ber. | C 73.45 | H 5.34 | N 11.42 |
| Gef. | 73.25 | 5.50 | 11.32 |

Beispiel 239

(Z)-3-{1-[4-(2-Hydroxy-ethylaminomethyl)-phenylamino]-1-phenyl-methyliden}-5-nitro-2-indolinon

Hergestellt analog Beispiel 231.
Schmelzpunkt: 196-198°C
$C_{24}H_{22}N_4O_4$ (430.47)
Massenspektrum: $M^+$ 430

| | | | |
|---|---|---|---|
| Ber. | C 66.97 | H 5.15 | N 13.02 |
| Gef. | 66.67 | 5.35 | 12.80 |

Beispiel 240

(Z)-3-{1-[4-(Bis-(2-hydroxyethyl)-aminomethyl)-phenylamino]-1-phenyl-methyliden}-5-nitro-2-indolinon

Hergestellt analog Beispiel 231.
Schmelzpunkt: 196-198°C
$C_{26}H_{26}N_4O_5$ (474.52)
Massenspektrum: $M^+$= 474

| | | | |
|---|---|---|---|
| Ber. | C 65.81 | H 5.52 | N 11.81 |
| Gef. | 65.53 | 5.53 | 11.69 |

Beispiel 241

(Z)-3-{1-[4-(2-Ethoxycarbonyl-ethylaminomethyl)-phenylamino]-1-phenyl-methyliden}-5-nitro-2-indolinon

Hergestellt analog Beispiel 231.
Schmelzpunkt: 129-131°C
$C_{27}H_{26}N_4O_5$ (486.53)
Massenspektrum: $M^+ = 486$

| | | | |
|---|---|---|---|
| Ber. | C 66.66 | H 5.39 | N 11.52 |
| Gef. | 66.68 | 5.42 | 11.50 |

Beispiel 242

(Z)-3-{1-[4-(2-Carboxy-ethylaminomethyl)-phenylamino]-1-phenyl-methyliden}-5-nitro-2-indolinon

Hergestellt analog Beispiel 241 und 8.
Schmelzpunkt: 220-222°C
$C_{25}H_{22}N_4O_5$ (458.47)
Massenspektrum: $M^+ = 458$

Beispiel 243

(Z)-3-{1-[4-(2-Dimethylaminocarbonyl-ethylaminomethyl)-phenylamino]-1-phenyl-methyliden}-5-nitro-2-indolinon

Hergestellt analog Beispiel 242 und 18.
Schmelzpunkt: 215-217°C
$C_{27}H_{27}N_5O_4$ (485.54)
Massenspektrum: $M^+ = 485$

Beispiel 244

(Z)-3-{1-[4-(4-tert.Butoxycarbonyl-piperazin-1-ylmethyl)-phenylamino]-1-phenyl-methyliden}-5-nitro-2-indolinon

Hergestellt analog Beispiel 231.
Schmelzpunkt: 236-237°C (Zers.)
$C_{31}H_{33}N_5O_5$ (555.64)
Massenspektrum: $M^+ = 555$

| | | | |
|---|---|---|---|
| Ber. | C 67.01 | H 5.99 | N 12.60 |
| Gef. | 66.89 | 6.08 | 12.65 |

Beispiel 245

(Z)-3-{1-[4-(Piperazin-1-ylmethyl)-phenylamino]-1-phenyl-methyliden}-5-nitro-2-indolinon-dihydrochlorid

Hergestellt analog Beispiel 244 und 29a.
Schmelzpunkt: >370°C; sintert ab 240°C
$C_{26}H_{25}N_5O_3$ (455.52)
Massenspektrum: $M^+ = 455$
$C_{26}H_{25}N_5O_3$ x 2 HCl x 2 $H_2O$ (564.47)

| | | | |
|---|---|---|---|
| Ber. | C 55.32 | H 5.54 | N 12.41 |
| Gef. | 54.96 | 5.66 | 12.26 |

Beispiel 246

(Z)-3-{1-[4-(4-Acetylpiperazin-1-ylmethyl)-phenylamino]-1-phenyl-methyliden}-5-nitro-2-indolinon

Hergestellt analog Beispiel 245 und 1a.
Schmelzpunkt: 275-277°C
$C_{28}H_{27}N_5O_4$ (497.56)
Massenspektrum: $M^+$ = 497
$C_{28}H_{27}N_5O_4$ x 0.5 $H_2O$ (506.56)

| | | | |
|---|---|---|---|
| Ber. | C 66.39 | H 5.57 | N 13.83 |
| Gef. | 66.51 | 5.66 | 13.70 |

Beispiel 247

(Z)-3-{1-[4-(4-Aminocarbonyl-piperidinomethyl)-phenylamino]-1-phenyl-methyliden}-5-nitro-2-indolinon

Hergestellt analog Beispiel 177 und 20.
Schmelzpunkt: 296-297°C
$C_{28}H_{27}N_5O_4$ (497.56)
Massenspektrum: $M^+$ = 497
$C_{28}H_{27}N_5O_4$ x 1.5 $H_2O$ (524.58)

| | | | |
|---|---|---|---|
| Ber. | C 64.11 | H 5.76 | N 13.35 |
| Gef. | 64.33 | 5.32 | 13.19 |

Beispiel 248

(Z)-3-{1-[4-(4-Methylaminocarbonyl-piperidinomethyl)-phenylamino]-1-phenyl-methyliden}-5-nitro-2-indolinon

Hergestellt analog Beispiel 177 und 18.
Schmelzpunkt: 263-265°C
$C_{29}H_{29}N_5O_4$ (511.59)
Massenspektrum: $M^+$ = 511

| | | | |
|---|---|---|---|
| Ber. | C 68.09 | H 5.71 | N 13.69 |
| Gef. | 67.94 | 5.78 | 13.53 |

Beispiel 249

(Z)-3-{1-[4-(4-Dimethylaminocarbonyl-piperidinomethyl)-phenylamino]-1-phenyl-methyliden}-5-nitro-2-indolinon

Hergestellt analog Beispiel 177 und 18.
Schmelzpunkt: 272-273°C
$C_{30}H_{31}N_5O_4$ (525.61)
Massenspektrum: $M^+$ = 525
$C_{30}H_{31}N_5O_4$ x 0.5 $H_2O$ (534.61)

| | | | |
|---|---|---|---|
| Ber. | C 67.40 | H 6.03 | N 13.10 |
| Gef. | 67.52 | 6.00 | 13.15 |

Beispiel 250

(Z)-3-{1-[4-(4-Hydroxymethyl-piperidinomethyl)-phenylamino]-1-phenyl-methyliden}-5-nitro-2-indolinon

Hergestellt analog Beispiel 231.

Schmelzpunkt: 227-228°C
$C_{28}H_{28}N_4O_4$ (484.56)
Massenspektrum: $M^+ = 484$
$C_{28}H_{28}N_4O_4$ x 0.5 $H_2O$ (493.56)

| | | | |
|---|---|---|---|
| Ber. | C 68.14 | H 5.92 | N 11.35 |
| Gef. | 68.25 | 5.94 | 11.18 |

Beispiel 251

(Z)-3-{1-[4-(4-Hydroxy-4-methyl-piperidinomethyl)-phenylamino]-1-phenyl-methyliden}-5-nitro-2-indolinon

Hergestellt analog Beispiel 231.
Schmelzpunkt: 186-187°C
$C_{28}H_{28}N_4O_4$ (484.56)
Massenspektrum: $M^+ = 484$
$C_{28}H_{28}N_4O_4$ x 0.5 $H_2O$ (493.56)

| | | | |
|---|---|---|---|
| Ber. | C 68.14 | H 5.92 | N 11.35 |
| Gef. | 67.87 | 6.00 | 11.27 |

Beispiel 252

(Z)-3-{1-[3-(2-Carboxyethylaminomethyl)-phenylamino]-1-phenylmethyliden}-5-nitro-2-indolinon

Hergestellt analog Beispiel 112 und 8.
Schmelzpunkt: 247-249°C
$C_{25}H_{22}N_4O_5$ (458.47)
Massenspektrum: $M^+ = 458$
$C_{25}H_{22}N_4O_5$ x 1.5 $H_2O$ (485.50)

| | | | |
|---|---|---|---|
| Ber. | C 61.85 | H 5.19 | N 11.54 |
| Gef. | 61.80 | 5.16 | 11.46 |

Beispiel 253

(Z)-3-{1-[3-(2-Dimethylaminocarbonyl-ethylaminomethyl)-phenylamino]-1-phenyl-methyliden}-5-nitro-2-indolinon

Hergestellt analog Beispiel 253 und 18.
Schmelzpunkt: 177-179°C
$C_{27}H_{27}N_5O_4$ (485.54)
Massenspektrum: $M^+ = 485$
$C_{27}H_{27}N_5O_4$ x 0.5 $H_2O$ (494.55)

| | | | |
|---|---|---|---|
| Ber. | C 65.57 | H 5.71 | N 14.16 |
| Gef. | 65.43 | 5.61 | 13.83 |

Beispiel 254

(Z)-3-{1-[4-(2-Methylamino-ethyl)-phenylamino]-1-phenyl-methyliden}-5-nitro-2-indolinon

a) 4-(2-Methylamino-ethyl)-nitrobenzol

Unter Eiskühlung werden in 120 ml Dichlormethan 2.7 g (86 mMol) Methylamin gelöst. Man gibt 4.9 g (21 mMol) 4-(2-Bromethyl)-nitrobenzol zu und lässt langsam auf Raumtemperatur erwärmen. Nach 15 Stunden Rühren wird das Lösungsmittel im Vakuum entfernt und der Rückstand in Wasser aufgenommen. Man stellt mit 2 N Salzsäure

einen sauren pH-Wert ein und wäscht mit Dichlormethan. Anschließend stellt man in der wässrigen Phase einen basischen pH-Wert mit 4 N Natronlauge ein und extrahiert das Produkt mit Dichlormethan.
Ausbeute: 3.1 g (82 % der Theorie)

b) 4-(2-Methylamino-ethyl)-anilin

Hergestellt durch katalytische Hydrierung von 4-(2-Methylamino-ethyl)-nitrobenzol über Palladium-Kohle in Methanol analog Beispiel 39c.
Ausbeute: 96 % der Theorie

c) (Z)-3-{1-[4-(2-Methylamino-ethyl)-phenylamino]-1-phenylmethyliden}-5-nitro-2-indolinon

Hergestellt analog Beispiel 89 durch Umsetzung von 3-(1-Ethoxy-1-phenyl-methyliden)-5-nitro-2-indolinon und 4-(2-Methylamino-ethyl)-anilin.
Aubeute: 12 % der Theorie
Schmelzpunkt: 250-252°C
$C_{24}H_{22}N_4O_3$ (414.46)
Massenspektrum: $M^+$ = 414

Beispiel 255

(Z)-3-{1-[4-(2-Ethylamino-ethyl)-phenylamino]-1-phenyl-methyliden}-5-nitro-2-indolinon

Hergestellt analog Beispiel 254.
Schmelzpunkt: 235-237°C
$C_{25}H_{24}N_4O_3$ (428.49)
Massenspektrum: $M^+$ = 428

| | | | |
|---|---|---|---|
| Ber. | C 70.08 | H 5.65 | N 13.07 |
| Gef. | 69.73 | 5.72 | 12.92 |

Beispiel 256

(Z)-3-{1-[4-(2-(2-Hydroxyethylamino)-ethyl)-phenylamino]-1-phenyl-methyliden}-5-nitro-2-indolinon

Hergestellt analog Beispiel 254.
Schmelzpunkt: 236-238°C
$C_{25}H_{24}N_4O_4$ (444.49)
Massenspektrum: $M^+$ = 444
$C_{25}H_{24}N_4O_4$ x 1.5 $H_2O$ (471.51)

| | | | |
|---|---|---|---|
| Ber. | C 63.68 | H 5.77 | N 11.88 |
| Gef. | 63.77 | 5.82 | 11.60 |

Beispiel 257

(Z)-3-{1-[4-(2-(2-Methoxyethylamino)-ethyl)-phenylamino]-1-phenyl-methyliden}-5-nitro-2-indolinon-hydrobromid

Hergestellt analog Beispiel 254.
Schmelzpunkt: 297-299°C
$C_{26}H_{26}N_4O_4$ (458.52)
Massenspektrum: $M^+$ = 458

Beispiel 258

(Z)-3-{1-[4-(2-Carboxymethylamino-ethyl)-phenylamino]-1-phenyl-methyliden}-5-nitro-2-indolinon

Hergestellt analog Beispiel 178 und 8.
Schmelzpunkt: 242-243 °C (Zers.)
$C_{25}H_{22}N_4O_5$ (458.48)
Massenspektrum: $(M+H)^+$ = 459
$C_{25}H_{22}N_4O_5$ x 0.5 $H_2O$ (467.48)

| | | | |
|---|---|---|---|
| Ber. | C 64.23 | H 4.96 | N 11.98 |
| Gef. | 64.09 | 5.00 | 11.87 |

Beispiel 259

(Z)-3-{1-[4-(2-(4-Methylpiperidino)-ethyl)-phenylamino]-1-phenyl-methyliden}-5-nitro-2-indolinon

Hergestellt analog Beispiel 254.
Schmelzpunkt: 252-253°C
$C_{29}H_{30}N_4O_3$ (482.58)
Massenspektrum: $M^+$ = 483

Beispiel 260

(Z)-3-{1-[4-(2-(4-Hydroxypiperidino)-ethyl)-phenylamino]-1-phenyl-methyliden}-5-nitro-2-indolinon

Hergestellt analog Beispiel 254.
Schmelzpunkt: 274-276°C
$C_{28}H_{28}N_4O_4$ (484.55)
Massenspektrum: $[M+H]^+$ = 485

Beispiel 261

(Z)-3-{1-[4-(2-(4-Methoxypiperidino)-ethyl)-phenylamino]-1-phenyl-methyliden}-5-nitro-2-indolinon

Hergestellt analog Beispiel 254.
Schmelzpunkt: 212-214°C
$C_{29}H_{30}N_4O_4$ (498.58)
Massenspektrum: $[M+H]^+$ = 499

Beispiel 262

(Z)-3-{1-[4-(2-(4-Ethoxycarbonyl-piperidino)-ethyl)-phenylamino]-1-phenyl-methyliden}-5-nitro-2-indolinon

Hergestellt analog Beispiel 254.
Schmelzpunkt: 208-213°C
$C_{31}H_{32}N_4O_5$ (540.62)
Massenspektrum: $[M+H]^+$ = 541

Beispiel 263

(Z)-3-{1-[4-(2-(4-Carboxypiperidino)-ethyl)-phenylamino]-1-phenyl-methyliden}-5-nitro-2-indolinon

Hergestellt analog Beispiel 262 und 8.
Schmelzpunkt: 287-288°C
$C_{29}H_{28}N_4O_5$ (512.56)
Massenspektrum: $[M+H]^+$ = 513

Beispiel 264

(Z)-3-{1-[4-(2-(4-Dimethylaminocarbonyl-piperidino)-ethyl)-phenylamino]-1-phenyl-methyliden}-5-nitro-2-indolinon

Hergestellt analog Beispiel 263 und 18.
Schmelzpunkt: 288°C (Zers.)
$C_{31}H_{33}N_5O_4$ (539.63)
Massenspektrum: $[M+H]^+ = 540$

Beispiel 265

(Z)-3-{1-[4-(2-Hexamethylenimino-ethyl)-phenylamino]-1-phenylmethyliden}-5-nitro-2-indolinon

Hergestellt analog Beispiel 254.
Schmelzpunkt: 217-222°C
$C_{29}H_{30}N_4O_3$ (482.58)
Massenspektrum: $[M+H]^+ = 483$

Beispiel 266

(Z)-3-{1-[4-(Dimethylaminomethyl)-phenylamino]-1-phenyl-methyliden}-2-indolinon

Hergestellt analog Beispiel 1.
Schmelzpunkt: 237-240°C
$C_{24}H_{23}N_3O$ (369.47)
Massenspektrum: $[M+H]^+ = 370$

Beispiel 267

(Z) -3-{1-[4-(Piperidinomethyl)-phenylamino]-1-phenyl-methyliden}-2-indolinon

Hergestellt analog Beispiel 1.
Schmelzpunkt: 235-240°C
$C_{27}H_{27}N_3O$ (409.53)
Massenspektrum: $[M+H]^+ = 410$

Beispiel 268

(Z)-3-{1-[4-(2-Dimethylamino-ethyl)-phenylamino]-1-phenyl-methyliden}-2-indolinon

Hergestellt analog Beispiel 1 und 254.
Schmelzpunkt: 244-246°C
$C_{25}H_{25}N_3O$ (383.49)
Massenspektrum: $[M+H]^+=384$

Beispiel 269

(Z)-3-{1-[4-(2-(3,6-Dihydro-2H-pyridin-1-yl)-ethyl)-phenylamino]-1-phenyl-methyliden}-5-nitro-2-indolinon

a) 4-[2-(3,6-Dihydro-2H-pyridin-1-yl)-ethyl]-anilin

Zu einer Lösung von 1.5 g (6.46 mMol) 4-[2-(3,6-Dihydro-2H-pyridin-1-yl)-ethyl]-nitrobenzol, hergestellt analog Beispiel 254, in 7 ml Eisessig und 2.5 ml konzentrierter Salzsäure werden bei Raumtemperatur 2.5 g (11.1 mMol) Zinndichlorid-dihydrat gegeben. Man erhitzt 4 Stunden auf 100°C, gibt anschließend nochmals 2.5 g (11.1 mMol) Zinndichlorid-dihydrat zu und erhitzt 12 Stunden auf 100°C. Nach dem Abkühlen wird das Lösungsmittel in Vakuum entfernt und der Rückstand in Wasser aufgenommen. Man stellt mit 4N Natronlauge einen alkalischen pH-Wert ein und extrahiert mit Dichlormethan. Nach Entfernen des Lösungsmittels im Vakuum erhält man das Produkt als Öl.
Ausbeute: 1.14 g (88 % der Theorie)

$C_{13}H_{18}N_2$ (202.3)
Massenspektrum: $[M+H]^+$ = 203

b) (Z)-3-{1-[4-(2-(3,6-Dihydro-2H-pyridin-1-yl)-ethyl)-phenylamino]-1-phenyl-methyliden}-5-nitro-2-indolinon

Hergestellt analog Beispiel 89 durch Umsetzung von 3-(1-Ethoxy-1-phenyl-methyliden)-5-nitro-2-indolinon und 4-[2-(3,6-Dihydro-2H-pyridin-1-yl)-ethyl]-anilin.
Aubeute: 88 % der Theorie
Schmelzpunkt: 249-254°C (Zers.)
$C_{28}H_{26}N_4O_3$ (466.54)
Massenspektrum: $[M+H]^+$ = 467

Beispiel 270

(Z)-3-{1-[4-(3,6-Dihydro-2H-pyridin-1-ylmethyl)-phenylamino]-1-phenyl-methyliden}-5-nitro-2-indolinon

Hergestellt analog Beispiel 269.
Schmelzpunkt: 222-225°C
$C_{27}H_{24}N_4O_3$ (452.51)
Massenspektrum: $M^+$ = 452

Beispiel 271

(Z)-3-{1-[4-(Pyrimidin-2-ylaminomethyl)-phenylamino]-1-phenylmethyliden}-5-nitro-2-indolinon

a) 4-(Pyrimidin-2-ylaminomethyl)-nitrobenzol

Zu einer Lösung von 5.7 g (50 mMol) 2-Chlorpyrimidin in 250 ml Ethanol gibt man 9.4 g (50 mMol) 4-Nitrobenzylamin-hydrochlorid, 11.7 g (110 mMol) Natriumcarbonat und 7.5 g (50 mMol) Natriumjodid. Man erhitzt 20 Stunden zum Rückfluß. Anschließend saugt man von Salzen ab, dampft das Filtrat ein und nimmt in 300 ml Essigester auf. Man wäscht mit Wasser, entfernt das Lösungsmittel im Vakuum und chromatographiert den Rückstand an Kieselgel (Dichlormethan/Methanol = 97:3).
Ausbeute: 2.4 g (21 % der Theorie),
Schmelzpunkt: 157-158°C
$C_{11}H_{10}N_4O_2$ (230.23)
Massenspektrum: $[M+H]^+$ = 231

b) 4-(Pyrimidin-2-ylaminomethyl)-anilin

Hergestellt analog Beispiel 55 durch katalytische Hydrierung von 4-(Pyrimidin-2-ylaminomethyl)-nitrobenzol mit Raney-Nickel.
Ausbeute: 89 % der Theorie,
Schmelzpunkt: 145-146°C
$C_{11}H_{12}N_4$ (200.25)
Massenspektrum: $[M+H]^+$ = 201

c) (Z)-3-{1-[4-(Pyrimidin-2-ylaminomethyl)-phenylamino]-1-phenyl-methyliden}-5-nitro-2-indolinon

Hergestellt analog Beispiel 89 aus durch Umsetzung von 3-(1-Ethoxy-1-phenyl-methyliden)-5-nitro-2-indolinon mit 4-(Pyrimidin-2-ylaminomethyl)-anilin.
Ausbeute: 80 % der Theorie,
Schmelzpunkt: 284°-286°C
$C_{26}H_{20}N_6O_3$ (464.49)
Massenspektrum: $M^+$ = 464

| | | | |
|---|---|---|---|
| Ber. | C 67.23 | H 4.34 | N 18.09 |
| Gef. | 66.86 | 4.42 | 17.85 |

Beispiel 272

(Z)-3-{1-[4-((N-Methyl-N-pyrimidin-2-yl-amino)-methyl)-phenylamino]-1-phenyl-methyliden}-5-nitro-2-indolinon

Hergestellt analog Beispiel 271.
Schmelzpunkt: 236°-239°C
$C_{27}H_{22}N_6O_3$ (478.51)
Massenspektrum: $M^+$ = 478

Beispiel 273

(Z)-3-{1-[4-(Azetidin-1-yl-methyl)-phenylamino]-1-phenyl-methyliden}-5-nitro-2-indolinon

a) 4-(Azetidin-1-yl-methyl)-nitrobenzol

Man löst in 120 ml Ethanol 6.2 g (41 mMol) 4-Nitrobenzaldehyd und 3.8 g (40.6 mMol) Azetidin-hydrochlorid. Bei 0°C werden 2.6 g (41 mMol) Natriumcyanoborhydrid zugegeben. Man läßt langsam auf Raumtemperatur erwärmen und rührt anschließend 18 Stunden. Dann wird das Lösungsmittel im Vakuum entfernt, der Rückstand in Essigester aufgenommen und mit Wasser gewaschen. Man entfernt das Lösungsmittel im Vakuum und erhält nach Chromatographie des Rückstands an Kieselgel (Essigester/Methanol/-$NH_4OH$ = 95:5:0.5) ein hellbraunes Öl.
Ausbeute: 0.9 g (11 % der Theorie),
$C_{10}H_{12}N_2O_2$ (192.22)
Massenspektrum: $[M+H]^+$ = 193

b) 4-(Azetidin-1-yl-methyl)-anilin

Hergestellt analog Beispiel 55 durch katalytische Hydrierung von 4-(Azetidin-1-yl-methyl)-nitrobenzol mit Raney-Nickel als hellbraunes Öl.
Ausbeute: 94 % der Theorie;
$C_{10}H_{14}N_2$ (162.24)
Massenspektrum: $[M+H]^+$ = 163

c) (Z)-3-{1-[4-(Azetidin-1-yl-methyl)-phenylamino]-1-phenylmethyliden}-5-nitro-2-indolinon

Hergestellt analog Beispiel 89 aus durch Umsetzung von 3-(1-Ethoxy-1-phenyl-methyliden)-5-nitro-2-indolinon mit 4-(Azetidin-1-yl-methyl)-anilin.
Ausbeute: 84 % der Theorie,
Schmelzpunkt: 228-229°C
$C_{25}H_{22}N_4O_3$ (426.48)
Massenspektrum: $M^+$ = 426

Beispiel 274

(Z)-3-{1-[4-(Cyclopropylaminomethyl)-phenylamino]-1-phenylmethyliden}-5-nitro-2-indolinon

Hergestellt analog Beispiel 273.
Schmelzpunkt: 220-221°C (Zers.)
$C_{25}H_{22}N_4O_3$ (426.48)
Massenspektrum: $M^+$ = 426

Beispiel 275

(Z)-3-{1-[4-((N-Cyclopropyl-N-methyl-amino)-methyl)-phenylamino]-1-phenyl-methyliden}-5-nitro-2-indolinon

Hergestellt analog Beispiel 273.
Schmelzpunkt: 216-217°C
$C_{26}H_{24}N_4O_3$ (440.51)
Massenspektrum: $M^+$ = 440

| | | | |
|---|---|---|---|
| Ber. | C 70.89 | H 5.49 | N 12.72 |
| Gef. | 70.42 | 5.52 | 12.48 |

Beispiel 276

(Z)-3-{1-[4-(Cyclopentylaminomethyl)-phenylamino]-1-phenyl-methyliden}-5-nitro-2-indolinon

Hergestellt analog Beispiel 273.
Schmelzpunkt:
$C_{27}H_{26}N_4O_3$ (454.53)
Massenspektrum: $M^+$ = 454
$C_{27}H_{26}N_4O_3$ x $H_2O$ (472.55)

| | | | |
|---|---|---|---|
| Ber. | C 68.63 | H 5.97 | N 11.86 |
| Gef. | 68.93 | 6.12 | 11.62 |

Beispiel 277

(Z)-3-{1-[4-(N-Cyclopentyl-N-methyl-aminomethyl)-phenylamino]-1-phenyl-methyliden}-5-nitro-2-indolinon

Hergestellt analog Beispiel 273.
Schmelzpunkt: 228°C
$C_{28}H_{28}N_4O_3$ (468.56)
Massenspektrum: $M^+$ = 468
$C_{28}H_{28}N_4O_3$ x 1.5 $H_2O$ (495.58)

| | | | |
|---|---|---|---|
| Ber. | C 67.86 | H 6.30 | N 11.31 |
| Gef. | 68.35 | 6.42 | 11.16 |

Beispiel 278

(Z)-3-{1-[4-(Cyclohexylaminomethyl)-phenylamino]-1-phenyl-methyliden}-5-nitro-2-indolinon

Hergestellt analog Beispiel 273.
Schmelzpunkt: 245°C
$C_{28}H_{28}N_4O_3$ (468.55)
Massenspektrum: $M^+$ = 468
$C_{28}H_{28}N_4O_3$ x 0.5 $H_2O$ (477.56)

| | | | |
|---|---|---|---|
| Ber. | C 70.42 | H 6.12 | N 11.73 |
| Gef. | 70.60 | 6.20 | 11.83 |

Beispiel 279

(Z)-3-{1-[4-(Pyridin-2-ylaminomethyl)-phenylamino]-1-phenylmethyliden}-5-nitro-2-indolinon

Hergestellt analog Beispiel 273.
Schmelzpunkt: 266-268°C
$C_{27}H_{21}N_5O_3$ (463.49)
Massenspektrum: $M^+$ = 463

| | | | |
|---|---|---|---|
| Ber. | C 69.97 | H 4.57 | N 15.11 |
| Gef. | 69.76 | 4.62 | 14.87 |

Beispiel 280

(Z)-3-{1-[4-(3-Dimethylaminoprop-1-inyl)-phenylamino]-1-phenyl-methyliden}-5-nitro-2-indolinon

a) 4-(3-Hydroxyprop-1-inyl)-nitrobenzol

Zu einer Lösung von 20.2 g (0.1 Mol) 4-Bromnitrobenzol in 285 ml Acetonitril werden 8.55 (0.15 Mol) Propargylalkohol und 152 ml (110 g, 1.09 Mol) Triethylamin gegeben. Die Reaktionslösung wird auf 100°C erwärmt. Man gibt 11.9 g (10 mMol) $Pd(PPh_3)_4$ und 3.94 g (20 mMol) Kupfer(I)jodid zu. Nach 10 Minuten wird das Lösungsmittel im Vakuum entfernt und der Rückstand in Essigester aufgenommen. Man wäscht mit Wasser und Ammoniakwasser, filtriert über Celite und entfernt das Lösungsmittel im Vakuum. Chromatographie an Kieselgel (Dichlormethan/Methanol = 10:1) ergibt das Produkt.
Ausbeute: 5.95 g (34 % der Theorie)
Schmelzpunkt: 98-105°C
$C_9H_7NO_3$ (177.2)
Massenspektrum: $[M-H]^-$ = 176

b) 4-[3-(p-Tolylsulfonyloxy)-prop-1-inyl]-nitrobenzol

Zu einer Lösung von 5.8 g (33 mMol) 4-(3-Hydroxyprop-1-inyl)-nitrobenzol und 5.2 g (27 mMol) p-Toluolsulfonsäurechlorid in 50 ml Dichlormethan werden bei 0°C 4.4 ml (54 mMol) Pyridin zugetropft. Nach 2 Stunden bei 0°C werden ca. 25 g Eis und 8 ml konz. Salzsäure zugesetzt. Die organische Phase wird abgetrennt und mit Wasser gewaschen. Nach Entfernen des Lösungsmittels im Vakuum und Chromatographie des Rückstands an Kieselgel (Dichlormethan/Methanol = 1:1) erhält man das Produkt als Öl.
Ausbeute: 0.7 g (8 % der Theorie)
$C_{16}H_{13}NO_5S$ (331.3)
Massenspektrum: $M^+$ = 331

c) 4-(3-Dimethylaminoprop-1-inyl)-nitrobenzol

Zu einer Lösung von 0.7 g (2.1 mMol) 4-[3-(p-Tolylsulfonyloxy)-prop-1-inyl]-nitrobenzol in 10 ml Dichlormethan werden bei 0°C 190 ml (4.2 mMol) Dimethylamin gelöst in 2.5 ml Dichlormethan getropft. Man entfernt die Kühlung und rührt 18 Stunden bei Raumtemperatur. Anschließend wird die Reaktionslösung mit Wasser gewaschen und vom Lösungsmittel befreit. Der Rückstand wird an Kieselgel (Dichlormethan/Methanol = 10:1) chromatographiert. Man erhält das Produkt als Öl.
Ausbeute: 278 mg (65 % der Thoerie)
$C_{11}H_{12}N_2O_2$ (204.2)
Massenspektrum: $[M+H]^+$ = 205

d) 4-(3-Dimethylaminoprop-1-inyl)-anilin

Die Umsetzung von 4-(3-Dimethylaminoprop-1-inyl)-nitrobenzol mit Zinndichlorid analog Beispiel 269 liefert folgende drei Produkte:
4-(3-Dimethylaminoprop-1-inyl)-anilin
$C_{11}H_{14}N_2$ (174.2)
Massenspektrum: $M^+$ = 174
(Z)-4-(3-Dimethylamino-2-chlorprop-1-enyl)-anilin
$C_{11}H_{15}ClN_2$ (210.7)
Massenspektrum: $M^+$ = 212/210
(E)-4-(3-Dimethylaminoprop-1-enyl)-anilin
$C_{11}H_{16}N_2$ (176.2)
Massenspektrum: $M^+$ = 176

e) (Z)-3-{1-[4-(3-Dimethylaminoprop-1-inyl)-phenylamino]-1-phenyl-methyliden}-5-nitro-2-indolinon

Hergestellt analog Beispiel 89 aus durch Umsetzung von 3-(1-Ethoxy-1-phenyl-methyliden)-5-nitro-2-indolinon mit 4-(3-Dimethylaminoprop-1-inyl)-anilin.
Ausbeute: 22 % der Theorie

$C_{26}H_{22}N_4O_3$ (438.48)
Massenspektrum: $[M+H]^+ = 439.5$
$R_f$-Wert: 0.54 (Kieselgel; Dichlormethan/Methanol = 5:1)

Beispiel 281

(Z)-3-{1-[(Z)-4-(3-Dimethylamino-2-chlorprop-1-enyl)-phenylamino]-1-phenyl-methyliden}-5-nitro-2-indolinon

Hergestellt analog Beispiel 280.
$C_{26}H_{23}ClN_4O_3$ (474.95)
Massenspektrum: $[M+H]^+ = 477/475$
$R_f$-Wert: 0.48 (Kieselgel; Dichlormethan/Methanol = 5:1)

Beispiel 282

(Z)-3-{1-[(E)-4-(3-Dimethylaminoprop-1-enyl)-phenylamino]-1-phenyl-methyliden}-5-nitro-2-indolinon

Hergestellt analog Beispiel 280.
$C_{26}H_{24}N_4O_3$ (440.50)
Massenspektrum: $M^+ = 440$
$R_f$-Wert: 0.51 (Kieselgel; Dichlormethan/Methanol 5:1)

Beispiel 283

(Z)-3-{1-[4-(3-Dimethylamino-propyl)-phenylamino]-1-phenylmethyliden}-5-nitro-2-indolinon

a) 4-(3-Dimethylaminopropyl)-anilin

Hergestellt durch katalytische Hydrierung von 4-(3-Dimethylaminoprop-1-inyl)-nitrobenzol (Beispiel 280°C) analog Beispiel 39c.
$C_{11}H_{18}N_2$ (178.3)
Massenspektrum: $M^+ = 178$

b) (Z)-3-{1-[4-(3-Dimethylaminopropyl)-phenylamino]-1-phenylmethyliden}-5-nitro-2-indolinon

Hergestellt analog Beispiel 89 aus durch Umsetzung von 3-(1-Ethoxy-1-phenyl-methyliden)-5-nitro-2-indolinon mit 4-(3-Dimethylaminopropyl)-anilin.
Ausbeute: 35 % der Theorie
Schmelzpunkt: 269°C (Zers.)
$C_{26}H_{26}N_4O_3$ (442.52)
Massenspektrum: $M^+ = 442$

Beispiel 284

(Z)-3-{1-[4-(2-Dimethylaminoethyloxy)-phenylamino]-1-phenylmethyliden}-2-indolinon

a) 4-(2-Bromethyloxy)-nitrobenzol

Zu einer Lösung von 20.8 g (150 mMol) 4-Nitrophenol in 100 ml Dimethylformamid werden 18 g (161 mMol) Kalium-tert.butylat gegeben. Dabei wird die Temperatur der Reaktionslösung bei <50°C gehalten. Nach 30 Minuten wird die Reaktionslösung zu einer Lösung von 113 g (602 mMol) 1.2-Dibromethan in 50 ml Dimethylformamid getropft. Danach wird 18 Stunden auf 80°C erhitzt. Anschließend wird das Lösungsmittel im Vakuum entfernt, der Rückstand in Dichlormethan aufgenommen, mit verdünnter Natronlauge gewaschen, getrocknet und bis zur Trockene eingeengt. Der ölige Rückstand wird an Kieselgel chromatographiert (Dichlormethan/Cyclohexan = 6:4)
Ausbeute: 13 g (35 % der Theorie)
Schmelzpunkt: 66°C
$R_f$-Wert: 0.53 (Kieselgel; Dichlormethan/Cyclohexan = 6:4)

b) 4-(2-Dimethylaminoethyloxy)-nitrobenzol

In einem Bombenrohr werden 4.9 g (20 mMol) 4-(2-Bromethyloxy)-nitrobenzol und 2.7 g (60 mMol) Dimethylamin in 50 ml Dimethylformamid 24 Stunden auf 100°C erhitzt. Nach Entfernen des Lösungsmittels im Vakuum wird der Rückstand in Wasser aufgenommen und mit Dichlormethan extrahiert. Die organische Phase wird getrocknet und eingedampft.
Ausbeute: 2.9 g (69 % der Theorie)
$C_{10}H_{14}N_2O_3$ (210.224)
Massenspektrum: $[M+H]^+$ = 211
$R_f$-Wert: 0.45 (Kieselgel; Dichlormethan/Ethanol = 9:1)

c) 4-(2-Dimethylaminoethyloxy)-anilin

Hergestellt durch katalytische Hydrierung von 4-(2-Dimethylaminoethyloxy)-nitrobenzol analog Beispiel 39°C.
Ausbeute: 93 % der Theorie
$C_{10}H_{16}N_2O$ (180.25)
Massenspektrum: $[M+H]^+$ = 181

d) (Z)-3-{1-[4-(2-Dimethylaminoethyloxy)-phenylamino]-1-phenyl-methyliden}-2-indolinon

Hergestellt analog Beispiel 11 durch Umsetzung von 3-(1-Ethoxy-1-phenyl-methyliden)-indolinon mit 4-(2-Dimethylaminoethyloxy)-anilin.
Ausbeute: 35 % der Theorie
Schmelzpunkt: 258-260°C
$C_{25}H_{25}N_3O_2$ (399.49)
Massenspektrum: $M^+$ = 399

| | | | |
|---|---|---|---|
| Ber. | C 76.79 | H 6.89 | N 9.26 |
| Gef. | 76.43 | 6.83 | 9.20 |

Beispiel 285

(Z)-3-{1-[4-(2-Piperidinoethyloxy)-phenylamino]-1-phenyl-methyliden}-2-indolinon

Hergestellt analog Beispiel 284.
Schmelzpunkt: 198-200°C
$C_{28}H_{29}N_3O_2$ (439.56)
Massenspektrum: $M^+$ = 439

Beispiel 286

(Z)-3-{1-[4-(3-Dimethylaminopropyloxy)-phenylamino]-1-phenylmethyliden}-2-indolinon

Hergestellt analog Beispiel 284.
Schmelzpunkt: 215-217°C
$C_{26}H_{27}N_3O_2$ (413.52)
Massenspektrum: $M^+$ = 413

Beispiel 287

(Z)-3-{1-[4-(3-Piperidinopropyloxy)-phenylamino]-1-phenyl-methyliden}-2-indolinon

Hergestellt analog Beispiel 284.
Schmelzpunkt: 223-225°C
$C_{29}H_{31}N_3O_2$ (453.58)
Massenspektrum: $M^+$ = 453

Beispiel 288

(Z)-3-{1-[4-(3-(N-Benzyl-N-methyl-amino)-propyloxy)-phenylamino]-1-phenyl-methyliden}-2-indolinon

Hergestellt analog Beispiel 284.
Schmelzpunkt: 187-189°C
$C_{32}H_{31}N_3O_2$ (489.62)
Massenspektrum: $M^+$ = 489

Beispiel 289

(Z)-3-{1-[4-(Ethyloxycarbonylmethyloxy)-phenylamino]-1-phenylmethyliden}-2-indolinon

Hergestellt analog Beispiel 284.
Schmelzpunkt: 175-177°C
$C_{25}H_{22}N_2O_4$ (414.46)
Massenspektrum: $M^+$ = 414

Beispiel 290

(Z)-3-{1-[4-(Carboxymethyloxy)-phenylamino]-1-phenyl-methyliden}-2-indolinon

Hergestellt analog Beispiel 289 und 8.
Schmelzpunkt: 238-240°C
$C_{23}H_{18}N_2O_4$ (386.41)
Massenspektrum: $M^+$ = 386

Beispiel 291

(Z)-3-{1-[4-(Dimethylaminocarbonylmethyloxy)-phenylamino]-1-phenyl-methyliden}-2-indolinon

Hergestellt analog Beispiel 290 und 18.
Schmelzpunkt: 224-226°C
$C_{25}H_{23}N_3O_3$ (413.47)
Massenspektrum: $M^+$ = 413

Beispiel 292

(Z)-3-{1-[4-(N-(2-Dimethylamino-ethylaminocarbonylmethyl)-N-methylsulfonyl-amino)-phenylamino]-1-phenyl-methyliden}-5-nitro-2-indolinon

Hergestellt analog Beispiel 192.
Schmelzpunkt: 145°C
$C_{28}H_{30}N_6O_6S$ (578.65)
Massenspektrum: $M^+$ = 578
$C_{28}H_{30}N_6O_6S$ x 1.5 $H_2O$ (605.67)

| | | | |
|---|---|---|---|
| Ber. | C 55.53 | H 5.49 | N 13.88 |
| Gef. | 55.54 | 5.59 | 13.68 |

Beispiel 293

(Z)-3-{1-[4-(N-((N-(2-Dimethylaminoethyl)-N-methyl-amino)-carbonylmethyl)-N-methylsulfonyl-amino)-phenylamino]-1-phenyl-methyliden}-2-indolinon

Hergestellt analog Beispiel 192.
Schmelzpunkt: 170°C

$C_{29}H_{33}N_5O_4S$ (547.68)
Massenspektrum: $M^+$ = 547

| | | | |
|---|---|---|---|
| Ber | C 63.60 | H 6.07 | N 12.79 |
| Gef | 63.38 | 6.12 | 12.67 |

Beispiel 294

(Z)-3-{1-[4-(N-((N-(2-Dimethylaminoethyl)-N-methyl-amino)-carbonylmethyl)-N-methylsulfonyl-amino)-phenylamino]-1-phenyl-methyliden}-5-nitro-2-indolinon

Hergestellt analog Beispiel 192.
Schmelzpunkt: 138-140°C
$C_{29}H_{32}N_6O_6S$ (592.67)
Massenspektrum: $M^+$ = 592
$C_{29}H_{32}N_6O_6S$ x $H_2O$ (601.68)

| | | | |
|---|---|---|---|
| Ber | C 57.89 | H 5.53 | N 13.97 |
| Gef | 57.58 | 5.57 | 13.84 |

Beispiel 295

(Z)-3-{1-[4-(N-(2-Dimethylamino-ethylaminocarbonylmethyl)-N-ethylsulfonyl-amino)-phenylamino]-1-phenyl-methyliden}-5-nitro-2-indolinon

Hergestellt analog Beispiel 192.
Schmelzpunkt: 155°C
$C_{29}H_{32}N_6O_6S$ (592.67)
Massenspektrum: $M^+$ = 592
$C_{29}H_{32}N_6O_6S$ x $H_2O$ (610.69)

| | | | |
|---|---|---|---|
| Ber | C 57.04 | H 5.61 | N 13.76 |
| Gef | 56.96 | 5.63 | 13.73 |

Beispiel 296

(Z)-3-{1-[4-(N-((N-(2-Dimethylaminoethyl)-N-methyl-amino)-carbonylmethyl)-N-ethylsulfonyl-amino)-phenylamino]-1-phenyl-methyliden}-5-nitro-2-indolinon

Hergestellt analog Beispiel 192.
Schmelzpunkt: 117°C
$C_{30}H_{34}N_6O_6S$ (606.70)
Massenspektrum: $M^+$ = 606

| | | | |
|---|---|---|---|
| Ber | C 59.39 | H 5.65 | N 13.85 |
| Gef | 59.29 | 5.78 | 13.65 |

Beispiel 297

(Z)-3-{1-[4-(N-Ethoxycarbonylmethyl-N-ethylsulfonyl-amino)-phenylamino]-1-phenyl-methyliden}-2-indolinon

Hergestellt analog Beispiel 198.
Schmelzpunkt: 228-230°C
$C_{27}H_{27}N_3O_5S$ (505.59)
Massenspektrum: $M^+$ = 505
$C_{27}H_{27}N_3O_5S$ x 0.5 $H_2O$ (514.60)

| | | | |
|---|---|---|---|
| Ber. | C 63.02 | H 5.48 | N 8.17 |
| Gef. | 62.70 | 5.37 | 8.29 |

Beispiel 298

(Z)-3-{1-[4-(N-Carboxymethyl-N-ethylsulfonyl-amino)-phenylamino]-1-phenyl-methyliden}-2-indolinon

Hergestellt analog Beispiel 297 und 8.
Schmelzpunkt: 240-242°C
$C_{25}H_{23}N_3O_5S$ (477.54)
Massenspektrum: $M^+ = 477$,
$R_f$-Wert: 0.3 (Kieselgel; Dichlormethan/Methanol = 9:1)

Beispiel 299

(Z)-3-{1-[4-(N-Aminocarbonylmethyl-N-ethylsulfonyl-amino)-phenylamino]-1-phenyl-methyliden}-2-indolinon

Hergestellt analog Beispiel 298 und 20.
Schmelzpunkt: 259°C
$C_{25}H_{24}N_4O_4S$ (476.55)
Massenspektrum: $M^+ = 476$
$C_{25}H_{24}N_4O_4S \times 0.3\ H_2O$ (481.96)

| | | | |
|---|---|---|---|
| Ber | C 62.30 | H 5.14 | N 11.62 |
| Gef | 62.50 | 5.31 | 11.55 |

Beispiel 300

(Z)-3-{1-[4-(N-Methylaminocarbonylmethyl-N-ethylsulfonyl-amino)-phenylamino]-1-phenyl-methyliden}-2-indolinon

Hergestellt analog Beispiel 298 und 18.
Schmelzpunkt: 242°C
$C_{26}H_{26}N_4O_4S$ (490.58)
Massenspektrum: $M^+ = 490$

Beispiel 301

(Z)-3-{1-[4-(N-(2-Dimethylamino-ethylaminocarbonylmethyl)-N-ethylsulfonyl-amino)-phenylamino]-1-phenyl-methyliden}-2-indolinon

Hergestellt analog Beispiel 298 und 18.
Schmelzpunkt: 203°C
$C_{29}H_{33}N_5O_4S$ (547.68)
Massenspektrum: $M^+ = 547$

Beispiel 302

(Z)-3-{1-[4-(N-((N-(2-Dimethylaminoethyl)-N-methyl-amino)-carbonylmethyl)-N-ethylsulfonyl-amino)-phenylamino]-1-phenyl-methyliden}-2-indolinon

Hergestellt analog Beispiel 298 und 18.
Schmelzpunkt: 170-172°C
$C_{30}H_{35}N_5O_4S$ (561.70)
Massenspektrum: $M^+ = 561$

Beispiel 303

(Z)-3-{1-[4-(N-(Dimethylaminocarbonylmethyl)-N-benzylsulfonylamino)-phenylamino]-1-phenyl-methyliden}-5-nitro-2-indolinon

Hergestellt analog Beispiel 187.
Schmelzpunkt: 159-161°C
$C_{32}H_{29}N_5O_6S$ (611.68)
Massenspektrum: $M^+$ = 611

Beispiel 304

(Z)-3-{1-[4-(N-(Dimethylaminocarbonylmethyl)-N-isopropylsulfonyl-amino)-phenylamino]-1-phenyl-methyliden}-2-indolinon

Hergestellt analog Beispiel 187.
Schmelzpunkt: 146-148°C
$C_{28}H_{30}N_4O_4S$ (518.63)
Massenspektrum: $M^+$ = 518

| | | | |
|---|---|---|---|
| Ber. | C 64.84 | H 5.83 | N 10.80 |
| Gef. | 65.11 | 5.82 | 10.67 |

Beispiel 305

(Z)-3-{1-[4-(N-(Dimethylaminocarbonylmethyl)-N-propylsulfonylamino)-phenylamino]-1-phenyl-methyliden}-2-indolinon

Hergestellt analog Beispiel 187.
Schmelzpunkt: 178-180°C
$C_{28}H_{30}N_4O_4S$ (518.63)
Massenspektrum: $M^+$ = 518

Beispiel 306

(Z)-3-{1-[4-(N-(Dimethylaminocarbonylmethyl)-N-butylsulfonylamino)-phenylamino]-1-phenyl-methyliden}-2-indolinon

Hergestellt analog Beispiel 187.
Schmelzpunkt: 121-123°C
$C_{29}H_{32}N_4O_4S$ (532.66)
Massenspektrum: $M^+$ = 532
$C_{29}H_{32}N_4O_4S$ x 2 $H_2O$ (568.69)

| | | | |
|---|---|---|---|
| Ber. | C 61.25 | H 6.38 | N 9.85 |
| Gef. | 61.59 | 6.49 | 10.00 |

Beispiel 307

(Z)-3-{1-[4-(N-(2-Dimethylaminoethyl)-N-ethylsulfonyl-amino)-phenylamino]-1-phenyl-methyliden}-2-indolinon

Hergestellt analog Beispiel 187.
Schmelzpunkt: 245°C
$C_{27}H_{30}N_4O_3S$ (490.63)
Massenspektrum: $M^+$ = 490
$C_{27}H_{30}N_4O_3S$ x 0.2 $H_2O$ (494.22)

| | | | |
|---|---|---|---|
| Ber | C 65.62 | H 6.20 | N 11.34 |
| Gef | 65.72 | 6.33 | 11.27 |

Beispiel 308

(Z)-3-{1-[4-(N-(2-Morpholinoethyl)-N-ethylsulfonyl-amino)-phenylamino]-1-phenyl-methyliden}-2-indolinon

Hergestellt analog Beispiel 187.
Schmelzpunkt: 222-224°C
$C_{29}H_{32}N_4O_4S$ (532.66)
Massenspektrum: $M^+$ = 532

Beispiel 309

(Z)-3-{1-[4-(N-(2-Dimethylaminoethyl)-N-isopropylsulfonyl-amino)-phenylamino]-1-phenyl-methyliden}-2-indolinon

a) Isopropylsulfonsäure-(4-tert.Butoxycarbonylamino-phenyl)-amid

Zu einer Lösung von 1.0 g (4.8 mMol) 4-tert.Butoxycarbonylamino-anilin in 10 ml Pyridin werden 1.2 g (10 mMol) Isopropylsulfonsäurechlorid zugetropft. Man rührt 18 Stunden bei Raumtemperatur. Danach wird die Reaktionslösung auf 150 ml Eiswasser gegossen und anschließend mit Essigester extrahiert. Die organischen Phasen werden mit Wasser gewaschen und vom Lösungsmittel befreit. Der Rückstand wird an Kieselgel chromatographiert (Dichlormethan/Methanol/$NH_4OH$ = 19:1:0.1).
Ausbeute: 0.8 g (53 % der Theorie)
$C_{14}H_{22}N_2O_4S$ (314.41)
Massenspektrum: $[M-H]^-$ = 313

b) 4-Isopropylsulfonylamino-anilin

Hergestellt analog Beispiel 29a aus Isopropylsulfonsäure-(4-tert.Butoxycarbonylamino-phenyl)-amid.
$C_9H_{14}N_2O_2S$ (214.28)
Massenspektrum: $M^+$ = 214

c) (Z)-1-Acetyl-3-[1-(4-isopropylsulfonylamino-phenylamino)-1-phenyl-methyliden]-2-indolinon

Hergestellt analog Beispiel 1c aus 1-Acetyl-3-(1-ethoxy-1-phenyl-methyliden)-2-indolinon und 4-Isopropylsulfonylamino-anilin.
Ausbeute: 27 % der Theorie
Schmelzpunkt: 258°C
$C_{26}H_{25}N_3O_4S$ (475.57)
Massenspektrum: $[M-H]^-$ = 474

d) (Z)-3-{1-[4-(N-(2-Dimethylaminoethyl)-N-isopropylsulfonylamino)-phenylamino]-1-phenyl-methyliden}-2-indolinon

Hergestellt analog Beispiel 36 aus (Z)-3-{1-[4-(Isopropylsulfonylamino)-phenylamino]-1-phenyl-methyliden}-2-indolinon, 1-Chlor-2-dimethylamino-ethan, Kaliumcarbonat und Natriumiodid in Aceton.
Ausbeute: 14 % der Theorie
Schmelzpunkt: 247°C
$C_{28}H_{32}N_4O_3S$ (504.65)
Massenspektrum: $[M+H]^+$ = 505
$C_{28}H_{32}N_4O_3S$ x 0.2 $H_2O$ (508.25)

| | | | |
|---|---|---|---|
| Ber. | C 66.17 | H 6.43 | N 11.02 |
| Gef. | 66.19 | 6.40 | 10.78 |

Beispiel 310

(Z)-3-{1-[4-(N-(2-Dimethylaminoethyl)-N-propylsulfonyl-amino)-phenylamino]-1-phenyl-methyliden}-5-nitro-2-indolinon

Hergestellt analog Beispiel 187.
Schmelzpunkt: 212°C
$C_{28}H_{31}N_5O_5S$ (549.65)
Massenspektrum: $M^+$ = 549

Beispiel 311

(Z)-3-{1-[4-(N-(2-Dimethylaminoethyl)-N-propylsulfonyl-amino)-phenylamino]-1-phenyl-methyliden}-2-indolinon

Hergestellt analog Beispiel 187.
Schmelzpunkt: 245°C
$C_{28}H_{32}N_4O_3S$ (504.65)
Massenspektrum: $M^+$ = 504

| | | | |
|---|---|---|---|
| Ber | C 66.64 | H 6.39 | N 11.10 |
| Gef | 66.40 | 6.44 | 11.00 |

Beispiel 312

(Z)-3-{1-[4-(N-(2-Dimethylaminoethyl)-N-phenylsulfonyl-amino)-phenylamino]-1-phenyl-methyliden}-2-indolinon

Hergestellt analog Beispiel 187.
Schmelzpunkt: 241-243°C
$C_{31}H_{30}N_4O_3S$ (538.67)
Massenspektrum: $M^+$ = 538

Beispiel 313

(Z)-3-{1-[4-(N-(2-Dimethylaminoethyl)-N-benzylsulfonyl-amino)-phenylamino]-1-phenyl-methyliden}-5-nitro-2-indolinon

Hergestellt analog Beispiel 187.
Schmelzpunkt: 248°C
$C_{32}H_{31}N_5O_5S$ (597.69)
Massenspektrum: $M^+$ = 597

Beispiel 314

(Z)-3-{1-[4-(N-(2-Dimethylaminoethyl)-N-benzylsulfonyl-amino)-phenylamino]-1-phenyl-methyliden}-2-indolinon

Hergestellt analog Beispiel 187.
Schmelzpunkt: 244°C
$C_{32}H_{32}N_4O_3S$ (552.70)
Massenspektrum: $M^+$ = 552
$C_{32}H_{32}N_4O_3S$ x 0.5 $H_2O$ (560.69)

| | | | |
|---|---|---|---|
| Ber | C 68.55 | H 5.75 | N 9.99 |
| Gef | 68.99 | 5.99 | 9.83 |

Beispiel 315

(Z)-3-{1-[4-(N-(3-Dimethylaminopropyl)-N-methylsulfonyl-amino)-phenylamino]-1-phenyl-methyliden}-2-indolinon

Hergestellt analog Beispiel 187.
Schmelzpunkt: 227°C
$C_{27}H_{30}N_4O_3S$ (490.63)
Massenspektrum: $[M+H]^+$ = 491

| | | | |
|---|---|---|---|
| Ber. | C 66.10 | H 6.16 | N 11.42 |
| Gef. | 66.04 | 6.14 | 11.43 |

Beispiel 316

(Z)-3-{1-[4-(N-(3-Dimethylaminopropyl)-N-ethylsulfonyl-amino)-phenylamino]-1-phenyl-methyliden}-2-indolinon

Hergestellt analog Beispiel 187.
Schmelzpunkt: 194°C
$C_{28}H_{32}N_4O_3S$ (504.65)
Massenspektrum: $[M+H]^+$ = 505

| | | | |
|---|---|---|---|
| Ber. | C 66.64 | H 6.39 | N 11.10 |
| Gef. | 66.43 | 6.37 | 10.88 |

Beispiel 317

(Z)-3-{1-[3-(N-Ethoxycarbonylmethyl-N-methylsulfonyl-amino)-phenylamino]-1-phenyl-methyliden}-2-indolinon

Hergestellt analog Beispiel 187.
Schmelzpunkt: 188-190°C
$C_{26}H_{25}N_3O_5S$ (491.57)
Massenspektrum: $M^+$ = 491

| | | | |
|---|---|---|---|
| Ber. | C 63.53 | H 5.13 | N 8.55 |
| Gef. | 63.67 | 5.20 | 8.59 |

Beispiel 318

(Z)-3-{1-[3-(N-Carboxymethyl-N-methylsulfonyl-amino)-phenylamino]-1-phenyl-methyliden}-2-indolinon

Hergestellt analog Beispiel 317 und 8.
Schmelzpunkt: 270°C (Zers.)
$C_{24}H_{21}N_3O_5S$ (463.51)
Massenspektrum: $[M-H]^-$ = 462

Beispiel 319

(Z)-3-{1-[3-(N-Aminocarbonylmethyl-N-methylsulfonyl-amino)phenylamino]-1-phenyl-methyliden}-2-indolinon

Hergestellt analog Beispiel 318 und 20.
Schmelzpunkt: 227-230°C
$C_{24}H_{22}N_4O_4S$ (462.53)
Massenspektrum: $M^+$ = 462

Beispiel 320

(Z)-3-{1-[3-(N-Methylaminocarbonylmethyl-N-methylsulfonyl-amino)-phenylamino]-1-phenyl-methyliden}-2-indolinon

Hergestellt analog Beispiel 318 und 20.
Schmelzpunkt: 163°C
$C_{25}H_{24}N_4O_4S$ (476.55)
Massenspektrum: $M^+$ = 476

Beispiel 321

(Z)-3-{1-[3-(N-Dimethylaminocarbonylmethyl-N-methylsulfonylamino)-phenylamino]-1-phenyl-methyliden}-2-indolinon

Hergestellt analog Beispiel 318 und 20.
Schmelzpunkt: 213-216°C
$C_{26}H_{26}N_4O_4S$ (490.58)
Massenspektrum: $M^+$ = 490

Beispiel 322

(Z)-3-{1-[3-(N-(2-Dimethylamino-ethylaminocarbonylmethyl)-N-methylsulfonyl-amino)-phenylamino]-1-phenyl-methyliden}-2-indolinon

Hergestellt analog Beispiel 318 und 18.
Schmelzpunkt: 179-181°C
$C_{28}H_{31}N_5O_4S$ (533.65)
Massenspektrum: $M^+$ = 533

Beispiel 323

(Z)-3-{1-[3-(N-((N-(2-Dimethylaminoethyl)-N-methyl-amino)-carbonylmethyl)-N-methylsulfonyl-amino)-phenylamino]-1-phenyl-methyliden}-2-indolinon

Hergestellt analog Beispiel 318 und 18.
Schmelzpunkt: 197-199°C
$C_{29}H_{33}N_5O_4S$ (547.68)
Massenspektrum: $M^+$ = 547

| | | | | |
|---|---|---|---|---|
| Ber. | C 63.60 | H 6.07 | N 12.79 | S 5.85 |
| Gef. | 63.52 | 6.14 | 12.72 | 5.85 |

Beispiel 324

(Z)-3-{1-[3-(N-(2-Dimethylaminoethyl)-N-methylsulfonyl-amino)-phenylamino]-1-phenyl-methyliden}-2-indolinon

Hergestellt analog Beispiel 187.
Schmelzpunkt: 208-211°C
$C_{26}H_{28}N_4O_3S$ (476.60)
Massenspektrum: $M^+$ = 476

| | | | |
|---|---|---|---|
| Ber. | C 65.52 | H 5.92 | N 11.76 |
| Gef. | 65.22 | 5.84 | 11.64 |

Beispiel 325

(Z)-3-{1-[3-(N-(2-Morpholinoethyl)-N-methylsulfonyl-amino)-phenylamino]-1-phenyl-methyliden}-2-indolinon

Hergestellt analog Beispiel 187.
Schmelzpunkt: 177-179°C
$C_{28}H_{30}N_4O_4S$ (518.63)
Massenspektrum: $M^+$ = 518

Beispiel 326

(Z)-3-{1-[4-(2-Dimethylamino-ethylamino)-phenylamino]-1-phenyl-methyliden}-5-nitro-2-indolinon

a) 4-(2-Dimethylamino-ethylamino)-nitrobenzol

2.2 ml (20 mMol) 4-Fluornitrobenzol und 2.6 ml (24 mMol) N,N-Dimethylethylendiamin werden in 10 ml Ethanol für 1.5 Stunden bei 120°C im Mikrowellenofen erhitzt. Danach werden 50 ml 1 N Salzsäure zugesetzt. Die Reaktionslösung wird mit Essigester gewaschen. Dann wird die wässrige Phase mit 4 N Natronlauge versetzt bis zur alkalischen Reaktion und mit Essigester extrahiert. Die vereinigten organischen Phasen werden mit Wasser gewaschen, über Magnesiumsulfat getrocknet und vom Lösungsmittel befreit. Man erhält das Produkt als gelbes Öl.
Ausbeute: 11.7 g (61 % der Theorie)
$C_{10}H_{15}N_3O_2$ (209.25)
Massenspektrum: $[M+H]^+$ = 210

b) 4-(2-Dimethylamino-ethylamino)-anilin

Hergestellt analog Beispiel 39c durch katalytische Hydrierung von 4-(2-Dimethylamino-ethylamino)-nitrobenzol.
Aubeute: 94 % der Theorie
$C_{10}H_{17}N_3$ (179.27)
Massenspektrum: $[M+H]^+$ = 180

c) (Z)-3-{1-[4-(2-Dimethylamino-ethylamino)-phenylamino]-1-phenyl-methyliden}-5-nitro-2-indolinon

Hergestellt analog Beispiel 89 aus 3-(1-Ethoxy-1-phenyl-methyliden)-5-nitro-2-indolinon und 4-(2-Dimethylamino-ethylamino)-anilin.
Ausbeute: 25 % der Theorie
Schmelzpunkt: 227-229°C
$C_{25}H_{25}N_5O_3$ (443.50)
Massenspektrum: $M^+$ = 443
$C_{25}H_{25}N_5O_3$ x 0.5 $H_2O$ (452.51)

| | | | |
|---|---|---|---|
| Ber. | C 66.36 | H 5.79 | N 15.48 |
| Gef. | 66.25 | 5.60 | 15.52 |

Beispiel 327

(Z)-3-{1-[4-(N-(2-Dimethylaminoethyl)-N-formyl-amino)-phenylamino]-1-phenyl-methyliden}-5-nitro-2-indolinon

a) 4-(N-(2-Dimethylaminoethyl)-N-formyl-amino)-nitrobenzol

1.4 g (6.7 mMol) 4-(2-Dimethylamino-ethylamino)-nitrobenzol (Beispiel 326a) werden in 20 ml Ameisensäure 4 Stunden zum Rückfluß erhitzt. Anschließend wird das Lösungsmittel im Vakuum entfernt, der Rückstand in Wasser aufgenommen und mit 2 N Natronlauge bis zur alkalischen Reaktion versetzt. Man extrahiert mit Essigester, trocknet die vereinigten organischen Phasen über Magnesiumsulfat und entfernt das Lösungsmittel im Vakuum. Man erhält das Produkt als Öl.
Aubeute: 1.3 g (78 % der Theorie)

$C_{11}H_{15}N_3O_3$ (237.26)
Massenspektrum: $[M+H]^+$ = 238

b) 4-(N-(2-Dimethylaminoethyl)-N-formyl-amino)-anilin

Hergestellt analog Beispiel 39c durch katalytische Hydrierung von 4-(N-(2-Dimethylaminoethyl)-N-formyl-amino)-nitrobenzol. Ausbeute: 82 % der Theorie
$C_{11}H_{17}N_3O$ (207.28)
Massenspektrum: $M^+$ = 207

c) (Z)-3-{1-[4-(N-(2-Dimethylaminoethyl)-N-formyl-amino)-phenylamino]-1-phenyl-methyliden}-5-nitro-2-indolinon

Hergestellt analog Beispiel 89 aus 3-(1-Ethoxy-1-phenyl-methyliden)-5-nitro-2-indolinon und 4-(N-(2-Dimethylaminoethyl)-N-formyl-amino)-anilin.
Ausbeute: 47 % der Theorie
Schmelzpunkt: 215-218°C
$C_{26}H_{25}N_5O_4$ (471.51)
Massenspektrum: $M^+$ = 471

Beispiel 328

(Z)-3-{1-[4-(N-(2-Dimethylaminoethyl)-N-acetyl-amino)-phenylamino]-1-phenyl-methyliden}-5-nitro-2-indolinon

Hergestellt analog Beispiel 327.
Schmelzpunkt: 228-230°C
$C_{27}H_{27}N_5O_4$ (485.54)
Massenspektrum: $[M+H]^+$ = 486

Beispiel 329

(Z)-3-{1-[4-(N-(2-Dimethylaminoethyl)-N-propionyl-amino)-phenylamino]-1-phenyl-methyliden}-5-nitro-2-indolinon

Hergestellt analog Beispiel 327.
Schmelzpunkt: 263-265°C
$C_{28}H_{29}N_5O_4$ (499.57)
Massenspektrum: $M^+$ = 499

| | | | |
|---|---|---|---|
| Ber. | C 67.32 | H 5.85 | N 14.02 |
| Gef. | 67.16 | 6.00 | 13.81 |

Beispiel 330

(Z)-3-{1-[4-(N-(2-Dimethylaminoethyl)-N-isopropylcarbonyl-amino)-phenylamino]-1-phenyl-methyliden}-5-nitro-2-indolinon

Hergestellt analog Beispiel 327.
Schmelzpunkt: 296-298°C
$C_{29}H_{31}N_5O_4$ (513.59)
Massenspektrum: $M^+$ = 513

| | | | |
|---|---|---|---|
| Ber. | C 67.82 | H 6.08 | N 13.64 |
| Gef. | 67.53 | 6.29 | 13.51 |

Beispiel 331

(Z)-3-{1-[4-(N-(2-Dimethylaminoethyl)-N-propylcarbonyl-amino)-phenylamino]-1-phenyl-methyliden}-5-nitro-2-indolinon

Hergestellt analog Beispiel 327.
Schmelzpunkt: 275-277°C
$C_{29}H_{31}N_5O_4$ (513.59)
Massenspektrum: $M^+$ = 513

| | | | |
|---|---|---|---|
| Ber. | C 67.82 | H 6.08 | N 13.64 |
| Gef. | 67.71 | 6.31 | 13.54 |

Beispiel 332

(Z)-3-{1-[4-(2-Morpholinoethylamino)-phenylamino]-1-phenyl-methyliden}-5-nitro-2-indolinon

Hergestellt analog Beispiel 326.
Schmelzpunkt: 253°C
$C_{27}H_{27}N_5O_4$ (485.54)
Massenspektrum: $M^+$ = 485
$C_{27}H_{27}N_5O_4$ x 0.5 $H_2O$ (494.54)

| | | | |
|---|---|---|---|
| Ber. | C 65.57 | H 5.71 | N 14.16 |
| Gef. | 65.58 | 5.70 | 14.08 |

Beispiel 333

(Z)-3-{1-[4-(N-(2-Morpholinoethyl)-N-formyl-amino)-phenylamino]-1-phenyl-methyliden}-5-nitro-2-indolinon

Hergestellt analog Beispiel 327.
Schmelzpunkt: 207°C
$C_{28}H_{27}N_5O_5$ (513.55)
Massenspektrum: $M^+$ = 513

| | | | |
|---|---|---|---|
| Ber | C 65.49 | H 5.30 | N 13.64 |
| Gef | 65.19 | 5.30 | 13.51 |

Beispiel 334

(Z)-3-{1-[4-(N-(2-Morpholinoethyl)-N-acetyl-amino)-phenylamino]-1-phenyl-methyliden}-5-nitro-2-indolinon

486 mg (1.0 mMol) (Z)-3-{1-[4-(2-Morpholinoethylamino)-phenylamino]-1-phenyl-methyliden}-5-nitro-2-indolinon (Beispiel 332) werden in 30 ml Dichlormethan gelöst und mit 1.2 ml (16 mMol) Acetylchlorid versetzt. Man rührt 1 Stunde bei Raumtemperatur. Man saugt vom Niederschlag ab und wäscht die Reaktionslösung mit Wasser. Anschließend wird das Lösungsmittel im Vakuum entfernt, der Rückstand in 20 ml Methanol gelöst und mit 4 ml 1 N Natronlauge versetzt. Man rührt 30 Minuten bei Raumtemperatur und entfernt anschließend das Lösungsmittel im Vakuum. Der Rückstand wird in Wasser und wenig Ether suspendiert. Anschließend wird das Produkt abgesaugt und getrocknet.
Ausbeute: 35 % der Theorie
Schmelzpunkt: 229°C
$C_{29}H_{29}N_5O_5$ (527.58)
Massenspektrum: $M^+$ = 527
$C_{29}H_{29}N_5O_5$ x 0.5 $H_2O$ (536.59)

| | | | |
|---|---|---|---|
| Ber | C 64.91 | H 5.64 | N 13.05 |
| Gef | 65.29 | 5.62 | 12.98 |

Beispiel 335

(Z)-3-{1-[4-(N-(2-Morpholinoethyl)-N-propionyl-amino)-phenylamino]-1-phenyl-methyliden}-5-nitro-2-indolinon

Hergestellt analog Beispiel 327.
Schmelzpunkt: 232°C
$C_{30}H_{31}N_5O_5$ (541.60)

| | | | |
|---|---|---|---|
| Ber | C 66.53 | H 5.77 | N 12.93 |
| Gef | 66.60 | 5.99 | 12.65 |

Beispiel 336

(Z)-3-{1-[4-(N-(2-Morpholinoethyl)-N-isopropylcarbonyl-amino)-phenylamino]-1-phenyl-methyliden}-5-nitro-2-indolinon

Hergestellt analog Beispiel 327.
Schmelzpunkt: 254°C
$C_{31}H_{33}N_5O_5$ (555.63)
Massenspektrum: $M^+$ = 555

| | | | |
|---|---|---|---|
| Ber | C 67.01 | H 5.99 | N 12.60 |
| Gef | 66.80 | 6.01 | 12.54 |

Beispiel 337

(Z)-3-{1-[4-(N-(2-Morpholinoethyl)-N-propylcarbonyl-amino)-phenylamino]-1-phenyl-methyliden}-5-nitro-2-indolinon

Hergestellt analog Beispiel 327.
Schmelzpunkt: 228°C
$C_{31}H_{33}N_5O_5$ (555.63)
Massenspektrum: $M^+$ = 555

| | | | |
|---|---|---|---|
| Ber | C 67.01 | H 5.99 | N 12.60 |
| Gef | 66.85 | 6.00 | 12.52 |

Beispiel 338

(Z)-3-{1-[4-(2-Dimethylamino-ethylamino)-phenylamino]-1-phenyl-methyliden}-2-indolinon

Hergestellt analog Beispiel 326.
Schmelzpunkt: 258-260°C
$C_{25}H_{26}N_4O$ (398.51)
Massenspektrum: $M^+$ = 398

Beispiel 339

(Z)-3-{1-[4-(N-(2-Dimethylaminoethyl)-N-formyl-amino)-phenylamino]-1-phenyl-methyliden}-2-indolinon

Hergestellt analog Beispiel 327.
Schmelzpunkt: 246-248°C
$C_{26}H_{26}N_4O_2$ (426.52)
Massenspektrum: $M^+$ = 426

Beispiel 340

(Z)-3-{1-[4-(N-(2-Dimethylaminoethyl)-N-acetyl-amino)-phenylamino]-1-phenyl-methyliden}-2-indolinon

Hergestellt analog Beispiel 327.
Schmelzpunkt: 197-199°C
$C_{27}H_{28}N_4O_2$ (440.54)
Massenspektrum: $M^+$ = 440

Beispiel 341

(Z)-3-{1-[4-(N-(2-Dimethylaminoethyl)-N-propionyl-amino)-phenylamino]-1-phenyl-methyliden}-2-indolinon

Hergestellt analog Beispiel 327.
Schmelzpunkt: 272-274°C
$C_{28}H_{30}N_4O_2$ (454.57)
Massenspektrum: $M^+$ = 454

| | | | |
|---|---|---|---|
| Ber. | C 73.98 | H 6.65 | N 12.33 |
| Gef. | 73.71 | 6.79 | 12.32 |

Beispiel 342

(Z)-3-{1-[4-(N-(2-Dimethylaminoethyl)-N-isopropylcarbonyl-amino)-phenylamino]-1-phenyl-methyliden}-2-indolinon

Hergestellt analog Beispiel 327.
Schmelzpunkt: 280-282°C
$C_{29}H_{32}N_4O_2$ (468.60)
Massenspektrum: $M^+$ = 468
$C_{29}H_{32}N_4O_2$ x 0.5 $H_2O$ (477.61)

| | | | |
|---|---|---|---|
| Ber. | C 72.93 | H 6.96 | N 11.73 |
| Gef. | 72.71 | 6.86 | 11.87 |

Beispiel 343

(Z)-3-{1-[4-(N-(2-Dimethylaminoethyl)-N-propylcarbonyl-amino)-phenylamino]-1-phenyl-methyliden}-2-indolinon

Hergestellt analog Beispiel 327.
Schmelzpunkt: 268-270°C
$C_{29}H_{32}N_4O_2$ (468.60)
Massenspektrum: $M^+$ = 468

| | | | |
|---|---|---|---|
| Ber. | C 74.33 | H 6.88 | N 11.96 |
| Gef. | 74.27 | 6.95 | 11.97 |

Beispiel 344

(Z)-3-{1-[4-(N-(3-Dimethylaminopropyl)-N-acetyl-amino)-phenylamino]-1-phenyl-methyliden}-2-indolinon

Hergestellt analog Beispiel 327.
Schmelzpunkt: 227°C
$C_{28}H_{30}N_4O_2$ (454.57)
Massenspektrum: $M^+$ = 454

| | | | |
|---|---|---|---|
| Ber. | C 73.98 | H 6.65 | N 12.33 |

(fortgesetzt)

| | | | |
|---|---|---|---|
| Gef. | 73.62 | 6.61 | 12.13 |

Beispiel 345

(Z)-3-{1-[4-(N-(3-Dimethylaminopropyl)-N-propionyl-amino)-phenylamino]-1-phenyl-methyliden}-2-indolinon

Hergestellt analog Beispiel 327.
Schmelzpunkt: 224°C
$C_{29}H_{32}N_4O_2$ (468.60)
Massenspektrum: $M^+$ = 468
$C_{29}H_{32}N_4O_2$ x 0.5 $H_2O$ (477.61)

| | | | |
|---|---|---|---|
| Ber. | C 72.93 | H 6.96 | N 11.73 |
| Gef. | 72.99 | 6.85 | 11.63 |

Beispiel 346

(Z)-3-{1-[4-(2-Morpholinoethylamino)-phenylamino]-1-phenyl-methyliden}-2-indolinon

Hergestellt analog Beispiel 326.
Schmelzpunkt: 257°C
$C_{27}H_{28}N_4O_2$ (440.54)
Massenspektrum: $M^+$ = 440

| | | | |
|---|---|---|---|
| Ber | C 73.61 | H 6.41 | N 12.72 |
| Gef | 73.57 | 6.48 | 12.62 |

Beispiel 347

(Z)-3-{1-[4-(N-(2-Morpholinoethyl)-N-formyl-amino)-phenylamino]-1-phenyl-methyliden}-2-indolinon

Hergestellt analog Beispiel 327.
Schmelzpunkt: 218°C
$C_{28}H_{28}N_4O_3$ (468.55)
Massenspektrum: $M^+$ = 468

Beispiel 348

(Z)-3-{1-[4-(N-(2-Morpholinoethyl)-N-acetyl-amino)-phenylamino]-1-phenyl-methyliden}-2-indolinon

Hergestellt analog Beispiel 334.
Schmelzpunkt: ab 90°C (Sinterung)
$C_{29}H_{30}N_4O_3$ (482.58)
Massenspektrum: $M^+$ = 482

Beispiel 349

(Z)-3-{1-[4-(N-(2-Morpholinoethyl)-N-propionyl-amino)-phenylamino]-1-phenyl-methyliden}-2-indolinon

Hergestellt analog Beispiel 334.
Schmelzpunkt: 228°C
$C_{30}H_{32}N_4O_3$ (496.61)
Massenspektrum: $M^+$ = 496
$C_{30}H_{32}N_4O_3$ x 0.3 $H_2O$ (502.01)

| | | | |
|---|---|---|---|
| Ber. | C 71.78 | H 6.55 | N 11.16 |
| Gef. | 71.70 | 6.56 | 11.13 |

Beispiel 350

(Z)-3-{1-[4-(N-(2-Morpholinoethyl)-N-isopropylcarbonyl-amino)-phenylamino]-1-phenyl-methyliden}-2-indolinon

Hergestellt analog Beispiel 327.
Schmelzpunkt: 239°C
$C_{31}H_{34}N_4O_3$ (510.63)
Massenspektrum: $M^+ = 510$

Beispiel 351

(Z)-3-{1-[4-(N-(2-Morpholinoethyl)-N-propylcarbonyl-amino)-phenylamino]-1-phenyl-methyliden}-2-indolinon

Hergestellt analog Beispiel 327.
Schmelzpunkt: 219°C
$C_{31}H_{34}N_4O_3$ (510.63)
Massenspektrum: $M^+ = 510$
$C_{31}H_{34}N_4O_3 \times 0.3\ H_2O$ (516.04)

| | | | |
|---|---|---|---|
| Ber. | C 72.15 | H 6.76 | N 10.86 |
| Gef. | 72.10 | 6.66 | 10.79 |

Beispiel 352

(Z)-3-{1-[4-(N-Ethoxycarbonylmethyl-N-acetyl-amino)-phenylamino]-1-phenyl-methyliden}-2-indolinon

Hergestellt analog Beispiel 187.
Schmelzpunkt: 244-247°C
$C_{27}H_{25}N_3O_4$ (455.51)
Massenspektrum: $M^+ = 455$

| | | | |
|---|---|---|---|
| Ber. | C 71.19 | H 5.53 | N 9.22 |
| Gef. | 71.01 | 5.59 | 9.36 |

Beispiel 353

(Z)-3-{1-[4-(N-Carboxymethyl-N-acetyl-amino)-phenylamino]-1-phenyl-methyliden}-2-indolinon

Hergestellt analog Beispiel 352 und 8.
Schmelzpunkt: 276°C (Zers.)
$C_{25}H_{21}N_3O_4$ (427.46)
Massenspektrum: $M^+ = 427$
$C_{25}H_{21}N_3O_4 \times 0.3\ H_2O$ (432.86)

| | | | |
|---|---|---|---|
| Ber. | C 69.37 | H 5.03 | N 9.71 |
| Gef. | 69.41 | 5.16 | 9.70 |

Beispiel 354

(Z)-3-{1-[4-(N-Methylaminocarbonylmethyl-N-acetyl-amino)-phenylamino]-1-phenyl-methyliden}-2-indolinon

Hergestellt analog Beispiel 353 und 18.

Schmelzpunkt: 270°C (Zers.)
$C_{26}H_{24}N_4O_3$ (440.50)
Massenspektrum: $M^+$ = 440

Beispiel 355

(Z)-3-{1-[4-(N-Dimethylaminocarbonylmethyl-N-acetyl-amino)-phenylamino]-1-phenyl-methyliden}-2-indolinon

Hergestellt analog Beispiel 353 und 18.
Schmelzpunkt: 264-268°C
$C_{27}H_{26}N_4O_3$ (454.53)
Massenspektrum: $M^+$ = 454
$C_{27}H_{26}N_4O_3$ x 0.3 $H^2O$ (459.93)

| | | | |
|---|---|---|---|
| Ber. | C 70.51 | H 5.83 | N 12.18 |
| Gef. | 70.52 | 5.86 | 12.10 |

Beispiel 356

(Z)-3-{1-[4-(N-Ethoxycarbonylmethyl-N-propionyl-amino)-phenylamino]-1-phenyl-methyliden}-2-indolinon

Hergestellt analog Beispiel 187.
Schmelzpunkt: 229-232°C
$C_{28}H_{27}N_3O_4$ (469.54)
Massenspektrum: $M^+$ = 469

| | | | |
|---|---|---|---|
| Ber. | C 71.63 | H 5.80 | N 8.95 |
| Gef. | 71.49 | 5.85 | 8.92 |

Beispiel 357

(Z)-3-{1-[4-(N-Carboxymethyl-N-propionyl-amino)-phenylamino]-1-phenyl-methyliden}-2-indolinon

Hergestellt analog Beispiel 356 und 8.
Schmelzpunkt: 270°C (Zers.)
$C_{26}H_{23}N_3O_4$ (441.48)
Massenspektrum: $M^+$ = 441

| | | | |
|---|---|---|---|
| Ber. | C 70.74 | H 5.25 | N 9.52 |
| Gef. | 70.46 | 5.44 | 9.39 |

Beispiel 358

(Z)-3-{1-[4-(N-Methylaminocarbonylmethyl-N-propionyl-amino)-phenylamino]-1-phenyl-methyliden}-2-indolinon

Hergestellt analog Beispiel 357 und 18.
Schmelzpunkt: 268°C
$C_{27}H_{26}N_4O_3$ (454.53)
Massenspektrum: $M^+$ = 454
$C_{27}H_{26}N_4O_3$ x 0.5 $H_2O$ (463.54)

| | | | |
|---|---|---|---|
| Ber. | C 69.96 | H 5.87 | N 12.09 |
| Gef. | 69.53 | 6.01 | 12.17 |

Beispiel 359

(Z)-3-{1-[4-(N-Dimethylaminocarbonylmethyl-N-propionyl-amino)-phenylamino]-1-phenyl-methyliden}-2-indolinon

Hergestellt analog Beispiel 357 und 18.
Schmelzpunkt: 274-277°C
$C_{28}H_{28}N_4O_3$ (468.55)
Massenspektrum: $M^+ = 468$

| | | | |
|---|---|---|---|
| Ber. | C 71.78 | H 6.02 | N 11.96 |
| Gef. | 71.70 | 6.21 | 11.94 |

Beispiel 360

(Z)-3-{1-[4-(N-Ethoxycarbonylmethyl-N-benzoyl-amino)-phenylamino]-1-phenyl-methyliden}-2-indolinon

Hergestellt analog Beispiel 187.
Schmelzpunkt: 209-211°C
$C_{32}H_{27}N_3O_4$ (517.58)
Massenspektrum: $M^+ = 517$

Beispiel 361

(Z)-3-{1-[4-(N-Carboxymethyl-N-benzoyl-amino)-phenylamino]-1-phenyl-methyliden}-2-indolinon

Hergestellt analog Beispiel 360 und 8.
Schmelzpunkt: 277°C (Zers.)
$C_{30}H_{23}N_3O_4$ (489.53)
Massenspektrum: $M^+ = 489$

Beispiel 362

(Z)-3-{1-[4-(N-Methylaminocarbonylmethyl-N-benzoyl-amino)-phenylamino]-1-phenyl-methyliden}-2-indolinon

Hergestellt analog Beispiel 361 und 18.
Schmelzpunkt: 260-262°C
$C_{31}H_{26}N_4O_3$ (502.57)
Massenspektrum: $M^+ = 502$

| | | | |
|---|---|---|---|
| Ber. | C 74.09 | H 5.21 | N 11.15 |
| Gef. | 74.01 | 5.36 | 11.09 |

Beispiel 363

(Z)-3-{1-[4-(N-Dimethylaminocarbonylmethyl-N-benzoyl-amino)phenylamino]-1-phenyl-methyliden}-2-indolinon

Hergestellt analog Beispiel 361 und 18.
Schmelzpunkt: 284-287°C
$C_{32}H_{28}N_4O_3$ (516.60)
Massenspektrum: $M^+ = 516$
$C_{32}H_{28}N_4O_3 \times 0.25\ H_2O$ (521.10)

| | | | |
|---|---|---|---|
| Ber. | C 73.76 | H 5.51 | N 10.75 |
| Gef. | 73.71 | 5.67 | 10.89 |

Beispiel 364

(Z)-3-{1-[4-(N-(Pyrrolidin-1-ylmethylcarbonyl)-N-methyl-amino)-phenylamino]-1-phenyl-methyliden}-5-nitro-2-indolinon

Hergestellt anälog Beispiel 43.
Schmelzpunkt: 246-247°C
$C_{28}H_{27}N_5O_4$ (497.55)
Massenspektrum: $M^+$ = 497

| | | | |
|---|---|---|---|
| Ber. | C 67.59 | H 5.47 | N 14.08 |
| Gef. | 67.34 | 5.53 | 14.00 |

Beispiel 365

(Z)-3-{1-[4-(N-Phthalimidomethylcarbonyl-N-methyl-amino)-phenylamino]-1-phenyl-methyliden}-2-indolinon

Hergestellt analog Beispiel 43.
Schmelzpunkt: 278-280°C
$C_{32}H_{24}N_4O_4$ (528.57)
Massenspektrum: $M^+$ = 528

Beispiel 366

(Z)-3-{1-[4-(N-Aminomethylcarbonyl-N-methyl-amino)-phenylamino]-1-phenyl-methyliden}-2-indolinon

Hergestellt analog Beispiel 365 und 139b.
Schmelzpunkt: 238-239°C
$C_{24}H_{22}N_4O_2$ (398.46)
Massenspektrum: $M^+$ = 398
$C_{24}H_{22}N_4O_2$ x 0.5 $H_2O$ (407.47)

| | | | |
|---|---|---|---|
| Ber. | C 70.74 | H 5.69 | N 13.75 |
| Gef. | 70.91 | 5.76 | 13.73 |

Beispiel 367

(Z)-3-{1-[4-(N-Acetylaminomethylcarbonyl-N-methyl-amino)-phenylamino]-1-phenyl-methyliden}-2-indolinon

Hergestellt analog Beispiel 366 und 140.
Schmelzpunkt: 255-256°C
$C_{26}H_{24}N_4O_3$ (440.50)
Massenspektrum: $[M-H]^-$ = 439

Beispiel 368

(Z)-3-{1-[4-(Acetylaminomethylcarbonylamino)-phenylamino]-1-phenyl-methyliden}-2-indolinon

Hergestellt analog Beispiel 43.
Schmelzpunkt: 245-247°C
$C_{25}H_{22}N_4O_3$ (426.47)
Massenspektrum: $M^+$ = 426

Beispiel 369

(Z)-3-{1-[4-(N-(Pyrrolidin-1-ylmethylcarbonyl)-N-methyl-amino)-phenylamino]-1-phenyl-methyliden}-2-indolinon

Hergestellt analog Beispiel 43.
Schmelzpunkt: 253-255°C
$C_{28}H_{28}N_4O_2$ (452.56)
Massenspektrum: $M^+$ = 452

Beispiel 370

(Z)-3-{1-[4-(N-(N-Benzyl-N-methyl-aminomethylcarbonyl)-N-methyl-amino)-phenylamino]-1-phenyl-methyliden}-2-indolinon

Hergestellt analog Beispiel 43.
Schmelzpunkt: 195-197°C
$C_{32}H_{30}N_4O_2$ (502.62)
Massenspektrum: $[M+H]^+$ = 503
$C_{32}H_{30}N_4O_2$ x 0.5 $H_2O$ (511.62)

| | | | |
|---|---|---|---|
| Ber. | C 75.12 | H 6.11 | N 10.95 |
| Gef. | 75.41 | 6.00 | 10.92 |

Beispiel 371

(Z)-3-{1-[4-(N-(2-Methoxyethylaminomethylcarbonyl)-N-methylamino)-phenylamino]-1-phenyl-methyliden}-2-indolinon

Hergestellt analog Beispiel 43.
Schmelzpunkt: 186-188°C
$C_{27}H_{28}N_4O_3$ (456.54)
Massenspektrum: $M^+$ = 456

Beispiel 372

(Z)-3-{1-[4-(N-(N-(2-Methoxyethyl)-N-methyl-aminomethylcarbonyl)-N-methyl-amino)-phenylamino]-1-phenyl-methyliden}-2-indolinon

Hergestellt analog Beispiel 43.
Schmelzpunkt: 197-199°C
$C_{28}H_{30}N_4O_3$ (470.57)
Massenspektrum: $M^+$ = 470

Beispiel 373

(Z)-3-{1-[4-(N-(2-Morpholinoethylaminomethylcarbonyl)-N-methyl-amino)-phenylamino]-1-phenyl-methyliden}-2-indolinon

Hergestellt analog Beispiel 43.
Schmelzpunkt: 117-119°C
$C_{30}H_{33}N_5O_3$ (511.62)
Massenspektrum: $M^+$ = 511

Beispiel 374

(Z)-3-{1-[4-(N-(N-(2-Morpholinoethyl)-N-methyl-aminomethylcarbonyl)-N-methyl-amino)-phenylamino]-1-phenyl-methyliden}-2-indolinon

Hergestellt analog Beispiel 43.
Schmelzpunkt: 116-118°C
$C_{31}H_{35}N_5O_3$ (525.65)
Massenspektrum: $M^+$ = 525

Beispiel 375

(Z)-3-{1-[4-(N-(N-(2-Dimethylaminoethyl)-N-methyl-aminomethylcarbonyl)-N-methyl-amino)-phenylamino]-1-phenyl-methyliden}-2-indolinon

Hergestellt analog Beispiel 43.
Schmelzpunkt: 167-169°C
$C_{29}H_{33}N_5O_2$ (483.61)
Massenspektrum: $M^+$ = 483

Beispiel 376

(Z)-3-{1-[4-(N-(2-tert.Butoxycarbonylamino-ethylcarbonyl)-N-methyl-amino)-phenylamino]-1-phenyl-methyliden}-2-indolinon

Hergestellt analog Beispiel 39.
Schmelzpunkt: 246-248°C
$C_{30}H_{32}N_4O_4$ (512.61)
Massenspektrum: $M^+$ = 512

| | | | |
|---|---|---|---|
| Ber. | C 70.29 | H 6.29 | N 10.93 |
| Gef. | 70.43 | 6.15 | 11.12 |

Beispiel 377

(Z)-3-{1-[4-(N-(2-Aminoethylcarbonyl)-N-methyl-amino)-phenylamino]-1-phenyl-methyliden}-2-indolinon-hydrochlorid

Hergestellt analog Beispiel 376 und 29a.
Schmelzpunkt: 97-99°C
$C_{25}H_{24}N_4O_2$ (412.49)
Massenspektrum: M+ = 412

Beispiel 378

(Z)-3-{1-[4-(N-(2-Acetylamino-ethylcarbonyl)-N-methyl-amino)-phenylamino]-1-phenyl-methyliden}-2-indolinon

Hergestellt analog Beispiel 376 und 31.
Schmelzpunkt: 187-189°C
$C_{27}H_{26}N_4O_3$ (454.53)
Massenspektrum: $M^+$ = 454

Beispiel 379

(Z)-3-{1-[4-(2-Dimethylamino-ethylsulfonylamino)-phenylamino]-1-phenyl-methyliden}-2-indolinon

a) 4-(2-Dimethylamino-ethylsulfonylamino)-nitrobenzol

Zu einer Lösung von 1.4 g (10 mMol) Nitroanilin in 25 ml Pyridin werden bei 0°C langsam 2.45 g (15 mMol) 2-Chlorethansulfonsäure-chlorid zugetropft. Man rührt anschließend 2 Stunden bei Raumtemperatur. Nach Entfernen des Lösungsmittels im Vakuum wird der Rückstand mit Wasser versetzt. Der Niederschlag wird abgesaugt und mit Wasser gewaschen. Man erhält 3.0 g 1-[2-(4-Nitrophenylsulfamoyl)-ethyl]-pyridinium-chlorid als Rohprodukt. 2.6 g dieses Rohprodukts werden in 25 ml DMF gelöst und mit 2 g (20 mMol) Triethylamin und 1.2 g (15 mMol) Dimethylamin-hydrochlorid versetzt. Man rührt 1.5 Stunden bei 100°C, gießt die Reaktionslösung anschließend in Wasser und extrahiert mit Essigester. Die organischen Extrakte werden über Magnesiumsulfat getrocknet und bis zur Trockene eingeengt.
Ausbeute: 1.6 g (59 % der Theorie)
$R_f$-Wert: 0.26 (Kieselgel; Dichlormethan/Methanol/$NH_4OH$ = 7:3:0.1)

b) 4-(2-Dimethylamino-ethylsulfonylamino)-anilin

Hergestellt analog Beispiel 39c durch katalytische Hydrierung von 4-(2-Dimethylamino-ethylsulfonylamino)-nitrobenzol.
Ausbeute: 88 % der Theorie
$R_f$-Wert: 0.34 (Kieselgel; Dichlormethan/Methanol = 7:3)

c) (Z)-3-{1-[4-(2-Dimethylamino-ethylsulfonylamino)-phenylamino]-1-phenyl-methyliden}-2-indolinon

Hergestellt analog Beispiel 39 aus 3-(1-Ethoxy-1-phenylmethyliden)-2-indolinon und 4-(2-Dimethylamino-ethylsulfonylamino)-anilin.
Ausbeute: 50 % der Theorie
Schmelzpunkt: 214-216°C
$C_{25}H_{26}N_4O_3S$ (462.57)
Massenspektrum: $M^+$ = 462

| | | | |
|---|---|---|---|
| Ber. | C 64.92 | H 5.67 | N 12.11 |
| Gef. | 64.88 | 5.71 | 11.98 |

Beispiel 380

(Z)-3-{1-[4-(2-Piperidinoethylsulfonylamino)-phenylamino]-1-phenyl-methyliden}-2-indolinon

Hergestellt analog Beispiel 379.
Schmelzpunkt: 225-227°C
$C_{28}H_{30}N_4O_3$ S (502.64)
Massenspektrum: $M^+$ = 502

| | | | |
|---|---|---|---|
| Ber. | C 66.91 | H 6.02 | N 11.15 |
| Gef. | 67.09 | 5.95 | 11.10 |

Beispiel 381

(Z)-3-{1-[4-(2-Morpholinoethylsulfonylamino)-phenylamino]-1-phenyl-methyliden}-2-indolinon

Hergestellt analog Beispiel 379.
Schmelzpunkt: 240-242°C
$C_{27}H_{28}N_4O_4S$ (504.61)
Massenspektrum: $M^+$ = 504

Beispiel 382

(Z)-3-{1-[4-(N-(2-Dimethylamino-ethylsulfonyl)-N-methyl-amino)-phenylamno]-1-phenyl-methyliden}-2-indolinon

a) 4-[N-(2-Dimethylamino-ethylsulfonyl)-N-methyl-amino]-nitrobenzol

Zu einer Lösung von 1.1 g (4 mMol) 4-(2-Dimethylamino-ethylsulfonylamino)-nitrobenzol (Beispiel 379a) in 20 ml DMSO werden bei Raumtemperatur 0.49 g (4.4 mMol) Kalium-tert.butylat zugegeben. Nach 1.5 Stunden Rühren werden 0.85 g (6 mMol) Methyliodid zugesetzt. Man rührt 18 Stunden, gießt die Reaktionslösung anschließend in Wasser und extrahiert mit Essigester. Die vereinigten organischen Phasen werden über Magnesiumsulfat getrocknet und vom Lösungsmittel im Vakuum befreit. Man erhält 0.9 g Ethensulfonsäure-N-(4-nitrophenyl)-N-methylamid als Rohprodukt. 0.75 g dieses Rohprodukts werden in Ethanol gelöst und mit einem Überschuß von Dimethylamin versetzt. Nach 18 Stunden Rühren wird bis zur Trockene eingeengt.
Ausbeute: 81 % der Theorie
$R_f$-Wert: 0.35 (Kieselgel; Dichlormethan/Methanol 19:1)

b) 4- [N- (2-Dimethylamino-ethylsulfonyl)-N-methyl-amino]-anilin

Hergestellt analog Beispiel 39c durch katalytische Hydrierung von 4-[N-(2-Dimethylamino-ethylsulfonyl)-N-methyl-amino]-nitrobenzol.
Ausbeute: 89 % der Theorie
$R_f$-Wert: 0.29 (Kieselgel; Dichlormethan/Methanol = 9:1)

c) (Z)-3-{1-[4-(N-(2-Dimethylamino-ethylsulfonyl)-N-methylamino)-phenylamino]-1-phenyl-methyliden}-2-indolinon

Hergestellt analog Beispiel 39 aus 3-(1-Ethoxy-1-phenyl-methyliden)-2-indolinon und 4-[N-(2-Dimethylamino-ethylsulfonyl)-N-methyl-amino]-anilin.
Ausbeute: 42 % der Theorie
Schmelzpunkt: 165-168°C
$C_{26}H_{28}N_4O_3S$ (476.60)
Massenspektrum: $[M+H]^+ = 476$

Beispiel 383

(Z)-3-{1-[4-(N-(2-Piperidinoethylsulfonyl)-N-methyl-amino)-phenylamino]-1-phenyl-methyliden}-2-indolinon

Hergestellt analog Beispiel 382.
Schmelzpunkt: 121-123°C
$C_{29}H_{32}N_4O_3S$ (516.66)
Massenspektrum: $M^+ = 516$

Beispiel 384

(Z)-3-{1-[4-(N-(2-Morpholinoethylsulfonyl)-N-methyl-amino)-phenylamino]-1-phenyl-methyliden}-2-indolinon

Hergestellt analog Beispiel 382.
Schmelzpunkt: 115-117°C
$C_{28}H_{30}N_4O_4S$ (518.63)
Massenspektrum: $M^+ = 518$

Beispiel 385

(Z)-3-{1-[4-(Diethylaminocarbonyl)-phenylamino]-1-phenyl-methyliden}-2-indolinon

Hergestellt analog Beispiel 18.
Schmelzpunkt: 202-204°C
$C_{26}H_{25}N_3O_2$ (411.50)
Massenspektrum: $M^+ = 411$

Beispiel 386

(Z)-3-{1-[4-(Pyrrolidin-1-ylcarbonyl)-phenylamino]-1-phenylmethyliden}-2-indolinon

Hergestellt analog Beispiel 18.
Schmelzpunkt: 127-129°C
$C_{26}H_{23}N_3O_2$ (409.49)
Massenspektrum: $M^+$ = 409

Beispiel 387

(Z)-3-{1-[4-(Piperidinocarbonyl)-phenylamino]-1-phenyl-methyiden}-2-indolinon

Hergestellt analog Beispiel 18.
Schmelzpunkt: 212-214°C
$C_{27}H_{25}N_3O_2$ (423.51)
Massenspektrum: $M^+$ = 423

Beispiel 388

(Z)-3-{1-[4-(2-Methoxyethylaminocarbonyl)-phenylamino]-1-phenyl-methyliden}-2-indolinon

Hergestellt analog Beispiel 18.
Schmelzpunkt: 277-279°C
$C_{25}H_{23}N_3O_3$ (413.47)
Massenspektrum: $M^+$ = 413

Beispiel 389

(Z)-3-{1-[4-(N-(2-Methoxyethyl)-N-methyl-aminocarbonyl)-phenylamino]-1-phenyl-methyliden}-2-indolinon

Hergestellt analog Beispiel 18.
Schmelzpunkt: 198-200°C
$C_{26}H_{25}N_3O_3$ (427.50)
Massenspektrum: $M^+$ = 427

| | | | |
|---|---|---|---|
| Ber. | C 73.05 | H 5.89 | N 9.83 |
| Gef. | 72.75 | 6.04 | 9.75 |

Beispiel 390

(Z)-3-{1-[4-(2-Dimethylamino-ethylaminocarbonyl)-phenylamino]-1-phenyl-methyliden}-2-indolinon

Hergestellt analog Beispiel 18.
Schmelzpunkt: 145-147°C
$C_{26}H_{26}N_4O_2$ (426.52)
Massenspektrum: $M^+$ = 426

Beispiel 391

(Z)-3-{1-[4-(N-(2-Dimethylaminoethyl)-N-methyl-aminocarbonyl)-phenylamino]-1-phenyl-methyliden}-2-indolinon

Hergestellt analog Beispiel 18.
Schmelzpunkt: 181-183°C
$C_{27}H_{28}N_4O_2$ (440.54)
Massenspektrum: $M^+$ = 440

| | | | |
|---|---|---|---|
| Ber. | C 73.61 | H 6.41 | N 12.72 |
| Gef. | 73.51 | 6.59 | 12.75 |

Beispiel 392

(Z)-3-{1-[4-(Ethoxycarbonylmethylaminocarbonyl)-phenylamino]-1-phenyl-methyliden}-2-indolinon

Hergestellt analog Beispiel 18.
Schmelzpunkt: 235-237°C
$C_{26}H_{23}N_3O_4$ (441.48)
Massenspektrum: $M^+$ = 441

Beispiel 393

(Z)-3-{1-[4-(Carboxymethylaminocarbonyl)-phenylamino]-1-phenyl-methyliden}-2-indolinon

Hergestellt analog Beispiel 392 und 8.
Schmelzpunkt: 245-247°C
$C_{24}H_{19}N_3O_4$ (413.43)
Massenspektrum: $M^+$ = 413

Beispiel 394

(Z)-3-{1-[4-(N-Ethoxycarbonylmethyl-N-methyl-aminocarbonyl)-phenylamino]-1-phenyl-methyliden}-2-indolinon

Hergestellt analog Beispiel 18.
Schmelzpunkt: 95-98°C
$C_{27}H_{25}N_3O_4$ (455.51)
Massenspektrum: $M^+$ = 455

Beispiel 395

(Z)-3-{1-[4-(N-Carboxymethyl-N-methyl-aminocarbonyl)-phenylamino]-1-phenyl-methyliden}-2-indolinon

Hergestellt analog Beispiel 394 und 8.
Schmelzpunkt: 168-170°C
$C_{25}H_{21}N_3O_4$ (427.46)
Massenspektrum: $M^+$ = 427

Beispiel 396

(Z)-3-{1-[4-(Aminosulfonyl)-phenylamino]-1-phenyl-methyliden}-2-indolinon

Hergestellt analog Beispiel 39d.
Schmelzpunkt: 254°C
$C_{21}H_{17}N_3O_3S$ (391.45)
Massenspektrum: $M^+$ = 391
$C_{21}H_{17}N_3O_3S$ x $H_2O$ (409.35)

| | | | |
|---|---|---|---|
| Ber. | C 61.60 | H 4.68 | N 10.26 |
| Gef. | 61.86 | 4.72 | 10.27 |

Beispiel 397

(Z)-3-{1-[4-(Pyrrolidin-1-ylsulfonyl)-phenylamino]-1-phenylmethyliden}-2-indolinon

a) 4-(Pyrrolidin-1-ylsulfonyl)-anilin

525 mg (3 mMol) Sulfanilsäurefluorid und 1.07 g (15 mMol) Pyrrolidin werden zusammen 15 Minuten auf 80°C erhitzt. Danach gibt man Wasser zum Reaktionsgemisch. Der ausgefallene Niederschlag wird abfiltiert und aus Methanol umkristallisiert. Ausbeute: 375 mg (55 % der Theorie)
Schmelzpunkt: 170-172°C
$R_f$-Wert: 0.44 (Kieselgel; Dichlormethan/Essigester = 9:1)

b) (Z)-3-{1-[4-(Pyrrolidin-1-ylsulfonyl)-phenylamino]-1-phenyl-methyliden}-2-indolinon

Hergestellt analog Beispiel 1c aus 1-Acetyl-3-(1-ethoxy-1-phenyl-methyliden)-2-indolinon und 4-(Pyrrolidin-1-ylsulfonyl)-anilin.
Ausbeute: 45 % der Theorie
Schmelzpunkt: 293-294°C
$C_{25}H_{23}N_3O_3S$ (445.54)
Massenspektrum: $M^+$ = 445
$C_{25}H_{23}N_3O_3S$ x 0.25 $H_2O$ (450.04)

| | | | |
|---|---|---|---|
| Ber. | C 66.72 | H 5.26 | N 9.34 |
| Gef. | 66.62 | 5.29 | 9.12 |

Beispiel 398

(Z)-3-{1-[4-(Diethylaminosulfonyl)-phenylamino]-1-phenyl-methyliden}-2-indolinon

Hergestellt analog Beispiel 397.
Schmelzpunkt: 252-254°C
$C_{25}H_{25}N_3O_3S$ (447.56)
Massenspektrum: $M^+$ = 447

| | | | |
|---|---|---|---|
| Ber. | C 67.09 | H 5.63 | N 9.39 |
| Gef. | 66.96 | 5.68 | 9.25 |

Beispiel 399

(Z)-3-{1-[4-(2-Dimethylamino-ethylaminosulfonyl)-phenylamino]-1-phenyl-methyliden}-2-indolinon

Hergestellt analog Beispiel 397.
Schmelzpunkt: 233-235°C
$C_{25}H_{26}N_4O_3S$ (462.57)
Massenspektrum: $M^+$ = 462
$C_{25}H_{26}N_4O_3S$ x 0.25 $H_2O$ (467.07)

| | | | |
|---|---|---|---|
| Ber. | C 64.29 | H 5.72 | N 12.00 |
| Gef. | 64.15 | 5.64 | 12.00 |

Beispiel 400

(Z)-3-{1-[4-(N-(2-Dimethylaminoethyl)-N-methyl-aminosulfonyl)-phenylamino]-1-phenyl-methyliden}-2-indolinon

Hergestellt analog Beispiel 397.
Schmelzpunkt: 200-203°C

$C_{26}H_{28}N_4O_3S$ (476.60)
Massenspektrum: $M^+$ = 476

Beispiel 401

(Z)-3-{1-[4-(2-Dimethylamino-ethylaminosulfonyl)-phenylamino]-1-phenyl-methyliden}-5-nitro-2-indolinon

Hergestellt analog Beispiel 397.
Schmelzpunkt: 260-261°C
$C_{25}H_{25}N_5O_5S$ (507.57)
Massenspektrum: $M^+$ = 507

Beispiel 402

(Z)-3-{1-[4-(N-(2-Dimethylaminoethyl)-N-methyl-aminosulfonyl)-phenylamino]-1-phenyl-methyliden}-5-nitro-2-indolinon

Hergestellt analog Beispiel 397.
Schmelzpunkt: 215-218°C
$C_{26}H_{27}N_5O_5S$ (521.60)
Massenspektrum: $[M+H]^+$ = 522
$C_{26}$ $H_{27}$ $N_5$ $O_5$ S x 0.3 H2O (527.00)

| | | | |
|---|---|---|---|
| Ber. | C 59.26 | H 5.28 | N 13.29 |
| Gef. | 59.25 | 5.19 | 13.17 |

Beispiel 403

(Z)-3-{1-[4-(3-Dimethylamino-propylaminosulfonyl)-phenylamino]-1-phenyl-methyliden}-5-nitro-2-indolinon

Hergestellt analog Beispiel 397.
Schmelzpunkt: 268-269°C
$C_{26}H_{27}N_5O_5S$ (521.60)
Massenspektrum: $M^+$ = 521

| | | | |
|---|---|---|---|
| Ber. | C 59.87 | H 5.22 | N 13.43 |
| Gef. | 59.65 | 5.32 | 13.26 |

Beispiel 404

(Z)-3-{1-[4-(N-(3-Dimethylaminopropyl)-N-methyl-aminosulfonyl)-phenylamino]-1-phenyl-methyliden}-5-nitro-2-indolinon

Hergestellt analog Beispiel 397.
Schmelzpunkt: 269-270°C (Zers.)
$C_{27}H_{29}N_5O_5S$ (535.62)
Massenspektrum: $M^+$ = 535

| | | | |
|---|---|---|---|
| Ber. | C 60.55 | H 5.46 | N 13.08 |
| Gef. | 60.28 | 5.56 | 12.90 |

Beispiel 405

(Z)-3-{1-[4-(Dimethylaminosulfonyl)-phenylamino]-1-phenyl-methyliden}-2-indolinon

a) 4-Nitrobenzolsulfonsäure-dimethylamid

Zu einer Lösung von 2.45 g (30 mMol) Dimethylamin-hydrochlorid und 6.46 g (50 mMol) N,N-Diisopropyl-N-methylamin in 30 ml Dichlormethan werden bei 0°C 4.43 g (20 mMol) 4-Nitrobenzolsulfonsäurechlorid zugetropft. Man rührt 18 Stunden bei Raumtemperatur. Anschließend wird die Reaktionslösung mit Wasser und verdünnter Salzsäure gewaschen, über Magnesiumsulfat getrocknet und bis zur Trockene eingedampft.
Ausbeute: 4.4 g (90 % der Theorie)

b) 4-Aminobenzol sulfonsäure-dimethylamid

Hergestellt analog Beispiel 39c durch katalytische Hydrierung von 4-Nitrobenzolsulfonsäure-dimethylamid.
Ausbeute: 78 % der Theorie
Schmelzpunkt: 172-173°C

c) (Z)-3-{1-[4-(Dimethylaminosulfonyl)-phenylamino]-1-phenylmethyliden}-2-indolinon

Hergestellt analog Beispiel 1c aus 1-Acetyl-3-(1-ethoxy-1-phenyl-methyliden)-2-indolinon und 4-Aminobenzolsulfonsäure-dimethylamid.
Ausbeute: 78 % der Theorie
Schmelzpunkt: 280°C
$C_{23}H_{21}N_3O_3S$ (419.50)
Massenspektrum: $M^+ = 419$

| | | | |
|---|---|---|---|
| Ber. | C 65.85 | H 5.05 | N 10.02 |
| Gef. | 65.54 | 5.24 | 9.96 |

Beispiel 406

(Z)-3-{1-[4-(Ethoxycarbonylmethylaminosulfonyl)-phenylamino]-1-phenyl-methyliden}-2-indolinon

Hergestellt analog Beispiel 405.
Schmelzpunkt: 196-199°C
$C_{25}H_{23}N_3O_5S$ (477.54)
Massenspektrum: $M^+ = 477$

| | | | |
|---|---|---|---|
| Ber. | C 62.88 | H 4.85 | N 8.80 |
| Gef. | 62.79 | 5.04 | 8.68 |

Beispiel 407

(Z)-3-{1-[4-(Carboxymethylaminosulfonyl)-phenylamino]-1-phenyl-methyliden}-2-indolinon

Hergestellt analog Beispiel 406 und 8.
Schmelzpunkt: 236°C (Zers.)
$C_{23}H_{19}N_3O_5S$ (449.49)
Massenspektrum: $M^+ = 449$

Beispiel 408

(Z)-3-{1-[4-(N-Ethoxycarbonylmethyl-N-methyl-aminosulfonyl)-phenylamino]-1-phenyl-methyliden}-2-indolinon

Hergestellt analog Beispiel 405.
Schmelzpunkt: 178-180°C

$C_{26}H_{25}N_3O_5S$ (491.57)
Massenspektrum: $M^+$ = 491

Beispiel 409

(Z)-3-{1-[4-(N-Carboxymethyl-N-methyl-aminosulfonyl)-phenylamino]-1-phenyl-methyliden}-2-indolinon

Hergestellt analog Beispiel 408 und 8.
Schmelzpunkt: 237°C (Zers.)
$C_{24}H_{21}N_3O_5$ S (463.51)
Massenspektrum: $M^+$ = 463

Beispiel 410

(Z)-3-{1-[4-(Ethoxycarbonylmethylaminosulfonyl)-phenylamino]-1-phenyl-methyliden}-5-nitro-2-indolinon

Hergestellt analog Beispiel 405.
Schmelzpunkt: 247-249°C
$C_{25}H_{22}N_4O_7S$ (522.54)
Massenspektrum: $M^+$ = 522

| | | | |
|---|---|---|---|
| Ber. | C 57.47 | H 4.24 | N 10.72 |
| Bef. | 57.44 | 4.22 | 10.66 |

Beispiel 411

(Z)-3-{1-[4-(Carboxymethylaminosulfonyl)-phenylamino]-1-phenyl-methyliden}-5-nitro-2-indolinon

Hergestellt analog Beispiel 410 und 8.
Schmelzpunkt: 177-180°C (Zers.)
$C_{23}H_{18}N_4O_7S$ (494.48)
Massenspektrum: $M^+$ = 494
$C_{23}H_{18}N_4O_7S \times H_2O$ (512.50)

| | | | |
|---|---|---|---|
| Ber. | C 53.90 | H 3.93 | N 10.93 |
| Gef. | 53.98 | 3.95 | 10.86 |

Beispiel 412

(Z)-3-{1-[4-(Dimethylaminocarbonylmethylaminosulfonyl)-phenylamino]-1-phenyl-methyliden}-5-nitro-2-indolinon

Hergestellt analog Beispiel 411 und 18.
Schmelzpunkt: 281-283°C
$C_{25}H_{23}N_5O_6S$ (521.55)
Massenspektrum: $M^+$ = 521
$C_{25}H_{23}N_5O_6S \times 0.5\ H_2O$ (530.56)

| | | | |
|---|---|---|---|
| Ber. | C 56.60 | H 4.56 | N 13.20 |
| Gef. | 56.51 | 4.56 | 13.15 |

Beispiel 413

(Z)-3-{1-[4-(N-Ethoxycarbonylmethyl-N-methyl-aminosulfonyl)-phenylamino]-1-phenyl-methyliden}-5-nitro-2-indolinon

Hergestellt analog Beispiel 405.

Schmelzpunkt: 206-207°C
$C_{26}H_{24}N_4O_7S$ (536.56)
Massenspektrum: $M^+$ = 536

Beispiel 414

(Z)-3-{1-[4-(N-Carboxymethyl-N-methyl-aminosulfonyl)-phenylamino]-1-phenyl-methyliden}-5-nitro-2-indolinon

Hergestellt analog Beispiel 413 und 8.
Schmelzpunkt: 259-260°C
$C_{24}H_{20}N_4O_7S$ (508.51)
Massenspektrum: $M^+$ = 508

Beispiel 415

(Z)-3-{1-[4-(N-Dimethylaminocarbonylmethyl-N-methyl-aminosulfonyl)-phenylamino]-1-phenyl-methyliden}-5-nitro-2-indolinon

Hergestellt analog Beispiel 414 und 18.
Schmelzpunkt: 277-278°C
$C_{26}H_{25}N_5O_6S$ (535.58)
Massenspektrum: $M^+$ = 535

| | | | |
|---|---|---|---|
| Ber. | C 58.31 | H 4.71 | N 13.08 |
| Gef. | 58.07 | 4.68 | 13.03 |

Beispiel 416

(Z)-3-{1-[3-(N-Aminocarbonylmethyl-N-methylsulfonyl-amino)-phenylamino]-1-(4-aminomethyl-phenyl)-methyliden}-2-indolinon

Hergestellt analog Beispiel 146, 148 und 191.
Schmelzpunkt: 167°C
$C_{25}H_{25}N_5O_4S$ (491.57)
Massenspektrum: $[M+H]^+$ = 492

Beispiel 417

(Z)-3-{1-[3-(N-Aminocarbonylmethyl-N-methylsulfonyl-amino)-phenylamino]-1-(4-acetylaminomethyl-phenyl)-methyliden}-2-indolinon

Hergestellt analog Beispiel 416 und 31.
Schmelzpunkt: 215°C
$C_{27}H_{27}N_5O_5S$ (533.61)
Massenspektrum: $[M-H]^-$ = 532

Beispiel 418

(Z)-3-{1-[3-(N-Methylaminocarbonylmethyl-N-methylsulfonyl-amino)-phenylamino]-1-(4-aminomethyl-phenyl)-methyliden}-2-indolinon

Hergestellt analog Beispiel 146, 148 und 192.
Schmelzpunkt: 164°C
$C_{26}H_{27}N_5O_4S$ (505.60)
Massenspektrum: $M^+$ = 505
$C_{26}H_{27}N_5O_4S$ x 0.7 $H_2O$ (518.21)

| | | | |
|---|---|---|---|
| Ber. | C 60.26 | H 5.52 | N 13.51 |
| Gef. | 60.28 | 5.51 | 13.78 |

Beispiel 419

(Z)-3-{1-[3-(N-Methylaminocarbonylmethyl-N-methylsulfonyl-amino)-phenylamino]-1-(4-acetylaminomethyl-phenyl)-methyliden}-2-indolinon

Hergestellt analog Beispiel 418 und 31.
Schmelzpunkt: 242°C
$C_{28}H_{29}N_5O_5S$ (547.63)
Massenspektrum: $M^+$ = 547
$C_{28}H_{29}N_5O_5S$ x 0.5 $H_2O$ (556.64)

| | | | |
|---|---|---|---|
| Ber. | C 60.42 | H 5.43 | N 12.58 |
| Gef. | 60.67 | 5.67 | 12.30 |

Beispiel 420

(Z)-3-{1-[3-(N-Dimethylaminocarbonylmethyl-N-methylsulfonylamino)-phenylamino]-1-(4-aminomethyl-phenyl)-methyliden}-2-indolinon

Hergestellt analog Beispiel 146, 148 und 192.
Schmelzpunkt: 220°C
$C_{27}H_{29}N_5O_4S$ (519.62)
Massenspektrum: $[M+H]^+$ = 520
$C_{27}H_{29}N_5O_4S$ 0.2 $H_2O$ (523.23)

| | | | |
|---|---|---|---|
| Ber. | C 61.98 | H 5.66 | N 13.38 |
| Gef. | 61.95 | 5.73 | 13.27 |

Beispiel 421

(Z)-3-{1-[3-(N-Dimethylaminocarbonylmethyl-N-methylsulfonylamino)-phenylamino]-1-(4-acetylaminomethyl-phenyl)-methyliden}-2-indolinon

Hergestellt analog Beispiel 420 und 31.
Schmelzpunkt: 194°C (Sinterung)
$C_{29}H_{31}N_5O_5S$ (561.66)
Massenspektrum: $[M-H]^-$ = 560

Beispiel 422

(Z)-3-{1-[3-(N-(2-Dimethylaminoethyl)-N-methylsulfonyl-amino)-phenylamino]-1-(4-aminomethyl-phenyl)-methyliden}-2-indolinon

Hergestellt analog Beispiel 146, 148 und 324.
Schmelzpunkt: 161°C
$C_{27}H_{31}N_5O_3S$ (505.64)
Massenspektrum: $M^+$ = 505

Beispiel 423

(Z)-3-{1-[3-(N-(2-Dimethylaminoethyl)-N-methylsulfonyl-amino)-phenylamino]-1-(4-acetylaminomethyl-phenyl)-methyliden}-2-indolinon

Hergestellt analog Beispiel 422 und 31.
Schmelzpunkt: 180°C
$C_{29}H_{33}N_5O_4S$ (547.68)
Massenspektrum: $M^+ = 547$

Beispiel 424

(Z) -3-{1-[3-(N-(3-Dimethylaminopropyl)-N-methylsulfonyl-amino)-phenylamino]-1-(4-aminomethyl-phenyl)-methyliden}-2-indolinon

Hergestellt analog Beispiel 146, 148 und 324.
Schmelzpunkt: 197°C
$C_{28}H_{33}N_5O_3S$ (519.67)
Massenspektrum: $M^+ = 519$
$C_{28}H_{33}N_5O_3S$ 0.5 $H_2O$ (528.67)

| | | | |
|---|---|---|---|
| Ber. | C 63.61 | H 6.48 | N 13.25 |
| Gef. | 63.64 | 6.47 | 13.39 |

Beispiel 425

(Z)-3-{1-[3-(N-(3-Dimethylaminopropyl)-N-methylsulfonyl-amino)-phenylamino]-1-(4-acetylaminomethyl-phenyl)-methyliden}-2-indolinon

Hergestellt analog Beispiel 424 und 31.
Schmelzpunkt: 208°C
$C_{30}H_{35}N_5O_4S$ (561.70)
Massenspektrum: $M^+ = 561$
$C_{30}H_{35}N_5O_4S$ 0.8 H2O (576.12)

| | | | |
|---|---|---|---|
| Ber. | C 62.54 | H 6.40 | N 12.16 |
| Gef. | 62.51 | 6.37 | 12.13 |

Beispiel 426

(Z)-3-{1-[4-(N-(2-Dimethylaminoethyl)-N-methylsulfonyl-amino)-phenylamino]-1-(4-aminomethyl-phenyl)-methyliden}-2-indolinon

Hergestellt analog Beispiel 146, 148 und 188.
Schmelzpunkt: 203-205°C
$C_{27}H_{31}N_5O_3S$ (505.64)
Massenspektrum: $M^+ = 505$

Beispiel 427

(Z)-3-{1-[4-(N-(2-Dimethylaminoethyl)-N-methylsulfonyl-amino)-phenylamino]-1-(4-acetylaminomethyl-phenyl)-methyliden}-2-indolinon

Hergestellt analog Beispiel 426 und 31.
Schmelzpunkt: 225-227°C
$C_{29}H_{33}N_5O_4S$ (547.68)
Massenspektrum: $M^+ = 547$

Beispiel 428

(Z)-3-{1-[4-(N-Dimethylaminocarbonylmethyl)-N-methylsulfonylamino)-phenylamino]-1-(4-aminomethyl-phenyl)-methyliden}-2-indolinon

Hergestellt analog Beispiel 146, 148 und 193.
Schmelzpunkt: 118-120°C
$C_{27}H_{29}N_5O_4S$ (519.62)
Massenspektrum: $[M+H]^+ = 520$

Beispiel 429

(Z)-3-{1-[4-(N-Dimethylaminocarbonylmethyl)-N-methylsulfonylamino)-phenylamino]-1-(4-acetylaminomethyl-phenyl)-methyliden}-2-indolinon

Hergestellt analog Beispiel 428 und 31.
Schmelzpunkt: 147-149°C
$C_{29}H_{31}N_5O_5S$ (561.66)
Massenspektrum: $[M-H]^- = 560$

Beispiel 430

(Z)-3-{1-[4-(N-Dimethylaminomethylcarbonyl-N-methyl-amino)-phenylamino]-1-(4-aminomethyl-phenyl)-methyliden}-2-indolinon

Hergestellt analog Beispiel 146, 148 und 48.
Schmelzpunkt: 188-190°C
$C_{27}H_{29}N_5O_2$ (455.56)
Massenspektrum: $M^+ = 455$

Beispiel 431

(Z)-3-{1-[4-(N-Dimethylaminomethylcarbonyl-N-methyl-amino)-phenylamino]-1-(4-acetylaminomethyl-phenyl)-methyliden}-2-indolinon

Hergestellt analog Beispiel 430 und 31.
Schmelzpunkt: 123-125°C
$C_{29}H_{31}N_5O_3$ (497.60)
Massenspektrum: $M^+ = 497$

Beispiel 432

(Z)-3-[1-(4-Piperdinomethyl-phenylamino)-1-(4-bromphenyl)-methyliden]-5-nitro-2-indolinon

Hergestellt analog Beispiel 146.
Schmelzpunkt: 295-297°C
$C_{27}H_{25}BrN_4O_3$ (533.42)
Massenspektrum: $M^+ = 534/532$

Beispiel 433

(Z)-3-[1-(4-Piperdinomethyl-phenylamino)-1-(4-iodphenyl)-methyliden]-5-nitro-2-indolinon

Hergestellt analog Beispiel 146.
Schmelzpunkt: 280-283°C
$C_{27}H_{25}IN_4O_3$ (580.42)
Massenspektrum: $[M+H]^+ = 581$

Beispiel 434

(Z)-3-[1-(4-Methoxyphenylamino)-1-(4-iodphenyl)-methyliden]-5-nitro-2-indolinon

Hergestellt analog Beispiel 146.
Schmelzpunkt: 280-283°C
$C_{22}H_{16}IN_3O_4$ (513.29)
Massenspektrum: $M^+$ = 513

Beispiel 435

(Z)-3-{1-(4-Methoxyphenylamino)-1-[(E)-4-(2-methoxycarbonylethenyl)-phenyl]-methyliden}-5-nitro-2-indolinon

Unter einer Stickstoffatmosphäre werden 257 mg (0.5 mMol) (Z)-3-[1-(4-Methoxyphenylamino)-1-(4-iodphenyl)-methyliden]-5-nitro-2-indolinon (Beispiel 434), 0.06 ml (0.75 mMol) Acrylsäuremethylester, 4.5 mg (0.02 mMol) Palladium-II-acetat und 1 ml (7.2 mMol) Triethylamin in 20 ml Acetonitril gelöst. Man erhitzt 10 Stunden auf 80°C. Danach wird die Reaktionslösung über Celite filtriert und vom Lösungsmittel im Vakuum befreit. Der Rückstand wird an Kieselgel chromatographiert (Dichlormethan/Methanol = 20:1).
Ausbeute: 0.2 g (85 % der Theorie)
Schmelzpunkt: 266-270°C
$C_{26}H_{21}N_3O_6$ (471.47)
Massenspektrum: $M^+$ = 471

Beispiel 436

(Z)-3-{1-[4-(N-(2-Dimethylaminoethyl)-N-methylsulfonyl-phenylamino]-1-[4-methoxyphenyl]-methyliden}-2-indolinon

Hergestellt analog Beispiel 146 und 188.
Schmelzpunkt: 219°C
$C_{27}H_{30}N_4O_4S$ (506.62)
Massenspektrum: $M^+$ = 506
$C_{27}H_{30}N_4O_4S$ x 0.2 $H_2O$ (510.23)

| | | | |
|---|---|---|---|
| Ber. | C 63.56 | H 6.01 | N 10.98 |
| Gef. | 63.61 | 6.11 | 10.97 |

Beispiel 437

(Z)-3-{1-[4-(N-(2-Dimethylaminoethyl)-N-methylsulfonyl-phenylamino]-1-[4-chlorphenyl]-methyliden}-2-indolinon

Hergestellt analog Beispiel 146 und 188.
Schmelzpunkt: 263°C
$C_{26}H_{27}ClN_4O_3$ S (511.04)
Massenspektrum: $M^+$ = 512/510

Beispiel 438

(Z)-3-{1-[4-Bromphenylamino]-1-[4-(imidazol-1-ylmethyl)-phenyl]-methyliden}-5-nitro-2-indolinon

Hergestellt analog Beispiel 146.
Schmelzpunkt: 260-265°C
$C_{25}H_{18}BrN_5O_3$ (516.35)
Massenspektrum: $M^+$ = 517/515

Beispiel 439

(Z)-3-{1-[4-Piperidinomethyl-phenylamino]-1-[4-(imidazol-1-ylmethyl)-phenyl]-methyliden}-5-nitro-2-indolinon

Hergestellt analog Beispiel 146.
Schmelzpunkt: 226-228°C
$C_{31}H_{30}N_6O_3$ (534.62)
Massenspektrum: $M^+$ = 535

Beispiel 440

(Z)-3-{1-[4-(N-Benzyl-N-methyl-aminomethyl)-phenylamino]-1-[4-(imidazol-1-ylmethyl)-phenyl]-methyliden}-5-nitro-2-indolinon

Hergestellt analog Beispiel 146.
Schmelzpunkt: 195-198°C
$C_{34}H_{30}N_6O_3$ (570.65)
Massenspektrum: $[M+H]^+$ = 571

Beispiel 441

(Z)-3-[1-(4-Piperidinomethyl-phenylamino)-1-(4-methoxyphenyl)-methyliden]-5-nitro-2-indolinon

a) [Methoxy-(4-methoxyphenyl)-methyl]-phosphonsäurediethylester

Unter einer Stickstoffatmosphäre werden zu einer Lösung von 5.6 ml (33 mMol) Anisaldehyddimethylacetal und 5.7 ml (33 mMol) Triethylphosphit in 60 ml Dichlormethan bei -20°C 4.3 ml (35 mMol) Bortrifluorid-etherat zugetropft. Man rührt 18 Stunden bei Raumtemperatur und setzt anschließend Wasser zu. Nach 1 Stunde Rühren werden die Phasen getrennt. Die organische Phase wird über Magnesiumsulfat getrocknet und vom Lösungsmittel im Vakuum befreit. Der Rückstand wird an Kieselgel chromatographiert (Dichlormethan/Essigester, 10:1).
Ausbeute: 7.5 g (79 % der Theorie)
$R_f$-Wert: 0.5 (Kieselgel; Dichlormethan/Essigester = 10:1)

b) 3-[1-Methoxy-1-(4-methoxyphenyl)-methyliden]-5-nitro-2-indolinon

Unter einer Stickstoffatmosphäre werden 6.3 g (22 mMol) [Methoxy-(4-methoxyphenyl)-methyl]-phosphonsäurediethylester in 40 ml DMF gelöst. Man gibt bei -40°C portionsweise 5.1 g (45 mMol) Kalium-tert.butylat zu und rührt anschließend noch 30 Minuten bei -10°C. Dann werden 3.84 g (20 mMol) 5-Nitroisatin zugegeben. Man rührt 1 Stunde bei Raumtemperatur und gießt anschließend auf Eiswasser, das 20 ml gesättigte Kaliumhydrogensulfatlösung enthält. Der Niederschlag wird abgesaugt und über Kieselgel chromatographiert (Dichlormethan/Methanol = 10:1).
Ausbeute: 4.4 g (67 % der Theorie)
Schmelzpunkt: 220-225°C
$C_{17}H_{14}N_2O_5$ (326.31)
Massenspektrum: $[M-H]^-$ = 325

c) (Z)-3-[1-(4-Piperidinomethyl-phenylamino)-1-(4-methoxyphenyl)-methyliden]-5-nitro-2-indolinon

Hergestellt analog Beispiel 39d aus 3-[1-Methoxy-1-(4-methoxyphenyl)-methyliden]-5-nitro-2-indolinon und 4-Piperidinomethyl-anilin.
Ausbeute: 90 % der Theorie
Schmelzpunkt: 230-233°C
$C_{28}H_{28}N_4O_4$ (484.55)
Massenspektrum: $[M+H]^+$ = 484

Beispiel 442

(Z)-3-[1-(4-Piperidinomethyl-phenylamino)-1-(4-trifluormethylphenyl}-methyliden]-5-nitro-2-indolinon

Hergestellt analog Beispiel 441.
Schmelzpunkt: 300-302°C
$C_{28}H_{25}F_3N_4O_3$ (522.52)
Massenspektrum: $M^+$ = 522

Beispiel 443

(Z)-3-[1-(4-Piperidinomethyl-phenylamino)-1-(4-chlorphenyl)-methyliden]-5-nitro-2-indolinon

Hergestellt analog Beispiel 441.
Schmelzpunkt: 309-311°C
$C_{27}H_{25}ClN_4O_3$ (488.97)
Massenspektrum: $[M+H]^+$ = 491/489

Beispiel 444

(Z)-3-[1-(4-Piperidinomethyl-phenylamino)-1-(4-methoxycarbonyl-phenyl)-methyliden]-5-nitro-2-indolinon

Hergestellt analog Beispiel 441.
Schmelzpunkt: 178-83°C
$C_{29}H_{28}N_4O_5$ (512.56)
Massenspektrum: $M^+$ = 512

Beispiel 445

(Z)-3-[1-(4-Piperidinomethyl-phenylamino)-1-(4-carboxyphenyl)methyliden]-5-nitro-2-indolinon

Hergestellt analog Beispiel 444 und 8.
Schmelzpunkt: 230°C
$C_{28}H_{26}N_4O_5$ (498.54)
Massenspektrum: $[M-H]^-$ = 497

Beispiel 446

(Z)-3-[1-(4-Piperidinomethyl-phenylamino)-1-(4-methoxycarbonylmethylaminocarbonyl-phenyl)-methyliden]-5-nitro-2-indolinon

Hergestellt analog Beispiel 445 und 18.
Schmelzpunkt: 230-235°C
$C_{31}H_{31}N_5O_6$ (569.61)
Massenspektrum: $[M+H]^+$ = 570

Beispiel 447

(Z)-3-[1-(4-Piperidinomethyl-phenylamino)-1-(4-(2-methoxycarbonyl-ethylaminocarbonyl)-phenyl)-methyliden]-5-nitro-2-indolinon

Hergestellt analog Beispiel 445 und 18.
Schmelzpunkt: 130°C
$C_{32}H_{33}N_5O_6$ (583.64)
Massenspektrum: $M^+$ = 583

Beispiel 448

(Z)-3-[1-(4-Piperidinomethyl-phenylamino)-1-phenyl-methyliden]-6-nitro-2-indolinon

a) 1-Hydroxy-6-nitro-2-indolinon

31 g (137 mMol) 2,4-Dinitrophenylessigsäure werden in 400 ml Essigester gelöst und analog Beispiel 39c über Pd-Kohle hydriert. Ausbeute: 13.2 g (50 % der Theorie).
$R_f$-Wert: 0.6 (Kieselgel; Dichlormethan/Methanol 9:1)
$C_8H_6N_2O_4$ (194.15)
Massenspektrum: $[M-H]^-$ = 193

b) 1-Acetoxy-3-(1-ethoxy-1-phenyl-methyliden)-6-nitro-2-indolinon

Hergestellt analog Beispiel 1b aus 1-Hydroxy-6-nitro-2-indolinon und Orthobenzoesäuretriethylester i Acetanhydrid.
Ausbeute: 62 % der Theorie
$R_f$-Wert: 0.3 (Kieselgel; Dichlormethan)
$C_{19}H_{16}N_2O_6$ (368.35)
Massenspektrum: $[M+Na]^+$ = 391

c) (Z)-3-[1-(4-Piperidinomethyl-phenylamino)-1-phenyl-methyliden]-1-hydroxy-6-nitro-2-indolinon

Hergestellt analog Beispiel 1c aus 1-Acetoxy-3-(1-ethoxy-1-phenyl-methyliden)-6-nitro-2-indolinon und 4-Piperidinomethyl-anilin.
Ausbeute: 88 % der Theorie
$R_f$-Wert: 0.48 (Kieselgel; Dichlormethan/Methanol = 9:1)
$C_{27}H_{26}N_4O_4$ (470.53)
Massenspektrum: $[M+H]^+$ = 471

d) (Z)-3-[1-(4-Piperidinomethyl-phenylamino)-1-phenyl-methyliden]-6-nitro-2-indolinon

Hergestellt analog Beispiel 39c durch katalytische Hydrierung von (Z)-3-[1-(4-Piperidinomethyl-phenylamino)-1-phenyl-methyliden]-1-hydroxy-6-nitro-2-indolinon.
Ausbeute: 4 % der Theorie
$R_f$-Wert: 0.4 (Kieselgel; Dichlormethan/Methanol = 9:1)
$C_{27}H_{26}N_4O_3$ (454.53)
Massenspektrum: $[M^+]$ = 454

Beispiel 449

(Z)-3-[1-(4-Piperidinomethyl-phenylamino)-1-phenyl-methyliden]-6-brom-2-indolinon

Hergestellt analog Beispiel 1.
$R_f$-Wert: 0.24 (Kieselgel; Dichlormethan/Ethanol = 9:1)
$C_{27}H_{26}BrN_3O$ (488.43)
Massenspektrum: $M^+$ = 489/487

Beispiel 450

(Z)-3-[1-(4-Piperidinomethyl-phenylamino)-1-phenyl-methylidenl-5-brom-2-indolinon

Hergestellt analog Beispiel 1.
Schmelzpunkt: 170°C
$C_{27}H_{26}BrN_3O$ (488.43)
Massenspektrum: $M^+$ = 489/487

Beispiel 451

(Z)-3-[1-(4-(N-(2-Dimethylaminoethyl)-N-methoxymethylcarbonylamino)-phenylamino)-1-phenyl-methyliden]-2-indolinon

Hergestellt analog Beispiel 327.
Schmelzpunkt: 246-249°C
$C_{28}H_{30}N_4O_3$ (470.48)
Massenspektrum: $[M+H]^+$ = 471

Beispiel 452

(Z)-3-[1-(4-(N-(2-Dimethylaminoethyl)-N-benzoyl-amino)-phenylamino)-1-phenyl-methyliden]-2-indolinon

Hergestellt analog Beispiel 327.
Schmelzpunkt: 272-274°C
$C_{32}H_{30}N_4O_2$ (505.62)
Massenspektrum: $M^+$ = 502

Beispiel 453

(Z)-3-[1-(4-(N-(2-Dimethylaminoethyl)-N-butylsulfonyl-amino)-phenylamino)-1-phenyl-methyliden]-2-indolinon

Hergestellt analog Beispiel 188.
Schmelzpunkt: 225-227°C
$C_{29}H_{34}N_4O_3S$ (518.68)
Massenspektrum: $[M+H]^+$ = 519

Beispiel 454

(Z)-3-[1-(4-(N-(2-Dimethylaminoethyl)-N-(p-tolylsulfonyl)-amino)-phenylamino)-1-phenyl-methyliden]-2-indolinon

Hergestellt analog Beispiel 188.
Schmelzpunkt: 213-215°C
$C_{32}H_{32}N_4O_3S$ (552.70)
Massenspektrum: $M^+$ = 552

Beispiel 455

(Z)-3-{1-[4-((2,6-Dimethylpiperidino)-methyl)-1-phenyl-methyliden]-2-indolinon

Hergestellt analog Beispiel 231.
Schmelzpunkt: 215-17°C
$C_{29}H_{31}N_3O$ (437.58)
Massenspektrum: $(M+H)^+$ = 438

Analog den vorstehenden Beispielen können folgende Verbindungen hergestellt werden:

(1) (Z)-3-[1-(4-Piperidinomethyl-phenylamino)-1-phenyl-methyliden]-5-methyl-2-indolinon

(2) (Z)-3-[1-(4-Piperidinomethyl-phenylamino)-1-phenyl-methyliden]-5-chlor-2-indolinon

(3) (Z)-3-[1-(4-Piperidinomethyl-phenylamino)-1-phenyl-methyliden]-6-methyl-2-indolinon

(4) (Z)-3-[1-(4-Piperidinomethyl-phenylamino)-1-phenyl-methyliden]-6-chlor-2-indolinon

(5) (Z)-3-{1-[4-(N-(2-Dimethylaminoethyl)-N-methylsulfonylamino)-phenylamino]-1-(4-methylphenyl)-methyliden}-2-indolinon

(6) (Z)-3-{1-[4-(N-(2-Dimethylaminoethyl)-N-methylsulfonylamino)-phenylamino]-1-(3-methylphenyl)-methyliden}-2-indolinon

(7) (Z)-3-{1-[4-(N-(2-Dimethylaminoethyl)-N-methylsulfonyl-amino)-phenylamino]-1-(3-methoxyphenyl)-methyliden}-2-indolinon

(8) (Z)-3-{1-[4-(N-(2-Dimethylaminoethyl)-N-methylsulfonyl-amino)-phenylamino]-1-(3-chlorphenyl)-methyliden}-2-indolinon

(9) (Z)-3-{1-[4- (N-(2-Dimethylaminoethyl)-N-methylsulfonyl-amino)-phenylamino]-1-(4-nitrophenyl)-methyliden}-2-indolinon

(10) (Z)-3-{1-[4-(N-(2-Dimethylaminoethyl)-N-methylsulfonylamino)-phenylamino]-1-(3-nitrophenyl)-methyliden}-2-indolinon

(11) (Z)-3-{1-[4-(N-(2-Dimethylaminoethyl)-N-methylsulfonylamino)-phenylamino]-1-(3-nitrophenyl)-methyliden}-2-indolinon

(12) (Z)-3-{1-[4- (N-(2-Aminoethyl)-N-methylsulfonyl-amino)-phenylamino]-1-phenyl-methyliden}-2-indolinon

(13) (Z)-3-{1-[4-(N-(2-Acetylaminoethyl)-N-methylsulfonyl-amino)-phenylamino]-1-phenyl-methyliden}-2-indolinon

(14) (Z)-3-{1-[4-(N-(2-Methylaminoethyl)-N-methylsulfonyl-amino)-phenylamino]-1-phenyl-methyliden}-2-indolinon

(15) (Z)-3-{1-[4-(N-(2-(N-Acetyl-N-methyl-amino)-ethyl)-N-methylsulfonyl-amino)-phenylamino]-1-phenyl-methyliden}-2-indolinon

(16) (Z)-3-{1-[4-(N-(2-Ethylamino-ethyl)-N-methylsulfonyl-amino)-phenylamino]-1-phenyl-methyliden}-2-indolinon

(17) (Z)-3-{1-[4-(N-(2-(N-Acetyl-N-ethyl-amino)-ethyl)-N-methylsulfonyl-amino)-phenylamino]-1-phenyl-methyliden}-2-indolinon

(18) (Z)-3-{1-[4-(N-Diethylaminoethyl-N-methylsulfonyl-amino)-phenylamino]-1-phenyl-methyliden}-2-indolinon

(19) (Z)-3-{1-[4-(N-(3-Aminopropyl)-N-methylsulfonyl-amino)-phenylamino]-1-phenyl-methyliden}-2-indolinon

(20) (Z)-3-{1-[4-(N-(3-Aminopropyl)-N-ethylsulfonyl-amino)-phenylamino]-1-phenyl-methyliden}-2-indolinon

(21) (Z)-3-{1-[4-(N-(3-Methylaminopropyl)-N-methylsulfonylamino)-phenylamino]-1-phenyl-methyliden}-2-indolinon

(22) (Z)-3-{1-[4-(N-(3-Methylaminopropyl)-N-ethylsulfonyl-amino)-phenylamino]-1-phenyl-methyliden}-2-indolinon

(23) (Z)-3-{1-[4-(N-(3-Aminopropyl)-N-acetyl-amino)-phenylamino]-1-phenyl-methyliden}-2-indolinon

(24) (Z)-3-{1-[4-(N-(3-Aminopropyl)-N-propionyl-amino)-phenylamino]-1-phenyl-methyliden}-2-indolinon

(25) (Z)-3-{1-[4-(N-(3-Methylaminopropyl)-N-acetyl-amino)-phenylamino]-1-phenyl-methyliden}-2-indolinon

(26) (Z)-3-{1-[4-(N-(3-Methylaminopropyl)-N-propionyl-amino)-phenylamino]-1-phenyl-methyliden}-2-indolinon

(27) (Z)-3-{1-[4-(N-Methylaminomethylcarbonyl-N-methyl-amino)-phenylamino]-1-phenyl-methyliden}-2-indolinon

(28) (Z)-3-{1-[4-(N-(N-Acetyl-N-methyl-aminomethylcarbonyl)-N-methyl-amino)-phenylamino]-1-phenyl-methyliden}-2-indolinon

(29) (Z)-3-{1-[4-(N-Ethylaminomethylcarbonyl-N-methyl-amino)-phenylamino]-1-phenyl-methyliden}-2-indolinon

(30) (Z)-3-{1-[4-(N-(N-Acetyl-N-ethyl-aminomethylcarbonyl)-N-methyl-amino)-phenylamino]-1-phenyl-methyliden}-2-indolinon

(31) (Z)-3-{1-[4-(N-(2-Hydroxyethyl-aminomethylcarbonyl)-N-methyl-amino)-phenylamino]-1-phenyl-methyliden}-2-indolinon

(32) (Z)-3-{1-[4-(N-(N-(2-Hydroxyethyl)-N-methyl-aminomethylcarbonyl)-N-methyl-amino)-phenylamino]-1-phenyl-methyliden}-2-indolinon

(33) (Z)-3-{1-[4-(N-(N-(2-Hydroxyethyl)-N-methyl-aminomethylcarbonyl)-N-methyl-amino)-phenylamino]-1-phenyl-methyliden}-2-indolinon

(34) (Z)-3-{1-[4-(N-(2-Dimethylamino-ethylcarbonyl)-N-methylamino)-phenylamino]-1-phenyl-methyliden}-2-indolinon

Beispiel 456

| Trockenampulle mit 75 mg Wirkstoff pro 10 ml | |
|---|---|
| Zusammensetzung: | |
| Wirkstoff | 75,0 mg |
| Mannitol | 50,0 mg |
| Wasser für Injektionszwecke | ad 10,0 ml |

Herstellung:

Wirkstoff und Mannitol werden in Wasser gelöst. Nach Abfüllung wird gefriergetrocknet. Die Auflösung zur gebrauchsfertigen Lösung erfolgt mit Wasser für Injektionszwecke.

Beispiel 457

| Trockenampulle mit 35 mg Wirkstoff pro 2 ml | |
|---|---|
| Zusammensetzung: | |
| Wirkstoff | 35,0 mg |
| Mannitol | 100,0 mg |
| Wasser für Injektionszwecke | ad 2,0 ml |

Herstellung:

Wirkstoff und Mannitol werden in Wasser gelöst. Nach Abfüllung wird gefriergetrocknet.
Die Auflösung zur gebrauchsfertigen Lösung erfolgt mit Wasser für Injektionszwecke.

Beispiel 458

| Tablette mit 50 mg Wirkstoff | |
|---|---|
| Zusammensetzung: | |
| (1) Wirkstoff | 50,0 mg |
| (2) Milchzucker | 98,0 mg |
| (3) Maisstärke | 50,0 mg |
| (4) Polyvinylpyrrolidon | 15,0 mg |
| (5) Magnesiumstearat | 2,0 mg |
| | 215,0 mg |

Herstellung:

(1), (2) und (3) werden gemischt und mit einer wäßrigen Lösung von (4) granuliert. Dem getrockneten Granulat wird (5) zugemischt. Aus dieser Mischung werden Tabletten gepreBt, biplan mit beidseitiger Facette und einseitiger Teilkerbe. Durchmesser der Tabletten: 9 mm.

Beispiel 459

| Tablette mit 350 mg Wirkstoff | |
|---|---|
| Zusammensetzung: | |
| (1) Wirkstoff | 350,0 mg |
| (2) Milchzucker | 136,0 mg |
| (3) Maisstärke | 80,0 mg |
| (4) Polyvinylpyrrolidon | 30,0 mg |
| (5) Magnesiumstearat | 4,0 mg |
| | 600,0 mg |

Herstellung:

(1), (2) und (3) werden gemischt und mit einer wäßrigen Lösung von (4) granuliert. Dem getrockneten Granulat wird (5) zugemischt. Aus dieser Mischung werden Tabletten gepreßt, biplan mit beidseitiger Facette und einseitiger Teilkerbe. Durchmesser der Tabletten: 12 mm.

Beispiel 460

| Kapseln mit 50 mg Wirkstoff | |
|---|---|
| Zusammensetzung: | |
| (1) Wirkstoff | 50,0 mg |
| (2) Maisstärke getrocknet | 58,0 mg |
| (3) Milchzucker pulverisiert | 50,0 mg |
| (4) Magnesiumstearat | 2,0 mg |
| | 160,0 mg |

Herstellung:

(1) wird mit (3) verrieben. Diese Verreibung wird der Mischung aus (2) und (4) unter intensiver Mischung

zugegeben.

Diese Pulvermischung wird auf einer Kapselabfüllmaschine in Hartgelatine-Steckkapseln Größe 3 abgefüllt.

Beispiel 461

| Kapseln mit 350 mg Wirkstoff | |
|---|---|
| Zusammensetzung: | |
| (1) Wirkstoff | 350,0 mg |
| (2) Maisstärke getrocknet | 46,0 mg |
| (3) Milchzucker pulverisiert | 30,0 mg |
| (4) Magnesiumstearat | 4,0 mg |
| | 430,0 mg |

Herstellung:

(1) wird mit (3) verrieben. Diese Verreibung wird der Mischung aus (2) und (4) unter intensiver Mischung zugegeben.

Diese Pulvermischung wird auf einer Kapselabfüllmaschine in Hartgelatine-Steckkapseln Größe 0 abgefüllt.

Beispiel 462

| Suppositorien mit 100 mg Wirkstoff | |
|---|---|
| 1 Zäpfchen enthält: | |
| Wirkstoff | 100,0 mg |
| Polyethylenglykol (M.G. 1500) | 600,0 mg |
| Polyethylenglykol (M.G. 6000) | 460,0 mg |
| Polyethylensorbitanmonostearat | 840,0 mg |
| | 2 000,0 mg |

Herstellung:

Das Polyethylenglykol wird zusammen mit Polyethylensorbitanmonostearat geschmolzen. Bei 40°C wird die gemahlene Wirk substanz in der Schmelze homogen dispergiert. Es wird auf 38°C abgekühlt und in schwach vorgekühlte Suppositorienformen ausgegossen.

## Patentansprüche

1. Substituierte Indolinone der allgemeinen Formel

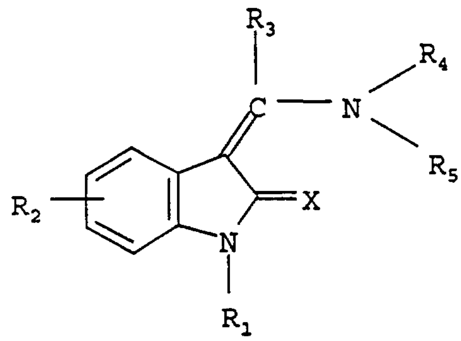

(I),

in der
X ein Sauerstoff- oder Schwefelatom,
$R_1$ ein Wasserstoffatom, $C_{1-3}$-Alkyl- oder Hydroxygruppe,
$R_2$ ein Wasserstoff-, Fluor-, Chlor-, Brom- oder Jodatom, eine $C_{1-3}$-Alkyl- oder Nitrogruppe,
$R_3$ eine Phenyl- oder Naphthylgruppe, die jeweils durch Fluor-, Chlor-, Brom- oder Jodatome, durch $C_{1-3}$-Alkyl-, $C_{1-3}$-Alkoxy-, Carboxy-, Cyano-, Trifluormethyl-, Nitro-, Amino-, $C_{1-3}$-Alkylamino-, Di-($C_{1-3}$-alkyl)-amino-, $C_{1-3}$-Alkylsulfonylamino-, Amino-$C_{1-3}$-alkyl-, 2-Carboxy-phenylcarbonylaminomethyl-, $C_{1-3}$-Alkylamino-$C_{1-3}$-alkyl-, $C_{2-4}$-Alkanoylamino-$C_{1-3}$-alkyl-, N-($C_{2-4}$-Alkanoyl)-$C_{1-3}$-alkylamino-$C_{1-3}$-alkyl-, Di-($C_{1-3}$-Alkyl)-amino-$C_{1-3}$-alkyl-, Carboxy-$C_{2-3}$-alkenyl-, N-(Carboxy-$C_{1-3}$-alkyl)-aminocarbonyl-, N-(Carboxy-$C_{1-3}$-alkyl)-N-($C_{1-3}$-alkyl)-aminocarbonyl- oder Imidazolyl-$C_{1-3}$-alkylgruppen mono- oder disubstituiert sein können, wobei die Substituenten gleich oder verschieden sein können,
$R_4$ ein Wasserstofatom oder eine $C_{1-3}$-Alkylgruppe und
$R_5$ ein gegebenenfalls durch eine $C_{1-3}$-Alkylgruppe substituierte Phenyl- oder Naphthylgruppe, die jeweils zusätzlich im aromatischen Teil

durch ein Fluor-, Chlor-, Brom- oder Jodatom, durch eine $C_{1-3}$-Alkyl-, $C_{1-3}$-Alkoxy-, Cyano-, Nitro- oder Trifluormethylgruppe,

durch eine $C_{1-3}$-Alkoxygruppe, die durch eine Carboxy-, Aminocarbonyl-, $C_{1-3}$-Alkylaminocarbonyl- oder Di-($C_{1-3}$-Alkyl)-aminocarbonylgruppe oder in 2- oder 3-Stellung auch durch eine Amino-, $C_{1-3}$-Alkylamino-, Di-($C_{1-3}$-Alkyl)-amino-, Phenyl-$C_{1-3}$-alkylamino-, N-(Phenyl-$C_{1-3}$-alkyl)-N-($C_{1-3}$-alkyl)-amino-, Pyrrolidino-, Piperidino- oder Hexamethyleniminogruppe substituiert ist,

durch eine gegebenenfalls durch eine Di-($C_{1-3}$-Alkyl)-aminogruppe substituierte $C_{2-3}$-Alkenylgruppe, die im Alkenylteil zusätzlich durch ein Chlor- oder Bromatom substituiert sein kann,

durch eine gegebenenfalls durch eine Di-($C_{1-3}$-Alkyl)-aminogruppe substituierte $C_{2-3}$-Alkinylgruppe,

durch eine $C_{1-3}$-Alkylgruppe, die durch eine 3- bis 7-gliedrige Cycloalkyleniminogruppe, durch eine Dehydropiperidino-, Morpholino-, Thiomorpholino-, 1-Oxido-thiomorpholino-, 1,1-Dioxido-thiomorpholino-, Piperazino-, N-($C_{1-3}$-Alkyl)-piperazino-, N-($C_{1-3}$-Alkanoyl)-piperazino- oder N-($C_{1-5}$-Alkoxycarbonyl)-piperazinogruppe substituiert ist, wobei die vorstehend erwähnten Substituenten durch eine $C_{1-3}$-Alkyl-, Phenyl- oder Phenyl-$C_{1-3}$-alkylgruppe substituiert und die vorstehend erwähnten Piperidino- oder Hexamethyleniminogruppen zusätzlich durch eine $C_{1-3}$-Alkylgruppe oder in 3- oder 4-Stellung durch eine Hydroxy-, $C_{1-3}$-Alkoxy-, Hydroxy-$C_{1-3}$-alkyl-, Carboxy-, Aminocarbonyl-, N-($C_{1-3}$-Alkyl)-aminocarbonyl- oder N,N-Di-($C_{1-3}$-Alkyl)-aminocarbonylgruppe substituiert sein können,

durch eine durch eine Hydroxy-, $C_{1-3}$-Alkoxy-, Carboxy- oder Cyanogruppe substituierte $C_{1-3}$-Alkylgruppe, wobei eine durch eine Carboxygruppe substituierte $C_{1-3}$-Alkylgruppe zusätzlich im Alkylteil durch eine Amino- oder $C_{1-5}$-Alkoxycarbonylaminogruppe substituiert sein kann,
durch eine gegebenenfalls durch eine oder zwei $C_{1-3}$-Alkylgruppen substituierte Aminocarbonylamino-, Amidino- oder Guanidinogruppe,
durch eine Piperidino-, Hexamethylenimino-, Morpholino-, Piperazino- oder N-($C_{1-3}$-Alkyl)-piperazinogruppe,
durch eine Formyl-, Carboxy- oder Trifluoracetylgruppe,
durch eine Carbonylgruppe, die

durch eine $C_{1-3}$-Alkyl-, $C_{1-3}$-Alkoxy-$C_{1-3}$-alkyl-, Amino-, $C_{1-5}$-Alkylamino- oder Di-($C_{1-3}$-Alkyl)-aminogruppe substituiert ist, wobei die vorstehend erwähnten Amino- und $C_{1-3}$-Alkylaminogruppen zusätzlich am Stickstoffatom durch eine Carboxy-$C_{1-3}$-alkylgruppe oder durch eine $C_{2-3}$-Alkylgruppe, die in 2- oder 3-Stellung durch eine Hydroxy-, $C_{1-3}$-Alkoxy-, Amino-, $C_{1-3}$-Alkylamino- oder Di-($C_{1-3}$-Alkyl)-aminogruppe substituiert sein können,
durch eine Pyrrolidinocarbonyl-, Piperidinocarbonyl-, Hexamethyleniminocarbonyl-, Morpholinocarbonyl-, Pipera-

zinocarbonyl-, N-($C_{1-3}$-Alkyl)-piperazinocarbonyl- oder N-(Phenyl-$C_{1-3}$-alkyl)-piperazinocarbonylgruppe, durch eine Amidosulfonyl-, Pyrrolidinosulfonyl-, Piperidinosulfonyl- oder Hexamethyleniminosulfonylgruppe, durch eine $C_{1-3}$-Alkylamidosulfonyl- oder Di-($C_{1-3}$-alkyl)-amidosulfonylgruppe, in denen ein Alkylteil jeweils durch eine Carboxy-, Aminocarbonyl-, N-($C_{1-3}$-Alkyl)-aminocarbonyl- oder N,N-Di-($C_{1-3}$-Alkyl)-aminocarbonylgruppe oder auch in 2- oder 3-Stellung durch eine $C_{1-3}$-Alkylamino- oder Di-($C_{1-3}$-Alkyl)-aminogruppe substituiert sein kann, durch eine Amino-, $C_{1-5}$-Alkylamino-, $C_{3-7}$-Cycloalkylamino-, Phenyl-$C_{1-3}$-alkylamino-, Phenylamino-, 6-gliedrige Heteroarylamino-, Amino-$C_{1-3}$-alkyl-, N-($C_{1-5}$-Alkyl)-amino-$C_{1-3}$-alkyl-, Di-($C_{1-5}$-alkyl)-amino-$C_{1-3}$-alkyl-, $C_{3-7}$-Cycloalkylamino-$C_{1-3}$-alkyl-, N-($C_{1-5}$-Alkyl)-$C_{3-7}$-cycloalkylamino-$C_{1-3}$-alkyl-, Phenylamino-$C_{1-3}$-alkyl-, N-($C_{1-3}$-Alkyl)-phenylamino-$C_{1-3}$-alkyl-, Phenyl-$C_{1-3}$-alkylamino-$C_{1-3}$-alkyloder N-($C_{1-5}$-Alkyl)-phenyl-$C_{1-3}$-alkylamino-$C_{1-3}$-alkylgruppe oder durch eine gegebenenfalls am Stickstoffatom durch $C_{1-5}$-Alkylgruppe substituierte 6-gliedrige Heteroarylamino-$C_{1-3}$-alkylgruppe, wobei der N-Alkylteil der vorstehend erwähnten Gruppen jeweils durch eine Cyano-, Carboxy-, Aminocarbonyl-, $C_{1-3}$-Alkylaminocarbonyl-, Di-($C_{1-3}$-alkyl)-aminocarbonyl-, 2-[Di-($C_{1-3}$-alkyl)-amino]-ethylaminocarbonyl-, 3-[Di-($C_{1-3}$-alkyl)-amino]-propylaminocarbonyl-, N-{2-[Di-($C_{1-3}$-alkyl)-amino]-ethyl}-N-($C_{1-3}$-alkyl)-aminocarbonyl- oder N-{3-[Di-($C_{1-3}$-alkyl)-amino]-propyl}-N-($C_{1-3}$-alkyl)-aminocarbonylgruppe oder in 2- oder 3-Stellung

durch eine Hydroxy-, $C_{1-3}$-Alkoxy-, Amino-, $C_{1-3}$-Alkylamino-, Di-($C_{1-3}$-alkyl)-amino-, Pyrrolidino-, Piperidino-, Hexamethylenimino-, Morpholino-, Piperazino- oder N-($C_{1-3}$-Alkyl)-piperazinogruppe substituiert sein können und das Stickstoffatom der vorstehend erwähnten Amino-, N-($C_{1-5}$-Alkyl)-amino-, $C_{3-7}$-Cycloalkylamino-, Phenyl-$C_{1-3}$-alkylamino-, Phenylamino-, 6-gliedrige Heteroarylamino-, Amino-$C_{1-3}$-alkyl- und N-($C_{1-5}$-Alkylamino)-$C_{1-3}$-alkylgruppen zusätzlich

durch eine $C_{1-5}$-Alkoxycarbonylgruppe,

durch eine Formyl-, Trifluoracetyl- oder Benzoylgruppe,

durch eine Carboxy-$C_{1-3}$-alkyl-, Aminocarbonyl-$C_{1-3}$-alkyl-, N-($C_{1-3}$-Alkyl)-aminocarbonyl-$C_{1-3}$-alkyl- oder N,N-Di-($C_{1-3}$-Alkyl)-aminocarbonyl-$C_{1-3}$-alkylgruppe,

durch eine $C_{1-5}$-Alkylgruppe, die mit Ausnahme der 1-Stellung durch eine Hydroxy-, $C_{1-3}$-Alkoxy-, Amino-, $C_{1-3}$-Alkylamino- oder Di-($C_{1-3}$-alkyl)-aminogruppe substituiert sein kann,

durch eine $C_{2-4}$-Alkanoylgruppe, die im Alkanoylteil durch eine Carboxy-, Hydroxy-, $C_{1-3}$-Alkoxy-, Phenyl-, Amino-, Phthalimido-, $C_{1-3}$-Alkylamino-, Di-($C_{1-3}$-alkyl)-amino-, Pyrrolidino-, Piperidino-, Hexamethylenimino- oder Morpholinogruppe oder durch eine gegebenenfalls am Stickstoffatom durch eine $C_{1-3}$-Alkyl- oder Phenyl-$C_{1-3}$-alkylgruppe substituierte Piperazinogruppe substituiert sein kann, wobei der Alkylteil der vorstehend erwähnten $C_{1-3}$-Alkylaminound Di-($C_{1-3}$-alkyl)-aminosubstituenten in 2- oder 3-Stellung durch eine Hydroxy-, $C_{1-3}$-Alkoxy-, Amino-, $C_{1-5}$-Alkoxycarbonylamino-, $C_{1-3}$-Alkylamino-, Di-($C_{1-3}$-alkyl)-amino-, Phenyl-, Pyrrolidino-, Piperidino-, Hexamethyleniminooder Morpholinogruppe substituiert sein,kann,

durch eine $C_{1-5}$-Alkylsulfonylgruppe, in der der Alkylteil mit Ausnahme der 1-Stellung durch eine Di-($C_{1-3}$-alkyl)-amino-, Pyrrolidino-, Piperidino-, Hexamethylenimino- oder Morpholinogruppe substituiert sein kann,

durch eine gegebenenfalls im Phenylteil durch ein Fluor-, Chlor- oder Bromatom oder durch eine $C_{1-3}$-Alkyl- oder $C_{1-3}$-Alkoxygruppe substituierte Phenyl-($C_{1-3}$)-alkylsulfonyl- oder Phenylsulfonylgruppe substituiert sein kann,

substituiert sein können,

bedeuten, wobei zusätzlich eine vorhandene Carboxy-, Aminooder Iminogruppe durch einen in-vivo abspaltbaren Rest substituiert sein kann, mit der Maßgabe, daß die Verbindung
3-[(α-Phenylamino)-benzyliden]-indolin-2-on
ausgeschlossen ist,
deren Isomere und deren Salze.

**2.** Substituierte Indolinone der allgemeinen Formel I gemäß Anspruch 1, in denen
X ein Sauerstoff- oder Schwefelatom,
$R_1$ ein Wasserstoffatom, eine $C_{1-3}$-Alkyl-, Hydroxy-, $C_{1-4}$-Alkoxycarbonyl- oder $C_{2-4}$-Alkanoylgruppe,
$R_2$ ein Wasserstoff-, Fluor-, Chlor-, Brom- oder Jodatom, eine $C_{1-3}$-Alkyl- oder Nitrogruppe,
$R_3$ eine Phenyl- oder Naphthylgruppe, die jeweils durch Fluor-, Chlor-, Brom- oder Jodatome, durch $C_{1-3}$-Alkyl-, Imidazolylmethyl-, 2-Carboxy-ethenyl-, 2-($C_{1-3}$-Alkoxycarbonyl)-ethenyl-, $C_{1-3}$-Alkoxy-, Cyano-, Carboxy-, $C_{1-3}$-Alkoxycarbonyl-, Trifluormethyl-, Nitro-, Amino-, Phthalimidomethyl-, 2-Carboxy-phenylcarbonylaminomethyl-, $C_{1-3}$-Alkylamino-, Di-($C_{1-3}$-alkyl)-amino-, $C_{1-3}$-Alkylsulfonylamino-, Amino-$C_{1-3}$-alkyl-, $C_{1-3}$-Alkylamino-$C_{1-3}$-alkyl-, $C_{2-4}$-Alkanoyl-amino-$C_{1-3}$-alkyl-, N-($C_{2-4}$-Alkanoyl)-$C_{1-3}$-alkylamino-$C_{1-3}$-alkyl-, Di-($C_{1-3}$-Alkyl)-amino-$C_{1-3}$-alkyl-, Carboxy-$C_{1-3}$-alkylaminocarbonyl- oder $C_{1-3}$-Alkoxycarbonyl-$C_{1-3}$-alkylaminocarbonylgruppen mono- oder disubstituiert sein können, wobei die Substituenten gleich oder verschieden sein können,
$R_4$ ein Wasserstoffatom oder eine $C_{1-3}$-Alkylgruppe und
$R_5$ ein gegebenenfalls durch eine $C_{1-3}$-Alkylgruppe substituierte Phenyl- oder Naphthylgruppe, die jeweils zusätzlich im aromatischen Teil

durch ein Fluor-, Chlor-, Brom- oder Jodatom, durch eine $C_{1-3}$-Alkyl-, $C_{1-3}$-Alkoxy-, Cyano-, Nitro- oder Trifluormethylgruppe, wobei die vorstehend erwähnte Alkylgruppe gleichzeitig durch eine Carboxy- oder $C_{1-3}$-Alkoxycarbonylgruppe und eine Amino- oder $C_{1-4}$-Alkoxycarbonylaminogruppe substituiert sein kann,

eine $C_{1-3}$-Alkylgruppe, die durch eine 4- bis 7-gliedrige Cycloalkyleniminogruppe, durch eine Dehydropiperidino-, Morpholino-, Thiomorpholino-, 1-Oxido-thiomorpholino-, 1,1-Dioxido-thiomorpholino-, Piperazino- oder N-($C_{1-4}$-Alkoxycarbonyl)-piperazinogruppe substituiert ist, wobei die vorstehend erwähnten Piperidino-, Hexamethylenimino-, Morpholino-, Thiomorpholino-, 1-Oxido-thiomorpholino-, 1,1-Dioxido-thiomorpholino- und Piperazinogruppen durch eine $C_{1-3}$-Alkyl-, Phenyl- oder Phenyl-$C_{1-3}$-alkylgruppe und die vorstehend erwähnten Piperidinogruppen zusätzlich durch eine $C_{1-3}$-Alkylgruppe oder in 3- oder 4-Stellung durch eine Hydroxy-, $C_{1-3}$-Alkoxy-, Hydroxy-$C_{1-3}$-alkyl-, Carboxy-, Aminocarbonyl-, N-($C_{1-3}$-Alkyl)-aminocarbonyl- oder N,N-Di-($C_{1-3}$-Alkyl)-aminocarbonylgruppe substituiert sein können,

durch eine gegebenenfalls durch eine Hydroxy-, $C_{1-3}$-Alkoxy-, Carboxy-, $C_{1-3}$-Alkoxycarbonyl- oder Cyanogruppe substituierte $C_{1-3}$-Alkylgruppe,

durch eine gegebenenfalls durch eine oder zwei $C_{1-3}$-Alkylgruppen substituierte Aminocarbonylamino-, Amidino- oder Guanidinogruppe,

durch eine Piperidino-, Hexamethylenimino-, Morpholino-, Piperazino- oder N-($C_{1-3}$-Alkyl)-piperazinogruppe,

durch eine Formyl-, Carboxy-, $C_{1-3}$-Alkoxycarbonyl- oder Trifluoracetylgruppe,

durch eine Carbonylgruppe, die

durch eine $C_{1-3}$-Alkyl-, $C_{1-3}$-Alkoxy-$C_{1-3}$-alkyl-, Amino-, $C_{1-5}$-Alkylamino- oder Di-($C_{1-3}$-Alkyl)-aminogruppe substituiert ist, wobei die vorstehend erwähnten Amino- und $C_{1-3}$-Alkylaminogruppen zusätzlich am Stickstoffatom durch eine Carboxy-$C_{1-3}$-alkyl- oder $C_{1-3}$-Alkoxycarbonyl-$C_{1-3}$-alkylgruppe oder durch eine $C_{2-3}$-Alkylgruppe, die in 2- oder 3-Stellung durch eine Hydroxy-, $C_{1-3}$-Alkoxy-, Amino-, $C_{1-3}$-Alkylamino- oder Di-($C_{1-3}$-Alkyl)-aminogruppe substituiert sein können,

durch eine Pyrrolidinocarbonyl-, Pyrrolidinosulfonyl-, Piperidinocarbonyl-, Hexamethyleniminocarbonyl-, Morpholinocarbonyl-, Piperazinocarbonyl-, N-($C_{1-3}$-Alkyl)-piperazinocarbonyloder N-(Phenyl-$C_{1-3}$-alkyl)-piperazinocarbonylgruppe,

durch eine Amidosulfonyl-, $C_{1-3}$-Alkylamidosulfonyl- oder Di-($C_{1-3}$-alkyl)-amidosulfonylgruppe, in denen ein Alkylteil durch eine Carboxy-, $C_{1-3}$-Alkoxycarbonyl-, Aminocarbonyl-, $C_{1-3}$-Alkylaminocarbonyl- oder Di-($C_{1-3}$-alkyl)-aminocarbonylgruppe oder in 2- oder 3-Stellung durch eine Amino-, $C_{1-3}$-Alkylamino- oder Di-($C_{1-3}$-alkyl)-aminogruppe substituiert sein kann,

durch eine Amino-, $C_{1-5}$-Alkylamino-, Amino-$C_{1-3}$-alkyl-, N-($C_{1-3}$-Alkyl)-amino-$C_{1-3}$-alkyl-, N-(2-Hydroxyethyl)-amino-$C_{1-3}$-alkyl-, N-(3-Hydroxypropyl)-amino-$C_{1-3}$-alkyl-, Di-($C_{1-5}$-alkyl)-amino-$C_{1-3}$-alkyl-, N-($C_{3-7}$-Cycloalkyl)-amino-$C_{1-3}$-alkyl-, N-($C_{3-7}$-Cycloalkyl)-N-($C_{1-3}$-alkyl)-amino-$C_{1-3}$-alkyl- oder N-(Phenyl-$C_{1-3}$-alkyl)-amino-$C_{1-3}$-alkylgruppe, wobei der N-Alkylteil der vorstehend erwähnten Gruppen durch eine Cyano-, Carboxy-, $C_{1-3}$-Alkylcarbonyl-, Aminocarbonyl-, $C_{1-3}$-Alkylaminocarbonyl-, Di-($C_{1-3}$-alkyl)-aminocarbonyl-, 2-[Di-($C_{1-3}$-alkyl)-amino]-ethylaminocarbonyl-, 3-[Di-($C_{1-3}$-alkyl)-amino]-propylaminocarbonyl-, N-{2-[Di-($C_{1-3}$-alkyl)-amino]-ethyl}-N-($C_{1-3}$-alkyl)-aminocarbonyl- oder N-{3-[Di-($C_{1-3}$-alkyl)-amino]-propyl}-N-($C_{1-3}$-alkyl)-aminocarbonylgruppe oder in 2- oder 3-Stellung durch eine Hydroxy-, $C_{1-3}$-Alkoxy-, Amino-, $C_{1-3}$-Alkylamino-, Di-($C_{1-3}$-alkyl)-amino- oder Morpholinogruppe substituiert sein können, wobei das Stickstoffatom der vorstehend erwähnten Amino-, $C_{1-3}$-Alkylamino-, Amino-$C_{1-3}$-alkyl- oder N-($C_{1-5}$-Alkylamino)-$C_{1-3}$-alkylteile zusätzlich

durch eine $C_{1-5}$-Alkoxycarbonylgruppe,

durch eine Formyl-, Trifluoracetyl- oder Benzoylgruppe,

durch eine $C_{1-5}$-Alkylgruppe, die mit Ausnahme der 1-Stellung durch eine Hydroxy-, $C_{1-3}$-Alkoxy-, Amino-, $C_{1-3}$-Alkylamino- oder Di-($C_{1-3}$)-alkylaminogruppe substituiert sein kann,

durch eine $C_{2-4}$-Alkanoylgruppe, die im Alkanoylteil durch eine Hydroxy-, $C_{1-3}$-Alkoxy-, Amino-, $C_{2-4}$-Alkanoylamino-, $C_{1-5}$-Alkoxycarbonylamino-, Phthalimido-, $C_{1-3}$-Alkylamino-, Di-($C_{1-3}$-alkyl)-amino-, N-($C_{1-3}$-alkyl)-phenylamino-, Pyrrolidino-, Piperidino- oder Morpholinogruppe oder durch eine gegebenenfalls am Stickstoffatom durch eine $C_{1-3}$-Alkyl- oder Phenyl-$C_{1-3}$-alkylgruppe substituierte Piperazinogruppe substituiert sein kann, wobei der N-Alkylteil der vorstehend erwähnten Gruppen in 2- oder 3-Stellung durch eine Methoxy-, Di-($C_{1-3}$-alkyl)-amino- oder Morpholinogruppe substituiert sein kann,

durch eine $C_{1-5}$-Alkylsulfonylgruppe, in der der Alkylteil mit Ausnahme der 1-Stellung durch eine Di-($C_{1-3}$-alkyl)-amino-, Pyrrolidino-, Piperidino-, Hexamethylenimino- oder Morpholinogruppe substituiert sein kann,

durch eine Pyridinyl- oder Pyrimidinylgruppe,

durch eine gegebenenfalls im Phenylteil durch eine $C_{1-3}$-Alkylgruppe substituierte Phenyl-, Phenyl-($C_{1-3}$)-alkylsulfonyl- oder Phenylsulfonylgruppe substituiert sein kann,

durch eine $C_{1-3}$-Alkoxygruppe, die durch eine Carboxy-, $C_{1-3}$-Alkoxycarbonyl-, Aminocarbonyl-, $C_{1-3}$-Alkylaminocarbonyl- oder Di-($C_{1-3}$-Alkyl)-aminocarbonylgruppe oder in 2- oder 3-Stellung durch eine Amino-, $C_{1-3}$-Alkylami-

no-, Di-($C_{1-3}$-Alkyl)-amino-, N-($C_{1-3}$-Alkyl)-N-(phenyl-$C_{1-3}$-alkyl)-amino-, Piperidino- oder Hexamethyleniminogruppe substituiert ist,

durch eine Prop-1-enyl-, 2-Chlor-prop-1-enyl- oder Prop-1-inyl-Gruppe, die in 3-Stellung durch eine Di-($C_{1-3}$-Alkyl)-aminogruppe substituiert ist,

substituiert sein können,

bedeuten, deren Isomere und deren Salze.

**3.** Substituierte Indolinone der allgemeinen Formel I gemäß Anspruch 1, in denen
X ein Sauerstoffatom,
$R_1$ ein Wasserstoffatom, eine $C_{1-3}$-Alkyl-, $C_{1-4}$-Alkoxycarbonyloder $C_{2-4}$-Alkanoylgruppe,
$R_2$ ein Wasserstoff-, Fluor-, Chlor-, Brom- oder Jodatom, eine $C_{1-3}$-Alkyl- oder Nitrogruppe,
$R_3$ eine Phenylgruppe, die durch Fluor-, Chlor-, Brom- oder Jodatome, durch $C_{1-3}$-Alkyl-, Trifluormethyl-, Imidazolylmethyl-, 2-Carboxy-ethenyl-, 2-$C_{1-3}$-Alkoxycarbonyl-ethenyl-, $C_{1-3}$-Alkoxy-, Cyano-, Carboxy-, $C_{1-3}$-Alkoxycarbonyl-, Nitro-, Amino-, Phthalimidomethyl-, 2-Carboxy-benzoylaminomethyl-, $C_{1-3}$-Alkylamino-, Di-($C_{1-3}$-alkyl)-amino-, $C_{1-3}$-Alkylsulfonylamino-, Amino-$C_{1-3}$-alkyl-, $C_{1-3}$-Alkylamino-$C_{1-3}$-alkyl-, $C_{2-4}$-Alkanoylamino-$C_{1-3}$-alkyl-, N-($C_{2-4}$-Alkanoyl)-$C_{1-3}$-alkylamino-$C_{1-3}$-alkyl-, Di-($C_{1-3}$-Alkyl)-amino-$C_{1-3}$-alkyl-, Carboxy-$C_{1-3}$-alkylaminocarbonyloder $C_{1-3}$-Alkoxycarbonyl-$C_{1-3}$-alkylaminocarbonylgruppen monooder disubstituiert sein können, wobei die Substituenten gleich oder verschieden sein können,
$R_4$ ein Wasserstoffatom oder eine $C_{1-3}$-Alkylgruppe und
$R_5$ ein gegebenenfalls durch eine $C_{1-3}$-Alkylgruppe substituierte Phenyl- oder Naphthylgruppe, die jeweils zusätzlich im aromatischen Teil

durch ein Fluor-, Chlor-, Brom- oder Jodatom, durch eine $C_{1-3}$-Alkoxy-, Cyano-, Nitro- oder Trifluormethylgruppe,

eine $C_{1-3}$-Alkylgruppe, die durch eine 4- bis 7-gliedrige Cycloalkyleniminogruppe, durch eine Dehydropiperidino-, Morpholino-, Thiomorpholino-, 1-Oxido-thiomorpholino-, 1,1-Dioxido-thiomorpholino-, Piperazino- oder N-($C_{1-4}$-Alkoxycarbonyl)-piperazinogruppe substituiert ist, wobei die vorstehend erwähnten Piperidino-, Hexamethylenimino-, Morpholinound Piperazinogruppen durch eine $C_{1-3}$-Alkyl-, Phenyl- oder Phenyl-$C_{1-3}$-alkylgruppe und die vorstehend erwähnten Piperidinogruppen zusätzlich durch eine $C_{1-3}$-Alkylgruppe oder in 3-oder 4-Stellung durch eine Hydroxy-, $C_{1-3}$-Alkoxy-, Hydroxy-$C_{1-3}$-alkyl-, Carboxy-, Aminocarbonyl-, N-($C_{1-3}$-Alkyl)-aminocarbonyl- oder N,N-Di-($C_{1-3}$-Alkyl)-aminocarbonylgruppe substituiert sein können,

durch eine gegebenenfalls durch eine Hydroxy-, $C_{1-3}$-Alkoxy-, Carboxy-, $C_{1-3}$-Alkoxycarbonyl- oder Cyanogruppe substituierte $C_{1-3}$-Alkylgruppe,

durch eine gegebenenfalls durch eine oder zwei $C_{1-3}$-Alkylgruppen substituierte Aminocarbonylamino-, Amidino- oder Guanidinogruppe,

durch eine Piperidino-, Hexamethylenimino-, Morpholino-, Piperazino- oder N-($C_{1-3}$-Alkyl)-piperazinogruppe,
durch eine Formyl-, Carboxy-, $C_{1-3}$-Alkoxycarbonyl- oder Trifluoracetylgruppe,
durch eine Carbonylgruppe, die

durch eine $C_{1-3}$-Alkyl-, $C_{1-3}$-Alkoxy-$C_{1-3}$-alkyl-, Amino-, $C_{1-5}$-Alkylamino- oder Di-($C_{1-3}$-Alkyl)-aminogruppe substituiert ist, wobei die vorstehend erwähnten Amino- und $C_{1-3}$-Alkylaminogruppen zusätzlich am Stickstoffatom durch eine Carboxy-$C_{1-3}$-alkyl-, $C_{1-3}$-Alkoxycarbonyl-$C_{1-3}$-alkyloder $C_{1-3}$-Alkoxycarbonyl-$C_{1-3}$-alkylgruppe oder durch eine $C_{2-3}$-Alkylgruppe, die in 2- oder 3-Stellung durch eine Hydroxy-, $C_{1-3}$-Alkoxy-, Amino-, $C_{1-3}$-Alkylamino- oder Di-($C_{1-3}$-Alkyl)-aminogruppe substituiert sein können,

durch eine Pyrrolidinocarbonyl-, Pyrrolidinosulfonyl-, Piperidinocarbonyl- oder Hexamethyleniminocarbonylgruppe,

durch eine Amidosulfonyl-, $C_{1-3}$-Alkylamidosulfonyl- oder Di-($C_{1-3}$-alkyl)-amidosulfonylgruppe, in denen ein Alkylteil durch eine Carboxy-, $C_{1-3}$-Alkoxycarbonyl- oder Dimethylaminocarbonylgruppe oder in 2- oder 3-Stellung durch eine Dimethylaminogruppe substituiert sein kann,

durch eine geradkettige $C_{1-2}$-Alkylgruppe, die endständig durch eine Amino-, Benzylamino-, Pyridylamino- oder Pyrimidylaminogruppe, durch eine $C_{1-4}$-Alkylaminogruppe, in der der Alkylteil in Position 2, 3 oder 4 durch eine Hydroxy- oder Methoxygruppe substituiert sein kann, oder durch eine im $C_{1-2}$-Alkylteil durch eine Carboxy-, $C_{1-3}$-Alkoxycarbonyl- oder Di-($C_{1-3}$-Alkyl)-aminocarbonylgruppe substituierte $C_{1-2}$-Alkylaminogruppe substituiert ist, wobei in den vorstehend erwähnten Gruppen ein am Aminstickstoffatom vorhandenes Wasserstoffatom zusätzlich

durch eine $C_{3-6}$-Cycloalkylgruppe, durch eine $C_{1-4}$-Alkylgruppe, in der der Alkylteil in Position 2, 3 oder 4 durch eine Hydroxygruppe substituiert sein kann, durch eine gegebenenfalls durch eine Methoxy-, Carboxy-, $C_{1-3}$-Alkoxycarbonyl-, Amino-, Methylamino-, Dimethylamino-, Acetylamino-, $C_{1-5}$-Alkoxycarbonylamino-, N-Methyl-$C_{1-5}$-alkoxycarbonylamino- oder Morpholinocarbonylaminogruppe substituierte $C_{1-2}$-Alkylcarbonylgruppe, durch eine $C_{1-5}$-Alkoxycarbonyl-, $C_{1-4}$-Alkylsulfonyl-, Phenylsulfonyl- oder Tolylsulfonylgruppe ersetzt sein kann,
durch eine 3-Dimethylaminopropyl- oder 3-Dimethylamino-prop-1-enylgruppe,

durch eine Ethylgruppe, die in 1-Stellung durch eine Aminooder $C_{1-5}$-Alkoxycarbonylaminogruppe substituiert ist, durch eine Ethylgruppe, die in 2-Stellung durch eine Aminooder $C_{1-5}$-Alkoxycarbonylaminogruppe und durch eine Carboxyoder $C_{1-3}$-Alkoxycarbonylgruppe substituiert ist,

durch eine Amino- oder $C_{1-3}$-Alkylaminogruppe, in der der Alkylteil durch eine Cyano-, Carboxy-, $C_{1-3}$-Alkoxycarbonyl-, Aminocarbonyl-, Methylaminocarbonyl- oder Dimethylaminocarbonylgruppe oder in 2- oder 3-Stellung durch eine Amino-, Methylamino-, Dimethylamino-, Acetylamino-, N-Methyl-acetylamino- oder Morpholinogruppe, durch eine gegebenenfalls in 2-oder 3-Position des $C_{1-3}$-Alkylteils durch eine Dimethylaminogruppe substituierte N-($C_{1-3}$-Alkyl)-aminocarbonyl- oder N-($C_{1-3}$-Alkyl)-methylaminocarbonylgruppe substituiert sein kann, wobei ein am Aminstickstoffatom vorhandenes Wasserstoffatom in den vorstehend erwähnten Gruppen zusätzlich

durch eine Formyl-, Trifluoracetyl-, Benzoyl-, $C_{1-4}$-Alkoxycarbonyl- oder $C_{1-4}$-Alkylaminocarbonylgruppe,

durch eine $C_{2-4}$-Alkanoylgruppe, die endständig durch eine Amino-, Acetylamino-, $C_{1-4}$-Alkoxycarbonylamino-, Pyrrolidino-, Piperidino-, Morpholino-, Piperazino-, 4-Methylpiperazino-, 4-Benzylpiperazino- oder Phthalimidogruppe oder durch eine $C_{1-3}$-Alkylamino-, N-Acetyl-$C_{1-3}$-alkyl-amino- oder Di-($C_{1-3}$-alkyl)-aminogruppe substituiert sein kann, wobei in den vorstehend erwähnten $C_{1-3}$-Alkylamino-, N-Acetyl-$C_{1-3}$-alkyl-amino- und Di-($C_{1-3}$-alkyl)-aminogruppen jeweils ein $C_{1-3}$-Alkylteil zusätzlich durch eine Phenylgruppe oder in 2- oder 3-Position durch eine Methoxy-, Dimethylamino- oder Morpholinogruppe substituiert sein kann,

durch eine $C_{1-4}$-Alkylsulfonylgruppe, in der der Alkylteil in 2- oder 3-Position zusätzlich durch eine Dimethylamino-, Piperidino- oder Morpholinogruppe substituiert sein kann,

durch eine Phenylsulfonyl- oder Toluolsulfonylgruppe ersetzt sein kann,

durch eine $C_{1-3}$-Alkoxygruppe, die durch eine Carboxy-, $C_{1-3}$-Alkoxycarbonyl-, Aminocarbonyl-, Methylaminocarbonyloder Dimethylaminocarbonylgruppe oder in 2- oder 3-Stellung durch eine Amino-, Methylamino-, Dimethylamino-, N-Methylbenzylamino-, Piperidino- oder Hexamethyleniminogruppe substituiert ist,

durch eine $C_{1-3}$-Alkylaminocarbonyl- oder Di-($C_{1-3}$-Alkyl)-aminocarbonylgruppe, in der ein $C_{1-3}$-Alkylteil in 2- oder 3-Stellung durch eine Methoxy- oder Dimethylaminogruppe substituiert sein kann,

substituiert sein können,

bedeuten, deren Isomere und deren Salze.

**4.** Substituierte Indolinone der allgemeinen Formel I gemäß Anspruch 1, in denen
X ein Sauerstoffatom
$R_1$ ein Wasserstoffatom,
$R_2$ ein Wasserstoff-, Chlor- oder Bromatom, eine Methyl- oder Nitrogruppe,
$R_3$ eine Phenylgruppe, die durch ein Fluor-, Chlor- oder Bromatom, durch ein Methyl-, Methoxy-, Aminomethyl-, Acetylaminomethyl-, Carboxy-, Methoxycarbonyl- oder Imidazolylmethylgruppe substituiert sein kann,
$R_4$ ein Wasserstoffatom,
$R_5$ eine Phenylgruppe, die

durch ein Fluor-, Chlor- oder Bromatom, durch eine Methyl-, Methoxy-, Nitro-, Cyano- oder Trifluormethylgruppe,

durch eine Methyl oder Ethylgruppe, die jeweils durch eine Carboxy-, $C_{1-3}$-Alkoxycarbonyl-, Cyano-, Azetidin-1-yl-, Pyrrolidino-, Piperidino-, 4-Phenylpiperidino-, 3,6-Dihydro-2H-pyridin-1-yl-, Hexamethylenimino, Morpholino-, Thiomorpholino-, 1-Oxido-thiomorpholino-, Piperazino-, 4-Methylpiperazino- oder 4-Acetylpiperazinogruppe substituiert ist, wobei die vorstehend erwähnten Piperidinogruppen zusätzlich durch eine oder zwei Methylgruppen oder in 3- oder 4-Position durch eine Hydroxy-, Methoxy-, Carboxy-, Hydroxymethyl-, $C_{1-3}$-Alkoxycarbonyl-, Aminocarbonyl-, Methylaminocarbonyl- oder Dimethylaminocarbonylgruppe substituiert sein können,

durch eine geradkettige $C_{1-2}$-Alkygruppe, die endständig durch eine Amino- oder Benzylaminogruppe, durch eine $C_{1-4}$-Alkylaminogruppe, in der der Alkylteil in den Positionen 2, 3 oder 4 durch eine Hydroxy- oder Methoxygruppe substituiert sein kann, durch eine im $C_{1-2}$-Alkylteil durch eine Carboxy-, $C_{1-3}$-Alkoxycarbonyl- oder Dimethylaminocarbonylgruppe substituierte $C_{1-2}$-Alkylaminogruppe substituiert ist, wobei in den vorstehend erwähnten Gruppen ein am Aminstickstoff vorhandenes Wasserstoffatom zusätzlich

durch eine $C_{3-6}$-Cycloalkylgruppe, durch eine $C_{1-4}$-Alkylgruppe, in der der Alkylteil in den Positionen 2, 3 oder 4 durch eine Hydroxygruppe substituiert sein kann, oder durch eine gegebenenfalls durch eine Amino-, Methylaminooder Dimethylaminogruppe substituierte $C_{1-2}$-Alkylcarbonylgruppe ersetzt sein kann,

durch eine 3-Dimethylamino-prop-1-enylgruppe,

durch eine Ethylgruppe, die in 1-Position durch eine Aminooder $C_{1-4}$-Alkoxycarbonylaminogruppe substituiert ist,

durch eine Amino- oder $C_{1-3}$-Alkylaminogruppe, in der der Alkylteil endständig durch eine Carboxy-, Aminocarbonyl-, Methylaminocarbonyl- oder Dimethylaminocarbonylgruppe oder in 2- oder 3-Stellung durch eine Amino-, Methylamino-, Dimethylamino-, Acetylamino-, N-Acetyl-methylamino- oder Morpholinogruppe oder durch eine gegebenenfalls in 2- oder 3-Position

durch eine Dimethylaminogruppe substituierte N-($C_{1-3}$-Alkyl)-aminocarbonyl- oder N-($C_{1-3}$-Alkyl)-methylaminocar-

bonylgruppe substituiert sein kann, wobei ein am Aminstickstoff vorhandenes Wasserstoffatom in den vorstehend erwähnten Gruppen zusätzlich

durch eine Formyl- oder Benzoylgruppe,

durch eine $C_{2-4}$-Alkanoylgruppe, die endständig durch eine Amino-, Acetylamino-, Pyrrolidino-, Piperidino-, Morpholino-, Piperazino- oder 4-Methylpiperazinogruppe oder durch eine $C_{1-3}$-Alkylamino-, N-Acetyl-$C_{1-3}$-alkylaminooder Di-($C_{1-3}$-alkyl)-aminogruppe substituiert sein kann, wobei in den vorstehend erwähnten $C_{1-3}$-Alkylamino-, N-Acetyl-$C_{1-3}$-alkylamino- oder Di-($C_{1-3}$-alkyl)-aminogruppen ein $C_{1-3}$-Alkylteil zusätzlich in 2- oder 3-Position durch eine Methoxy-, Dimethylamino- oder Morpholinogruppe substituiert sein kann,

durch eine $C_{1-4}$-Alkylsulfonylgruppe, die in 2- oder 3-Stellung durch eine Dimethylaminogruppe substituiert sein kann, ersetzt sein kann,

durch eine Pyrrolindinosulfonylgruppe, eine Aminosulfonyl-, $C_{1-3}$-Alkylaminosulfonyl- oder Di-($C_{1-3}$-alkyl)-aminosulfonylgruppe, in denen jeweils ein $C_{1-3}$-Alkylteil durch eine Carboxy-, $C_{1-3}$-Alkoxycarbonyl-, Aminocarbonyl-, Methylaminocarbonyl- oder Dimethylaminocarbonylgruppe oder mit Ausnahme der 1-Stellung durch eine Dimethylaminogruppe substituiert sein kann,

durch eine $C_{2-3}$-Alkoxygruppe, die in 2- oder 3-Stellung durch eine Dimethylamino- oder Piperidinogruppe substituiert ist,

durch eine Aminocarbonyl-, $C_{1-3}$-Alkylaminocarbonyl- oder Di-($C_{1-3}$-alkyl)-aminocarbonylgruppe, in denen jeweils die $C_{1-3}$-Alkylteile mit Ausnahme der 1-Position durch eine Methoxy- oder Dimethylaminogruppe substituiert sein können,

substituiert sein kann, bedeuten,

deren Isomere und deren Salze.

**5.** Substituierte Indolinone der allgemeinen Formel I gemäß Anspruch 1, in denen

X und $R_2$ bis $R_4$ wie vorstehend erwähnt definiert sind,

$R_1$ ein Wasserstoffatom und

$R_5$ eine Phenylgruppe bedeutet, die

durch eine Methyl oder Ethylgruppe, die jeweils durch eine Azetidin-1-yl-, Pyrrolidino-, Piperidino-, Hexamethylenimino-, Morpholino-, 1-Oxido-thiomorpholino-, Piperazino-, 4-Methylpiperazino- oder 4-Acetylpiperazinogruppe substituiert ist, wobei die vorstehend erwähnten Piperidinogruppen zusätzlich durch eine oder zwei Methylgruppen oder in 4-Position durch eine Hydroxy-, Methoxy-, Hydroxymethyl-, Aminocarbonyl-, Methylaminocarbonyl- oder Dimethylaminocarbonylgruppe substituiert sein können,

durch eine geradkettige $C_{1-2}$-Alkygruppe, die endständig durch eine Aminogruppe oder durch eine $C_{1-3}$-Alkylaminogruppe substituiert ist, wobei der Alkylteil der $C_{1-3}$-Alkylaminogruppe in den Positionen 2 oder 3 durch eine Hydroxy- oder Methoxygruppe substituiert sein kann und in den vorstehend erwähnten Gruppen das am Aminstickstoff vorhandene Wasserstoffatom zusätzlich

durch eine $C_{3-6}$-Cycloalkylgruppe, durch eine $C_{1-3}$-Alkylgruppe, in der der Alkylteil in den Positionen 2 oder 3 durch eine Hydroxygruppe substituiert sein kann, oder durch eine durch eine Amino-, Methylamino- oder Dimethylaminogruppe substituierte $C_{1-2}$-Alkylcarbonylgruppe ersetzt sein kann,

durch eine Ethylgruppe, die in 1-Position durch eine Aminogruppe substituiert ist,

durch eine Amino- oder $C_{1-3}$-Alkylaminogruppe, in der der Alkylteil endständig durch eine Carboxy-, Aminocarbonyl-, Methylaminocarbonyl-, Dimethylaminocarbonyl-, N-(2-Dimethylamino-ethyl)-aminocarbonyl- oder N-(2-Dimethylamino-ethyl)-N-methyl-aminocarbonylgruppe oder in 2- oder 3-Stellung durch eine Amino-, Methylamino-, Dimethylamino-, Acetylamino-, N-Acetyl-methylamino- oder Morpholinogruppe substituiert sein kann, wobei das vorhandene Wasserstoffatom am Aminstickstoff der vorstehend erwähnten Gruppen zusätzlich

durch eine $C_{2-4}$-Alkanoylgruppe, die endständig durch eine Amino-, Acetylamino-, Pyrrolidino-, Piperidino-, Morpholino-, Piperazino- oder 4-Methylpiperazinogruppe oder durch eine $C_{1-3}$-Alkylamino-, N-Acetyl-$C_{1-3}$-alkylaminooder Di-($C_{1-3}$--alkyl)-aminogruppe substituiert sein kann, wobei in den vorstehend erwähnten $C_{1-3}$-Alkylamino-, N-Acetyl-$C_{1-3}$-alkylamino- oder Di-($C_{1-3}$--alkyl)-aminogruppen ein $C_{1-3}$-Alkylteil zusätzlich in 2- oder 3-Position durch eine Methoxy-, Dimethylamino- oder Morpholinogruppe substituiert sein kann,

durch eine $C_{1-4}$-Alkylsulfonylgruppe, die in 2- oder 3-Stellung durch eine Dimethylaminogruppe substituiert sein kann, ersetzt sein kann,

durch eine Pyrrolindinosulfonylgruppe, eine Aminosulfonyl-, $C_{1-3}$-Alkylaminosulfonyl- oder Di-($C_{1-3}$-alkyl)-aminosulfonylgruppe, in denen jeweils ein $C_{1-3}$-Alkylteil durch eine Carboxy-, Methoxycarbonyl-, Aminocarbonyl-, Methylaminocarbonyl- oder Dimethylaminocarbonylgruppe oder mit Ausnahme der 1-Stellung durch eine Dimethylaminogruppe substituiert sein kann,

durch eine $C_{1-3}$-Alkoxygruppe, die in 2- oder 3-Stellung durch eine Dimethylamino- oder Piperidinogruppe substituiert ist,

durch eine Aminocarbonyl-, $C_{1-3}$-Alkylaminocarbonyl- oder Di-($C_{1-3}$-alkyl)-aminocarbonylgruppe, in denen

jeweils ein $C_{1-3}$-Alkylteil mit Ausnahme der 1-Position durch eine Methoxy- oder Dimethylaminogruppe substituiert sein können,
substituiert sein kann, bedeuten,
deren Isomere und deren Salze.

**6.** Folgende Verbindungen der allgemeinen Formel I:

(a) (Z)-3-[1-(4-Dimethylaminomethyl-phenylamino)-1-phenyl-methyliden]-5-nitro-2-indolinon,

(b) (Z)-3-[1-(4-Piperidinomethyl-phenylamino)-1-phenyl-methyliden]-5-nitro-2-indolinon,

(c) (Z)-3-{1-[4-(2-Morpholinoethyl)-phenylamino]-1-phenyl-methyliden}-5-nitro-2-indolinon,

(d) (Z)-3-{1-[4-(2-Dimethylamino-ethyl)-phenylamino]-1-phenylmethyliden}-5-nitro-2-indolinon und

(e) (Z)-3-{1-[4-(N-(2-Dimethylamino-ethyl)-N-methylsulfonylamino)-phenylamino]-1-phenyl-methyliden}-2-indolinon

sowie deren Salze.

**7.** Physiologisch verträgliche Salze der Verbindungen gemäß den Ansprüchen 1 bis 6.

**8.** Arzneimittel, enthaltend eine Verbindung der allgemeinen Formel I nach mindestens einem der Ansprüche 1 bis 6, in der $R_1$ ein Wasserstoffatom, eine $C_{1-3}$-Alkylgruppe oder einen Prodrugrest darstellt oder ein physiologisch verträgliches Salz hiervon neben gegebenenfalls einem oder mehreren inerten Trägerstoffen und/oder Verdünnungsmitteln.

**9.** Verwendung einer Verbindung der allgemeinen Formel I nach mindestens einem der Ansprüche 1 bis 6, in der $R_1$ ein Wasserstoffatom, eine $C_{1-3}$-Alkylgruppe oder einen Prodrugrest darstellt oder ein physiologisch verträgliches Salz hiervon zur Herstellung eines Arzneimittels, welches zur Behandlung von exzessiven oder anomalen Zellproliferationen geeignet ist.

**10.** Verfahren zur Herstellung eines Arzneimittels gemäß Anspruch 8, **dadurch gekennzeichnet, daß** auf nichtchemischem Wege eine Verbindung der allgemeinen Formel I nach mindestens einem der Ansprüche 1 bis 6, in der $R_1$ ein Wasserstoffatom, eine $C_{1-3}$-Alkylgruppe oder einen Prodrugrest darstellt oder ein physiologisch verträgliches Salz hiervon in einen oder mehrere inerte Trägerstoffe und/oder Verdünnungsmittel eingearbeitet wird.

**11.** Verfahren zur Herstellung der Verbindungen gemäß den Ansprüchen 1 bis 7, **dadurch gekennzeichnet, daß**

a. eine Verbindung der allgemeinen Formel

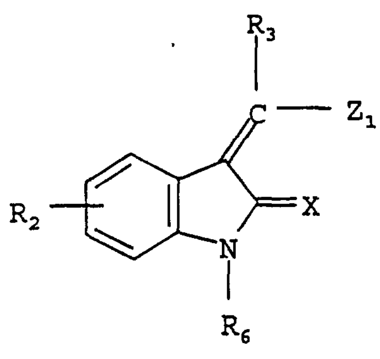

(II),

in der
X, $R_2$ und $R_3$ wie in den Ansprüchen 1 bis 6 erwähnt definiert sind,
$R_6$ ein Wasserstoffatom, eine Schutzgruppe für das Stickstoffatom der Lactamgruppe oder eine Bindung an

eine Festphase und
$Z_1$ ein Halogenatom, eine Hydroxy-, Alkoxy- oder Aralkoxygruppe bedeuten,
mit einem Amin der allgemeinen Formel

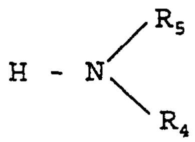

(III),

in der
$R_4$ und $R_5$ wie in den Ansprüchen 1 bis 6 erwähnt definiert sind, umgesetzt und erforderlichenfalls anschließend ein verwendete Schutzgruppe für das Stickstoffatom der Lactamgruppe oder eine so erhaltene Verbindung von einer Festphase abgespalten wird oder

b. zur Herstellung einer Verbindung der allgemeinen Formel I, die eine Aminomethylgruppe enthält und X ein Sauerstoffatom darstellt, eine Verbindung der allgemeinen Formel

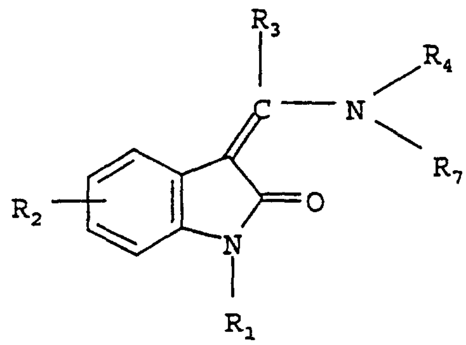

(IV),

in der
$R_1$ bis $R_4$ wie in den Ansprüchen 1 bis 6 erwähnt definiert sind und
$R_7$ mit der Maßgabe die für $R_5$ in den Ansprüchen 1 bis 5 erwähnten Bedeutungen aufweist, daß $R_5$ eine Cyanogruppe enthält, reduziert wird oder

c. zur Herstellung einer Verbindung der allgemeinen Formel I, in der $R_1$ ein Wasserstoffatom und X ein Sauerstoffatom darstellen, eine Verbindung der allgemeinen Formel

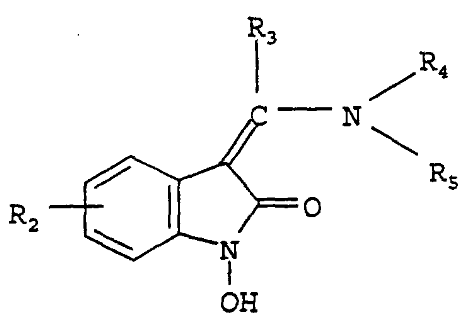

(V),

in der
$R_2$ bis $R_5$ wie in den Ansprüchen 1 bis 6 erwähnt definiert sind, reduziert wird und
gewünschtenfalls anschließend eine so erhaltene Verbindung der allgemeinen Formel I, die eine Alkoxycarbonylgruppe enthält, mittels Hydrolyse in eine entsprechende Carboxyverbindung übergeführt wird, oder

eine so erhaltene Verbindung der allgemeinen Formel I, die eine Amino- oder Alkylaminogruppe enthält, mittels Alkylierung oder reduktiver Alkylierung in eine entsprechende Alkylaminooder Dialkylaminoverbindung übergeführt wird, oder
eine so erhaltene Verbindung der allgemeinen Formel I, die eine Amino- oder Alkylaminogruppe enthält, mittels Acylierung in eine entsprechende Acylverbindung übergeführt wird, oder
eine so erhaltene Verbindung der allgemeinen Formel I, die eine Carboxygruppe enthält, mittels Veresterung oder Amidierung in eine entsprechende Ester- oder Aminocarbonylverbindung übergeführt wird, oder
eine so erhaltene Verbindung der allgemeinen Formel I, in der $R_3$ einen Phenylrest darstellt, der ein Chlor-, Brom- oder Jodatom enthält, mittels Umsetzung mit einer Alkenylverbindung in eine entsprechende alkenylierte Verbindung übergeführt wird, oder
eine so erhaltene Verbindung der allgemeinen Formel I, in der $R_3$ einen Phenylrest darstellt, der ein Chlor-, Brom- oder Jodatom enthält, mittels Umsetzung mit einer Alkinylverbindung in eine entsprechende alkenylierte Verbindung übergeführt wird und
erforderlichenfalls ein während den Umsetzungen zum Schutze von reaktiven Gruppen verwendeter Schutzrest abgespalten wird, oder
gewünschtenfalls anschließend eine so erhaltene Verbindung der allgemeinen Formel I in ihre Stereoisomere aufgetrennt wird, oder
eine so erhaltene Verbindung der allgemeinen Formel I in ihre Salze, insbesondere für die pharmazeutische Anwendung in ihre physiologisch verträglichen Salze mit einer anorganischen oder organischen Säure oder Base, übergeführt wird.

## Claims

**1.** Substituted indolinones of general formula

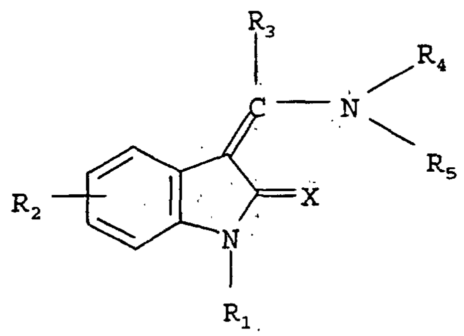

(I),

X denotes an oxygen or sulphur atom,
$R_1$ denotes a hydrogen atom, $C_{1-3}$-alkyl or hydroxy group,
$R_2$ denotes a hydrogen, fluorine, chlorine, bromine or iodine atom, a $C_{1-3}$-alkyl or nitro group,
$R_3$ denotes a phenyl or naphthyl group, each of which may be mono- or disubstituted by fluorine, chlorine, bromine or iodine atoms, by $C_{1-3}$-alkyl, $C_{1-3}$-alkoxy, carboxy, cyano, trifluoromethyl, nitro, amino, $C_{1-3}$-alkylamino, di-($C_{1-3}$-alkyl)-amino, $C_{1-3}$-alkylsulphonylamino, amino-$C_{1-3}$-alkyl, 2-carboxy-phenylcarbonylaminomethyl, $C_{1-3}$-alkylamino-$C_{1-3}$-alkyl, $C_{2-4}$-alkanoylamino-$C_{1-3}$-alkyl, N-($C_{2-4}$-alkanoyl)-$C_{1-3}$-alkylamino-$C_{1-3}$-alkyl, di-($C_{1-3}$-alkyl)-amino-$C_{1-3}$-alkyl, carboxy-$C_{2-3}$-alkenyl, N-(carboxy-$C_{1-3}$-alkyl)-aminocarbonyl, N-(carboxy-$C_{1-3}$-alkyl)-N-($C_{1-3}$-alkyl)-aminocarbonyl or imidazolyl-$C_{1-3}$-alkyl groups, while the substituents may be identical or different,
$R_4$ denotes a hydrogen atom or a $C_{1-3}$-alkyl group and
$R_5$ denotes a phenyl or naphthyl group optionally substituted by a $C_{1-3}$-alkyl group, each of which may additionally be substituted in the aromatic moiety

by a fluorine, chlorine, bromine or iodine atom, by a $C_{1-3}$-alkyl, $C_{1-3}$-alkoxy, cyano, nitro or trifluoromethyl group,

by a $C_{1-3}$-alkoxy group which is substituted by a carboxy, aminocarbonyl, $C_{1-3}$-alkylaminocarbonyl or di-($C_{1-3}$-alkyl)-aminocarbonyl group or in the 2 or 3 position by an amino, $C_{1-3}$-alkylamino, di-($C_{1-3}$-alkyl)-amino,phenyl-$C_{1-3}$-alkylamino, N-(phenyl-$C_{1-3}$-alkyl)-N-($C_{1-3}$-alkyl)-amino, pyrrolidino, piperidino or hexamethyleneimino group,

by a $C_{2-3}$-alkenyl group optionally substituted by a di-($C_{1-3}$-alkyl)-amino group, which may additionally be substituted in the alkenyl moiety by a chlorine or bromine atom,

by a $C_{2-3}$-alkynyl group optionally substituted by a di-($C_{1-3}$-alkyl)-amino group,

by a $C_{1-3}$-alkyl group which is substituted by a 3- to 7-membered cycloalkyleneimino group, by a dehydropiperidino, morpholino, thiomorpholino, 1-oxido-thiomorpholino, 1,1-dioxido-thiomorpholino, piperazino, N-($C_{1-3}$-alkyl)-piperazino, N-($C_{1-3}$-alkanoyl)-piperazino or N-($C_{1-5}$-alkoxycarbonyl)-piperazino group, whilst the abovementioned substituents may be substituted by a $C_{1-3}$-alkyl, phenyl or phenyl-$C_{1-3}$-alkyl group and the abovementioned piperidino or hexamethyleneimino groups may additionally be substituted by a $C_{1-3}$-alkyl group or in the 3 or 4 position by a hydroxy, $C_{1-3}$-alkoxy, hydroxy-$C_{1-3}$-alkyl, carboxy, aminocarbonyl, N-($C_{1-3}$-alkyl)-aminocarbonyl or N,N-di-($C_{1-3}$-alkyl)-aminocarbonyl group,

by a $C_{1-3}$-alkyl group substituted by a hydroxy, $C_{1-3}$-alkoxy, carboxy or cyano group, whilst a $C_{1-3}$-alkyl group substituted by a carboxy group may additionally be substituted in the alkyl moiety by an amino or $C_{1-5}$-alkoxycarbonylamino group,

by an aminocarbonylamino, amidino or guanidino group optionally substituted by one or two $C_{1-3}$-alkyl groups,

by a piperidino, hexamethyleneimino, morpholino, piperazino or N-($C_{1-3}$-alkyl)-piperazino group,

by a formyl, carboxy or trifluoroacetyl group,

by a carbonyl group which

is substituted by a $C_{1-3}$-alkyl, $C_{1-3}$-alkoxy-$C_{1-3}$-alkyl, amino, $C_{1-5}$-alkylamino or di-($C_{1-3}$-alkyl)-amino group, while the abovementioned amino and $C_{1-3}$-alkylamino groups may additionally be substituted at the nitrogen atom by a carboxy-$C_{1-3}$-alkyl group or by a $C_{2-3}$-alkyl group which is substituted in the 2 or 3 position by a hydroxy, $C_{1-3}$-alkoxy, amino, $C_{1-3}$-alkylamino or di-($C_{1-3}$-alkyl)-amino group,

by a pyrrolidinocarbonyl, piperidinocarbonyl, hexamethyleneiminocarbonyl, morpholinocarbonyl, piperazinocarbonyl, N-($C_{1-3}$-alkyl)-piperazinocarbonyl or N-(phenyl-$C_{1-3}$-alkyl)-piperazinocarbonyl group,

by an amidosulphonyl, pyrrolidinosulphonyl, piperidinosulphonyl or hexamethyleneiminosulphonyl group, by a $C_{1-3}$-alkylamidosulphonyl or di-($C_{1-3}$-alkyl)-amidosulphonyl group, wherein an alkyl moiety may be substituted in each case by a carboxy, aminocarbonyl, N-($C_{1-3}$-alkyl)-aminocarbonyl or N,N-di-($C_{1-3}$-alkyl)-aminocarbonyl group or, in the 2 or 3 position, by a $C_{1-3}$-alkylamino or di-($C_{1-3}$-alkyl)-amino group,

by an amino, $C_{1-5}$-alkylamino, $C_{3-7}$-cycloalkylamino, phenyl-$C_{1-3}$-alkylamino, phenylamino, 6-membered heteroarylamino, amino-$C_{1-3}$-alkyl, N-($C_{1-5}$-alkyl)-amino-$C_{1-3}$-alkyl, di-($C_{1-5}$-alkyl)-amino-$C_{1-3}$-alkyl, $C_{3-7}$-cycloalkylamino-$C_{1-3}$-alkyl, N-($C_{1-5}$-alkyl)-$C_{3-7}$-cycloalkylamino-$C_{1-3}$-alkyl, phenylamino-$C_{1-3}$-alkyl, N-($C_{1-3}$-alkyl)-phenylamino-$C_{1-3}$-alkyl, phenyl-$C_{1-3}$-alkylamino-$C_{1-3}$-alkyl or N-($C_{1-5}$-alkyl)-phenyl-$C_{1-3}$-alkylamino-$C_{1-3}$-alkyl group or by a 6-membered heteroarylamino-$C_{1-3}$-alkyl group optionally substituted at the nitrogen atom by a $C_{1-5}$-alkyl group, while the N-alkyl moiety of the abovementioned groups may be substituted in each case by a cyano, carboxy, aminocarbonyl, $C_{1-3}$-alkylaminocarbonyl, di-($C_{1-3}$-alkyl)-aminocarbonyl, 2-[di-($C_{1-3}$-alkyl)-amino]-ethylaminocarbonyl, 3-[di-($C_{1-3}$-alkyl)-amino]-propylaminocarbonyl, N-{2-[di-($C_{1-3}$-alkyl)-amino]-ethyl}-N-($C_{1-3}$-alkyl)-aminocarbonyl or N-{3-[di-($C_{1-3}$-alkyl)-amino]-propyl}-N-($C_{1-3}$-alkyl)-aminocarbonyl group or in the 2 or 3 position by a hydroxy, $C_{1-3}$-alkoxy, amino, $C_{1-3}$-alkylamino, di-($C_{1-3}$-alkyl)-amino, pyrrolidino, piperidino, hexamethyleneimino, morpholino, piperazino or N-($C_{1-3}$-alkyl)-piperazino group and the nitrogen atom of the abovementioned amino, N-($C_{1-5}$-alkyl)-amino, $C_{3-7}$-cycloalkylamino, phenyl-$C_{1-3}$-alkylamino, phenylamino, 6-membered heteroarylamino, amino-$C_{1-3}$-alkyl- and N-($C_{1-5}$-alkylamino)-$C_{1-3}$-alkyl groups may additionally be substituted

by a $C_{1-5}$-alkoxycarbonyl group,

by a formyl, trifluoroacetyl or benzoyl group,

by a carboxy-$C_{1-3}$-alkyl, aminocarbonyl-$C_{1-3}$-alkyl, N-($C_{1-3}$-alkyl)-aminocarbonyl-$C_{1-3}$-alkyl or N,N-di-($C_{1-3}$-alkyl)-aminocarbonyl-$C_{1-3}$-alkyl group,

by a $C_{1-5}$-alkyl group which may be substituted, except in the 1 position, by a hydroxy, $C_{1-3}$-alkoxy, amino, $C_{1-3}$-alkylamino or di-($C_{1-3}$-alkyl)-amino group,

by a $C_{2-4}$-alkanoyl group which may be substituted in the alkanoyl moiety by a carboxy, hydroxy, $C_{1-3}$-alkoxy, phenyl, amino, phthalimido, $C_{1-3}$-alkylamino, di-($C_{1-3}$-alkyl)-amino, pyrrolidino, piperidino, hexamethyleneimino or morpholino group or by a piperazino group optionally substituted at the nitrogen atom by a $C_{1-3}$-alkyl or phenyl-$C_{1-3}$-alkyl group, while the alkyl moiety of the abovementioned $C_{1-3}$-alkylamino- and di-($C_{1-3}$-alkyl)-amino substituents may be substituted in the 2 or 3 position by a hydroxy, $C_{1-3}$-alkoxy, amino, $C_{1-5}$-alkoxycarbonylamino, $C_{1-3}$-alkylamino, di-($C_{1-3}$-alkyl)-amino, phenyl, pyrrolidino, piperidino, hexamethyleneimino or morpholino group,

by a $C_{1-5}$-alkylsulphonyl group in which the alkyl moiety may be substituted except in the 1 position by a di-($C_{1-3}$-alkyl)-amino, pyrrolidino, piperidino, hexamethyleneimino or morpholino group,

by a phenyl-($C_{1-3}$)-alkylsulphonyl or phenylsulphonyl group optionally substituted in the phenyl moiety by a fluorine, chlorine or bromine atom or by a $C_{1-3}$-alkyl or $C_{1-3}$-alkoxy group,

while additionally any carboxy, amino or imino group present may be substituted by a group which can be cleaved *in vivo,* with the proviso that the compound

3-[($\alpha$-phenylamino)-benzylidene]-indolin-2-one

is excluded,

the isomers and the salts thereof.

**2.** Substituted indolinones of general formula I according to claim 1, wherein
X denotes an oxygen or sulphur atom,
$R_1$ denotes a hydrogen atom, a $C_{1-3}$-alkyl, hydroxy, $C_{1-4}$-alkoxycarbonyl or $C_{2-4}$-alkanoyl group,
$R_2$ denotes a hydrogen, fluorine, chlorine, bromine or iodine atom, a $C_{1-3}$-alkyl or nitro group,
$R_3$ denotes a phenyl or naphthyl group, each of which may be mono- or disubstituted by fluorine, chlorine, bromine or iodine atoms, by $C_{1-3}$-alkyl, imidazolylmethyl, 2-carboxy-ethenyl, 2-($C_{1-3}$-alkoxycarbonyl)-ethenyl, $C_{1-3}$-alkoxy, cyano, carboxy, $C_{1-3}$-alkoxycarbonyl, trifluoromethyl, nitro, amino, phthalimidomethyl, 2-carboxy-phenylcarbonylaminomethyl, $C_{1-3}$-alkylamino, di-($C_{1-3}$-alkyl)-amino, $C_{1-3}$-alkylsulphonylamino, amino-$C_{1-3}$-alkyl, $C_{1-3}$-alkylamino-$C_{1-3}$-alkyl, $C_{2-4}$-alkanoyl-amino-$C_{1-3}$-alkyl, N-($C_{2-4}$-alkanoyl)-$C_{1-3}$-alkylamino-$C_{1-3}$-alkyl, di-($C_{1-3}$-alkyl)-amino-$C_{1-3}$-alkyl, carboxy-$C_{1-3}$-alkylaminocarbonyl or $C_{1-3}$-alkoxycarbonyl-$C_{1-3}$-alkylaminocarbonyl groups, while the substituents may be identical or different,
$R_4$ denotes a hydrogen atom or a $C_{1-3}$-alkyl group and
$R_5$ denotes a phenyl or naphthyl group optionally substituted by a $C_{1-3}$-alkyl group, each of which may additionally be substituted in the aromatic moiety

by a fluorine, chlorine, bromine or iodine atom, by a $C_{1-3}$-alkyl, $C_{1-3}$-alkoxy, cyano, nitro or trifluoromethyl group, while the abovementioned alkyl group may simultaneously be substituted by a carboxy or $C_{1-3}$-alkoxycarbonyl group and an amino or $C_{1-4}$-alkoxycarbonylamino group,

a $C_{1-3}$-alkyl group which is substituted by a 4- to 7-membered cycloalkyleneimino group, by a dehydropiperidino, morpholino, thiomorpholino, 1-oxido-thiomorpholino, 1,1-dioxido-thiomorpholino, piperazino or N-($C_{1-4}$-alkoxycarbonyl)-piperazino group, while the abovementioned piperidino, hexamethyleneimino, morpholino, thiomorpholino, 1-oxido-thiomorpholino, 1,1-dioxido-thiomorpholino- and piperazino groups may be substituted by a $C_{1-3}$-alkyl, phenyl or phenyl-$C_{1-3}$-alkyl group and the abovementioned piperidino groups may additionally be substituted by a $C_{1-3}$-alkyl group or in the 3 or 4 position by a hydroxy, $C_{1-3}$-alkoxy, hydroxy-$C_{1-3}$-alkyl, carboxy, aminocarbonyl, N-($C_{1-3}$-alkyl)-aminocarbonyl or N,N-di-($C_{1-3}$-alkyl)-aminocarbonyl group,
by a $C_{1-3}$-alkyl group optionally substituted by a hydroxy, $C_{1-3}$-alkoxy, carboxy, $C_{1-3}$-alkoxycarbonyl or cyano group,
by an aminocarbonylamino, amidino or guanidino group optionally substituted by one or two $C_{1-3}$-alkyl groups,
by a piperidino, hexamethyleneimino, morpholino, piperazino or N-($C_{1-3}$-alkyl)-piperazino group,
by a formyl, carboxy, $C_{1-3}$-alkoxycarbonyl or trifluoroacetyl group,
by a carbonyl group which

is substituted by a $C_{1-3}$-alkyl, $C_{1-3}$-alkoxy-$C_{1-3}$-alkyl, amino, $C_{1-5}$-alkylamino or di-($C_{1-3}$-alkyl)-amino group, while the abovementioned amino- and $C_{1-3}$-alkylamino groups may additionally be substituted at the nitrogen atom by a carboxy-$C_{1-3}$-alkyl or $C_{1-3}$-alkoxycarbonyl-$C_{1-3}$-alkyl group or by a $C_{2-3}$-alkyl group which may be substituted in the 2 or 3 position by a hydroxy, $C_{1-3}$-alkoxy, amino, $C_{1-3}$-alkylamino or di-($C_{1-3}$-alkyl)-amino group,
by a pyrrolidinocarbonyl, pyrrolidinosulphonyl, piperidinocarbonyl, hexamethyleneiminocarbonyl, morpholinocarbonyl, piperazinocarbonyl, N-($C_{1-3}$-alkyl)-piperazinocarbonyl or N-(phenyl-$C_{1-3}$-alkyl)-piperazinocarbonyl group,
by an amidosulphonyl, $C_{1-3}$-alkylamidosulphonyl or di-($C_{1-3}$-alkyl)-amidosulphonyl group, wherein an alkyl moiety may be substituted by a carboxy, $C_{1-3}$-alkoxycarbonyl, aminocarbonyl, $C_{1-3}$-alkylaminocarbonyl or di-($C_{1-3}$-alkyl)-aminocarbonyl group or in the 2 or 3 position may be , substituted by an amino, $C_{1-3}$-alkylamino or di-($C_{1-3}$-alkyl)-amino group,
by an amino, $C_{1-5}$-alkylamino, amino-$C_{1-3}$-alkyl, N-($C_{1-3}$-alkyl)-amino-$C_{1-3}$-alkyl, N-(2-hydroxyethyl)-amino-$C_{1-3}$-alkyl, N-(3-hydroxypropyl)-amino-$C_{1-3}$-alkyl, di-($C_{1-5}$-alkyl)-amino-$C_{1-3}$-alkyl, N-($C_{3-7}$-cycloalkyl)-amino-$C_{1-3}$-alkyl, N-($C_{3-7}$-cycloalkyl)-N-($C_{1-3}$-alkyl)-amino-$C_{1-3}$-alkyl or N-(phenyl-$C_{1-3}$-alkyl)-amino-$C_{1-3}$-alkyl group, while the N-alkyl moiety of the abovementioned groups may be substituted by a cyano, carboxy, $C_{1-3}$-alkylcarbonyl, aminocarbonyl, $C_{1-3}$-alkylaminocarbonyl, di-($C_{1-3}$-alkyl)-aminocarbonyl, 2-[di-($C_{1-3}$-alkyl)-amino]-ethylaminocarbonyl, 3-[di-($C_{1-3}$-alkyl)-amino]-propylaminocarbonyl, N-{2-[di-($C_{1-3}$-alkyl)-amino]-ethyl}-N-($C_{1-3}$-alkyl)-aminocarbonyl or N-{3-[di-($C_{1-3}$-alkyl)-amino]-propyl}-N-($C_{1-3}$-alkyl)-aminocarbonyl group or may be substituted in the 2 or 3 position by a hydroxy, $C_{1-3}$-alkoxy, amino, $C_{1-3}$-alkylamino, di-($C_{1-3}$-alkyl)-amino or morpholino group, while the nitrogen atom of the abovementioned amino, $C_{1-3}$-alkylamino, amino-$C_{1-3}$-alkyl or N-($C_{1-5}$-alkylamino)-$C_{1-3}$-alkyl moieties may additionally be substituted

by a $C_{1-5}$-alkoxycarbonyl group,

by a formyl, trifluoroacetyl or benzoyl group,

by a $C_{1-5}$-alkyl group which may be substituted, except in the 1 position, by a hydroxy, $C_{1-3}$-alkoxy, amino, $C_{1-3}$-alkylamino or di-($C_{1-3}$)-alkylamino group,

by a $C_{2-4}$-alkanoyl group which may be substituted in the alkanoyl moiety by a hydroxy, $C_{1-3}$-alkoxy, amino, $C_{2-4}$-alkanoylamino, $C_{1-5}$-alkoxycarbonylamino, phthalimido, $C_{1-3}$-alkylamino, di-($C_{1-3}$-alkyl)-amino, N-($C_{1-3}$-alkyl)-phenylamino, pyrrolidino, piperidino or morpholino group or by a piperazino group optionally substituted at

the nitrogen atom by a $C_{1-3}$-alkyl or phenyl-$C_{1-3}$-alkyl group, while the N-alkyl moiety of the abovementioned groups may be substituted in the 2 or 3 position by a methoxy, di-($C_{1-3}$-alkyl)-amino or morpholino group,

by a $C_{1-5}$-alkylsulphonyl group in which the alkyl moiety may be substituted, except in the 1 position, by a di-($C_{1-3}$-alkyl)-amino, pyrrolidino, piperidino, hexamethyleneimino or morpholino group,

by a pyridinyl or pyrimidinyl group,

by a phenyl, phenyl-($C_{1-3}$)-alkylsulphonyl or phenylsulphonyl group optionally substituted in the phenyl moiety by a $C_{1-3}$-alkyl group,

by a $C_{1-3}$-alkoxy group which is substituted by a carboxy, $C_{1-3}$-alkoxycarbonyl, aminocarbonyl, $C_{1-3}$-alkylaminocarbonyl or di-($C_{1-3}$-alkyl)-aminocarbonyl group or is substituted in the 2 or 3 position by an amino, $C_{1-3}$-alkylamino, di-($C_{1-3}$-alkyl)-amino, N-($C_{1-3}$-alkyl)-N-(phenyl-$C_{1-3}$-alkyl)-amino, piperidino or hexamethyleneimino group,

by a prop-1-enyl, 2-chloro-prop-1-enyl or prop-1-ynyl group which is substituted in the 3 position by a di-($C_{1-3}$-alkyl)-amino group,

the isomers and the salts thereof.

**3.** Substituted indolinones of general formula I according to claim 1, wherein

X denotes an oxygen atom,

$R_1$ denotes a hydrogen atom, a $C_{1-3}$-alkyl, $C_{1-4}$-alkoxycarbonyl or $C_{2-4}$-alkanoyl group,

$R_2$ denotes a hydrogen, fluorine, chlorine, bromine or iodine atom, a $C_{1-3}$-alkyl or nitro group,

$R_3$ denotes a phenyl group which may be mono- or disubstituted by fluorine, chlorine, bromine or iodine atoms, by $C_{1-3}$-alkyl, trifluoromethyl, imidazolylmethyl, 2-carboxy-ethenyl, 2-$C_{1-3}$-alkoxycarbonyl-ethenyl, $C_{1-3}$-alkoxy, cyano, carboxy, $C_{1-3}$-alkoxycarbonyl, nitro, amino, phthalimidomethyl, 2-carboxybenzoylaminomethyl, $C_{1-3}$-alkylamino, di-($C_{1-3}$-alkyl)-amino, $C_{1-3}$-alkylsulphonylamino, amino-$C_{1-3}$-alkyl, $C_{1-3}$-alkylamino-$C_{1-3}$-alkyl, $C_{2-4}$-alkanoylamino-$C_{1-3}$-alkyl, N-($C_{2-4}$-alkanoyl)-$C_{1-3}$-alkylamino-$C_{1-3}$-alkyl, di-($C_{1-3}$-alkyl)-amino-$C_{1-3}$-alkyl, carboxy-$C_{1-3}$-alkylaminocarbonyl or $C_{1-3}$-alkoxycarbonyl-$C_{1-3}$-alkylaminocarbonyl groups, while the substituents may be identical or different,

$R_4$ denotes a hydrogen atom or a $C_{1-3}$-alkyl group and

$R_5$ denotes a phenyl or naphthyl group optionally substituted by a $C_{1-3}$-alkyl group, each of which may additionally be substituted in the aromatic moiety

by a fluorine, chlorine, bromine or iodine atom, by a $C_{1-3}$-alkoxy, cyano, nitro or trifluoromethyl group,

a $C_{1-3}$-alkyl group which is substituted by a 4- to 7-membered cycloalkyleneimino group, by a dehydropiperidino, morpholino, thiomorpholino, 1-oxido-thiomorpholino, 1,1-dioxido-thiomorpholino, piperazino or N-($C_{1-4}$-alkoxycarbonyl)-piperazino group, while the abovementioned piperidino, hexamethyleneimino, morpholino and piperazino groups may be substituted by a $C_{1-3}$-alkyl, phenyl or phenyl-$C_{1-3}$-alkyl group and the abovementioned piperidino groups may additionally be substituted by a $C_{1-3}$-alkyl group or may be substituted in the 3 or 4 position by a hydroxy, $C_{1-3}$-alkoxy, hydroxy-$C_{1-3}$-alkyl, carboxy, aminocarbonyl; N-($C_{1-3}$-alkyl)-aminocarbonyl or N,N-di-($C_{1-3}$-alkyl)-aminocarbonyl group,

by a $C_{1-3}$-alkyl group optionally substituted by a hydroxy, $C_{1-3}$-alkoxy, carboxy, $C_{1-3}$-alkoxycarbonyl or cyano group,

by an aminocarbonylamino, amidino or guanidino group optionally substituted by one or two $C_{1-3}$-alkyl groups,

by a piperidino, hexamethyleneimino, morpholino, piperazino or N-($C_{1-3}$-alkyl)-piperazino group,

by a formyl, carboxy, $C_{1-3}$-alkoxycarbonyl or trifluoroacetyl group,

by a carbonyl group which

is substituted by a $C_{1-3}$-alkyl, $C_{1-3}$-alkoxy-$C_{1-3}$-alkyl, amino, $C_{1-5}$-alkylamino or di-($C_{1-3}$-alkyl)-amino group, while the abovementioned amino and $C_{1-3}$-alkylamino groups may additionally be substituted at the nitrogen atom by a carboxy-$C_{1-3}$-alkyl, $C_{1-3}$-alkoxycarbonyl-$C_{1-3}$-alkyl or $C_{1-3}$-alkoxycarbonyl-$C_{1-3}$-alkyl group or by a $C_{2-3}$-alkyl group which may be substituted in the 2 or 3 position by a hydroxy, $C_{1-3}$-alkoxy, amino, $C_{1-3}$-alkylamino or di-($C_{1-3}$-alkyl)-amino group,

by a pyrrolidinocarbonyl, pyrrolidinosulphonyl, piperidinocarbonyl or hexamethyleneiminocarbonyl group,

by an amidosulphonyl, $C_{1-3}$-alkylamidosulphonyl or di-($C_{1-3}$-alkyl)-amidosulphonyl group, wherein an alkyl moiety may be substituted by a carboxy, $C_{1-3}$-alkoxycarbonyl or dimethylaminocarbonyl group or in the 2 or 3 position by a dimethylamino group,

by a straight-chain $C_{1-2}$-alkyl group which is terminally substituted by an amino, benzylamino, pyridylamino or pyrimidylamino group, by a $C_{1-4}$-alkylamino group in which the alkyl moiety may be substituted in position 2, 3 or 4 by a hydroxy or methoxy group, or by a $C_{1-2}$-alkylamino group substituted in the $C_{1-2}$-alkyl moiety by a carboxy, $C_{1-3}$-alkoxycarbonyl or di-($C_{1-3}$-alkyl)-aminocarbonyl group, while in the abovementioned groups any hydrogen atom present at the amino nitrogen atom may additionally be replaced

by a $C_{3-6}$-cycloalkyl group, by a $C_{1-4}$-alkyl group in which the alkyl moiety may be substituted in position 2,

3 or 4 by a hydroxy group, by a $C_{1-2}$-alkylcarbonyl group optionally substituted by a methoxy, carboxy, $C_{1-3}$-alkoxycarbonyl, amino, methylamino, dimethylamino, acetylamino, $C_{1-5}$-alkoxycarbonylamino, N-methyl-$C_{1-5}$-alkoxycarbonylamino or morpholinocarbonylamino group, by a $C_{1-5}$- alkoxycarbonyl, $C_{1-4}$-alkylsulphonyl, phenylsulphonyl or tolylsulphonyl group,
by a 3-dimethylaminopropyl or 3-dimethylamino-prop-1-enyl group,
by an ethyl group which is substituted in the 1 position by an amino or $C_{1-5}$-alkoxycarbonylamino group,
by an ethyl group which is.substituted in the 2 position by an amino or $C_{1-5}$-alkoxycarbonylamino group and by a carboxy or $C_{1-3}$-alkoxycarbonyl group,
by an amino or $C_{1-3}$-alkylamino group in which the alkyl moiety may be substituted by a cyano, carboxy, $C_{1-3}$-alkoxycarbonyl, aminocarbonyl, methylaminocarbonyl or dimethylaminocarbonyl group or may be substituted in the 2 or 3 position by an amino, methylamino, dimethylamino, acetylamino, N-methyl-acetylamino or morpholino group, by an N-($C_{1-3}$-alkyl)-aminocarbonyl or N-($C_{1-3}$-alkyl)-methylaminocarbonyl group optionally substituted in the 2 or 3 position of the $C_{1-3}$-alkyl moiety by a dimethylamino group, while any hydrogen atom present at the amino nitrogen atom in the abovementioned groups may additionally be replaced

by a formyl, trifluoroacetyl, benzoyl, $C_{1-4}$-alkoxycarbonyl or $C_{1-4}$-alkylaminocarbonyl group,

by a $C_{2-4}$-alkanoyl group which may be terminally substituted by an amino, acetylamino, $C_{1-4}$-alkoxycarbonylamino, pyrrolidino, piperidino, morpholino, piperazino, 4-methylpiperazino, 4-benzylpiperazino or phthalimido group or by a $C_{1-3}$-alkylamino, N-acetyl-$C_{1-3}$-alkyl-amino or di-($C_{1-3}$-alkyl)-amino group, while in the abovementioned $C_{1-3}$-alkylamino, N-acetyl-$C_{1-3}$-alkyl-amino- and di-($C_{1-3}$-alkyl)-amino groups any $C_{1-3}$-alkyl moiety may additionally be substituted by a phenyl group or in the 2 or 3 position by a methoxy, dimethylamino or morpholino group,

by a $C_{1-4}$-alkylsulphonyl group in which the alkyl moiety may additionally be substituted in the 2 or 3 position by a dimethylamino, piperidino or morpholino group,

by a phenylsulphonyl or toluenesulphonyl group,

by a $C_{1-3}$-alkoxy group which is substituted by a carboxy, $C_{1-3}$-alkoxycarbonyl, aminocarbonyl, methylaminocarbonyl or dimethylaminocarbonyl group or is substituted in the 2 or 3 position by an amino, methylamino, dimethylamino, N-methylbenzylamino, piperidino or hexamethyleneimino group,
by a $C_{1-3}$-alkylaminocarbonyl or di-($C_{1-3}$-alkyl)-aminocarbonyl group wherein a $C_{1-3}$-alkyl moiety may be substituted in the 2 or 3 position by a methoxy or dimethylamino group,
the isomers and the salts thereof.

**4.** Substituted indolinones of general formula I according to claim 1, wherein
X denotes an oxygen atom.
$R_1$ denotes a hydrogen atom,
$R_2$ denotes a hydrogen, chlorine or bromine atom, a methyl or nitro group,
$R_3$ denotes a phenyl group which may be substituted by a fluorine, chlorine or bromine atom, by a methyl, methoxy, aminomethyl, acetylaminomethyl, carboxy, methoxycarbonyl or imidazolylmethyl group,
$R_4$ denotes a hydrogen atom,
$R_5$ denotes a phenyl group which may be substituted

by a fluorine, chlorine or bromine atom, by a methyl, methoxy, nitro, cyano or trifluoromethyl group,

by a methyl or ethyl group, each of which is substituted by a carboxy, $C_{1-3}$-alkoxycarbonyl, cyano, azetidin-1-yl, pyrrolidino, piperidino, 4-phenylpiperidino, 3,6-dihydro-2H-pyridin-1-yl, hexamethyleneimino, morpholino, thiomorpholino, 1-oxido-thiomorpholino, piperazino, 4-methylpiperazino or 4-acetylpiperazino group, while the abovementioned piperidino groups may additionally be substituted by one or two methyl groups or may be substituted in the 3 or 4 position by a hydroxy, methoxy, carboxy, hydroxymethyl, $C_{1-3}$-alkoxycarbonyl, aminocarbonyl, methylaminocarbonyl or dimethylaminocarbonyl group,

by a straight-chain $C_{1-2}$-alkyl group which may be terminally substituted by an amino or benzylamino group, by a $C_{1-4}$-alkylamino group in which the alkyl moiety in positions 2, 3 or 4 is substituted by a hydroxy or methoxy group, by a $C_{1-2}$-alkylamino group substituted in the $C_{1-2}$-alkyl moiety by a carboxy, $C_{1-3}$-alkoxycarbonyl or dimethylaminocarbonyl group, while in the abovementioned groups a hydrogen atom present at the amino nitrogen may additionally be replaced

by a $C_{3-6}$-cycloalkyl group, by a $C_{1-4}$-alkyl group in which the alkyl moiety may be substituted in positions 2, 3 or 4 by a hydroxy group, or by a $C_{1-2}$-alkylcarbonyl group optionally substituted by an amino, methylamino or dimethylamino group,
by a 3-dimethylamino-prop-1-enyl group,
by an ethyl group which is substituted in the 1-position by an amino or $C_{1-4}$-alkoxycarbonylamino group,
by an amino or $C_{1-3}$-alkylamino group in which the alkyl moiety may be terminally substituted by a carboxy, aminocarbonyl, methylaminocarbonyl or dimethylaminocarbonyl group or in the 2 or 3 position by an amino, methylamino, dimethylamino, acetylamino, N-acetyl-methylamino or morpholino group or by an N-($C_{1-3}$-alkyl)-aminocar-

bonyl or N-($C_{1-3}$-alkyl)-methylaminocarbonyl group optionally substituted in the 2 or 3 position by a dimethylamino group, while a hydrogen atom present at the amino nitrogen in the abovementioned groups may additionally be substituted

by a formyl or benzoyl group,

by a $C_{2-4}$-alkanoyl group which may be terminally substituted by an amino, acetylamino, pyrrolidino, piperidino, morpholino, piperazino or 4-methylpiperazino group or by a $C_{1-3}$-alkylamino, N-acetyl-$C_{1-3}$-alkylamino or di-($C_{1-3}$-alkyl)-amino group, while in the abovementioned $C_{1-3}$-alkylamino, N-acetyl-$C_{1-3}$-alkylamino or di-($C_{1-3}$-alkyl)-amino groups a $C_{1-3}$-alkyl moiety may additionally be substituted in the 2 or 3 position by a methoxy, dimethylamino or morpholino group,

by a $C_{1-4}$-alkylsulphonyl group which may be substituted in the 2 or 3 position by a dimethylamino group,

by a pyrrolidinosulphonyl group, an aminosulphonyl, $C_{1-3}$-alkylaminosulphonyl or di-($C_{1-3}$-alkyl)-aminosulphonyl group, wherein in each case a $C_{1-3}$-alkyl moiety may be substituted by a carboxy, $C_{1-3}$-alkoxycarbonyl, aminocarbonyl, methylaminocarbonyl or dimethylaminocarbonyl group or, except in the 1 position, by a dimethylamino group,

by a $C_{2-3}$-alkoxy group which is substituted in the 2 or 3 position by a dimethylamino or piperidino group,

by an aminocarbonyl, $C_{1-3}$-alkylaminocarbonyl or di-($C_{1-3}$-alkyl)-aminocarbonyl group, wherein in each case the $C_{1-3}$-alkyl moieties may be substituted by a methoxy or dimethylamino group, except in the 1 position,

the isomers and the salts thereof.

**5.** Substituted indolinones of general formula I according to claim 1, wherein

X and $R_2$ to $R_4$ are as hereinbefore defined,

$R_1$ denotes a hydrogen atom and

$R_5$ denotes a phenyl group which may be substituted

by a methyl or ethyl group, each of which is substituted by an azetidin-1-yl, pyrrolidino, piperidino, hexamethyleneimino, morpholino, 1-oxido-thiomorpholino, piperazino, 4-methylpiperazino or 4-acetylpiperazino group, while the abovementioned piperidino groups may additionally be substituted by one or two methyl groups or in the 4 position may be substituted by a hydroxy, methoxy, hydroxymethyl, aminocarbonyl, methylaminocarbonyl or dimethylaminocarbonyl group,

by a straight-chain $C_{1-2}$-alkyl group which is terminally substituted by an amino group or by a $C_{1-3}$-alkylamino group, while the alkyl moiety of the $C_{1-3}$-alkylamino group may be substituted in positions 2 or 3 by a hydroxy or methoxy group and in the abovementioned groups the hydrogen atom present at the amino nitrogen may additionally be replaced

by a $C_{3-6}$-cycloalkyl group, by a $C_{1-3}$-alkyl group in which the alkyl moiety in positions 2 or 3 may be substituted by a hydroxy group, or by a $C_{1-2}$-alkylcarbonyl group substituted by an amino, methylamino or dimethylamino group,

by an ethyl group substituted in the 1 position by an amino group,

by an amino or $C_{1-3}$-alkylamino group in which the alkyl moiety may be terminally substituted by a carboxy, aminocarbonyl, methylaminocarbonyl, dimethylaminocarbonyl, N-(2-dimethylamino-ethyl)-aminocarbonyl or N-(2-dimethylamino-ethyl)-N-methyl-aminocarbonyl group or may be substituted in the 2 or 3 position by an amino, methylamino, dimethylamino, acetylamino, N-acetyl-methylamino or morpholino group, while the hydrogen atom present at the amino nitrogen of the abovementioned groups may additionally be replaced

by a $C_{2-4}$-alkanoyl group which may be terminally substituted by an amino, acetylamino, pyrrolidino, piperidino, morpholino, piperazino or 4-methylpiperazino group or by a $C_{1-3}$-alkylamino, N-acetyl-$C_{1-3}$-alkylamino or di-($C_{1-3}$-alkyl)-amino group, while in the abovementioned $C_{1-3}$-alkylamino, N-acetyl-$C_{1-3}$-alkylamino or di-($C_{1-3}$-alkyl)-amino groups a $C_{1-3}$-alkyl moiety may additionally be substituted in the 2 or 3 position by a methoxy, dimethylamino or morpholino group,

by a $C_{1-4}$-alkylsulphonyl group which may be substituted in the 2 or 3 position by a dimethylamino group,

by a pyrrolidinosulphonyl group, an aminosulphonyl, $C_{1-3}$-alkylaminosulphonyl or di-($C_{1-3}$-alkyl)-aminosulphonyl group, wherein in each case a $C_{1-3}$-alkyl moiety may be substituted by a carboxy, methoxycarbonyl, aminocarbonyl, methylaminocarbonyl or dimethylaminocarbonyl group or, except in the 1 position, by a dimethylamino group,

by a $C_{1-3}$-alkoxy group substituted in the 2 or 3 position by a dimethylamino or piperidino group,

by an aminocarbonyl, $C_{1-3}$-alkylaminocarbonyl or di-($C_{1-3}$-alkyl)-aminocarbonyl group, wherein in each case a $C_{1-3}$-alkyl moiety may be substituted by a methoxy or dimethylamino group, except in the 1 position,

the isomers and the salts thereof.

**6.** The following compounds of general formula I:

(a) (Z)-3-[1-(4-dimethylaminomethyl-phenylamino) -1-phenylmethylidene]-5-nitro-2-indolinone,

(b) (Z)-3-[1-(4-piperidinomethyl-phenylamino)-1-phenylmethylidene]-5-nitro-2-indolinone,

(c) (Z)-3-{1-[4-(2-morpholinoethyl)-phenylamino]-1-phenylmethylidene}-5-nitro-2-indolinone,

(d) (Z)-3-{1-[4-(2-dimethylamino-ethyl)-phenylamino]-1-phenylmethylidene}-5-nitro-2-indolinone and

(e) (Z)-3-{1-[4-(N-(2-dimethylamino-ethyl)-N-methylsulphonylamino)-phenylamino]-1-phenyl-methylidene}-2-indolinone

and the salts thereof.

**7.** Physiologically acceptable salts of the compounds according to claims 1 to 6.

**8.** Pharmaceutical compositions containing a compound of general formula I according to at least one of claims 1 to 6 wherein $R_1$ denotes a hydrogen atom, a $C_{1-3}$-alkyl group or a prodrug group or a physiologically acceptable salt thereof, optionally together with one or more inert carriers and/or diluents.

**9.** Use of a compound of general formula I according to at least one of claims 1 to 6, wherein $R_1$ denotes a hydrogen atom, a $C_{1-3}$-alkyl group or a prodrug group or a physiologically acceptable salt thereof, for preparing a pharmaceutical composition which is suitable for treating excessive or anomalous cell proliferation.

**10.** Process for preparing a pharmaceutical composition according to claim 8, **characterised in that** a compound of formula I according to at least one of claims 1 to 6 wherein $R_1$ denotes a hydrogen atom, a $C_{1-3}$-alkyl group or a prodrug group or a physiologically acceptable salt thereof is incorporated in one or more inert carriers and/or diluents by a non-chemical method.

**11.** Process for preparing the compounds according to claims 1 to 7, **characterised in that**

a. a compound of general formula

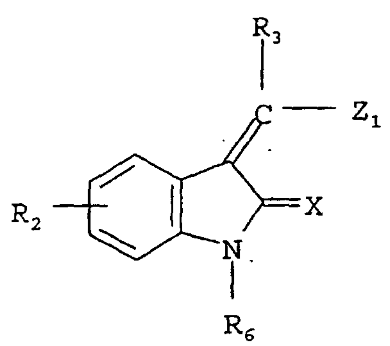

(II),

wherein
X, $R_2$ and $R_3$ are defined as in claims 1 to 6,
$R_6$ denotes a hydrogen atom, a protecting group for the nitrogen atom of the lactam group or a bond to a solid phase and
$Z_1$ denotes a halogen atom, a hydroxy, alkoxy or aralkoxy group,
is reacted with an amine of general formula

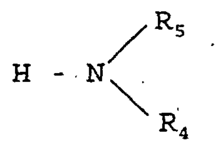

(III),

wherein

$R_4$ and $R_5$ are defined as in claims 1 to 6, and if necessary any protecting group used for the nitrogen atom of the lactam group is cleaved, or a compound thus obtained is cleaved from a solid phase, or

b. in order to prepare a compound of general formula I which contains an aminomethyl group and wherein X denotes an oxygen atom, a compound of general formula

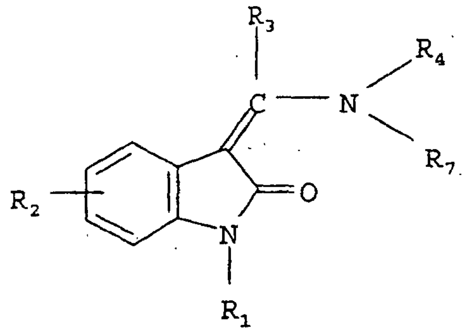

(IV),

wherein
$R_1$ to $R_4$ are defined as in claims 1 to 6 and
$R_7$ has the meanings given for $R_5$ win claims 1 to 5, with the proviso that $R_5$ contains a cyano group, is reduced, or

c. in order to prepare a compound of general formula I wherein $R_1$ denotes a hydrogen atom and X denotes an oxygen atom, a compound of general formula

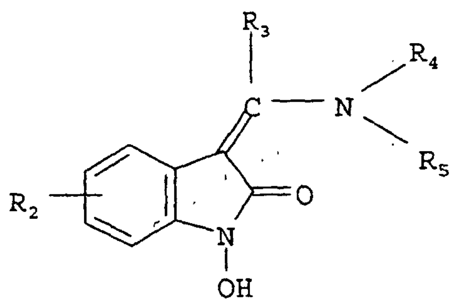

(V),

wherein
$R_2$ to $R_5$ are defined as in claims 1 to 6, is reduced and
subsequently, if desired, a compound of general formula I thus obtained which contains an alkoxycarbonyl group is converted by hydrolysis into a corresponding carboxy compound, or
a compound of general formula I thus obtained which contains an amino or alkylamino group is converted by alkylation or reductive alkylation into a corresponding alkylamino or dialkylamino compound or
a compound of general formula I thus obtained which contains an amino or alkylamino group is converted by acylation into a corresponding acyl compound, or
a compound of general formula I thus obtained which contains a carboxy group is converted by esterification or amidation into a corresponding ester or aminocarbonyl, or
a compound of general formula I thus obtained wherein $R_3$ denotes a phenyl group which contains a chlorine, bromine or iodine atom is converted into a corresponding alkenylated compound by reacting with an alkenyl compound, or
a compound of general formula I thus obtained wherein $R_3$ denotes a phenyl group which contains a chlorine, bromine or iodine atom is converted into a corresponding alkenylated compound by reacting with an alkynyl compound, and
if necessary any protecting group used to protect reactive groups during the reactions is cleaved or
if desired a compound of general formula I thus obtained is subsequently resolved into the stereoisomers thereof or

a compound of general formula I thus obtained is converted into the salts thereof, in particular for pharmaceutical use into the physiologically acceptable salts thereof with an inorganic or organic acid or base.

## Revendications

1. Indolinones substituées de formule générale

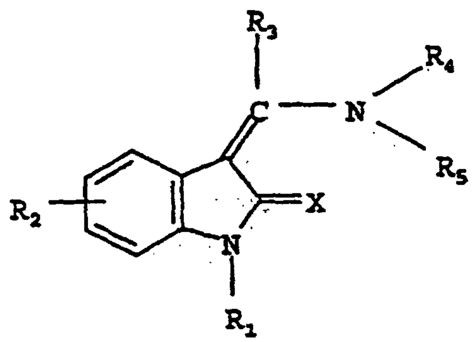

(I),

où

X représente un atome d'oxygène ou de soufre,

$R_1$ représente un atome d'hydrogène, un groupe alkyle en $C_{1-3}$ ou un groupe hydroxyle,

$R_2$ représente un atome d'hydrogène, de fluor, de chlore, de brome ou d'iode, un groupé alkyle en $C_{1-3}$ ou un groupe nitro,

$R_3$ représente un groupe phényle ou naphtyle qui peut être mono- ou disubstitué dans chaque cas par des atomes de fluor, de chlore, de brome ou d'iode, par des groupes alkyle en $C_{1-3}$, alcoxy en $C_{1-3}$, carboxyle, cyano, trifluorométhyle, nitro, amino, alkyle en $C_{1-3}$-amino, di(alkyle en $C_{1-3}$)amino, alkyle en $C_{1-3}$-sulfonylamino, amino-alkyle en $C_{1-3}$, 2-carboxyphénylcarbonylaminométhyle, alkyle en $C_{1-3}$-amino-alkyle en $C_{1-3}$, alcanoyle en $C_{2-4}$-amino-alkyle en $C_{1-3}$, N-(alcanoyle en $C_{2-4}$)-alkyle en $C_{1-3}$-amino-alkyle en $C_{1-3}$, di(alkyle en $C_{1-3}$)aminoalkyle en $C_{1-3}$, carboxy-alcényle en $C_{2-3}$, N-(carboxyalkyle en $C_{1-3}$)aminocarbonyle, N-(carboxy-alkyle en $C_{1-3}$)-N-(alkyle en $C_{1-3}$) aminocarbonyle ou imidazolylalkyle en $C_{1-3}$, les substituants pouvant être identiques ou différents,

$R_4$ représente un atome d'hydrogène ou un groupe alkyle en $C_{1-3}$ et

$R_5$ représente un groupe phényle ou naphtyle éventuellement substitué par un groupe alkyle en $C_{1-3}$, qui peut être substitué en outre dans chaque cas, dans la partie aromatique

par un atome de fluor, de chlore, de brome ou d'iode, par un groupe alkyle en $C_{1-3}$, alcoxy en $C_{1-3}$, cyano, nitro ou trifluorométhyle,

par un groupe alcoxy en $C_{1-3}$, qui est substitué par un groupe carboxyle, aminocarbonyle, alkyle en $C_{1-3}$-aminocarbonyle ou di(alkyle en $C_{1-3}$)aminocarbonyle ou en position 2 ou 3 aussi par un groupe amino, alkyle en $C_{1-3}$-amino, di(alkyle en $C_{1-3}$)amino, phényl-alkyle en $C_{1-3}$-amino, N-(phényl-alkyle en $C_{1-3}$)-N-(alkyle en $C_{1-3}$) amino, pyrrolidino, pipéridino ou hexaméthylèneimino,

par un groupe alcényle en $C_{2-3}$ éventuellement substitué par un groupe di(alkyle en $C_{1-3}$)amino, qui peut être substitué en outre dans la partie alcényle par un atome de chlore ou de brome,

par un groupe alcynyle en $C_{2-3}$ éventuellement substitué par un groupe di(alkyle en $C_{1-3}$)amino,

par un groupe alkyle en $C_{1-3}$, qui est substitué par un groupe cycloalkylèneimino à 3 à 7 chaînons, par un groupe déshydropipéridino, morpholino, thiomorpholino, 1-oxydo-thiomorpholino, 1,1-dioxydo-thiomorpholino, pipérazino, N-(alkyle en $C_{1-3}$) pipérazino, N-(alcanoyle en $C_{1-3}$)pipérazino ou N-(alcoxy en $C_{1-5}$-carbonyl)pipérazino, où les substituants cités précédemment peuvent être substitués par un groupe alkyle en $C_{1-3}$, phényle ou phényl-alkyle en $C_{1-3}$ et les groupes pipéridino ou hexaméthylèneimino cités précédemment peuvent être substitués en outre par un grouper alkyle en $C_{1-3}$ ou en position 3 ou 4 par un groupe hydroxyle, alcoxy en $C_{1-3}$, hydroxy-alkyle en $C_{1-3}$, carboxyle, aminocarbonyle, N-(alkyle en $C_{1-3}$)aminocarbonyle ou N,N-di(alkyle en $C_{1-3}$)aminocarbonyle, par un groupe alkyle en $C_{1-3}$ substitué par un groupe hydroxyle, alcoxy en $C_{1-3}$, carboxyle ou cyano, où un groupe alkyle en $C_{1-3}$ substitué par un groupe carboxyle peut être substitué en outre dans la partie alkyle par un groupe amino ou alcoxy en $C_{1-5}$-carbonylamino,

par un groupe aminocarbonylamino, amidino ou guanidino éventuellement substitué par un ou deux groupes alkyle en $C_{1-3}$,

par un groupe pipéridino, hexaméthylèneimino, morpholino, pipérazino ou N-(alkyle en $C_{1-3}$)-pipérazino,

par un groupe formyle, carboxyle ou trifluoroacétyle,
par un groupe carbonyle qui peut être substitué

par un groupe alkyle en $C_{1-3}$, alcoxy en $C_{1-3}$-alkyle en $C_{1-3}$, amino, alkyle en $C_{1-5}$-amino ou di(alkyle en $C_{1-3}$) amino, où les groupes amino et alkyle en $C_{1-3}$-amino cités précédemment peuvent être substitués en outre sur l'atome d'azote par un groupe carboxy-alkyle en $C_{1-3}$ ou par un groupe alkyle en $C_{2-3}$, qui peut être substitué en position 2 ou 3 par un groupe hydroxyle, alcoxy en $C_{1-3}$, amino, alkyle en $C_{1-3}$-amino ou di(alkyle en $C_{1-3}$)amino,

par un groupe pyrrolidinocarbonyle, pipéridinocarbonyle, hexaméthylèneiminocarbonyle, morpholinocarbonyle, pipérazinocarbonyle, N-(alkyle en $C_{1-3}$)pipérazinocarbonyle ou N-(phényl-alkyle en $C_{1-3}$)-pipérazinocarbonyle,

par un groupe amidosulfonyle, pyrrolidinosulfonyle, pipéridinosulfonyle ou hexaméthylèneiminosulfonyle, par un groupe alkyle en $C_{1-3}$-amidosulfonyle ou di(alkyle en $C_{1-3}$) amidosulfonyle, dans lesquels une partie alkyle peut être substituée dans chaque cas par un groupe carboxyle, aminocarbonyle, N-(alkyle en $C_{1-3}$)aminocarbonyle ou N,N-di(alkyle en $C_{1-3}$)-aminocarbonyle ou aussi en position 2 ou 3 par un groupe alkyle en $C_{1-3}$-amino ou di(alkyle en $C_{1-3}$)amino,

par un groupe amino, alkyle en $C_{1-5}$-amino, cycloalkyle en $C_{3-7}$-amino, phényl-alkyle en $C_{1-3}$-amino, phénylamino, hétéroarylamino à 6 chaînons, amino-alkyle en $C_{1-3}$, N-(alkyle en $C_{1-5}$)-amino-alkyle en $C_{1-3}$, di(alkyle en $C_{1-5}$) amino-alkyle en $C_{1-3}$, cycloalkyle en $C_{3-7}$-amino-alkyle en $C_{1-3}$, N-(alkyle en $C_{1-5}$)-cycloalkyle en $C_{3-7}$-amino-alkyle en $C_{1-3}$, phénylamino-alkyle en $C_{1-3}$, N-(alkyle en $C_{1-3}$)-phénylamino-alkyle en $C_{1-3}$, phényl-alkyle en $C_{1-3}$-amino-alkyle en $C_{1-3}$ ou N-(alkyle en $C_{1-5}$)phénylalkyle en $C_{1-3}$-amino-alkyle en $C_{1-3}$ ou par un groupe hétéroarylamino-alkyle en $C_{1-3}$ à 6 chaînons éventuellement substitué sur l'atome d'azote par un groupe alkyle en $C_{1-5}$, où la partie N-alkyle des groupes cités précédemment peut être substituée dans chaque cas par un groupé cyano, carboxyle, aminocarbonyle, alkyle en $C_{1-3}$-aminocarbonyle, di(alkyle en $C_{1-3}$)aminocarbonyle, 2-[di(alkyle en $C_{1-3}$)amino] éthylaminocarbonyle, 3-[di(alkyle en $C_{1-3}$)amino]propylaminocarbonyle, N-{2-[di(alkyle en $C_{1-3}$)amino]éthyl}-N-(alkyle en $C_{1-3}$)aminocarbonyle ou N-{3-[di(alkyle en $C_{1-3}$)amino]propyl}-N-(alkyle en $C_{1-3}$)aminocarbonyle ou en position 2 ou 3 par un groupe hydroxyle, alcoxy en $C_{1-3}$, amino-, alkyle en $C_{1-3}$-amino, di(alkyle en $C_{1-3}$)amino, pyrrolidino, pipéridino, hexaméthylèneimino, morpholino, pipérazino ou N-(alkyle en $C_{1-3}$)pipérazino et l'atome d'azote des groupes amino, N-(alkyle en $C_{1-5}$)-amino, cycloalkyle en $C_{3-7}$-amino, phényl-alkyle en $C_{1-3}$-amino, phénylamino, hétéroarylamino à 6 chaînons, amino-alkyle en $C_{1-3}$ et N-(alkyle en $C_{1-5}$-amino)-alkyle en $C_{1-3}$ peut être substitué en outre

par un groupe alcoxy en $C_{1-5}$-carbonyle,

par un groupe formyle, trifluoroacétyle ou benzoyle,

par un groupe carboxy-alkyle en $C_{1-3}$, aminocarbonyl-alkyle en $C_{1-3}$, N-(alkyle en $C_{1-3}$)-aminocarbonyl-alkyle en $C_{1-3}$ ou N,N-di(alkyle en $C_{1-3}$)aminocarbonyl-alkyle en $C_{1-3}$,

par un groupe alkyle en $C_{1-5}$, qui, à l'exception de la position 1, peut être substitué par un groupe hydroxyle, alcoxy en $C_{1-3}$, amino, alkyle en $C_{1-3}$-amino ou di(alkyle en $C_{1-3}$)amino,

par un groupe alcanoyle en $C_{2-4}$, qui peut être substitué dans la partie alcanoyle par un groupé carboxyle, hydroxyle, alcoxy en $C_{1-3}$, phényle, amino, phtalimido, alkyle en $C_{1-3}$-amino, di(alkyle en $C_{1-3}$)amino, pyrrolidino, pipéridino, hexaméthylèneimino ou morpholino ou par un groupe pipérazino éventuellement substitué sur l'atome d'azote par un groupe alkyle en $C_{1-3}$ ou phényl-alkyle en $C_{1-3}$, ou la partie alkyle des substituants alkyle en $C_{1-3}$-amino et di(alkyle en $C_{1-3}$)amino cités précédemment peut être substituée en position 2 ou 3 par un groupe hydroxyle, alcoxy en $C_{1-3}$, amino, alcoxy en $C_{1-5}$-carbonylamino, alkyle en $C_{1-3}$-amino, di(alkyle en $C_{1-3}$)amino, phényle, pyrrolidino, pipéridino, hexaméthylèneimino ou morpholino,

par un groupe alkyle en $C_{1-5}$-sulfonyle, dans lequel la partie alkyle, à l'exception de la position. 1, peut être substituée par un groupe di(alkyle en $C_{1-3}$)amino, pyrrolidino, pipéridino, hexaméthylèneimino ou morpholino,

par un groupe phényl-alkyle en $C_{1-3}$-sulfonyle ou phénylsulfonyle éventuellement substitué dans la partie phényle par un atome de fluor, de chlore ou de brome ou par un groupe alkyle en $C_{1-3}$ ou alcoxy en $C_{1-3}$,

où, en outre, un groupe carboxyle, amino ou imino présent peut être substitué par un reste clivable in vivo, à condition que le composé 3-[(α-phénylamino)benzylidène]indolin-2-one
soit exclu,
leurs isomères et leurs sels.

**2.** Indolinones substituées de formule générale I selon la revendication 1 où
X représente un atome d'oxygène ou de soufre,
$R_1$ représente un atome d'hydrogène, un groupe alkyle en $C_{1-3}$, hydroxyle, alcoxy en $C_{1-4}$-carbonyle ou alcanoyle en $C_{2-4}$,
$R_2$ représente un atome d'hydrogène, de fluor, de chlore, de brome ou d'iode, un groupe alkyle en $C_{1-3}$ ou nitro,
$R_3$ représente un groupe phényle ou naphtyle qui peut être mono- ou disubstitué dans chaque cas par des atomes de fluor, de chlore, de brome ou d'iode, par des groupes alkyle en $C_{1-3}$, imidazolylméthyle, 2-carboxyéthényle, 2-(alcoxy en $C_{1-3}$-carbonyl)éthényle, alcoxy en $C_{1-3}$, cyano, carboxyle, alcoxy en $C_{1-3}$-carbonyle, trifluorométhyle,

nitro, amino, phtalimidométhyle, 2-carboxy-phénylcarbonylaminométhyle, alkyle en $C_{1-3}$-amino, di(alkyle en $C_{1-3}$) amino, alkyle en $C_{1-3}$-sulfonylamino, amino-alkyle en $C_{1-3}$, alkyle en $C_{1-3}$-amino-alkyle en $C_{1-3}$, alcanoyle en $C_{2-4}$-amino-alkyle en $C_{1-3}$, N-(alcanoyle en $C_{2-4}$)-alkyle en $C_{1-3}$-amino-alkyle en $C_{1-3}$, di(alkyle en $C_{1-3}$)amino-alkyle en $C_{1-3}$, carboxy-alkyle en $C_{1-3}$)-aminocarbonyle ou alcoxy en $C_{1-3}$-carbonyl-alkyle en $C_{1-3}$-aminocarbonyle, les substituants pouvant être identiques ou différents,

$R_4$ représente un atome d'hydrogène ou un groupe alkyle en $C_{1-3}$ et

$R_5$ représente un groupe phényle ou naphtyle éventuellement substitué par un groupe alkyle en $C_{1-3}$, qui peut être substitué en outre dans chaque cas, dans la partie aromatique

par un atome de fluor, de chlore, de brome ou d'iode, par un groupe alkyle en $C_{1-3}$, alcoxy en $C_{1-3}$, cyano, nitro ou trifluorométhyle, où le groupe alkyle mentionne précédemment peut être substitué simultanément par un groupe carboxyle ou alcoxy en $C_{1-3}$-carbonyle et un groupe amino ou alcoxy en $C_{1-4}$-carbonylamino,

par un groupe alkyle en $C_{1-3}$, qui est substitué par un groupe cycloalkylèneimino à 4 à 7 chaînons, par un groupe déshydropipéridino, morpholino, thiomorpholino, 1-oxydo-thiomorpholino, 1,1-dioxydo-thiomorpholino, pipérazino ou N-(alcoxy en $C_{1-4}$-carbonyl)pipérazino, où les substituants pipéridino, hexaméthylèneimino, morpholino, thiomorpholino, 1-oxydo-thiomorpholino, 1,1-dioxydo-thiomorpholino et pipérazino cités précédemment peuvent être substitués par un groupe alkyle en $C_{1-3}$, phényle ou phényl-alkyle en $C_{1-3}$ et les groupes pipéridino cités précédemment peuvent être substitués en outre par un groupe alkyle en $C_{1-3}$ ou en position 3 ou 4 par un groupe hydroxyle, alcoxy en $C_{1-3}$, hydroxy-alkyle en $C_{1-3}$, carboxyle, aminocarbonyle, N-(alkyle en $C_{1-3}$)aminocarbonyle ou N,N-di (alkyle en $C_{1-3}$) aminocarbonyle,

par un groupe alkyle en $C_{1-3}$ éventuellement substitué par un groupe hydroxyle, alcoxy en $C_{1-3}$, carboxyle, alcoxy en $C_{1-3}$-carbonyle ou cyano,

par un groupe aminocarbonylamino, amidino ou guanidino éventuellement substitué par un ou deux groupes alkyle en $C_{1-3}$,

par un groupe pipéridino, hexaméthylèneimino, morpholino, pipérazino ou N-(alkyle en $C_{1-3}$)-pipérazino,

par un groupe formyle, carboxyle, alcoxy en $C_{1-3}$-carbonyle ou trifluoroacétyle,

par un groupe carbonyle qui peut être substitué

par un groupe alkyle en $C_{1-3}$, alcoxy en $C_{1-3}$-alkyle en $C_{1-3}$, amino, alkyle en $C_{1-5}$-amino ou di(alkyle en $C_{1-3}$) amino, où les groupes amino et alkyle en $C_{1-3}$-amino cités précédemment peuvent être substitués en outre sur l'atome d'azote par un groupe carboxy-alkyle en $C_{1-3}$ ou alcoxy en $C_{1-3}$-carbonyl-alkyle en $C_{1-3}$ ou par un groupe alkyle en $C_{2-3}$, qui peuvent être substitués en position 2 ou 3 par un groupe hydroxyle., alcoxy en $C_{1-3}$, amino, alkyle en $C_{1-3}$-amino ou di(alkyle en $C_{1-3}$)amino,

par un groupe pyrrolidinocarbonyle, pyrrolidinosulfonyle, pipéridinocarbonyle, hexaméthylèneiminocarbonyle, morpholinocarbonyle, pipérazinocarbonyle, N-(alkyle en $C_{1-3}$)pipérazinocarbonyle ou N-(phényl-alkyle en $C_{1-3}$) pipérazinocarbonyle,

par un groupe amidosulfonyle, alkyle en $C_{1-3}$-amidosulfonyle ou di(alkyle en $C_{1-3}$)amidosulfonylé, dans lesquels une partie alkyle peut être substituée par un groupe carboxyle, alcoxy en $C_{1-3}$-carbonyle, aminocarbonyle, alkyle en $C_{1-3}$-aminocarbonyle ou di(alkyle en $C_{1-3}$)aminocarbonyle ou en position 2 ou 3 par un groupe amino, alkyle en $C_{1-3}$-amino ou di(alkyle en $C_{1-3}$)amino,

par un groupe amino, alkyle en $C_{1-5}$-amino, amino-alkyle en $C_{1-3}$, N-(alkyle en $C_{1-3}$)amino-alkyle en $C_{1-3}$, N-(2-hydroxyéthyl)amino-alkyle en $C_{1-3}$, N-(3-hydroxypropyl)amino-alkyle en $C_{1-3}$, di(alkyle en $C_{1-5}$)amino-alkyle en $C_{1-3}$, N-(cycloalkyle en $C_{3-7}$)-amino-alkyle en $C_{1-3}$, N-(cycloalkyle en $C_{3-7}$)-N-(alkyle en $C_{1-3}$)-amino-alkyle en $C_{1-3}$ ou N-(phénylalkyle en $C_{1-3}$)-amino-alkyle en $C_{1-3}$, où la partie N-alkyle des groupes cités précédemment peut être substituée par un groupe cyano, carboxyle, alkyle en $C_{1-3}$-carbonyle, aminocarbonyle, alkyle en $C_{1-3}$-aminocarbonyle, di(alkyle en $C_{1-3}$)aminocarbonyle, 2-[di(alkyle en $C_{1-3}$)amino]éthylaminocarbonyle, 3-[di(alkyle en $C_{1-3}$) amino]propylaminocarbonyle, N-{2-[di(alkyle en $C_{1-3}$)amino]éthyl}-N-(alkyle en $C_{1-3}$)-aminocarbonyle ou N-{3-[di (alkyle en $C_{1-3}$)-amino]propyl}-N-(alkyle en $C_{1-3}$)aminocarbonyle ou en position 2 ou 3 par un groupe hydroxyle, alcoxy en $C_{1-3}$, amino, alkyle en $C_{1-3}$-amino, di(alkyle en $C_{1-3}$)amino ou morpholino, où l'atome d'azote des parties amino, alkyle en $C_{1-3}$-amino, amino-alkyle en $C_{1-3}$ ou N-(alkyle en $C_{1-5}$-amino)-alkyle en $C_{1-3}$ peut être substitué en outre

par un groupe alcoxy en $C_{1-5}$-carbonyle,

par un groupe formyle, trifluoroacétyle ou benzoyle,

par un groupe alkyle en $C_{1-5}$, qui, à l'exception de la position 1, peut être substitué par un groupe hydroxyle, alcoxy en $C_{1-3}$, amino, alkyle en $C_{1-3}$-amino ou di(alkyle en $C_{1-3}$)amino,

par un groupe alcanoyle en $C_{2-4}$, qui peut être substitué dans la partie alcanoyle par un groupe hydroxyle, alcoxy en $C_{1-3}$, amino, alcanoyle en $C_{2-4}$-amino, alcoxy en $C_{1-5}$-carbonylamino, phtalimido, alkyle en $C_{1-3}$-amino, di(alkyle en $C_{1-3}$)amino, N-(alkyle en $C_{1-3}$)phénylamino, pyrrolidino, pipéridino ou morpholino ou par un groupe pipérazino éventuellement substitué sur l'atome d'azote par un groupe alkyle en $C_{1-3}$ ou phényl-alkyle en $C_{1-3}$, où la partie N-alkyle des groupes cités précédemment peut être substituée en position 2 ou 3 par un groupe méthoxy, di(alkyle

en $C_{1-3}$)amino ou morpholino,

par un groupe alkyle en $C_{1-5}$-sulfonyle, dans lequel la partie alkyle, à l'exception de la position 1, peut être substituée par un groupe di(alkyle en $C_{1-3}$)amino, pyrrolidino, pipéridino, hexaméthylèneimino ou morpholino,

par un groupe pyridinyle ou pyrimidinyle,

par un groupe phényle, phényl-alkyle en $C_{1-3}$-sulfonyle ou phénylsulfonyle éventuellement substitué dans la partie phényle par un groupe alkyle en $C_{1-3}$,

par un groupe alcoxy en $C_{1-3}$, qui est substitué par un groupe carboxyle, alcoxy en $C_{1-3}$-carbonyle, aminocarbonyle, alkyle en $C_{1-3}$-aminocarbonyle ou di(alkyle en $C_{1-3}$)aminocarbonyle ou en position 2 ou 3 par un groupe amino, alkyle en $C_{1-3}$-amino, di(alkyle en $C_{1-3}$)amino, N-(alkyle en $C_{1-3}$)-N-(phénylalkyle en $C_{1-3}$)amino, pipéridino ou hexaméthylèneimino,

par un groupe prop-1-ényle, 2-chloroprop-1-ényle ou prop-1-ynyle, qui est substitué en position 3 par un groupe di(alkyle en $C_{1-3}$) amino,

leurs isomères et leurs sels.

**3.** Indolinones substituées de formule générale I selon la revendication 1 où

X représente un atome d'oxygène,

$R_1$ représente un atome d'hydrogène, un groupe alkyle en $C_{1-3}$, alcoxy en $C_{1-4}$-carbonyle ou alcanoyle en $C_{2-4}$,

$R_2$ représente un atome d'hydrogène, de fluor, de chlore, de brome ou d'iode, un groupe alkyle en $C_{1-3}$ ou nitro,

$R_3$ représente un groupe phényle qui peut être mono- ou disubstitué par des atomes de fluor, de chlore, de brome ou d'iode, par des groupes alkyle en $C_{1-3}$, trifluorométhyle, imidazolylméthyle, 2-carboxyéthényle, 2-alcoxy en $C_{1-3}$-carbonyl-éthényle, alcoxy en $C_{1-3}$, cyano, carboxyle, alcoxy en $C_{1-3}$-carbonyle, nitro, amino, phtalimidométhyle, 2-carboxy-benzoylaminométhyle, alkyle en $C_{1-3}$-amino, di(alkyle en $C_{1-3}$)amino, alkyle en $C_{1-3}$-sulfonylamino, amino-alkyle en $C_{1-3}$, alkyle en $C_{1-3}$-amino-alkyle en $C_{1-3}$, alcanoyle en $C_{2-4}$-amino-alkyle en $C_{1-3}$, N-(alcanoyle en $C_{2-4}$)-alkyle en $C_{1-3}$-amino-alkyle en $C_{1-3}$, di(alkyle en $C_{1-3}$)amino-alkyle en $C_{1-3}$, carboxy-alkyle en $C_{1-3}$-aminocarbonyle ou alcoxy en $C_{1-3}$-carbonyl-alkyle en $C_{1-3}$-aminocarbonyle, les substituants pouvant être identiques ou différents,

$R_4$ représente un atome d'hydrogène ou un groupe alkyle en $C_{1-3}$ et

$R_5$ représente un groupe phényle ou naphtyle éventuellement substitué par un groupe alkyle en $C_{1-3}$, qui peut être substitué en outre dans chaque cas, dans la partie aromatique

par un atome de fluor, de chlore, de brome ou d'iode, par un groupe alcoxy en $C_{1-3}$, cyano, nitro ou trifluorométhyle,

par un groupe alkyle en $C_{1-3}$, qui est substitué par un groupe cycloalkylèneimino à 4 à 7 chaînons, par un groupe déshydropipéridino, morpholino, thiomorpholino, 1-oxydo-thiomorpholino, 1,1-dioxydo-thiomorpholino, pipérazino ou N-(alcoxy en $C_{1-4}$-carbonyl)pipérazino, où les substituants pipéridino, hexaméthylèneimino, morpholino et pipérazino cités précédemment peuvent être substitués par un groupe alkyle en $C_{1-3}$, phényle ou phénylalkyle en $C_{1-3}$ et les groupes pipéridino cités précédemment peuvent être substitués en outre par un groupe alkyle en $C_{1-3}$ ou en position 3 ou 4 par un groupe hydroxyle, alcoxy en $C_{1-3}$, hydroxy-alkyle en $C_{1-3}$, carboxyle, aminocarbonyle, N-(alkyle en $C_{1-3}$)aminocarbonyle ou N,N-di(alkyle en $C_{1-3}$)-aminocarbonyle,

par un groupe alkyle en $C_{1-3}$ éventuellement substitué par un groupe hydroxyle, alcoxy en $C_{1-3}$, carboxyle, alcoxy en $C_{1-3}$-carbonyle ou cyano,

par un groupe aminocarbonylamino, amidino ou guanidino éventuellement substitué par un ou deux groupes alkyle en $C_{1-3}$,

par un groupe pipéridino, hexaméthylèneimino, morpholino, pipérazino ou N-(alkyle en $C_{1-3}$)-pipérazino,

par un groupe formyle, carboxyle, alcoxy en $C_{1-3}$-carbonyle ou trifluoroacétyle,

par un groupe carbonyle qui peut être substitué

par un groupe alkyle en $C_{1-3}$, alcoxy en $C_{1-3}$-alkyle en $C_{1-3}$, amino, alkyle en $C_{1-5}$-amino ou di(alkyle en $C_{1-3}$) amino, où les groupes amino et alkyle en $C_{1-3}$-amino cités précédemment peuvent être substitués en outre sur l'atome d'azote par un groupe carboxy-alkyle en $C_{1-3}$, alcoxy en $C_{1-3}$-carbonyl-alkyle en $C_{1-3}$ ou alcoxy en $C_{1-3}$-carbonylalkyle en $C_{1-3}$ ou par un groupe alkyle en $C_{2-3}$, qui peut être substitué en position 2 ou 3 par un groupe hydroxyle, alcoxy en $C_{1-3}$, amino, alkyle en $C_{1-3}$-amino ou di(alkyle en $C_{1-3}$)amino,

par un groupe pyrrolidinocarbonyle, pyrrolidinosulfonyle, pipéridinocarbonyle ou hexaméthylèneiminocarbonyle,

par un groupe amidosulfonyle, alkyle en $C_{1-3}$-amidosulfonyle ou di(alkyle en $C_{1-3}$)amidosulfonyle, dans lesquels une partie alkyle peut être substituée par un groupe carboxyle, alcoxy en $C_{1-3}$-carbonyle ou diméthylaminocarbonyle ou en position 2 ou 3 par un groupe diméthylamino,

par un groupe alkyle en $C_{1-2}$ linéaire, qui peut être substitué en position terminale par un groupe amino, benzylamino, pyridylamino ou pyrimidylamino, par un groupe alkyle en $C_{1-4}$-amino, dans lequel la partie alkyle peut être substituée en position 2, 3 ou 4 par un groupe hydroxyle ou méthoxy, ou par un groupe alkyle en $C_{1-2}$-amino

substitué dans la partie alkyle en $C_{1-2}$ par un groupe carboxyle, alcoxy en $C_{1-3}$-carbonyle ou di(alkyle en $C_{1-3}$) aminocarbonyle, où, dans les groupes cités précédemment, un atome d'hydrogène présent sur l'atome d'azote d'amine peut être remplacé en outre

par un groupe cycloalkyle en $C_{3-6}$, par un groupe alkyle en $C_{1-4}$ dans lequel la partie alkyle peut être substituée en position 2, 3 ou 4 par un groupe hydroxyle, par un groupe alkyle en $C_{1-2}$-carbonyle éventuellement substitué par un groupe méthoxy, carboxyle, alcoxy en $C_{1-3}$-carbonyle, amino, méthylamino, diméthylamino, acétylamino, alcoxy en $C_{1-5}$-carbonylamino, N-méthyl-alcoxy en $C_{1-5}$-carbonylamino ou morpholinocarbonylamino, par un groupe alcoxy en $C_{1-5}$-carbonyle, alkyle en $C_{1-4}$-sulfonyle, phénylsulfonyle ou tolylsulfonyle,

par un groupe 3-diméthylaminopropyle ou 3-diméthylaminoprop-1-ényle,

par un groupe éthyle qui est substitué en position 1 par un groupe amino ou alcoxy en $C_{1-5}$-carbonylamino,

par un groupe éthyle qui est substitué en position 2 par un groupe amino ou alcoxy en $C_{1-5}$-carbonylamino et par un groupe carboxyle ou alcoxy en $C_{1-3}$-carbonyle,

par un groupe amino ou alkyle en $C_{1-3}$-amino, où la partie alkyle peut être substituée par un groupe cyano, carboxyle, alcoxy en $C_{1-3}$-carbonyle, aminocarbonyle, méthylaminocarbonyle ou diméthylaminocarbonyle ou en position 2 ou 3 par un groupe amino, méthylamino, diméthylamino, acétylamino, N-méthyl-acétylamino ou morpholino, par un groupe N-(alkyle en $C_{1-3}$)aminocarbonyle ou N-(alkyle en $C_{1-3}$)-méthylaminocarbonyle éventuellement substitué en position 2 ou 3 de la partie alkyle en $C_{1-3}$ par un groupe diméthylamino, où un atome d'hydrogène présent sur l'atome d'azote d'amine dans les groupes cités précédemment peut être remplacé en outre

par un groupe formyle, trifluoroacétyle, benzoyle, alcoxy en $C_{1-4}$-carbonyle ou alkyle en $C_{1-4}$-aminocarbonyle,

par un groupe alcanoyle en $C_{2-4}$, qui peut être substitué en position terminale par un groupe amino, acétylamino, alcoxy en $C_{1-4}$-carbonylamino, pyrrolidino, pipéridino, morpholino, pipérazino, 4-méthylpipérazino, 4-benzylpipérazino ou phtalimido ou par un groupe alkyle en $C_{1-3}$-amino, N-acétyl-alkyle en $C_{1-3}$-amino ou di(alkyle en $C_{1-3}$)amino, où, dans les groupes alkyle en $C_{1-3}$-amino, N-acétyl-alkyle en $C_{1-3}$-amino et di(alkyle en $C_{1-3}$)amino cités précédemment, dans chaque cas une partie alkyle en $C_{1-3}$ peut être substituée en outre par un groupe phényle ou en position 2 ou 3 par un groupe méthoxy, diméthylamino ou morpholino,

par un groupe alkyle en $C_{1-4}$-sulfonyle, dans lequel la partie alkyle peut être substituée en outre en position 2 ou 3 par un groupe diméthylamino, pipéridino ou morpholino,

par un groupe phénylsulfonyle ou toluènesulfonyle,

par un groupe alcoxy en $C_{1-3}$, qui est substitué par un groupe carboxyle, alcoxy en $C_{1-3}$-carbonyle, aminocarbonyle, méthylaminocarbonyle ou diméthylaminocarbonyle ou en position 2 ou 3 par un groupe amino, méthylamino, diméthylamino, N-méthylbenzylamino, pipéridino ou hexaméthylèneimino, par un groupe alkyle en $C_{1-3}$-aminocarbonyle ou di(alkyle en $C_{1-3}$)aminocarbonyle dans lequel une partie alkyle en $C_{1-3}$ peut être substituée en position 2 ou 3 par un groupe méthoxy ou diméthylamino,

leurs isomères et leurs sels.

**4.** Indolinones substituées de formule générale
I selon la revendication 1 où
X représente un atome d'oxygène,
$R_1$ représente un atome d'hydrogène,
$R_2$ représente un atome d'hydrogène, de chlore ou de brome, un groupe méthyle ou nitro,
$R_3$ représente un groupe phényle qui peut être substitué par un atome de fluor, de chlore ou de brome, par un groupe méthyle, méthoxy, aminométhyle, acétylaminométhyle, carboxyle, méthoxycarbonyle ou imidazolylméthyle,
$R_4$ représente un atome d'hydrogène,
$R_5$ représente un groupe phényle qui peut être substitué

par un atome de fluor, de chlore ou de brome, par un groupe méthyle, méthoxy, nitro, cyano ou trifluorométhyle,

par un groupe méthyle ou éthyle qui est substitué dans chaque cas par un groupe carboxyle, alcoxy en $C_{1-3}$-carbonyle, cyano, azétidin-1-yle, pyrrolidino, pipéridino, 4-phénylpipéridino, 3,6-dihydro-2H-pyridin-1-yle, hexaméthylèneimino, morpholino, thiomorpholino, 1-oxydo-thiomorpholino, pipérazino, 4-méthylpipérazino ou 4-acétylpipérazino, où les groupes pipéridino cités précédemment peuvent être substitués en outre par un ou deux groupes méthyle ou en position 3 ou 4 par un groupe hydroxyle, méthoxy, carboxyle, hydroxyméthyle, alcoxy en $C_{1-3}$-carbonyle, aminocarbonyle, méthylaminocarbonyle ou diméthylaminocarbonyle,

par un groupe alkyle en $C_{1-2}$ linéaire qui peut être substitué en position terminale par un groupe amino ou benzylamino, par un groupe alkyle en $C_{1-4}$-amino, dans lequel la partie alkyle peut être substituée en position 2, 3 ou 4 par un groupe hydroxyle ou méthoxy, par un groupe alkyle en $C_{1-2}$-amino substitué dans la partie alkyle en $C_{1-2}$ par un groupe carboxyle, alcoxy en $C_{1-3}$-carbonyle ou diméthylaminocarbonyle, où, dans les groupes cités précédemment, un atome d'hydrogène présent sur l'atome d'azote d'aminé peut être remplacé en outre par un groupe cycloalkyle en $C_{3-6}$, par un groupe alkyle en $C_{1-4}$, dans lequel la partie alkyle peut être substituée en position 2, 3 ou 4 par un groupe hydroxyle, ou par un groupe alkyle en $C_{1-2}$-carbonyle éventuellement substitué par un groupe amino, méthylamino ou diméthylamino,

par un groupe 3-diméthylaminoprop-1-ényle,

par un groupe éthyle qui est substitué en position 1 par un groupe amino ou alcoxy en $C_{1-4}$-carbonylamino,

par un groupe amino ou alkyle en $C_{1-3}$-amino, où la partie alkyle peut être substituée en position terminale par un groupe carboxyle, aminocarbonyle, méthylaminocarbonyle ou diméthylaminocarbonyle ou en position 2 ou 3 par un groupe amino, méthylamino, diméthylamino, acétylamino, N-acétyl-méthylamino ou morpholino ou par un groupe N-(alkyle en $C_{1-3}$)aminocarbonyle ou N-(alkyle en $C_{1-3}$)-méthylaminocarbonyle éventuellement substitué en position 2 ou 3 par un groupe diméthylamino, où un atome d'hydrogène présent sur l'atome d'azote d'amine dans les groupes cités précédemment peut être remplacé en outre

par un groupe formyle ou benzoyle,

par un groupe alcanoyle en $C_{2-4}$, qui peut être substitué en position terminale par un groupe amino, acétylamino, pyrrolidino, pipéridino, morpholino, pipérazino ou 4-méthylpipérazino ou par un groupe alkyle en $C_{1-3}$-amino, N-acétyl-alkyle en $C_{1-3}$-amino ou di(alkyle en $C_{1-3}$)amino, où, dans les groupes alkyle en $C_{1-3}$-amino, N-acétyl-alkyle en $C_{1-3}$-amino et di(alkyle en $C_{1-3}$)-amino cités précédemment, une partie, alkyle en $C_{1-3}$ peut être substituée en outre par un groupe phényle ou en position 2 ou 3 par un groupe méthoxy, diméthylamino ou morpholino,

par un groupe alkyle en $C_{1-4}$-sulfonyle, qui peut être substitué en position 2 ou 3 par un groupe diméthylamino,

par un groupe pyrrolidinosulfonyle, un groupe aminosulfonyle, alkyle en $C_{1-3}$-aminosulfonyle ou di(alkyle en $C_{1-3}$) aminosulfonyle, dans lesquels à chaque fois une partie alkyle en $C_{1-3}$ peut être substituée par un groupe carboxyle, alcoxy en $C_{1-3}$-carbonyle, aminocarbonyle, méthylaminocarbonyle ou diméthylaminocarbonyle ou, à l'exception de la position 1, par un groupe diméthylamino,

par un groupe alcoxy en $C_{2-3}$, qui est substitué en position 2 ou 3 par un groupe diméthylamino ou pipéridino,

par un groupe aminocarbonyle, alkyle en $C_{1-3}$-aminocarbonyle ou di(alkyle en $C_{1-3}$)aminocarbonyle dans lequel à chaque fois les parties alkyle en $C_{1-3}$ peuvent être substituées, à l'exception de la position 1, par un groupe méthoxy ou diméthylamino,

leurs isomères et leurs sels.

**5.** Indolinones substituées de formule générale I selon la revendication 1 où

X et $R_2$ à $R_4$ sont définis comme indiqué précédemment,

$R_1$ représente un atome d'hydrogène et

$R_5$ représente un groupe phényle qui peut être substitué

par un groupe méthyle ou éthyle qui est substitué dans chaque cas par un groupe azétidin-1-yle, pyrrolidino, pipéridino, hexaméthylèneimino, morpholino, 1-oxydo-thiomorpholino, pipérazino, 4-méthylpipérazino ou 4-acétylpipérazino, où les groupes pipéridino cités précédemment peuvent être substitués en outre par un ou deux groupes méthyle ou en position 4 par un groupé hydroxyle, méthoxy, hydroxyméthyle, aminocarbonyle, méthylaminocarbonyle ou diméthylaminocarbonyle,

par un groupe alkyle en $C_{1-2}$ linéaire qui peut être substitué en position terminale par un groupe amino ou par un groupe alkyle en $C_{1-3}$-amino, dans lequel la partie alkyle du groupe alkyle en $C_{1-3}$-amino peut être substituée en position 2 ou 3 par un groupe hydroxyle ou méthoxy, et, dans les groupes cités précédemment, l'atome d'hydrogène présent sur l'atome d'azote d'amine peut être remplacé en outre

par un groupe cycloalkyle en $C_{3-6}$, par un groupe alkyle en $C_{1-3}$, dans lequel la partie alkyle peut être substituée en position 2 ou 3 par un groupe hydroxyle, ou par un groupe alkyle en $C_{1-2}$-carbonyle substitué par un groupe amino, méthylamino ou diméthylamino,

par un groupe éthyle qui est substitué en position 1 par un groupe amino,

par un groupe amino ou alkyle en $C_{1-3}$-amino, où la partie alkyle peut être substituée en position terminale par un groupe carboxyle, aminocarbonyle, méthylaminocarbonyle, diméthylaminocarbonyle, N-(2-diméthylaminoéthyl) aminocarbonyle ou N-(2-diméthylaminoéthyl)-N-méthylaminocarbonyle ou en position 2

ou 3 par un groupé amino, méthylamino, diméthylamino, acétylamino, N-acétyl-méthylamino ou morpholino, où l'atome d'hydrogène présent sur l'atome d'azote d'amine des groupes cités précédemment peut être remplacé en outre

par un groupe alcanoyle en $C_{2-4}$, qui peut être substitué en position terminale par un groupe amino, acétylamino, pyrrolidino, pipéridino, morpholino, pipérazino ou 4-méthylpipérazino ou par un groupe alkyle en $C_{1-3}$-amino, N-acétyl-alkyle en $C_{1-3}$-amino ou di(alkyle en $C_{1-3}$)amino, où, dans les groupes alkyle en $C_{1-3}$-amino, N-acétyl-alkyle en $C_{1-3}$-amino et di(alkyle en $C_{1-3}$)-amino cités précédemment, une partie alkyle en $C_{1-3}$ peut être substituée en outre en position 2 ou 3 par un groupe méthoxy, diméthylamino ou morpholino,

par un groupe alkyle en $C_{1-4}$-sulfonyle, qui peut être substitué en position 2 ou 3 par un groupe diméthylamino, par un groupe pyrrolidinosulfonyle, un groupe aminosulfonyle, alkyle en $C_{1-3}$-aminosulfonyle ou di(alkyle en $C_{1-3}$)aminosulfonyle, dans lesquels à chaque fois une partie alkyle en $C_{1-3}$ peut être substituée par un groupe carboxyle, méthoxycarbonyle, aminocarbonyle, méthylaminocarbonyle ou diméthylaminocarbonyle ou, à l'exception de la' position 1, par un groupe diméthylamino,
par un groupe alcoxy en $C_{1-3}$, qui est substitué en position 2 ou 3 par un groupe diméthylamino ou pipéridino,
par un groupe aminocarbonyle, alkyle en $C_{1-3}$-aminocarbonyle ou di(alkyle en $C_{1-3}$)aminocarbonyle
dans lequel à chaque fois une partie alkyle en $C_{1-3}$ peut être substituée, à l'exception de la position 1, par un groupe méthoxy ou diméthylamino,
leurs isomères et leurs sels.

**6.** Composés de formule générale I suivants :

(a) (Z)-3-[1-(4-diméthylaminométhyl-phénylamino)-1-phényl-méthylidène]-5-nitro-2-indolinone,
(b) (Z)-3-[1-(4-pipéridinométhyl-phénylamino)-1-phénylméthylidène]-5-nitro-2-indolinone,
(c) (Z)-3-{1-[4-(2-morpholinoéthyl)-phénylamino)-1-phényl-méthylidène]-5-nitro-2-indolinone,
(d) (Z)-3-{1-[4-(2-diméthylaminoéthyl)-phénylamino)-1-phényl-méthylidène]-5-nitro-2-indolinone et
(e) (Z)-3-{1-[4-(N-(2-diméthylaminoéthyl)-N-méthylsulfonylamino)phénylamino]-1-phényl-méthylidène]-2-indolinone,

ainsi que leurs sels.

**7.** Sels physiologiquement acceptables des composés selon les revendications 1 à 6.

**8.** Médicament contenant un composé de formule générale I selon au moins l'une des revendications 1 à 6 où $R_1$ représente un atome d'hydrogène, un groupe alkyle en $C_{1-3}$ ou un reste de promédicament, ou un sel physiologiquement acceptable de celui-ci, et éventuellement un ou plusieurs supports et/ou diluants inertes.

**9.** Utilisation d'un composé de formule générale I selon au moins l'une des revendications 1 à 6 où $R_1$ représente un atome d'hydrogène, un groupe alkyle en $C_{1-3}$ ou un reste de promédicament ou d'un sel physiologiquement acceptable de celui-ci pour la préparation d'un médicament qui convient pour le traitement de proliférations cellulaires excessives ou anormales.

**10.** Procédé de préparation d'un médicament selon la revendication 8 **caractérisé en ce que**, par voie non chimique, un composé de formule générale I selon au moins l'une des revendications 1 à 6, où $R_1$ représente un atome d'hydrogène, un groupe alkyle en $C_{1-3}$ ou un reste de promédicament, ou un sel physiologiquement acceptable de celui-ci est incorporé dans un ou plusieurs supports et/ou diluants inertes.

**11.** Procédé de préparation des composés selon les revendications 1 à 7 **caractérisé en ce que**

a. un composé de formule générale

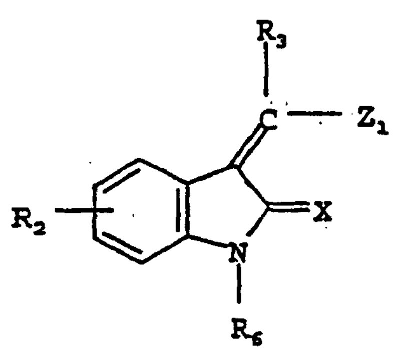

(II),

où
X, $R_2$ et $R_3$ sont définis comme indiqué dans les revendications 1 à 6,

$R_6$ représente un atome d'hydrogène, un groupe protecteur pour l'atome d'azote du groupe lactame ou une liaison à une phase solide et
$Z_1$ représente un atome d'halogène, un groupe hydroxyle, alcoxy ou aralcoxy,
est mis à réagir avec une amine de formule générale

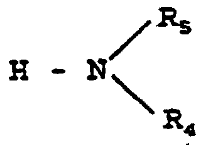

(III),

où
$R_4$ et $R_5$ sont définis comme indiqué dans les revendications 1 à 6 puis; si nécessaire, un groupe protecteur utilisé pour l'atome d'azote du groupe lactame est clivé ou un composé ainsi obtenu est clivé d'une phase solide ou
b. pour la préparation d'un composé de formule générale I qui contient un groupe aminométhyle et X représente un atome d'oxygène, un composé de formule générale

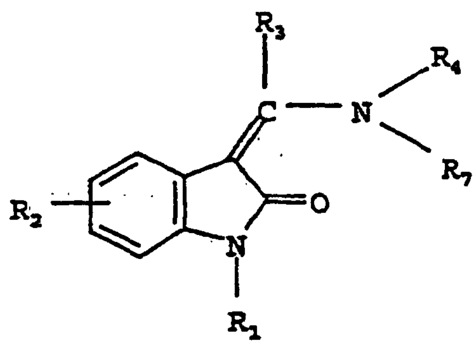

(IV),

où
$R_1$ à $R_4$ sont définis comme indiqué dans les revendications 1 à 6 et
$R_7$ possède les significations indiquées pour $R_5$ dans les revendications 1 à 5 à condition que $R_5$ contienne un groupe cyano, est réduit ou
c. pour la préparation d'un composé de formule générale I où $R_1$ représente un atome d'hydrogène et X représente un atome d'oxygène, un composé de formule générale

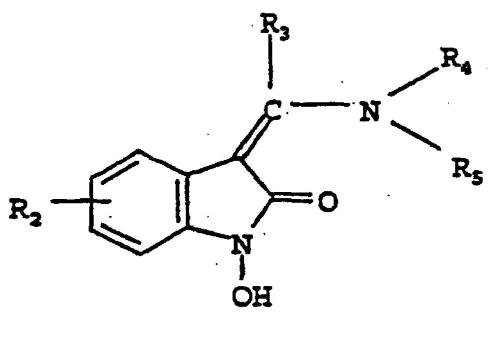

(V),

où
$R_2$ à $R_5$ sont définis comme indiqué dans les revendications 1 à 6, est réduit puis
si on le souhaite, un composé de formule générale I ainsi obtenu, qui contient un groupe alcoxycarbonyle, est converti par hydrolyse en un composé carboxy correspondant, ou

un composé de formule générale I ainsi obtenu, qui contient un groupe amino ou alkylamino, est converti par alkylation ou alkylation réductrice en un composé alkylamino ou dialkylamino correspondant, ou
un composé de formule générale I ainsi obtenu, qui contient un groupe amino ou alkylamino, est converti par acylation en un composé acyle correspondant, ou un composé de formule générale I ainsi obtenu, qui contient un groupe carboxyle, est converti par estérification ou amidation en un composé ester ou aminocarbonyle correspondant, ou
un composé de formule générale I ainsi obtenu, où $R_3$ représente un reste phényle qui contient un atome de chlore, de brome ou d'iode, est converti par réaction avec un composé alcényle en un composé alcénylé correspondant, ou
un composé de formule générale I ainsi obtenu, où $R_3$ représente un reste phényle qui contient un atome de chlore, de brome ou d'iode, est converti par réaction avec un composé alcynyle en un composé alcénylé correspondant, et
si nécessaire, un reste protecteur utilisé pendant les réactions pour protéger des groupes réactifs est clivé, ou si on le souhaite, un composé de formule générale I ainsi obtenu est ensuite résolu en ses stéréoisomères, ou un composé de formule générale I ainsi obenu est converti en ses sels, en particulier pour l'utilisation pharmaceutique en ses sels physiologiquement acceptables avec un acide ou une base inorganique ou organique.